# EUROPEAN PATENT SPECIFICATION

(11) **EP 3 606 921 B1**
(45) Date of publication and mention of the grant of the patent: **01.06.2022**
(21) Application number: 18717550.0
(22) Date of filing: 06.04.2018
(51) Int. Cl.: C07D 417/12, C07D 275/06, C07F 5/02, A61P 35/00, A61K 31/425

(54) **NEW COMPOUNDS INHIBITORS OF THE YAP/TAZ-TEAD INTERACTION AND THEIR USE IN THE TREATMENT OF MALIGNANT MESOTHELIOMA.**
NEUE VERBINDUNGSINHIBITOREN DER YAP/TAZ-TEAD-INTERAKTION UND VERWENDUNG ZUR BEHANDLUNG VON MALIGNEM MESOTHELIOM
NOUVEAUX COMPOSÉS INHIBITEURS DE L'INTERACTION YAP/TAZ-TEAD ET LEUR UTILISATION DANS LE TRAITEMENT DU MÉSOTHÉLIOME MALIN

(30) Priority: 06.04.2017 EP 17305410
(43) Date of publication of application: 12.02.2020
(73) Proprietor: Inventiva, 21121 Daix (FR)
(72) Inventor: BARTH, Martine, 21380 Asnières lès Dijon (RE); CONTAL, Sylvie, 21240 Talant (FR); JUNIEN, Jean-Louis, 75008 Paris (FR); MASSARDIER, Christine, 21000 Dijon (FR); MONTALBETTI, Christian, 21121 Fontaine les Dijon (FR); SOUDE, Anne, 21600 Longvic (FR)
(74) Representative: Cabinet Beau de Loménie
(86) International application number: PCT/EP2018/058823
(87) International publication number: WO 2018/185266

(56) References cited:
- EP-A1- 3 156 404
- WO-A1-2017/053706

## Description

### Field of the invention

The present invention concerns new compounds inhibitors of the YAP/TAZ-TEAD interaction, and the said compounds for use in therapy, particularly in the treatment of malignant mesothelioma.

The hippo pathway regulates cell proliferation, cell death and cell differentiation in multicellular organisms to ensure normal tissue development (Tumaneng K et al., Curr. Biol., 2013, 22, R368-379; Yu Fx et al., Genes Dev. 2013, 27, 355-371). Over the past years, various genetic and biochemical studies in Drosophila and mammals have defined a highly conserved core hippo signaling module (Huang et al., Cell, 2005, 122, 421-434; Zeng et al., Cancer Cell, 208, 13, 188-192; Badouel et al., Curr. Opin. Cell. Biol., 2009, 21, 837-843).

Essentially, the core hippo signaling module is composed of members of Ste20-like kinase, (MST1/2) and a large tumor suppressor 1/2 (LATS1/2), together with MOB activator 1A (MOB1A) and MOB1B and the AGC (protein kinase A(PKA)/PKG/PKC-like) kinase families (Hong W et al., Cell. Dev. Biol., 2012, 23, 785-793).

Lats1 and 2, AGC kinases (homologous to Drosophila Warts), are activated by association with Mob1 (Mps one binder kinase activator-like 1A and 1B) (Mats in Drosophila) and also by phosphorylation by the STE20 family protein kinases MST1 and 2 (Hippo in Drosophila).

The final output of hippo signaling is the inhibition of the transcriptional co-activators YAP (Yes-associated protein; Yorkie in drosophila)/ TAZ (transcriptional co-activator with PDZ-binding motif) by phosphorylation by the complex Lats/Mob, in flies and mammals (Hong W et al., Cell. Dev. Biol., 2012, 23, 785-793; Zhao et al., Cancer Res., 2009, 69, 1089-98; Lei et al., Mol. Cell. Biol., 2008, 28, 2426-2436).

Functionally, when the hippo pathway is activated, YAP and TAZ are sequestered in the cytoplasm and degraded. Conversely, when the Hippo pathway is deactivated, YAP and TAZ translocate into the nucleus and promote transcription of downstream genes by forming complexes with transcription factors, such as transcriptional enhancer factors (TEF; also referred to as TEAD) and others. TEADs seems to be the key mediators of the growth and the tumorigenic potential of YAP/TAZ. (Zhao et al., Genes Dev., 2008, 22, 1962-1971; Harvey et al., Nat. Rev. Cancer, 2013,13, 246-257) by inducing the expression of target genes, such as CTGF, Cyr61, FGF1 (Wang L et al., Tumor Biol., 2014, 14, 463-468).

Hyperactivation of YAP and TAZ subsequent to a deregulation of the hippo pathway is widespread in cancer, indeed, the levels and nuclear localization of YAP/TAZ are elevated in many tumors such as lung, thyroid, skin, ovarian, colorectal, prostate, pancreas, esophagus, liver and breast cancer (Harvey et al., Nat. Rev. Cancer, 2013,13, 246-257; Avruch et al., Cell Cycle, 2012, 1090-1096; De Christofaro T, Eur. J. Cancer, 2011, 926-933; Zhou et al., Oncogene, 2011, 30, 2181-2186; Wang et al., Cancer Sci., 2010, 101, 1279-85; Chad et al., Cancer Res., 2010, 70, 8517-25; Steinhardt et al., Hum.. Pathol., 2008, 39, 1582-9, Zhao et al. Genes Dev., 2007, 21: 2747-2761; Dong et al. Cell, 2007, 130: 1120-1133).

Although hippo signaling is clearly altered in human cancer, only few germline and somatic mutation of hippo signaling components have been described so far, this is especially true of the core hippo pathway genes. Only neurofibromin 2 (NF2 or merlin in Drosophila) an upstream component of the hippo pathway core component has been linked to a heritable cancer syndrome and classified as a tumor suppressor gene. Hundreds of somatically acquired mutation have been reported in NF2, predominantly in meningiomas, mesotheliomas and peripheral nerve sheath tumors, but also in other cancer types. (Harvey et al., Nat. Rev. Cancer, 2013, 13, 246-257; Bianchi et al., Nat. Genet., 1994, 6, 185-192; Ruttledge et al., Nat. Genet., 1994, 6, 180-184).

Malignant pleural mesothelioma (MPM) is an aggressive human malignancy, mostly associated with asbestos exposure (Carbone et al., Clin. Cancer Res., 2012, 18, 598-604). About 3 out of 4 mesotheliomas are pleural mesotheliomas. MPM is a rare disease with a 15-year cumulative frequency during 1994-2008 in the 56 countries reporting MPM to be 174300 cases (Park et al., Environ. Health Perspect., 2011, 119, 514-518). However the real incidence of MPM is unknown, since there are countries for which MPM mortality is not reported, including asbestos producers. Despite treatment with chemotherapy, radiation therapy or surgery, the prognosis is poor, the median survival time of patients after diagnosis is only 7-12 months. (Bianchi et al. Natl. Acad.. Sci., USA, 1995, 92, 10854-10858; Sekido et al., Cancer Res., 1995, 55, 1227; Deguen et al., Int. J. Cancer, 1998, 77, 554-560).

Malignant pleural mesothelioma shows frequent inactivation of the NF2-tumor suppressor gene, indeed data mining of the catalogue of somatic mutations in cancers shows that the genes that are mostly mutated in MPM are cyclin-dependent kinase activator (CDKN2A), neurofibromatosis type 2 and BRCA-associated protein 1 (BAP1) (Forbe et al., Nucleic Acids Res., 2011, 39, D945-950).

Recently, besides the NF2 mutation, genetic alterations in the components of the hippo signaling pathway have also been identified, including inactivating mutations of Lats1/2 and amplification of YAP. Together with NF2 mutation, MPM shows frequent Merlin-Hippo pathway inactivation, which leads to YAP activation over 70% of MPM cases (Bott et al., Nat. Genet., 2011, 43, 668-672; Murakami et al., Cancer Res., 2011, 71, 873-883; Yokoyama et al., Carcinogenesis, 2008, 29, 2139-2146; Sekido et al., Pathol. Int., 2011, 61, 331-344).

Inhibition of the activity of Hippo pathway effectors YAP and TAZ is likely to represent a valuable approach for the treatment of several cancers since the Hippo pathway deregulation is largely observed in many cancers, leading to YAP/TAZ nuclear translocation.

Therefore disruption of hippo pathway downstream YAP/TAZ-TEAD interaction is believed to abrogate the oncogenic property of YAP/TAZ. The compounds of invention are designed to block this interaction upon binding to TEAD and can be further developed into drugs for cancers especially for the treatment of malignant mesothelioma.

WO 2004/087153 and WO 2013/123071 disclose hundreds of small molecules susceptible to be used generally in relation with cancer treatments. Two hydrozobenzothiazole derivatives, different from the one disclosed in the present application, are disclosed but no YAP/TAZ-TEAD interaction inhibiting activity is reported, not to mention specific anticancer activity.

The invention provides new compounds identified as inhibitors of the YAP/TAZ-TEAD interaction, and particularly new hydrozobenzothiazole derivatives inhibiting YAP/TAZ-TEAD interaction.

### General disclosure of the invention

The present invention concerns a compound of formula (I), inhibitor of the YAP/TAZ-TEAD interaction: 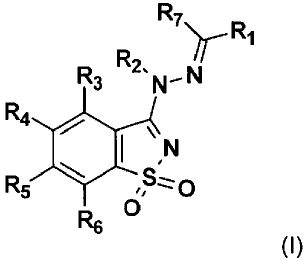 or a pharmaceutically acceptable salt thererof, wherein:
R₁ is an aryl group optionally substituted with one or more substituents independently selected from halo, hydroxyl, alkoxyl, alkylthio, OCF₃, alkyl, cycloalkyl, alkyl-R₈, -O-alkyl-R₈, -NR_{g}R₁₀, -CO-NR₉R₁₀, oxo, thioxo and phenyl;
R₂ is an alkyl optionally substituted by one or two R₈ or -aryl-R_{8:}
   R₃, R₄, R₅, R₆ are each independently H, halo, alkyl, alkoxyl, alkoxyl substituted with boronic acid or a boronic acid derivative, hydroxycarbonyl, aryl, -O-aryl, boronic acid or a boronic acid derivative, phosphonic acid or phosphonic acid derivative, alkyl-R₈, -O-alkyl-R₈, -COOR₁₁, -NO₂, -NR₉R₁₀ or -CO-NR₉R₁₀;
   R₇ is H or alkyl; or
   R₂ and R₇ are bound together to form a 5 or 6-membered heterocycle;
   R₈ is halo, hydroxyl, alkoxyl, -NR₉R₁₀, -CO-X-R₁₁, -CN, -CF₃, aryl, cycloalkyl, -O-alkyl-alkoxyl, -O-CO-alkyl, -CO-NR₉R₁₀ or -O-CO-NR₉R₁₀;
   R₉ and R₁₀ are independently H, alkyl, -CO-alkyl or form together with the nitrogen atom a 3 to 6-membered cyclic group;
   X is -O- or -NR₁₂-;
   R₁₁ is H or alkyl;
   R₁₂ is H, alkyl or hydroxyalkyl;
   and when X is -NR₁₂-, R₁₁ and R₁₂ can be bound together to form a 4- to 7-membered cyclic group;
   with the proviso that R₁ is not 3,4-dimethoxyphenyl or 3-methoxy-4-hydroxyphenyl,
   and with the proviso that R₁ is a substituted aryl group when R₂ and R₇ are bound together to form a 5 or 6-member heterocycle and R₃, R₄, R₅ and R₆ are each H.

The invention also concerns a pharmaceutical composition comprising a compound of formula (I) or one of its pharmaceutically acceptable salts and at least one pharmaceutically acceptable vehicle.

### Brief disclosure of the figure

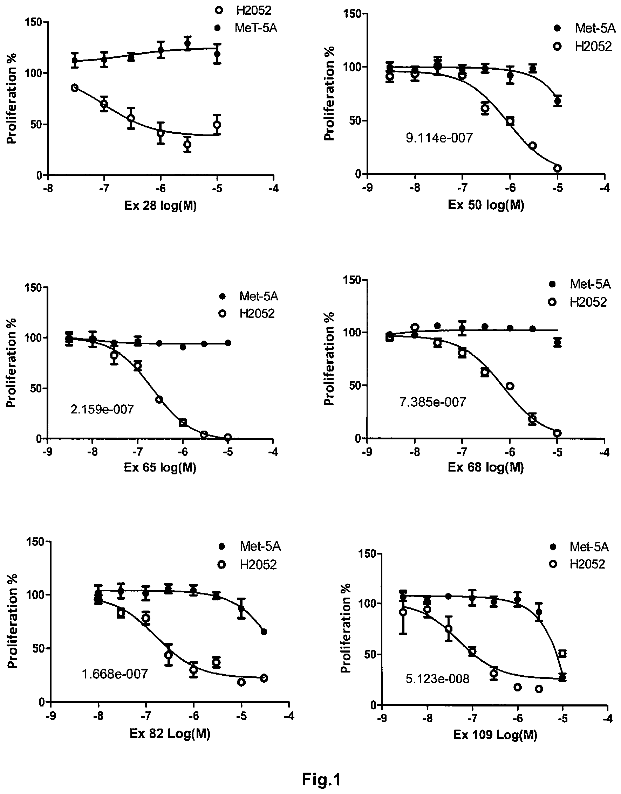

Figure 1 represents the anti-proliferative activity of representative compounds of the invention as inhibitors of the YAP/TAZ-TEAD interaction in mesothelioma cells NCI-H2052.

### Detailed disclosure of the invention

The compounds of formula (I) described herein and their pharmaceutically acceptable salts are representatives of this new class of compounds, inhibitors of the YAP/TAZ-TEAD interaction.

According to the present invention, the term "alkyl" or the prefix "alk" means a linear or branched C₁-C₆ alkyl moiety, particularly a C₁-C₄ alkyl moiety, more particularly C₁, C₂, C₃ or C₄ alkyl moieties, including the groups methyl or methylene, ethyl or ethylene, propyl or propylene, isopropyl or isopropylene, butyl or butylene, isobutyl or isobutylene and tertiobutyl or tertiobutylene. In particular embodiments, the alkyl moieties are selected among methyl or methylene, ethyl or ethylene, propyl or propylene.

According to the invention, the term "cycloalkyl" means a 3- to 6- membered cycle, particularly cyclopropyl, cyclobutyl, cyclopentyl and cyclohexyl.

According to the present invention, the "halo" group is selected among F, Cl, Br or I, particularly F or Cl, more particularly F.

A boronic acid derivative is preferably a derivative of formula -B(O-R₁₇)₂, wherein each R₁₇ independently is H or alkyl, or the two OR₁₇ together form a 5- to 8-membered heterocycle, such as 4,4,5,5-tetramethyl-1,3,2-dioxaborolan-2-yl, 5,5-dimethyl-1,3,2-dioxaborinan-2-yl or N-methyl-1,3,6,2-dioxazaborocane-4,8-dione.

An alkoxyl substituted with boronic acid or a boronic acid derivative can be represented by the formula -O-alkyl- B(O-R₁₇)₂, wherein alkyl and R₁₇ have the same meaning as above. A phosphonic acid derivative is preferably a derivative of formula -PO(O-R₁₇)₂, wherein each R₁₇ independently is H or alkyl.

When R₂ and R₇ are bound together with the nitrogen atoms to form a 5- or 6-membered heterocycle, they particularly represent an alkylene moiety selected from -C(R₁₅)₂-C(R₁₆)₂- and -C(R₁₅)₂-(CR₁₆)₂-C(R₁₈)₂- wherein R₁₅, R₁₆ and R₁₈ are each independently H, alkyl, alkyl-R₈ or -aryl-R₈. Independently means that both R₁₅, both R₁₆ and both R₁₈ respectively can be the same or different. In one embodiment, all R₁₅, R₁₆ and R₁₈ are the same. In another embodiment, at least one of R₁₅, R₁₆ and R₁₈ is different from the other R₁₅, R₁₆ or R₁₈. In a particular embodiment, at least one R₁₅ is H and at least one R₁₆ is an alkyl group. In another embodiment, at least one of R₁₅ and one of R₁₆ is H and one of the other R₁₅ or R₁₆ is an alkyl group, particularly one R₁₅ is an alkyl group and both R₁₆ are H. in preferred embodiments, both R₁₈ are H.

When R₉ and R₁₀ together with the nitrogen atom form a 3- to 6-membered cyclic group, including 3-, 4-, 5- or 6-membered cycles, said group is preferably selected from aziridinyl, azetidinyl, diazetidinyl, pyrrolidinyl, pyrrolinyl, pyrrolyl, imidazolidinyl, imidazolyl, pyrazolidinyl, pyrazolyl, piperidinyl, piperazinyl, pyrazinyl, triazinyl, morphonlinyl, oxazinyl, thiomorpholinyl, thiazinyl. A prefered cyclic group is morpholinyl. The 3- to 6-membered cyclic group may be substituted by one or more groups selected from alkyl and halo.

The same definition applies when R₁₁ and R₁₂ are bound together to form a 4- to 7-membered cyclic group, in case X is -NR₁₂-. Preferably the cyclic group is selected among azetidine, pyrrolidine, piperidine, morpholine, azepane and oxazepane.

R₇ is preferably H.

According to an embodiment of the invention, R₃, R₄, R₅ and R₆ are H.

According to another embodiment of the invention, one of R₃, R₄, R₅ and R₆ is boronic acid or a boronic acid derivative or an alkoxyl substituted with boronic acid or a boronic acid derivative. Particularly one of R₃, R₄, R₅ and R₆ is -B(O-R₁₇)₂ and the others are H.

In a preferred embodiment R₅ is -B(O-R₁₇)₂, R₃, R₄ and R₆ are H and R₁, R₂ and R₇ to R₁₆ are as defined above and below.

According to the invention, pharmaceutically acceptable salts are salts of acids or bases, known for their use in the preparation of active principles for their use in therapy. Examples of pharmaceutically acceptable acids suitable as source of anions are those disclosed in the Handbook of Pharmaceutical Salts: Properties, Selection and Use (P. H. Stahl and C. G. Wermuth, Weinheim/Zürich:Wiley-VCH/VHCA, 2002).

According to the present invention, "aryl" means an aromatic or partially unsaturated (hetero)cycle comprising 1, 2 or 3 rings, which can be fused, including phenyl, naphthyl, pyrazolyl, pyridyl, indolyl, isoindolyl, indolinyl, isoindolinyl, thienyl, thiazolyl, benzimidazolyl, benzotriazolyl, spiro[cyclopropane-1,3'-isoindoline], and derivatives thereof. The aryl groups when not related to R₁ are optionally substituted by one or more substituents selected from alkyl and halo groups.

Particularly, the aryl of R₁ is selected from phenyl, pyridyl, thiazolyl, indolyl, isoindolyl and benzimidazolyl, these last three groups being optionally substituted by oxo.

According to one embodiment, the invention concerns a compound of formula (l'): 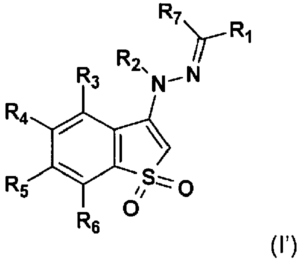 or a pharmaceutically acceptable salt thereof, wherein:
R₁ is an aryl group optionally substituted with one or more substituents independently selected from halo, hydroxyl, alkoxyl, alkyl, cycloalkyl, alkyl-R₈, -O-alkyl-R₈, -NR₉R₁₀ , -CO-NR₉R₁₀, oxo and phenyl;
R₂ is alkyl optionally substituted by one or two R₈, or -aryl-R₈;
R₃, R₄, R₅, R₆ are each independently H, halo, hydroxyl, alkyl, alkoxyl, hydroxycarbonyl, aryl, -O-aryl, boronic acid or a boronic acid derivative, phosphonic acid or phosphonic acid derivative, alkyl-R₈, -O-alkyl-R₈, -COOR₁₁, -NO₂, -NR₉R₁₀ or -CO-NR₉R₁₀;
R₇ is H or alkyl; or
R₂ and R₇ are bound together to form a 5- or 6-membered heterocycle;
R₈ is halo, hydroxyl, alkoxyl, -NR₉R₁₀, -CO-X-R₁₁, -CN, -CF₃, or aryl;
R₉ and R₁₀ are independently H, alkyl or -CO-alkyl, or form together with the nitrogen atom a 3- to 6-membered cyclic group;
X is -O- or -NR₁₂-;
R₁₁ is H or alkyl;
R₁₂ is H, alkyl or hydroxyalkyl;
with the proviso that R₁ is not 3,4-dimethoxyphenyl or 3-methoxy-4-hydroxyphenyl, and with the proviso that R₁ is a substituted aryl group when R₂ and R₇ are bound together to form a 5 or 6-membered heterocycle and R₃, R₄, R₅ and R₆ are H.

According to a first preferred embodiment, the invention concerns a compound of formula (I) wherein R₁ represents: 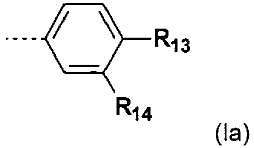 and R₁₃ and R₁₄ are each independently selected from H, halo, hydroxyl, alkoxyl, OCF₃, alkyl, cycloalkyl, alkylthio, alkyl-R₈, -O-alkyl-R₈, -NR₉R₁₀ and -CO-NR₉R₁₀, with the proviso that when R₁₄ is methoxyl, then R₁₃ is not hydroxyl or methoxyl.

Preferably, R₁₃ is halo, alkoxyl, alkyl or alkoxyalkoxyl, and R₁₄ is H, OH, OCF₃, alkoxyl or alkylthio.

The halo group is preferably F, the alkoxyl is preferably methoxyl or ethoxyl, the alkylthio is preferably methylsulfanyl and the alkoxyalkoxyl is preferably 2-methoxy-ethoxyl. According to a second preferred embodiment, the invention concerns a compound of formula (I) wherein R₁ represents: 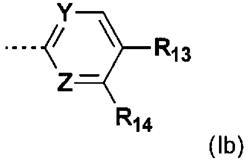 one of Y and Z is N and the other is CH, and
R₁₃ and R₁₄ are each independently selected from H, halo, hydroxyl, alkoxyl, alkyl, cycloalkyl, alkylthio, alkyl-R₈ and -O-alkyl-R₈.

Preferably, R₁₃ and R₁₄ are each independently selected from H, hydroxyl, alkoxyl and alkoxyalkoxyl. Particularly R₁₃ and R₁₄ are each independently selected from H, OH, methoxyl and ethoxyl.

According to a third preferred embodiment, the invention concerns a compound of formula (I) wherein R₁ represents: 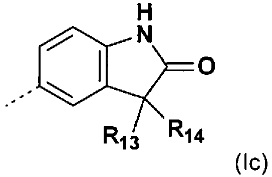 and R₁₃ and R₁₄ are each independently selected from H, halo, hydroxyl, alkyl, alkoxyl, alkoxyalkoxyl, alkylthio, alkyl, cycloalkyl, alkyl-R₈, -O-alkyl-R₈ or R₁₃ and R₁₄ can be bound together to form a 3- to 6-membered cycle. Preferably, the alkyl, linear or branched, is optionally substituted with one or more halo, particularly fluorine. The alkyl is advantageously selected from methyl, ethyl, n-propyl, isopropyl, n-butyl, isobutyl and isopropyl, each of which is optionally substituted with 1 or more halo, particularly fluoro. When the alkyl is substituted with more than one fluorine atom, particularly 2 or 3 fluorine atoms, the fluorine atoms are attached to the same carbon. Examples of such fluoro-substituted alkyls are trifluoromethyl and 1,1,1-trifluoroethyl.

Prefered 3- to 6- membered cycle are cyclopropane and cyclobutane.

In a particular embodiment, one of R₁₃ or R₁₄ is H and the other is alkyl as defined above, more particularly methyl or ethyl. In another particular embodiment, R₁₃ and R₁₄ are both alkyl, as defined above, more particularly methyl or ethyl.

According to a fourth preferred embodiment, the invention concerns a compound of formula (I) wherein R₁ represents: 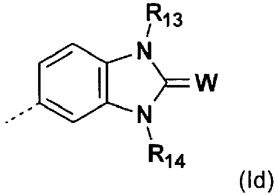 and
W is O or S, and
R₁₃ and R₁₄ are each independently selected from H, alkyl, cycloalkyl, and alkyl-R₈, and R₈ is preferably hydroxyl, alkoxyl, alkoxyalkoxyl, -O-CO-alkyl, -CO-NR₉R₁₀ or-CF₃.

Preferably, the alkyl, linear or branched, is optionally substituted with one or more halo, particularly fluorine. The alkyl is advantageously selected from methyl, ethyl, n-propyl, isopropyl, n-butyl, isobutyl and isopropyl, each of which optionally substituted with 1 or more halo, particularly fluoro. When the alkyl is substituted with more than one fluorine atom, particularly 2 or 3 fluorine atoms, the fluorine atoms are attached to the same carbon. Examples of such fluoro-substituted alkyls are trifluoromethyl and 1,1,1-trifluoroethyl.

The cycloalkyl is advantageously selected from cyclopropyl, cyclobutyl and cyclopentyl.

In a particular embodiment, one of R₁₃ or R₁₄ is H and the other is an alkyl or a cycloalkyl as defined above, more particularly selected from methyl, ethyl, 1,1,1-trifluoroethyl, n-propyl, isopropyl, cyclopropyl, n-butyl, isobutyl and cyclobutyl.

According to a fifth preferred embodiment, the invention concerns a compound of formula (I) wherein R₁ represents 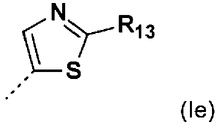 and R₁₃ is H, alkyl, cycloalkyl, phenyl, alkyl-R₈ or -NR₉R₁₀.

Preferably, R₁₃ is -NR₉R₁₀ where R₉ and R₁₀ form together with the nitrogen atom a 3- to 6-membered cyclic group, particularly a 4- or 5-membered cyclic ring.

According to a sixth preferred embodiment, the invention concerns a compound of formula (I) wherein R₁ represents: 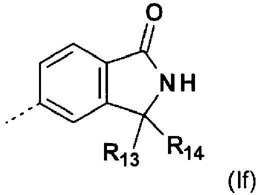 and R₁₃ and R₁₄ are each independently selected from H, halo, hydroxyl, alkoxyl, alkylthio, alkyl, cycloalkyl, alkyl-R₈, -0-alkyl-R₈ or R₁₃ and R₁₄ can be bound together to form a 3- to 6-membered cycle. Preferably, the alkyl, linear or branched, is optionally substituted with one or more halo, particularly fluorine. The alkyl is advantageously selected from methyl, ethyl, n-propyl, isopropyl, n-butyl, isobutyl and isopropyl, each of which optionally substituted with 1 or more halo, particularly fluoro. When the alkyl is substituted with more than one fluorine atom, particularly 2 or 3 fluorine atoms, the fluorine atoms are attached to the same carbon. Examples of such fluoro-substituted alkyls are trifluoromethyl and 1,1,1-trifluoroethyl. Prefered 3- to 6- membered cycle are cyclopropane and cyclobutane. In a particular embodiment, one of R₁₃ or R₁₄ is H and the other is alkyl as defined above, more particularly methyl or ethyl. In another particular embodiment, R₁₃ and R₁₄ are both alkyl, as defined above, more particularly methyl or ethyl.

R₂ is particularly selected from methyl, ethyl, n-propyl, i-propyl, i-butyl, hydroxyethyl, hydroxyl-n-propyl, 2-pyridylethyl, phenylethyl, methoxyethyl, ethoxyethyl, and 2-chloromethyl-3-hydroxypropyl.

The compounds in which R₁ is a group of formula (la) are particularly selected from:
- N-[(E)-(4-fluorophenyl)methyleneamino]-N-methyl-1,1-dioxo-1,2-benzothiazol-3-amine
- 2-[3-[(E)-[(1,1-dioxo-1,2-benzothiazol-3-yl)-methyl-hydrazono]methyl]phenoxy] ethanol
- N-methyl-N-[(E)-(4-morpholinophenyl)methyleneamino]-1,1-dioxo-1,2-benzothiazol-3-amine
- N-[(E)-[3-(dimethylamino)phenyl]methyleneamino]-N-methyl-1,1-dioxo-1,2-benzothiazol-3-amine
- N-[(E)-(4-fluoro-3-methoxy-phenyl)methyleneamino]-1,1-dioxo-N-[2-(2-pyridyl)ethyl]-1,2-benzothiazol-3-amine
- methyl 2-[3-[(E)-[(1,1-dioxo-1,2-benzothiazol-3-yl)-methyl-hydrazono] methyl]phenyl] acetate
- N-[(E)-(3-ethoxy-4-fluoro-phenyl)methyleneamino]-N-methyl-1,1-dioxo-1,2-benzothiazol-3-amine
- N-[(E)-(4-fluoro-3-propoxy-phenyl)methyleneamino]-N-methyl-1,1-dioxo-1,2-benzothiazol-3-amine
- N-[(E)-(4-fluoro-3-isopropoxy-phenyl)methyleneamino]-N-methyl-1,1-dioxo-1,2-benzothiazol-3-amine
- 2-[5-[(E)-[(1,1-dioxo-1,2-benzothiazol-3-yl)-methyl-hydrazono]methyl]-2-fluoro-phenoxy]ethanol
- N-[(E)-[4-fluoro-3-(2-methoxyethoxy)phenyl]methyleneamino]-N-methyl-1,1-dioxo-1,2-benzothiazol-3-amine
- N-[(E)-[3-fluoro-5-methoxy-4-(2-methoxyethoxy)phenyl]methyleneamino]-N-methyl-1,1-dioxo-1,2-benzothiazol-3-amine
- 5-[(E)-[(1,1-dioxo-1,2-benzothiazol-3-yl)-methyl-hydrazono]methyl]-2-fluoro-benzamide
- 2-(dimethylamino)-5-[(E)-[(1,1-dioxo-1,2-benzothiazol-3-yl)-methyl-hydrazono]methyl] benzamide
- [4-[(E)-[(1,1-dioxo-1,2-benzothiazol-3-yl)-methyl-hydrazono]methyl]-2-methoxyphenyl]methanol
- 2-[4-[(E)-[(1,1-dioxo-1,2-benzothiazol-3-yl)-methyl-hydrazono]methyl]-2-methoxyphenyl]-N-methyl-acetamide
- N-[(E)-[4-(2-methoxyethoxy)phenyl]methyleneamino]-N-methyl-1,1-dioxo-1,2-benzothiazol-3-amine
- N-[(E)-[3-(2-methoxyethoxy)phenyl]methylenearriino]-N-methyl-1,1-dioxo-1,2-benzothiazol-3-amine
- N-ethyl-N-[(E)-(4-fluoro-3-methoxy-phenyl)methyleneamino]-1,1-dioxo-1,2-benzothiazol-3-a mine
- N-[(E)-(4-fluoro-3-methoxy-phenyl)methyleneamino]-N-isopropyl-1,1-dioxo-1,2-benzothiazol-3-amine
- N-[(E)-(4-fluoro-3-methoxy-phenyl)methyleneamino]-N-isobutyl-1,1-dioxo-1,2-benzothiazol-3-amine
- 2-[(1,1-dioxo-1,2-benzothiazol-3-yl)-[(E)-(4-fluoro-3-methoxy-phenyl) methyleneamino]am ino]ethanol
- N-[(E)-(4-fluoro-3-methoxy-phenyl)methyleneamino]-N-(2-methoxyethyl)-1,1-dioxo-1,2-benzothiazol-3-amine
- N-(2-ethoxyethyl)-N-[(E)-(4-fluoro-3-methoxy-phenyl)methyleneamino]-1,1-dioxo-1,2-benzothiazol-3-amine
- 3-[(1,1-dioxo-1,2-benzothiazol-3-yl)-[(E)-(4-fluoro-3-methoxy-phenyl) methyleneamino]amino]propan-1-ol
- 2-(chloromethyl)-3-[(1,1-dioxo-1,2-benzothiazol-3-yl)-[(E)-(4-fluoro-3-methoxyphenyl)methyleneamino]amino]propan-1-ol
- N-[(E)-(4-fluoro-3-methoxy-phenyl)methyleneamino]-1,1-dioxo-N-(2-phenyl ethyl)-1,2-benzothiazol-3-amine
- N-[(E)-(4-fluoro-3-methoxy-phenyl)methyleneamino]-N-methyl-5-nitro-1,1-dioxo-1,2-benzothiazol-3-a mine
- N-[3-[[(E)-(4-fluoro-3-methoxy-phenyl)methyleneamino]-methyl-amino]-1,1-dioxo-1,2-benzothiazol-5-yl]acetamide
- N3-[(E)-(4-fluoro-3-methoxy-phenyl)methyleneamino]-N3-methyl-1,1-dioxo-1,2-benzothiazole-3,6-diamine
- 3-[[(E)-(4-fluoro-3-methoxy-phenyl)methyleneamino]-methyl-amino]-1,1-dioxo-1,2-benzothiazol-6-yl]boronic acid
- 3-[[(E)-(4-fluoro-3-methoxy-phenyl)methyleneamino]-methyl-amino]-1,1-dioxo-1,2-benzothiazol-6-ol
- 6-benzyloxy-N-[(E)-(4-fluoro-3-methoxy-phenyl)imethyleneamino]-N-methyl-1,1-dioxo-1,2-benzothiazol-3-amine
- 5-ethoxy-N-[(E)-(4-fluoro-3-methoxy-phenyl)methyleneamino]-N-methyl-1,1-dioxo-1,2-benzothiazol-3-amine
- N-[(E)-(4-fluoro-3-methoxy-phenyl)methyleneamino]-5-isopropoxy-N-methyl-1,1-dioxo-1,2-benzothiazol-3-amine
- N-[(E)-(4-fluoro-3-methoxy-phenyl)methyleneamino]-N-methyl-1,1-dioxo-5-phenoxy-1,2-benzothiazol-3-amine
- N-[(E)-(4-fluoro-3-methoxy-phenyl)methyleneamino]-5-(2-methoxyethoxy)-N-methyl-1,1-dioxo-1,2-benzothiazol-3-amine
- 5-[2-(dimethylarnino)ethoxy]-N-[(E)-(4-fluoro-3-methoxy-phenyl)methyleneamino]-N-methyl-1, 1-dioxo-1 ,2-benzothiazol-3-amine
- 2-[[3-[[(E)-(4-fluoro-3-methoxy-phenyl)methyleneamino]-methyl-amino]-1,1-dioxo-1,2-benzothiazol-5-yl]oxy]-N,N-dimethyl-acetamide
- 6-(5,5-dimethyl-1,3,2-d ioxaborinan-2-yl)-N-[(E)-(4-fluoro-3-methoxy-phenyl) methyleneamino]-N-methyl-1,1-dioxo-1,2-benzothiazol-3-amine
- 6-diethoxyphosphoryl-N-[(E)-(4-fluoro-3-methoxy-phenyl)methyleneamino]-N-methyl-1,1-dioxo-1,2-benzothiazol-3-amine
- [3-[ethyl-[(E)-(4-fluoro-3-methoxy-phenyl)methyleneamino]amino]-1,1-dioxo-1,2-benzothiazol-6-yl]boronic acid
- [3-[[(E)-(4-fluoro-3-methoxy-phenyl)methyleneamino]-isobutyl-amino]-1,1-dioxo-1,2-benzothiazol-6-yl]boronic acid
- [3-[[(E)-(4-fluorophenyl)methyleneamino]-methyl-amino]-1,1-dioxo-1,2-benzothiazol-6-yl]boronic acid
- 2-[3-[[(E)-(4-fluoro-3-methoxy-phenyl)methyleneamino]-methyl-amino]-1,1-dioxo-1,2-benzothiazol-6-yl]-6-methyl-1,3,6,2-dioxazaborocane-4,8-dione
- 2-[3-[[(E)-(4-fluoro-3-methoxy-phenyl)methyleneamino]-isobutyl-amino]-1,1-dioxo-1,2-benzothiazol-6-yl]-6-methyl-1,3,6,2-dioxazaborocane-4,8-dione
- [3-[[(E)-(4-fluoro-3-methoxy-phenyl)methyleneamino]-(2-methoxyethyl)amino]-1,1-dioxo-1,2-benzothiazol-6-yl]boronic acid
- N-[(E)-[4-fluoro-3-(trifluoromethoxy)phenyl]methyleneamino]-N-methyl-1,1-dioxo-1,2-benzothiazol-3-amine
- [3-[cyclobutylmethyl-[(E)-(4-fluoro-3-methoxy-phenyl)methyleneamino]amino]-1,1-dioxo-1,2-benzothiazol-6-yl]boronic acid
- [5-fluoro-3-[[(E)-(4-fluoro-3-methoxy-phenyl)methyleneamino]-isobutyl-amino]-1,1-dioxo-1,2-benzothiazol-6-yl]boronic acid
- N-[(E)-(4-fluoro-3-methylsulfanyl-phenyl)methyleneamino]-N-methyl-1,1-dioxo-1,2-benzothiazol-3-am ine
- N-[(E)-[3-(dimethylamino)-4-fluoro-phenyl]methyleneamino]-N-methyl-1,1-dioxo-1,2-benzothiazol-3-amine
- 3-[[(E)-(4-fluoro-3-methoxy-phenyl)methyleneamino]-isobutyl-amino]-1,1-dioxo-1,2-benzothiazol-6-ol
- [3-[[(E)-(4-fluoro-3-methoxy-phenyl)methyleneamino]-isobutyl-amino]-1,1-dioxo-1,2-benzothiazol-6-yl]oxymethylboronic acid
- 5-ethoxy-N-[(E)-(4-fluoro-3-methoxy-phenyl)methyleneamino]-N-isobutyl-1,1-dioxo-1,2-benzothiazol-3-amine
- [3-[[(E)-(4-fluoro-3-methoxy-phenyl)methyleneamino]-isobutyl-amino]-1,1-dioxo-1,2-benzothiazol-5-yl]boronic acid
- [3-[[(E)-(4-fluoro-3-methoxy-phenyl)methyleneamino]-isobutyl-amino]-1,1-dioxo-1,2-benzothiazol-5-yl]oxymethylboronic acid
- 4-[[3-[[(E)-(4-fluoro-3-methoxy-phenyl)methyleneamino]-isobutyl-amino]-1,1-dioxo-1,2-benzothiazol-6-yl]oxy]butan-1-ol
- [3-[[(E)-(4-fluoro-3-methoxy-phenyl)methyleneamino]-isopropyl-amino]-1,1-dioxo-1,2-benzothiazol-6-yl]boronic acid
- 1-(azetidin-1-yl)-2-[[3-[[(E)-(4-fluoro-3-methoxy-phenyl)methyleneamino]-isobutyl-amino]-1,1-dioxo-1,2-benzothiazol-5-yl]oxy]ethanone
- 2-[[3-[[(E)-(4-fluoro-3-methoxy-phenyl)methyleneamino]-isobutyl-amino]-1,1-dioxo-1,2-benzothiazol-6-yl]oxy]acetamide
- 2-[[3-[[(E)-(4-fluoro-3-methoxy-phenyl)methyleneamino]-isobutyl-amino]-1,1-dioxo-1,2-benzothiazol-5-yl]oxy]-N-methyl-acetamide
- 2-[[3-[[(E)-(4-fluoro-3-methoxy-phenyl)methyleneamino]-isobutyl-amino]-1,1-dioxo-1,2-benzothiazol-6-yl]oxymethyl]-6-methyl-1,3,6,2-dioxazaborocane-4,8-dione
- N-[(E)-(4-fluoro-3-methylsulfanyl-phenyl)methyleneamino]-N-isobutyl-1,1-dioxo-1,2-benzothiazol-3-amine
- 3-[(1,1-dioxo-1,2-benzothiazol-3-yl)-[(E)-(4-fluoro-3-methoxy-phenyl)methylene amino]amino]-2,2-dimethyl-propan-1-ol
- [3-[6-(4-fluoro-3-methoxy-phenyl)-3-methyl-4,5-dihydro-3H-pyridazin-2-yl]-1,1-dioxo-1,2-benzothiazol-6-yl]boronic acid
- 3-[6-(4-fluoro-3-methoxy-phenyl)-4,4-dimethyl-3,5-dihydropyridazin-2-yl]-1,2-benzothiazole 1,1-dioxide
- 6-diethoxyphosphoryl-N-[(E)-(4-fluoro-3-methoxy-phenyl)methyleneamino]-N-isobutyl-1,1 -dioxo-1,2-benzothiazol-3-amine
and pharmaceutically acceptable salts of these compounds.

The compounds in which R₁ is a group of formula (lb) are particularly selected from:
- 6-[(E)-[(1,1-dioxo-1,2-benzothiazol-3-yi)-methyl-hydrazono]methyl]-4-methoxy-pyridin-3-ol
- N-[(E)-(6-chloro-5-fluoro-2-pyridyl)methyleneamino]-N-methyl-1,1-dioxo-1,2-benzothiazol-3-amine
and pharmaceutically acceptable salts of these compounds.

The compounds in which R₁ is a group of formula (Ic) are particularly selected from:
- 5-[(E)-[(1,1-dioxo-1,2-benzothiazol-3-yl)-methyl-hydrazono]methyl]-3,3-dimethyl-indolin-2-one
- 5-[(E)-[(1,1-dioxo-1,2-benzothiazol-3-yl)-methyl-hydrazono]methyl]-3,3-diethyl-indolin-2-one
- 5-[(E)-[(1,1-dioxo-1,2-benzothiazol-3-yl)-ethyl-hydrazono]methyl]-3,3-diethyl-indolin-2-one
- [3-[[(E)-(3,3-diethyl-2-oxo-indolin-5-yl)methyleneamino]-(2-methoxyethyl)amino]-1,1-dioxo-1,2-benzothiazol-6-yl]boronic acid
- [3-[[(E)-(3,3-diethyl-2-oxo-indolin-5-yl)methyleneamino]-isobutyl-amino]-1,1-dioxo-1,2-benzothiazol-6-yl]boronic acid
- 3,3-diethyl-5-[(E)-[(6-hydroxy-1,1-dioxo-1,2-benzothiazol-3-yl)-isobutyl-hydrazono] methyl]indolin-2-one
- 5-[(E)-[(6-ethoxy-1,1-dioxo-1,2-benzothiazol-3-yl)-isobutyl-hydrazono]methyl]-3,3-diethyl-indolin-2-one
- 3,3-diethyl-5-[(E)-[isobutyl-(6-methoxy-1,1-dioxo-1,2-benzothiazol-3-yl)hydrazono] methyl]indolin-2-one
- 3,3-diethyl-5-[(E)-[isobutyl-(6-isopropoxy-1,1-dioxo-1,2-benzothiazol-3-yl)hydrazono] methyl]indolin-2-one
- 5-[(E)-[(5-ethoxy-1,1-dioxo-1,2-benzothiazol-3-yl)-isobutyl-hydrazono]methyl]-3,3-diethyl-indolin-2-one
and pharmaceutically acceptable salts of these compounds.

The compounds in which R₁ is a group of formula (Id) are particularly selected from:
- 5-[(E)-[(1,1-dioxo-1 ,2-benzothiazol-3-yl)-isobutyl-hydrazono]methyl]-3-methyl-1H-benzimidazol-2-one
- 5-[(E)-[(1,1-dioxo-1,2-benzothiazol-3-yl)-methyl-hydrazono]methyl]-3-ethyl-1H-benzimidazol-2-one
- 5-[(E)-[(1,1-dioxo-1,2-benzothiazol-3-yl)-methyl-hydrazono]methyl]-3-propyl-1H-benzimidazol-2-one
- 5-[(E)-[(1,1-dioxo-1,2-benzothiazol-3-yl)-methyl-hydrazono]methyl]-3-isopropyl-1H-benzimidazol-2-one
- 3-cyclopropyl-5-[(E)-[(1,1-dioxo-1,2-benzothiazol-3-yl)-methyl-hydrazono]methyl]-1H-benzimidazol-2-one
- 3-cyclobutyl-5-[(E)-[(1 ,1-dioxo-1,2-benzothiazol-3-yl)-methyl-hydrazono]methyl]-1H-benzimidazol-2-one
- 5-[(E)-[(1,1-dioxo-1 ,2-benzothiazol-3-yl)-methyl-hydrazono]methyl]-3-isobutyl-1H-benzimidazol-2-one
- 5-[(E)-[(1,1-dioxo-1,2-benzothiazol-3-yl)-methyl-hydrazono]methyl]-3-(2,2,2-trifluoroethyl)-1H-benzimidazol-2-one
- [3-[isobutyl-[(E)-(3-methyl-2-oxo-1H-benzimidazol-5-yl)methyleneamino]amino]-1,1-dioxo-1,2-benzothiazol-6-yl]boronic acid
- [3-[[(E)-(3-ethyl-2-oxo-1H-benzimidazol-5-yl)methyleneamino]-methyl-amino]-1,1-dioxo-1,2-benzothiazol-6-yl]boronic acid
- [3-[[(E)-(3-isopropyl-2-oxo-1H-benzimidazol-5-yl)methyleneamino]-methyl-amino]-1,1-dioxo-1,2-benzothiazol-6-yl]boronic acid
- 3-methyl-5-[(E)-[methyl-(5-methyl-1,1-dioxo-1,2-benzothiazol-3-yl) hydrazono]methyl] - 1H-benzimidazol-2-one
- [3-[isobutyl-[(E)-(3-isopropyl-2-oxo-1H-benzimidazol-5-yl)methyleneamino]amino]-1,1-dioxo-1,2-benzothiazol-6-yl]boronic acid
- 3-[[(E)-(3-isopropyl-2-oxo-1H-benzimidazol-5-yl)methyleneamino]-(2-methoxyethyl) amino]-1,1-dioxo-1,2-benzothiazol-6-yl]boronic acid
- 3-cyclobutyl-5-[(E)-[(1,1-dioxo-1,2-benzothiazol-3-yl)-methyl-hydrazono]methyl]-1H-benzimidazole-2-thione
- [3-[ethyl-[(E)-(3-isopropyl-2-oxo-1H-benzimidazol-5-yl)methyleneamino]amino]-1,1-dioxo-1,2-benzothiazol-6-yl]boronic acid
- [3-[[(E)-(3-cyclobutyl-2-oxo-1H-benzimidazol-5-yl)methyleneamino]-methyl-amino]-1,1-dioxo-1,2-benzothiazol-6-yl]boronic acid
- [3-[[(E)-(3-cyclobutyl-2-oxo-1H-benzimidazol-5-yl)methyleneamino]-ethyl-amino]-1,1-dioxo-1,2-benzothiazol-6-yl]boronic acid
- [3-[[(E)-(3-cyclobutyl-2-oxo-1H-benzimidazol-5-yl)methyleneamino]-(2-methoxyethyl) amino]-1,1-dioxo-1,2-benzothiazol-6-yl]boronic acid
- [3-[ethyl-[(E)-(3-ethyl-2-oxo-1H-benzimidazol-5-yl)methyleneamino]amino]-1,1-dioxo-1,2-benzothiazol-6-yl]boronic acid
- [3-[[(E)-(3-ethyl-2-oxo-1H-benzimidazol-5-yl)methyleneamino]-isobutyl-amino]-1,1-dioxo-1,2-benzothiazol-6-yl]boronic acid
- 3-cyclobutyl-5-[(E)-[(1,1-dioxo-1,2-benzothiazol-3-yl)-methyl-hydrazono]methyl]-1-(methoxymethyl)benzimidazol-2-one
- 5-[(E)-[(1,1-dioxo-1,2-benzothiazol-3-yl)-methyl-hydrazono]methyl]-3-isopropyl-1-(methoxymethyl)benzimidazol-2-one
- [3-cyclobutyl-5-[(E)-[(1,1-dioxo-1,2-benzothiazol-3-yl)-methyl-hydrazono]methyl]-2-oxo-benzimidazol-1-yl]methyl 2,2-dimethylpropanoate
- [3-cyclobutyl-5-[(E)-[(1,1-dioxo-1,2-benzothiazol-3-yl)-methyl-hydrazono]methyl]-2-oxo-benzimidazol-1-yl]methyl butanoate
- 3-cyclobutyl-5-[(E)-[(1,1-dioxo-1,2-benzothiazol-3-yl)-methyl-hydrazono]methyl]-1-(2-methoxyethoxymethyl)benzimidazol-2-one
- 3-cyclobutyl-5-[(E)-[(1,1-dioxo-1,2-benzothiazol-3-yl)-methyl-hydrazono]methyl]-1-(ethoxymethyl)benzimidazol-2-one
- [3-cyclobutyl-5-[(E)-[(1,1-dioxo-1,2-benzothiazol-3-yl)-methyl-hydrazono]methyl]-2-oxo-benzimidazol-1-yl]methyl N,N-dimethylcarbamate
and pharmaceutically acceptable salts of these compounds.

The compounds in which R₁ is a group of formula (le) are particularly selected from:
- N-[(E)-(2-aminothiazol-5-yl)methyleneamino]-N-methyl-1,1-dioxo-1,2-benzothiazol-3-amine
- N-methyl-1,1-dioxo-N-[(E)-(2-pyrrolidin-1-ylthiazol-5-yl)methyleneamino]-1,2-benzothiazol-3-amine
- N-methyl-N-[(E)-(2-morpholinothiazol-5-yl)methyleneamino]-1,1-dioxo-1,2-benzothiazol-3-amine
- N-methyl-1,1-dioxo-N-[(E)-(2-phenylthiazol-5-yl)methyleneamino]-1,2-benzothiazol-3-amine
- N-[(E)-[2-(azetidin-1-yl)thiazol-5-yl]methyleneamino]-N-methyl-1,1-dioxo-1,2-benzothiazol-3-amine
- N-methyl-N-[(E)-(2-methylthiazol-5-yl)methyleneamino]-1,1-dioxo-1,2-benzothiazol-3-amine
and pharmaceutically acceptable salts of these compounds.

The compounds in which R₁ is a group of formula (If) are particularly selected from:
- 5-[(E)-[(1,1-dioxo-1,2-benzothiazol-3-yl)-methyl-hydrazono]methyl]-3-methyl-isoindolin-1-one
- 5-[(E)-[(1,1-dioxo-1,2-benzothiazol-3-yl)-methyl-hydrazono]methyl]-3,3-dimethyl-isoindolin-1-one
- 5-[(E)-[(1,1-dioxo-1,2-benzothiazol-3-yl)-ethyl-hydrazono]methyl]-3,3-dimethyl-isoindolin-1-one
- 5'-[(E)-[(1,1-dioxo-1,2-benzothiazol-3-yl)-methyl-hydrazono]methyl]spiro[cyclopropane-1,3'-isoindoline]-1'-one
and pharmaceutically acceptable salts of these compounds.

In a sixth preferred embodiment of the invention, the invention concerns a compound of formula (II) or a pharmaceutically acceptable salt thereof: 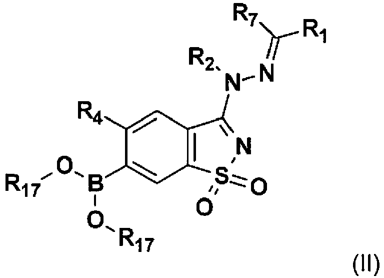 wherein R₁, R₂, R₄, R₇ and R₁₇ are as defined above, more particularly with R₂ being a linear or branched (C₁-C₄)alkyl, and R₁ being an aryl of formula (la), (lb), (Ic), (Id), (le) or (If) as defined above.

In a particular embodiment, the invention is a compound of formula (IIa) or a pharmaceutically acceptable salt thereof: 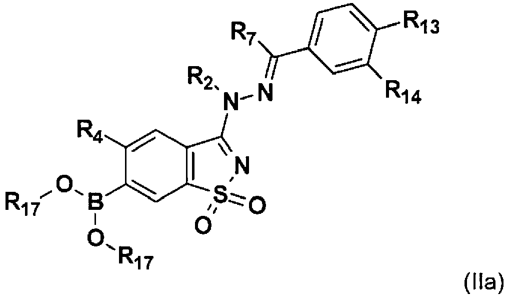 wherein:
R₂ and R₇ are as defined above, in particular R₂ is a linear or branched (C₁-C₄)alkyl or -alkyl-R₈ and R₇ is H or R₂ and R₇ are bound together to form a 6 membered heterocycle, R₄ is H, alkyl, or halo
R₁₃ and R₁₄ are each independently selected from H, halo, hydroxyl, alkoxyl, alkyl, cycloalkyl, alkyl-R₈, -O-alkyl-R₈, -NR₉R₁₀ and -CO-NR₉R₁₀, with the proviso that when R₁₄ is methoxyl, then R₁₃ is not hydroxyl or methoxyl,
R₈ is hydroxyl, alkoxyl, cycloalkyl or -CF₃,
each R₁₇ is independently H or alkyl, or the two OR₁₇ together form a 5- to 8-membered heterocycle such as 4,4,5,5-tetramethyl-1,3,2-dioxaborolan-2-yl, 5,5-dimethyl-1,3,2-dioxaborinan-2-yl or N-methyl-1,3,6,2-dioxazaborocane-4,8-dione.

In a preferred embodiment, each R₁₇ is H.

Preferably, R₁₃ is halo, alkoxyl, alkyl or alkoxyalkylhydroxyl, and R₁₄ is H, OH or alkoxyl. The halo group is preferably F, the alkoxyl is preferably methoxyl or ethoxyl and the alkoxyalkylhydroxyl is preferably 2-methoxy-ethoxyl.

In another particular embodiment, the invention is a compound of formula (IId) or a pharmaceutically acceptable salt thereof: 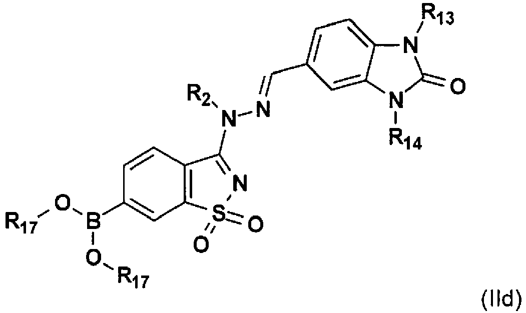 wherein:
R₂ is a linear or branched (C₁-C₄)alkyl or alkyl-R₈;
R₁₃ and R₁₄ are each independently selected from H, alkyl, cycloalkyl, and alkyl-R₈;
R₈ is hydroxyl, alkoxyl, cycloalkyl or -CF₃;
each R₁₇ is H or alkyl, or the two OR₁₇ together form a 5- to 8-membered heterocycle such as 4,4,5,5-tetramethyl-1,3,2-dioxaborolan-2-yl, 5,5-dimethyl-1,3,2-dioxaborinan-2-yl or N-methyl-1,3,6,2-dioxazaborocane-4,8-dione.

In a preferred embodiment, R₁₇ is H.

Preferably R₁₃ is H.

More preferably R₁₃ is H and R₁₄ is a linear or branched (C₁-C₄)alkyl or (C₃-C₄)cycloalkyl. The compounds of formula (IIa) are particularly selected from:
- 3-[[(E)-(4-fluoro-3-methoxy-phenyl)methyleneamino]-methyl-amino]-1,1-dioxo-1,2-benzothiazol-6-yl]boronic acid
- 6-(5,5-dimethyl-1,3,2-dioxaborinan-2-yl)-N-[(E)-(4-fluoro-3-methoxy-phenyl) methyleneamino]-N-methyl-1 ,1-dioxo-1,2-benzothiazol-3-amine
- [3-[ethyl-[(E)-(4-fluoro-3-methoxy-phenyl)methyleneamino]amino]-1,1-dioxo-1,2-benzothiazol-6-yl]boronic acid
- [3-[[(E)-(4-fluoro-3-methoxy-phenyl)methyleneamino]-isobutyl-amino]-1,1-dioxo-1,2-benzothiazol-6-yl]boronic acid
- [3-[[(E)-(4-fluorophenyl)methyleneamino]-methyl-amino]-1,1-dioxo-1,2-benzothiazol-6-yl]boronic acid
- 2-[3-[[(E)-(4-fluoro-3-methoxy-phenyl)methyleneamino]-methyl-amino]-1,1-dioxo-1,2-benzothiazol-6-yl]-6-methyl-1,3,6,2-dioxazaborocane-4,8-dione
- 2-[3-[[(E)-(4-fluoro-3-methoxy-phenyl)methyleneamino]-isobutyl-amino]-1,1-dioxo-1,2-benzothiazol-6-yl]-6-methyl-1,3,6,2-dioxazaborocane-4,8-dione
- [3-[[(E)-(4-fluoro-3-methoxy-phenyl)methyleneamino]-(2-methoxyethyl)amino]-1,1-dioxo-1,2-benzothiazol-6-yl]boronic acid
- [3-[cyclobutylmethyl-[(E)-(4-fluoro-3-methoxy-phenyl)methyleneamino]amino]-1,1-dioxo-1,2-benzothiazol-6-yl]boronic acid
- [5-fluoro-3-[[(E)-(4-fluoro-3-methoxy-phenyl)methyleneamino]-isobutyl-amino]-1,1-dioxo-1,2-benzothiazol-6-yl]boronic acid
- [3-[[(E)-(4-fluoro-3-methoxy-phenyl)methyleneamino]-isopropyl-amino]-1,1-dioxo-1,2-benzothiazol-6-yl]boronic acid
- [3-[6-(4-fluoro-3-methoxy-phenyl)-3-methyl-4,5-dihydro-3H-pyridazin-2-yl]-1,1-dioxo-1,2-benzothiazol-6-yl]boronic acid
and pharmaceutically acceptable salts of these compounds.

The compounds of formula (IId) are particularly selected from:
- [3-[isobutyl-[(E)-(3-methyl-2-oxo-1H-benzimidazol-5-yl)methyleneamino] amino]-1,1-dioxo-1,2-benzothiazol-6-yl]boronic acid
- [3-[[(E)-(3-ethyl-2-oxo-1H-benzimidazol-5-yl)methyleneamino]-methyl-amino]-1,1-dioxo-1,2-benzothiazol-6-yl]boronic acid
- [3-[[(E)-(3-isopropyl-2-oxo-1H-benzimidazol-5-yl)methyleneamino]-methyl-amino]-1,1-dioxo-1,2-benzothiazol-6-yl]boronic acid
- [3-[isobutyl-[(E)-(3-isopropyl-2-oxo-1H-benzimidazol-5-yl)methyleneamino]amino]-1,1-dioxo-1,2-benzothiazol-6-yl]boronic acid
- 3-[[(E)-(3-isopropyl-2-oxo-1H-benzimidazol-5-yl)methyleneamino]-(2-methoxyethyl) amino]-1,1-dioxo-1,2-benzothiazol-6-yl]boronic acid
- 75: [3-[ethyl-[(E)-(3-isopropyl-2-oxo-1H-benzimidazol-5-yl)methyleneamino]amino]-1,1-dioxo-1,2-benzothiazol-6-yl]boronic acid
- [3-[[(E)-(3-cyclobutyl-2-oxo-1H-benzimidazol-5-yl)methyleneamino]-methyl-amino]-1,1-dioxo-1,2-benzothiazol-6-yl]boronic acid
- [3-[[(E)-(3-cyclobutyl-2-oxo-1H-benzimidazol-5-yl)methyleneamino]-ethyl-amino]-1,1-dioxo-1,2-benzothiazol-6-yl]boronic acid
- [3-[[(E)-(3-cyclobutyl-2-oxo-1H-benzimidazol-5-yl)methyleneamino]-(2-methoxyethyl) amino]-1,1-dioxo-1,2-benzothiazol-6-yl]boronic acid
- [3-[ethyl-[(E)-(3-ethyl-2-oxo-1H-benzimidazol-5-yl)methyleneamino]amino]-1,1-dioxo-1,2-benzothiazol-6-yl]boronic acid
- [3-[[(E)-(3-ethyl-2-oxo-1H-benzimidazol-5-yl)methyleneamino]-isobutyl-amino]-1,1-dioxo-1,2-benzothiazol-6-yl]boronic acid
and pharmaceutically acceptable salts of these compounds.

In another particular embodiment of the invention, the invention concerns a compound of formula (III) or a pharmaceutically acceptable salt thereof: 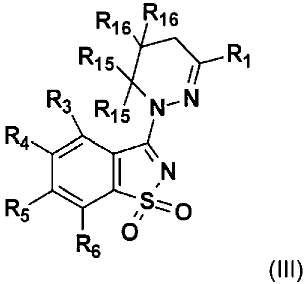 wherein R₁, R₃, R₄, R₅, R₆, R₁₅, R₁₆ are as defined above.

In a prefered embodiment, R₁ is an aryl of formula la as defined above, particularly with R₁₃ is halo, alkoxyl, alkyl or alkoxyalkylhydroxyl, and R₁₄ is H, OH or alkoxyl.

The halo group is preferably F, the alkoxyl is preferably methoxyl or ethoxyl and the alkoxyalkylhydroxyl is preferably 2-methoxy-ethoxyl.

Preferably, R5 is boronic acid or a boronic acid derivative as defined above.

The compounds of formula (III) are particularly selected from:
- [3-[6-(4-fluoro-3-methoxy-phenyl)-3-methyl-4,5-dihydro-3H-pyridazin-2-yl]-1,1-dioxo-1,2-benzothiazol-6-yl]boronic acid
- 3-[6-(4-fluoro-3-methoxy-phenyl)-4,4-dimethyl-3,5-dihydropyridazin-2-yl]-1,2-benzothiazole 1,1-dioxide
and pharmaceutically acceptable salts of these compounds.

The present invention also concerns a pharmaceutical composition comprising at least one compound of formula (I) as described herein or one of its pharmaceutically acceptable salts as the active principle. Pharmaceutical compositions and method for their preparation are well known in the art. Particularly, the composition comprises at least the compound of general formula (I) or one of its pharmaceutically acceptable salts as the active principle and at least one pharmaceutically acceptable excipient.

The pharmaceutical composition of the invention is formulated for its administration by usual routes particularly to be administered by oral, sublingual, subcutaneous, intramuscular, intravenous, transdermal, local or rectal route. The form of the pharmaceutical composition is particularly chosen among the group consisting of tablets, capsules, powders, granules and oral solutions or suspensions, sublingual forms of administration, subcutaneous, topical, intramuscular, intravenous, intranasal or intraocular forms of administration and rectal forms of administration.

Such forms, excipients and methods for their preparation are well known in the art, such as described (Handbook of pharmaceutical Excipients, Rowe et al, seventh edition, June 2012; Rules and Guidance For Pharma Manufacturers and distributors 205, Medecines and Healthcare products Regulatory Agency, London UK).

Therefore, the present invention also concerns the compounds of formula (I) as described herein, for their use in therapy, particularly in the treatment of any cancer indication where YAP is localized in the nucleus of the tumor cells, such as lung, thyroid, ovarian, colorectal, prostate, pancreas, esophagus, liver, breast and skin cancer, more particularly in the treatment of malignant mesothelioma.

The present disclosure also relates to a method for treating cancer in a patient in need thereof, comprising administering a therapeutically effective dose of a compound of formula (I) as described herein, wherein the cancer is any cancer indication where YAP is localized in the nucleus of the tumor cells, such as lung, thyroid, ovarian, colorectal, prostate, pancreas, esophagus, liver, breast and skin cancer, particularly malignant mesothelioma. The skilled practitioner, depending on the activity of the compound of formula (I) and the body weight of the patient, shall determine the appropriate dose of compound and the administration regimen. Such a dose is generally between 5 mg and 1000 mg per day orally for an adult. In general the doctor will determine the appropriate dosage depending on the age, weight and any other factors specific to the subject to be treated.

In a particular embodiment, the inhibitor of the YAP/TAZ-TEAD interaction, and particularly the compounds of formula (I) are used together or separately in combination with another treatment of cancer, particularly malignant mesothelioma, such as surgery, chemotherapy (with among other cisplatin, carboplatin, alimta (pemetrexed), gemcitabine and doxorubicin) and radiation.

### General Synthetic Schemes

Compounds of the invention may be prepared using the synthetic transformations illustrated in schemes I - VI, XVIII, XIX. Starting materials are commercially available or may be prepared by procedures described herein (schemes VII - XVII), by literature procedures, or by procedures well known to one skilled in the art of organic chemistry.

In scheme I, step a, aldehyde (commercially available or prepared following schemes IX - XVII) in solution in alcohol is reacted with commercially available hydrazine and an organic or mineral base (like sodium acetate, triethylamine, sodium hydrogenocarbonate, potassium carbonate.......) (for example, Kurian et al., Bioorg. Med. Chem. Lett., 2014, 24(17), 41764180; Loghmani-Khouzani et al., J. Chem. Res. Syn., 2001, (2), 80-81). Step b, saccharin analog (commercially available or prepared following schemes VIII) is reacted with thionyl-chloride in presence of dimethylformamide or other chlorinating agent such as phosphorous pentachloride, phosphorous oxychloride, oxalyl chloride (for example, Differding et al., Hel. Chim. Acta, 1989; 72(6), 1248-52; Raw et al., Tet. Lett., 2011, 52(50), 6775--78). Step c, hydrazono derivative (obtained in step a or following scheme VII) and 3-chloro-1,2-benzothiazole 1,1-dioxides analog obtained in step b, can react together using or not an organic or mineral base (for example, Haffner et al., Bioorg. Med. Chem. Lett., 2010, 20(23), 6989-92; Haffner et al., Bioorg. Med. Chem. Lett., 2010, 20(23), 6983-88) to give 1,1-dioxo-1,2-benzothiazol-3yl hydrazono derivatives.

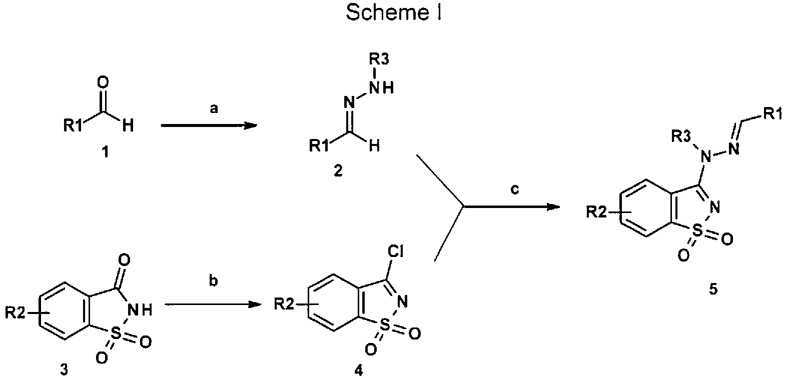

In scheme II, step a, aromatic substitution of chloro-saccharin (commercially available or prepared using transformations described in schemes I step b) with hydrazine may be done in similar conditions as described in scheme I step c. Step b, 1,1-dioxo-1,2-benzothiazol-3-yl-hydrazine derivatives obtained in step a, are reacted with aldehyde (commercially available or prepared following schemes IX - XVII) in similar conditions as described in scheme I, step a.

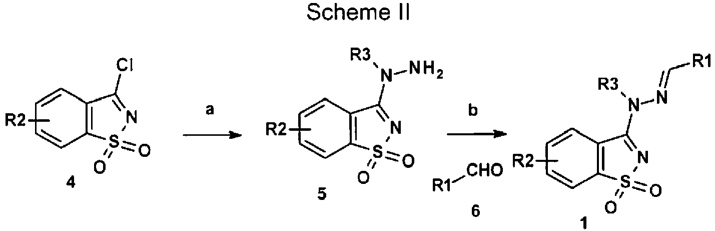

In scheme III, step a, aromatic substitution of bromo derivative (prepared following scheme I or II) with bis pinacolborane is done in presence of palladium catalyst (for example, Dzhevakov et al., Adv. Synth. Catal., 2016, 358(6), 977-983, Burns et al. WO 2009/029998). Step b, the corresponding boronic acid analog is obtained using sodium periodate in acetone/water followed by hydrochloric acid in acetonitrile (for example, Murphy et al., Org. Lett., 2007, 9(5), 757-760 ; Tzchucke et al., Org. Lett., 2007, 9(5), 761-764; Blaquiere et al. WO 2015/025025). Step c, boronic acid could be further dialkylated with diol reagents in toluene (for example, Li et al., Org. Proc. Res. Dev., 2016, 20(8), 1489-1499; Dastbaravardeh et al., Org. Lett., 2012, 14(7), 1930-1933) or di-acylated with di-acid reagents in toluene and/or demethylsulfoxide in presence or not of a dehydrating agent such as magnesium sulfate (for example, Naidu et al. WO 2014/028384). Step d, aromatic substitution of bromo derivative could also be done with trialkylphosphite in presence of palladium catalyst (like palladium acetate) in acetonitrile. Step e, the corresponding phosphonic acid is obtained using bromotrimethylsilane in dichloromethane (for example step d and e, Keglevich et al., Heteroatom Chem., 2012, 23(6), 574-582; Chen et al., JACS, 2014, 136(49), 17337-17342).

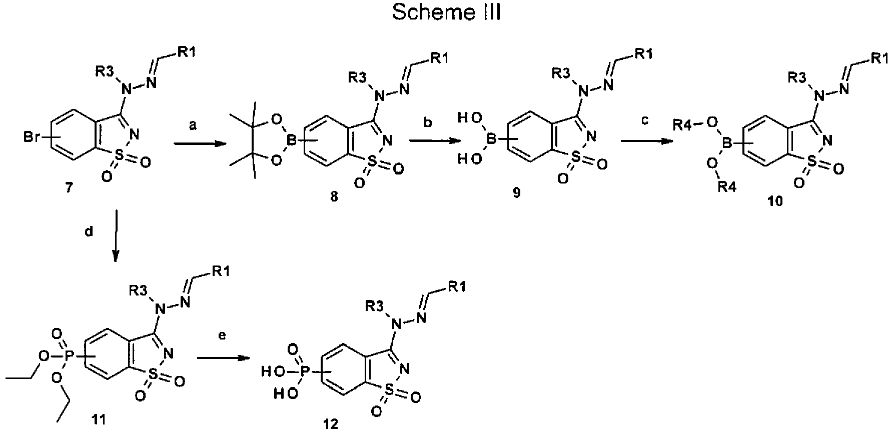

In scheme IV, step a, aromatic substitution of bromo derivative (prepared following scheme I or II) with phenols is done in presence of palladium catalyst (like palladium acetate), 2-(Di-tert-butylphosphino)biphenyl, potassium phosphate in toluene (for example, Aranyos et al., JACS, 1999, 121(18), 4369-4378).

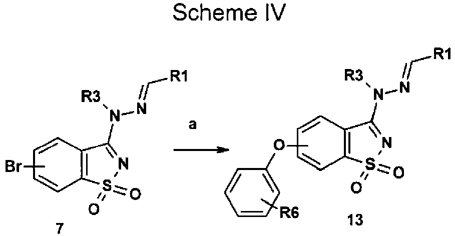

In scheme V, step a, pinacol borane analog (prepared following scheme III, step a) is hydrolyzed with hydrogen peroxide to give the corresponding phenol derivative (for example, Schumacher et al., WO 2007/038367). Step b, akylation of the hydroxyl group could be done either using halogeno alkylating agent with a mineral or organic base (like triethylamine, pyridine, potassium carbonate, cesium carbonate) or by a Mitsunobu reaction in presence triphenyl phosphine and azodicarboxylate reagents (like diethyl azodicarboxylate, diisopropyl azodicarboxylate..). Step c, the tert-butyl ester is hydrolyzed in acidic medium (like trifluoroacetic acid, hydrochloric acid...). Step d, amide is form using classical coupling reagents (such as o-(7-azabenzotriazol-1-yl)-N,N,N',N'-tetramethyluronium hexafluorophosphate, 1-hydroxy-7-azabenzotriazole, dicyclohexyl carbodiimide, 1-ethyl-3-(3-dimethylaminopropyl)carbodiimide, 1-hydroxy benzotriazole...)

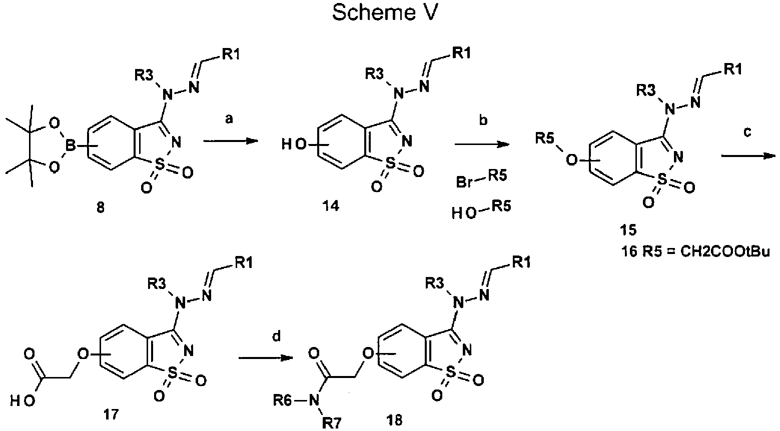

In scheme VI, step a, nitro derivatives (prepared following scheme I or II) are reduced using well known reducing methods (like catalytic hydrogenation, iron reduction...) (for example Hudlicky Reductions in Organic Chemistry ACS monograph 188 second edition). Step b, aniline is acylated using either acylchloride in presence of an organic base (like dimethylaminopyridine, triethylamine...) or acid with classical coupling agent reagents (as scheme V, step d).

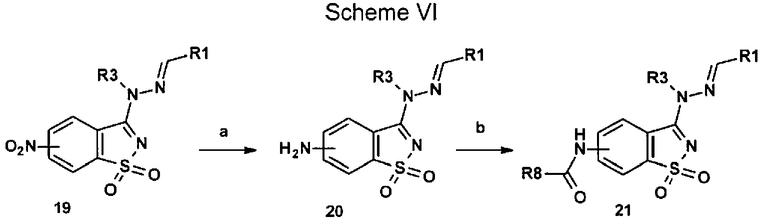

In scheme VII, step a, condensation of tert-butoxycarbonyl hydrazide with aldehyde (commercially available or prepared following schemes IX - XVII) is done using similar conditions as described in scheme I, step a. For sensitive function present on R1 some protection step using acid cleavable groups could be done before the next step of alkylation (for example Green et al. Protective group in organic synthesis, Wiley, third edition). Step b, alkylation is done using halogeno alkylating agent with a mineral or organic base (like triethylamine, pyridine, potassium carbonate, cesium carbonate...) in dimethylformamide. Step c, cleavage of the tert-butylcarboxyl protecting group is obtained using hydrochloric acid in dioxane.

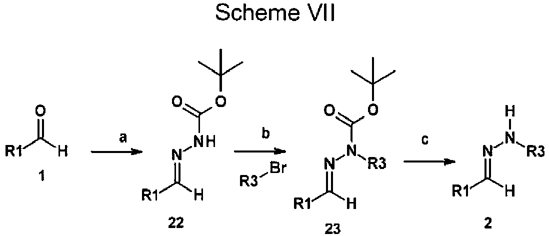

Saccharin preparations are described in scheme VIII. Step a sulfonylation is done starting from commercially available phenylsulfonyl chloride with ammonia in methanol or dichloromethane (for example, Blanchet et al., JOC, 2007, 72(9), 3199-3206; Schneider et al., 2011, Org. Lett., 13(14) 3588-3591). Step b, oxidation of methyl group is performed with potassium permanganate in acetone (for example, Sam et al., JACS, 1972, 94, 4024-4025). Step c, cyclisation into saccharin is obtained using sulfuric acid (for example, Perez et al. WO 2010/100139). Step d, saccharin derivative can also be prepared in one step by cyclisation of commercially available 2-methoxycabonyl-phenyl sulfonyl chloride with ammonia in tetrahydrofuran (for example Subramanyam et al. U.S., 5306818; Kim et al., Bioorg. Med. Chem. Lett., 2008, 18(14), 4181-4185).

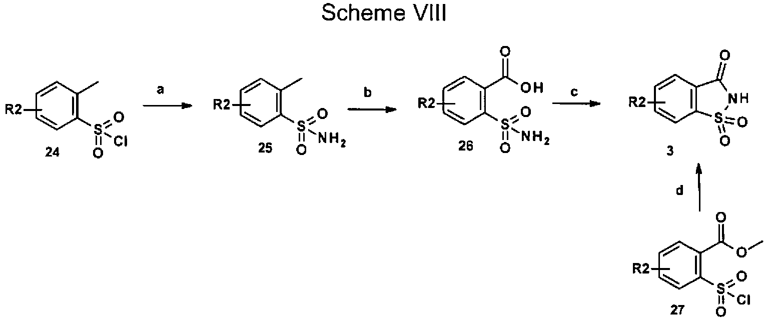

Scheme IX describes the different routes used to prepare 3-substituted-2-oxo-1H-benzimidazole-5-carbaldehyde. The first route, step a, aromatic substitution of commercially available 3-fluor-4-nitro-benzoic acid methyl ester with amine in tetrahydrofuran (for example, Liu et al., Molecules, 2012, 17, 4545-4559; Zhang et al., Bioorg. Med; Chem. Lett., 2008, 18(23), 6067-6070). Step b, the reduction of the nitro group is done either by catalytic hydrogenation with palladium on charcoal or iron reduction in acidic medium (for example, Bandarage et al., Bioorg. Med. Chem.Lett., 2009, 19(17), 5191-5194; Mukhina et al., Angew. Chem., Inter. Ed., 2015, 54(39), 11516-11520). Step c, cyclisation to benzimidazolone ester is obtained using carbonyldimidazole (for example, Zhang et al., J. Med. Chem., 2009, 52(18) 5703-5711), this benzimidazolone ester can be prepared by esterification of the commercially available corresponding acid (step f). Step d, reduction of the ester group is done using well known reducing agent (such as lithium aluminum hydride, lithium borohydride...) (for example, Hudlicky, Reductions in Organic Chemistry, ACS monograph 188, second edition). Step e, the aldehyde is obtained by oxidation using either Dess-Martin reagent, or pyridinium dichromate, or Svern reaction (for example, Dutta et al. U.S. Pat. Appl. Publ., 20140309427; Tohma et al., Adv. Syn. Catal., 2004, 346, 111-124; Omura et al., Tetrahedron, 1978, 34 (11), 1651-1660). The second route is used when the presence of ester group is not compatible with function in R9. Step g, reduction of ester is done with diisobutylaluminum hydride in dichloromethane. Step h, benzyl alcohol is protected with a tert-butyl-dimethylsilyl group using classical methods (like tert-butyl-dilethylsilyl chloride, imidazole in dimethylformamide) (for example Green et al., Protective Group in Organic Synthesis, Wiley, third edition). Step I, aromatic substitution is performed using the same conditions as step a, partial silyl group cleavage occurs during this reaction needing a new step of protection in step j (using same conditions as step h). Step k, reduction of nitro function is done using same conditions as step b. Cyclisation in step I is done using carbonyldiimidazole as in step c followed by silyl group cleavage with tert-butyl ammonium fluoride in tetrahydrofuran to benzyl alcohol derivative (step m) which is further oxidized into aldehyde (step e).

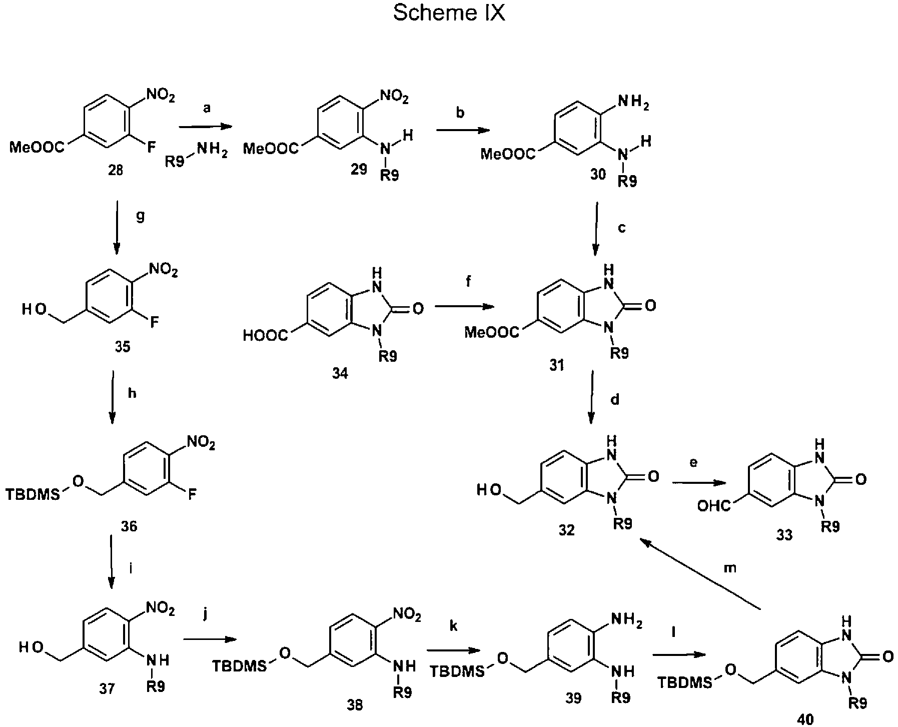

In scheme X, step a, protection of commercially available bromo isoindolin-1-one with either a tert-butyl carbamate or a p-methoxy benzyl using well described classical methods (for example Green et al. Protective Group in Organic Synthesis, Wiley, third edition). Step b, two methods are used for alkylation: alkyl iodide with lithium hexamethyldisilazane as base for mono alkylation (for example, Deniau et al., Tetrahedron, 2001, 57(13), 2581-2588) and with sodium hydride as base for di-alkylation (for example, Aebi et al., WO 2016/055394). Step c, cleavage of the protecting group using classical described methods. Step d, formation of aldehyde is done with methylmagnesium bromide, tert-butyl lithium and dimethylformamide in tetrahydrofuran (for example, Aiguade et al., WO 2013/068554).

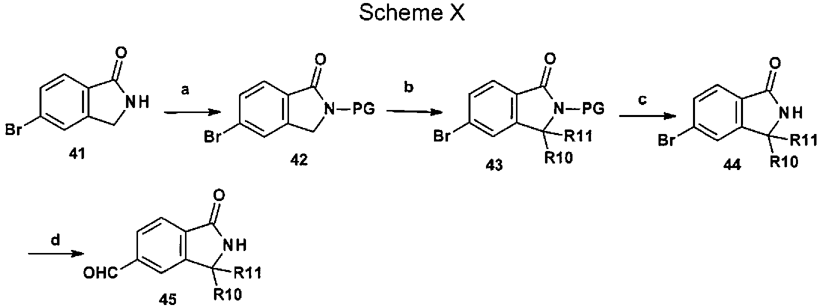

In scheme XI, step a, protection of commercially available bromo indolin-1-one with a tert-butyl carbamate using well described classical methods. Step b, alkylation is done with alkyl iodide and sodium hydride as base (for example, Meerpoel et al., WO 2012/084804), tert-butyl carbamate cleavage occurs during the work up. Step c, formation of aldehyde is done using the same conditions as scheme X step d.

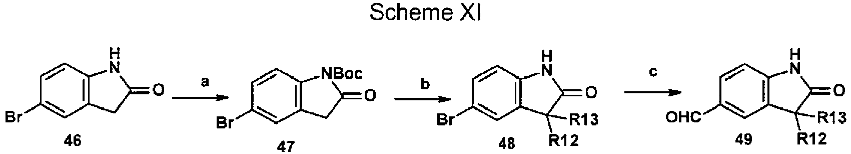

In scheme XII, step a, alkylation of commercially available hydroxyl-benzaldehyde is done with halogeno alkylating agent and an organic or mineral base (such as triethylamine, pyridine, potassium carbonate, cesium carbonate...) in dimethylformamide (for example, Luehr et al., Bioorg. Med. Chem., 2010, 18(4), 1388-1395; Ebright et a/., WO 2013/192352).

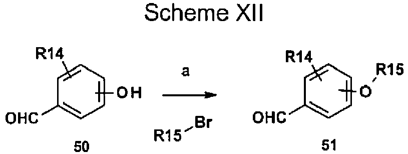

In scheme XII, step a, hydrolyze of commercially available cyano-benzaldehyde is performed in sulfuric acid (for example, Zil'berman et al., Russ. Chem. Rev., 1984, 53, 900-912).

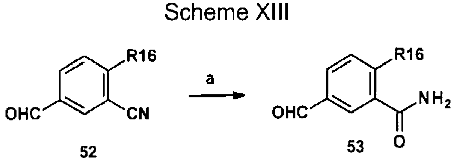

In scheme XIV, step a, amide is formed using classical coupling agents (such as carbonyl diimidazole, dicyclohexylcarbodiimide, 1-ethyl-3-(3-dimethylaminopropyl)carbodiimide, 1-hydroxy-7-azabenzotriazole, 1-hydroxybenzotriazole, o-(7-azabenzotriazol-1-yl)-N,N,N',N'-tetramethyluronium hexafluorophosphate...). Step b, formylation is done using the same conditions as scheme X, step d.

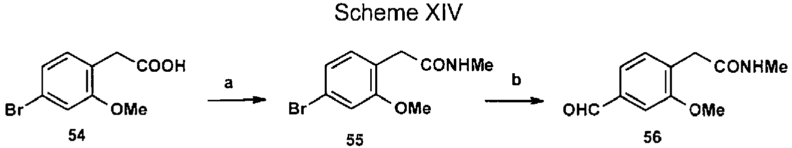

In scheme XV, step a, both alkylation and esterification are done in one step with methyl iodide and potassium carbonate in dimethylformamide. Step b, radical bromination is performed using N-bromosuccinimide, benzoylperoxide in tetrachlorocarbone (for example, Sun et al., 2008, JOC, 73(18), 7361-7364; Baldwin et al., WO 2008/156817). Step c, transformation to aldehyde is done with silver nitrate in acetone (for example, Traynelis et al., WO 2014/025942). Step d, protection of the aldehyde to dioxolane is obtained using well known methods (ethanediol in presence of acid such as p-toluene sulfonic acid, triethylorthoformate....) (for example, Green et al. Protective Group in Organic Synthesis, Wiley, 3rd edition). Step e, reduction of ester is done using lithium aluminum hydride as reducing agent. Step f, aldehyde is recovered in acid medium (like: hydrochloride acid, pyridinium p-toluene sulfonate...).

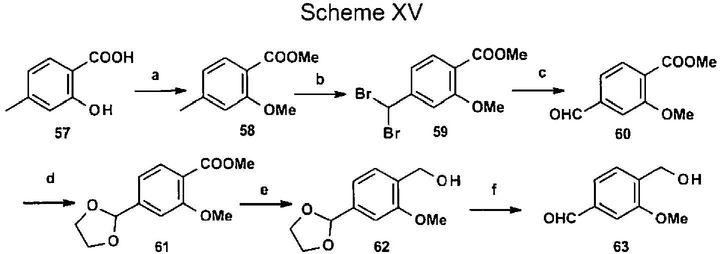

Preparation of pyridine carbaldehydes are described in schemes XVI and XVII. In Scheme XVI, step a, benzyl protection is done using classical methods with benzylbromide in basic medium (for example, Green et al. Protective Group in Organic Synthesis, Wiley, third edition). Step b, transformation into pyridinone is performed with alcoholic ammonia solution (for example, Xie et al., Eur. J. Med. Chem., 2016, 115, 132-140). Step c, alkylation is done with trimethylsilyl diazomethane (for example, Meyers et al., Bioorg. Med. Chem. Lett., 2010, 20(5), 1543-1547). Step d, aldehyde is obtained by oxidation using either manganese dioxide, Dess-Martin reagent, pyridinium dichromate or Svern reaction (for example, Timmer et al., Chem. Commun. (Cambridge, United Kingdom), 2016, 52(83), 12326-12329; Fuchida et al., Bul. Chem. Soc. Jp., 2015, 88(6) 784-791; Garcia et al., Chem. Commun. (Cambridge, United Kingdom), 2016, 52(58), 9059-9062). Step e, benzyl cleavage is done by catalytic hydrogenation with palladium on charcoal in methanol.

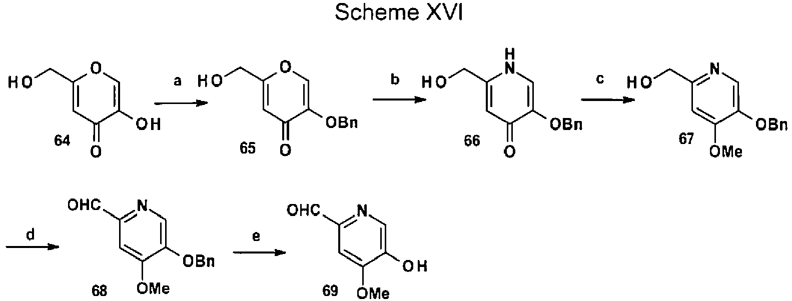

In scheme XVII, step a, reduction is done with lithium aluminum hydride in tetrahydrofuran. Step b, aldehyde is obtained using the same oxidation conditions as scheme XVI step c.

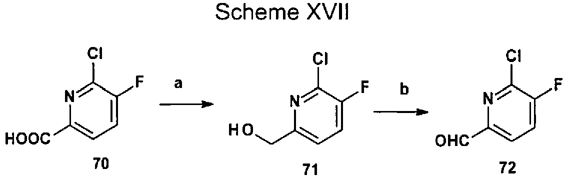

In scheme XVIII, step a, the formation of the Weinreb amide is done using classical conditions with methoxymethanamine in presence of pyridine in dichloromethane. Step b, the ketone is obtained from the corresponding bromo derivative with the Weinreb amide using butyl lithium as base in THF (for example, Goulet. et al WO 00/04013). Step c, cyclisation to tetrahydropyridazine is done with hydrazine hydrate in ethanol. Step d is one pot reaction done using similar conditions as describe for scheme I steps b and c, using phosphorous oxychloride (as saccharin chlorinating agent) without any solvent.

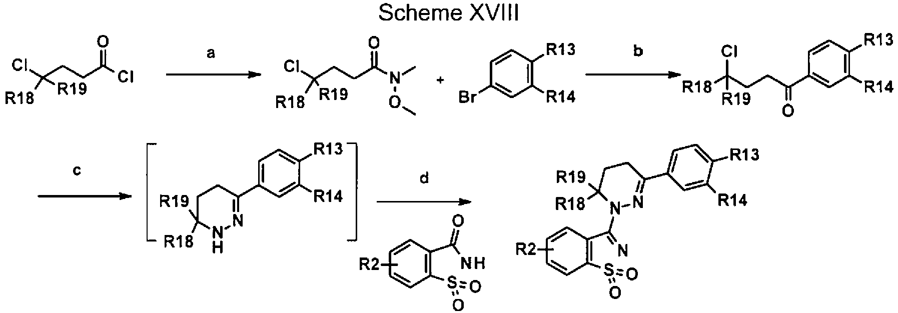

In scheme XIX, step a, succinic ring opening is done using a phenylmagnesiumlbromide (prepared from the corresponding phenylbromide with magnesium turnings) in THF (for example, Sakai et al. Chemistry Letters, 2015, 44(11), 1503-1505). Step b, cyclisation to tetrahydropyridazine is done with hydrazine hydrate in ethanol (for example, Gouault et al., Journal of Pharmacy and Pharmacology, 2001, 53(7), 981-985). Step c, the reduction id done using lithium aluminium hydride in THF (for example, Winton. et al., Journal of Heterocyclic Chemistry, 1984, 21(3), 889-91).

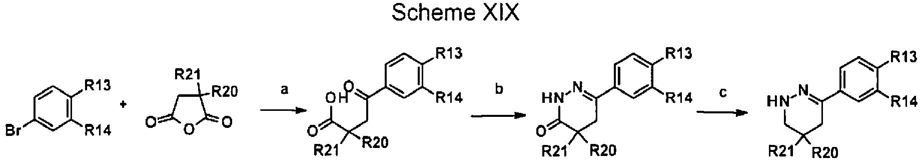

In scheme XX, alkylation of benzimidazolone is done using classical conditions with alkylbronine or chlorine as alkylating agent using sodium hydride as base in dimethylformamide.

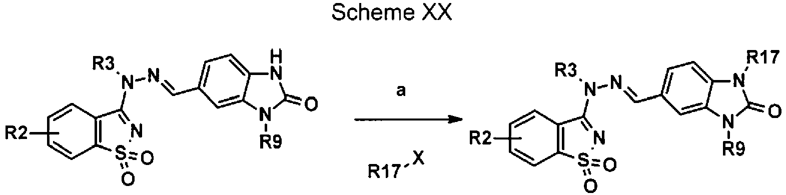

### ABBREVIATIONS

Ac = Acetyl; AcOH = Acetic acid; ACN = Acetonitrile; Bn = Benzyl; Boc = *t-*Butoxycarbonyl; Boc₂O = Di-*tert*-butyl-dicarbonate; BSA = Bivine serum albumin; Bt = Benzotriazolyl; n-BuLi = n-Butyllithium; s-BuLi = sec-Butyllithium; *t*-BuLi = *tert*-Butyllitium; Bu₄NBr = Tetrabutylammonium bromide; CaCl₂ = Calcium chloride; DCM = Dichloromethane; D₂O = deuterated water; CDCl₃ = Chloroform deutered; CDI = Carbonyl diimidazole; Cs₂CO₃ = Cesium carbonate; d = Doublet; dba = Dibenzylideneacetone; DCC = Dicyclohexylcarbodiimide; DCE = Dichlororethane; DCM = Dichloromethane; dd = Doublet of doublets; DEAD = Diethyl azodicarboxylate; DIAD = Diisopropyl azodicarboxylate; DIBAL = Diisobutylaluminum hydride; DIPEA = Diisopropylethylamine; DMF = *N*,*N*-Dimethylformamide; DMSO = Dimethylsulfoxide; dppf = 1,1'-bis(diphenyl-phosphino)ferrocene; EDCI = 1-ethyl -3-(3-dimethylaminopropyle) carbodiimide hydrochloride; eq = Equivalent(s); EtOAc = Ethyl acetate; Et₂O = Ether; EtOH = Ethanol; g = Gram(s); h = Hour(s); HATU = O-(7-azabenzotriazol-1-yl)-N, N, N', N'-tetramathyluroniom hexafluorophosphate; HCl = Hydrochloric acid; Hz = Hertz; HOAt = 1-hydroxy-7-azabenzotriazole; HCOOH = formic acid; HOBt = Hydroxybenzotriazole; HMDS = 1,1,1,3,3,3-hexamethyldisilazane; KF = Potassium fluoride; K₂CO₃ = Potassium carbonate; KOH = potassium hydroxide; LC/MS = Liquid chromatography/mass spectrometry; LDA = Lithium diisopropylamide; LiAlH₄ = Lithium aluminium hydride; LiHMDS = Lithium bis(trimethylsilyl)amide; m = Multiplet; min = Minute; MeOH = Methanol; mg = Milligram; MgSO₄ = Magnesium sulfate; min = Minute(s); mmol = Millimole; mp = Melting point; MW = Microwave; N = Normal; NaCl = Sodium chloride; NaH = Sodium hydride; NaHCO₃ = Sodium bicarbonate; NaBH₃CN = Sodium cyanoborohydride; Na₂CO₃ = Sodium carbonate; NaOH = Sodium hydroxide; Na₂SO₄ = Sodium sulfate; NBS = N-bromosuccinimide; NH₃ = Ammonia; NH₄Cl = Ammonium chloride; NMM = N-methylmorpholine; NMP = M-methylpyrrolidone; NMR = Nuclear magnetic resonance; PBS = Phosphate buffered saline; PCC = Pyridinium chlorochromate; PDC = Pyridinium dichromate; Pd/C = Palladium on carbon; PdCl₂(dppf) = Dichloro [1,1'-bis(diphenylphosphino) ferrocene] palladium (II); Pd₂(dba)₃ = Bis(dibenzylidenacetone)palladium(0); Pd(OAc)₂ = Palladium (II) acetate; Pd(PPh₃)₄ = Tetrakis(triphenylphosphine)palladium (0); Ph = Phenyl; ppm = Parts per million; PPTS = Pyridinium p-toluenesulfonate; PrOH = Propanol; PSI = Pounds per square inch; q = Quadruplet; quant = Quantitative ; quint = Quintuplet; Rt = Retention time ; rt = Rt; s = singlet; sept = septuplet; sext = sextuplet; SiO₂ = Silica; SnCl₂ = tin chloride; t = triplet; TBAF = Tetrabutyl ammonium fluoride; TBDMS = t-butyldimethylsilyl; TEA = Triethylamine; TFA = Trifluoroacetic acid; THF = Tetrahydrofuran; TMEDA = N, N, N', N'-tretramethyl ethylene diamine.

### General

Reagents and solvents obtained from commercial suppliers are used without further purification unless otherwise stated. Analytical data is included within the procedures below. 1H NMR spectra were recorded on a Bruker Advance spectrometer. Chemical shifts are reported in ppm (δ) and were calibrated using the undeuterated solvent resonance as internal standard. Melting points were determined on a hotstage apparatus and are uncorrected.

### Analytical Methods

| **Method** | **Description** | |
|---|---|---|
| A | *Column:* KINETEX XB-C18 core-shell (Dimensions: 30 x 3 mm, 2.6 µm) | |
| | *Column temperature:* 45 °C | |
| | *Mobile Phase:* A1: Water (0.1% v/v AcOH) | |
| | | B1: MeCN (0.1% v/v AcOH) |
| | *Gradient:* from 10% to 100% B1 within 3.15 min | |
| | *Flow rate:* 1.4 ml/min | |
| | *UV detection:* DAD 210-260 nm | |
| | *MS detection:* ESI positive | |
| B | *Column:* KINETEX EVO C18 (Dimensions: 50 x 4.6 mm, 5 µm) | |
| | *Column temperature:* 25 °C | |
| | *Mobile Phase:* A2: MeCN:H₂O = 5:95 with 20 mM NH₄HCO₂ buffer, pH = 7.4 | |
| | | B2: MeCN:H₂O = 80:20 with 20 mM NH₄HCO₂ buffer, pH = 7.4 |
| | *Gradient:* from 5% to 100% B2 within 4.50 min | |
| | *Flow rate:* 1.3 ml/min | |
| | *UV detection:* DAD 220-254 nm | |
| | *MS detection:* APCI positive | |
| C | *Column:* KINETEX EVO C18 (Dimensions: 50 x 4.6 mm, 5 µm) | |
| | *Column temperature:* 25 °C | |
| | *Mobile Phase:* A1: Water (0.05% v/v TFA) | |
| | | B1: MeCN (0.05% v/v TFA) |
| | *Gradient:* from 5% to 100% B1 within 4.50 min | |
| | *Flow rate:* 1.3 ml/min | |
| | *UV detection:* DAD 220-254 nm | |
| | *MS detection:* APCI positive | |
| D | *Column:* KINETEX EVO C18 (Dimensions: 50 x 4.6 mm, 5 µm) | |
| | *Column temperature:* 25 °C | |
| | *Mobile Phase:* A2: MeCN:H₂O = 5:95 with 20 mM NH₄HCO₂ buffer, pH = 7. | |
| | | B2: MeCN:H₂O = 80:20 with 20 mM NH₄HCO₂ buffer, pH = 7.4 |
| | *Gradient:* from 30% to 100% B2 within 4.50 min | |
| | *Flow rate:* 1.3 ml/min | |
| | *UV detection:* DAD 220-254 nm | |
| | *MS detection:* APCI positive | |
| E | *Column:* KINETEX EVO C18 (Dimensions: 50 x 4.6 mm, 5 µm) | |
| | *Column temperature:* 35 °C | |
| | *Mobile Phase:* A2: MeCN:H₂O = 5:95 with 20 mM NH₄HCO₂ buffer, pH = 7.4 | |
| | | B2: MeCN:H₂O = 80:20 with 20 mM NH₄HCO₂ buffer, pH = 7.4 |
| | *Gradient:* from 5% to 100% B2 within 4.00 min | |
| | *Flow rate:* 1.3 ml/min | |
| | *UV detection:* DAD 220nm | |
| | *MS detection:* APCI positive | |

### Example 1: N-[(E)-(4-fluorophenyl)methyleneamino]-N-methyl-1,1-dioxo-1,2-benzothiazol-3-amine

### Step A: 3-chloro-1,2-benzothiazole 1,1-dioxide

A mixture of saccharin (70.00 g; 382.13 mmol; 1.00 eq.), thionyl chloride (41.58 ml; 573.19 mmol; 1.50 eq.) and a catalytic amount of DMF (2.10 ml) in 1,4-dioxane (350 ml) was heated for 24h under reflux. The reaction mixture was concentrated, co-evaporated 4 times with toluene (200 ml) and dried to give 3-chloro-1,2-benzothiazole 1,1-dioxide (77.00 g; 99 %), used without further purification. 1H NMR (DMSO-d6, 300 MHz): δ 8.19 (dd, J = 1.0 Hz, J = 7.0 Hz, 1H); 7.99 (m, 3H).

### Step B: 1-(1,1-dioxo-1,2-benzothiazol-3-yl)-1-methyl-hydrazine

3-chloro-1,2-benzothiazole 1,1-dioxide (7.00 g; 0.03 mol; 1.00 eq.) was dissolved into dry THF (56 ml) and then added dropwise to a solution of methyl hydrazine (2.22 ml; 0.04 mol; 1.20 eq.) in THF (56 ml). The reaction was then stirred 1h under reflux and the solid formed was filtered. It was washed with water and dried at 50°C under vacuum to give 1-(1,1-dioxo-1,2-benzothiazol-3-yl)-1-methyl-hydrazine (5.92 g; 81 %) as a white powder. 1H NMR (DMSO-d6, 300 MHz): δ 8.97 (m, 1H), 7.93 (m, 1H), 7.77 (m, 2H), 5.61 (s, 2H), 3.43 (s, 3H).

### Step C: N-[(E)-(4-fluorophenyl)methyleneamino]-N-methyl-1,1-dioxo-1,2-benzothiazol-3-amine

A mixture of 1-(1,1-dioxo-1,2-benzothiazol-3-yl)-1-methyl-hydrazine (70.0 mg; 0.33 mmol; 1.00 eq.) and 4-fluorobenzaldehyde (54 µl; 0.50 mmol; 1.50 eq.) in 1,4-dioxane (2 ml) was stirred 6h under reflux. The reaction was cooled to rt and concentrated under reduced pressure. The residue was taken up into few dioxane and the solid was filtered, washed once with dioxane and dried under vacuum at 50°C to give N-[(E)-(4-fluorophenyl)methyleneamino]-N-methyl-1,1-dioxo-1,2-benzothiazol-3-amine (46.00 mg; 43.48 %) as a white solid. 1H NMR (DMSO-d6, 400 MHz): δ 8.85 (d, J = 7.7 Hz, 1H); 8.51 (s, 1H); 8.10 (d, J = 7.4 Hz, 1H); 7.93 (m, 4H) ; 7.42 (t, J = 8.8 Hz, 2H) ; 3.81 (s, 3H).

### Example 2: 2-[3-[(E)-[(1,1-dioxo-1,2-benzothiazol-3-yl)-methyl-hydrazono]methyl] phenoxy]ethanol

The compound was prepared using the same procedure as described in example 1 step C starting from 1-(1,1-dioxo-1,2-benzothiazol-3-yl)-1-methyl-hydrazine (example 1, step B, 70.0 mg; 0.33 mmol; 1.00 eq.) and 3-(2-hydroxyethoxy)benzaldehyde (82.6 mg, 0.5 mmol, 1.5 eq.) giving 2-[3-[(E)-[(1,1-dioxo-1,2-benzothiazol-3-yl)-methyl-hydrazono]methyl]-phenoxy]ethanol (24.0 mg; 20 %) as a white solid. 1H NMR (DMSO-d6, 400 MHz): δ 8.87 (m, 1H); 8.46 (s, 1H); 8.09 (m, 1H); 7.92 (m, 2H) ; 7.48 (m, 2H) ; 7.42 (m, 1H); 7.11 (m, 1H) ; 4.92 (t, J = 5.4 Hz, 1H) ; 4.10 (t, J = 5.0 Hz, 2H); 3.80 (s, 3H); 3.77 (q, J = 5.3 Hz, 2H).

### Example 3: N-methyl-N-[(E)-(4-morpholinophenyl)methyleneamino]-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 50.0 mg; 0.24 mmol; 1.00 eq.) and 4-(4-morpholinyl)benzaldehyde (49.8 mg, 0.26 mmol, 1.10 eq.) giving N-methyl-N-[(E)-(4-morpholinophenyl)methyleneamino]-1,1-dioxo-1,2-benzothiazol-3-amine (60.0 mg; 66 %) as a yellow solid. 1H NMR (DMSO-d6, 400 MHz): δ 8.90 (d, J = 7.1 Hz, 1H); 8.37 (s, 1H); 8.07 (d, J = 7.1 Hz, 1H); 7.89 (m, 2H) ; 7.75 (d, J = 8.5 Hz, 2H) ; 7.09 (d, J = 8.5 Hz, 2H) ; 3.79 (s, 3H) ; 3.75 (t, J = 4.9 Hz, 4H) ; 3.27 (t, J = 4.9 Hz, 4H). mp: 258-261 °C.

### Example 4: N-[(E)-[3-(dimethylamino)phenyl]methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 70.0 mg; 0.33 mmol; 1.00 eq.) and 3-(dimethylamino)benzaldehyde (74.2 mg; 0.50 mmol; 1.50 eq.) giving N-[(E)-[3-(dimethylamino)phenyl]methyleneamino]-N-methyl-1,1-dioxo-1,2-benzo-thiazol-3-amine (49.0 mg; 42 %) as a yellow solid. 1H NMR (DMSO-d6, 400 MHz): δ 8.92 (dd, J = 2.3 Hz, J = 6.3 Hz, 1H); 8.42 (s, 1H); 8.09 (m, 1H); 7.90 (m, 2H) ; 7.36 (t, J =7.8 Hz, 1H); 7.18 (m, 2H);6.90 (dd, J =2.3Hz, J =8.3Hz, 1H); 3.79 (s, 3H); 2.99 (s, 6H).

### Example 5: N-[(E)-(2-aminothiazol-5-yl)methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 50.0 mg; 0.24 mmol; 1.00 eq.) and 2-aminothiazole-5-carboxaldehyde (36.4 mg; 0.28 mmol; 1.2 eq.) giving N-[(E)-(2-aminothiazol-5-yl)methyleneamino]-N-methyl-1,1-dioxo-1,2-benzo thiazol-3-amine (11.2 mg; 14 %) as an orange solid. 1H NMR (DMSO-d6, 400 MHz): δ 8.74 (d, J = 7.5 Hz, 1H); 8.53 (s, 1H); 8.07 (m, 1H); 7.84 (m, 4H) ; 7.57 (s, 1H) ; 3.73 (s, 3H).

### Example 6: N-methyl-1,1-dioxo-N-[(E)-(2-pyrrolidin-1-ylthiazol-5-yl)methyleneamino]-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 50.0 mg; 0.24 mmol; 1.00 eq.) and 2-(1-pyrrolidinyl)-1,3-thiazole-5-carbaldehyde (51.8 mg; 0.28 mmol; 1.20 eq.) giving N-methyl-1,1-dioxo-N-[(E)-(2-pyrrolidin-1-yl-thiazol-5-yl)methyleneamino]-1,2-benzothiazol-3-amine (29.0 mg; 33 %) as an orange solid. 1H NMR (DMSO-d6, 400 MHz): δ 8.75 (m, 1H); 8.56 (s, 1H); 8.05 (m, 1H); 7.88 (m, 2H) ; 7.74 (s, 1H); 3.73 (s, 3H) ; 3.51 (m, 4H) ; 2.02 (m, 4H). mp: 205-246 °C.

### Example 7: N-methyl-N-[(E)-(2-morpholinothiazol-5-yl)methyleneamino]-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 50.0 mg; 0.24 mmol; 1.00 eq.) and 2-(4-morpholino)thiazole-5-carboxaldehyde (56.3 mg; 0.28 mmol; 1.20 eq.) giving N-methyl-N-[(E)-(2-morpholinothiazol-5-yl)methyleneamino]-1,1-dioxo-1,2-benzothiazol-3-amine (61.0 mg; 66 %) as a yellow solid. 1H NMR (DMSO-d6, 400 MHz): δ 8.74 (m, 1H); 8.59 (s, 1H); 8.05 (m, 1H); 7.89 (m, 2H) ; 7.76 (s, 1H) ; 3.75 (m, 4H); 3.74 (s, 3H) ; 3.59 (m, 4H). mp: 281-300 °C.

### Example 8: N-methyl-1,1-dioxo-N-[(E)-(2-phenylthiazol-5-yl)methyleneamino]-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 50.0 mg; 0.24 mmol; 1.00 eq.) and 2-phenyl-1,3-thiazole-5-carbaldehyde (53.8 mg; 0.28 mmol; 1.20 eq.) giving N-methyl-1,1-dioxo-N-[(E)-(2-phenylthiazol-5-yl)methyleneamino]-1,2-benzothiazol-3-amine (58.0 mg; 64 %) as a yellow solid. 1H NMR (DMSO-d6, 400 MHz): δ 8.81 (m, 1H); 8.77 (s, 1H); 8.38 (s, 1H); 8.10 (m, 3H); 8.03 (dt, J = 1.2 Hz, J = 7.8 Hz, 1H); 7.92 (dt, J = 1.1 Hz, J = 7.7 Hz, 1H); 7.58 (m, 3H); 3.80 (s, 3H). mp: 271-353 °C.

### Example 9: N-[(E)-(4-fluoro-3-methoxy-phenyl)methyleneamino]-1,1-dioxo-N-[2-(2-pyridyl)ethyl]-1,2-benzothiazol-3-amine

### Step A: 1-(1,1-dioxo-1,2-benzothiazol-3-yl)-1-[2-(2-pyridyl)ethyl]hydrazine

The compound was prepared using the same procedure as described in example 1 step B starting from 3-chloro-1,2-benzothiazole 1,1-dioxide (example 1, step A, 200 mg; 0.99 mmol; 1.00 eq.) and 2-(2-pyridyl)ethylhydrazine (158 mg; 1.09 mmol; 1.10 eq.) giving 1-(1,1-dioxo-1,2-benzothiazol-3-yl)-1-[2-(2-pyridyl)ethyl]hydrazine (144 mg; 48 %) as a pale yellow solid, used without purification in the next step. 1H NMR (DMSO-d6, 300 MHz): δ 9.00 (m, 1H); 8.55 (d, J = 5.6 Hz, 1H); 7.93 (m, 1H); 7.81 (m, 3H) ; 7.44 (d, J = 7.5 Hz, 1H); 7.33 (t, J = 6.4 Hz, 1H); 5.73 (br s, 2H) ; 4.14 (t, J = 7.5 Hz, 2H) ; 3.30 (t, J = 7.5 Hz, 2H).

### Step B: N-[(E)-(4-fluoro-3-methoxy-phenyl)methyleneamino]-1,1-dioxo-N-[2-(2-pyridyl)ethyl]-1,2-benzothiazol-3-amine

The compound was prepared using the same procedure as described in example 1 step C starting from 1-(1,1-dioxo-1,2-benzothiazol-3-yl)-1-[2-(2-pyridyl)ethyl]hydrazine (50.0 mg; 0.17 mmol; 1.00 eq.) and 4-fluoro-3-methoxybenzaldehyde (31.0 mg; 0.2 mmol; 1.20 eq.) giving N-[(E)-(4-fluoro-3-methoxy-phenyl)methyleneamino]-1,1-dioxo-N-[2-(2-pyridyl)-ethyl]-1,2-benzothiazol-3-amine (50.0 mg; 69 %) as a yellow solid. 1H NMR (DMSO-d6, 300 MHz): δ 8.78 (d, J = 7.5 Hz, 1H); 8.52 (m, 2H) ; 8.08 (d, J = 7.5 Hz, 1H); 7.90 (m, 2H); 7.73 (t, J = 7.5 Hz 1H); 7.59 (d, J = 8.4 Hz, 1H); 7.40 (m, 3H) ; 7.25 (m, 1H); 4.74 (t, J = 7.6 Hz, 2H); 3.96 (s, 3H); 3.23 (t, J = 7.6 Hz, 2H). mp: 211-213 °C.

### Example 10: methyl 2-[3-[(E)-[(1,1-dioxo-1,2-benzothiazol-3-yl)-methyl-hydrazono] methyl]phenyl]acetate

### Step A: methyl 2-(3-vinylphenyl)acetate

Tetrakis(triphenylphosphine)palladium(0) (252.2 mg; 0.22 mmol; 0.10 eq.) and tributyl(vinyl)tin (953 µl; 3.27 mmol; 1.50 eq.) were added to a solution of methyl 2-(3-bromophenyl)acetate (500.0 mg; 2.18 mmol; 1.00 eq.) in toluene (10 ml) under inert atmopshere. The reaction mixture was stirred 1h30 under reflux, then cooled to rt and concentrated to dryness. The residue was purified by chromatography (column SiO₂ 10g, flow rate 15ml/min, gradient from 100% cyclohexane to 75/25 cyclohexane / EtOAc) to give methyl 2-(3-vinylphenyl)acetate (422.0 mg; quant.) as a white solid. 1H NMR (DMSO-d6, 300 MHz): δ 7.36 (m, 2H) ; 7.29 (m, 1H); 7.17 (m, 1H); 6.72 (dd, J = 10.9 Hz, J = 17.7 Hz, 1H); 5.82 (dd, J = 1.0 Hz, J = 17.7 Hz, 1H); 5.26 (dd, J = 1.0 Hz, J = 10.9 Hz, 1H); 3.68 (s, 2H) ; 3.62 (s, 3H).

### Step B: methyl 2-[3-(1,2-dihydroxyethyl)phenyl]acetate

AD-Mix-alpha (4.00 g) was added to a solution of methyl 2-(3-vinylphenyl)acetate (422.0 mg; 2.39 mmol; 1.00 eq.) in a mixture of tert-butanol (4.20 ml) and water (4.20 ml). The reaction was then stirred 16h at rt. Sodium sulfite (4.27 g; 33.84 mmol; 14.13 eq.) was added and the reaction was stirred 1h. Then water and EtOAc were added. The organic layer was recovered and the aqueous layer one extracted twice with EtOAc. Combined organic layers were washed with brine, dried over MgSO4, filtered and concentrated to dryness. The residue was purified by chromatography (column SiO₂ 25g, flow rate 25ml/min, gradient from 100% cyclohexane to 50/50 cyclohexane / EtOAc) to give methyl 2-[3-(1,2-dihydroxyethyl)phenyl]acetate (245.0 mg; 49 %.) as a yellow oil. 1H NMR (DMSO-d6, 300 MHz): δ 7.24 (m, 3H) ; 7.12 (td, J = 1.8 Hz, J = 6.4 Hz, 1H); 5.22 (d, J = 4.3 Hz, 1H); 4.70 (t, J = 5.8 Hz, 1H); 4.50 (dt, J = 4.3 Hz, J = 5.9 Hz, 1H); 3.65 (s, 2H) ; 3.61 (s, 3H) ; 3.41 (t, J =5.9 Hz, 2H).

### Step C: methyl 2-(3-formylphenyl)acetate

Sodium metaperiodate (498.5 mg; 2.33 mmol; 2.00 eq.) was added to a solution of methyl 2-[3-(1,2-dihydroxyethyl)phenyl]acetate (245.0 mg; 1.17 mmol; 1.00 eq.) in water (5.90 ml) and acetone (11.70 ml). The reaction was stirred 1h at rt and quenched by addition of saturated aqueous sodium thiosulfate. The mixture was diluted with EtOAc and the organic layer was recovered. The aqueous layer was extracted twice with EtOAc and the combined organic layers were washed with brine, dried over MgSO4, filtered and concentrated under reduced pressure, giving methyl 2-(3-formylphenyl)acetate (220.0 mg; quant.) as a colorless oil, used without purification in the next step. 1H NMR (DMSO-d6, 300 MHz): δ 10.01 (s, 1H), 7.83 (m, 2H) ; 7.60 (m, 2H) ; 3.83 (s, 2H) ; 3.63 (s, 3H).

### Step D: methyl 2-[3-[(E)-[(1,1-dioxo-1,2-benzothiazol-3-yl)-methyl-hydrazono]methyl] phenyl]acetate

The compound was prepared using the same procedure as described in example 1 step C starting from 1-(1,1-dioxo-1,2-benzothiazol-3-yl)-1-methyl-hydrazine (example 1, step B, 79.0 mg; 0.37 mmol; 1.00 eq.) and methyl 2-(3-formylphenyl)acetate (100.0 mg; 0.56 mmol; 1.50 eq.) giving methyl 2-[3-[(E)-[(1,1-dioxo-1,2-benzothiazol-3-yl)-methyl-hydrazono]methyl]phenyl]acetate (70.0 mg; 49 %) as a beige solid. 1H NMR (DMSO-d6, 400 MHz): δ 8.89 (m, 1H); 8.48 (s, 1H); 8.09 (m, 1H); 7.93 (m, 2H) ; 7.81 (s, 1H); 7.79 (td, J = 1.1 Hz, J = 7.7 Hz, 1H); 7.53 (t, J = 7.6 Hz, 1H); 7.44 (td, J = 1.3 Hz, J = 7.5 Hz, 1H); 3.84 (s, 2H) ; 3.81 (s, 3H) ; 3.66 (s, 3H).

### Example 11: N-[(E)-(3-ethoxy-4-fluoro-phenyl)methyleneamino]-N-methyl-1,1-dioxo-1,2-benzothiazol-3-amine

### Step A: 3-ethoxy-4-fluoro-benzaldehyde

Bromoethane (213 µl; 2.85 mmol; 4.00 eq.) was added followed by potassium carbonate (493.2 mg; 3.57 mmol; 5.00 eq.) to a solution of 4-fluoro-3-hydroxybenzaldehyde (100.00 mg; 0.71 mmol; 1.00 eq.) in DMF (2.00 ml). The mixture was heated at 65°C for 3h30 and cooled to rt. Water was added and the mixture was extracted with EtOAc (3x). Combined organic layers were washed with brine, dried over MgSO4, filtered and concentrated. The residue was purified by chromatography (column SiO₂ 5g, flow rate 15ml/min, gradient from 100% cyclohexane to 80/20 cyclohexane / EtOAc) to give 3-ethoxy-4-fluorobenzaldehyde (108.70 mg; 91 %.) as a colorless oil. 1H NMR (DMSO-d6, 300 MHz): δ 9.94 (s, 1H); 7.63 (dd, J = 1.9 Hz, J = 8.3 Hz, 1H); 7.57 (ddd, J = 2.0 Hz, J = 4.6 Hz, J = 8.1 Hz, 1H); 7.47 (dd, J = 8.1 Hz, J = 10.9 Hz, 1H); 4.21 (q, J = 7.0 Hz, 2H); 1.38 (t, J = 7.0 Hz, 3H).

### Step B: N-[(E)-(3-ethoxy-4-fluoro-phenyl)methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 100.0 mg; 0.47 mmol; 1.00 eq.) and 3-ethoxy-4-fluoro-benzaldehyde (105.0 mg; 0.62 mmol; 1.32 eq.) giving N-[(E)-(3-ethoxy-4-fluoro-phenyl)methyleneamino]-N-methyl-1,1-dioxo-1,2-benzothiazol -3-amine (100.0 mg; 58 %) as a white solid. 1H NMR (DMSO-d6, 400 MHz): δ 8.60 (m, 1H) ; 8.46 (s, 1H) ; 8.09 (m, 1H ; 7.91 (m, 2H) ; 7.62 (dd, J = 1.8 Hz, J = 8.4 Hz, 1H); 7.47 (ddd, J = 2.2 Hz, J = 4.6 Hz, J = 8.4 Hz, 1H); 7.41 (dd, J = 8.1 Hz, J = 10.8 Hz, 1H) ; 4.24 (q, J = 6.9 Hz, 2H) ; 3.79 (s, 3H); 1.42 (t, J = 6.9 Hz, 3H). mp: 190-212 °C.

### Example 12: N-[(E)-(4-fluoro-3-propoxy-phenyl)methyleneamino]-N-methyl-1,1-dioxo-1,2-benzothiazol-3-amine

### Step A: 4-fluoro-3-propoxy-benzaldehyde

The compound was prepared using the same procedure as described in example 11 step A starting from 4-fluoro-3-hydroxybenzaldehyde (100.00 mg; 0.71 mmol; 1.00 eq.) and bromopropane (260 µl; 2.85 mmol; 4.00 eq.) giving 4-fluoro-3-propoxy-benzaldehyde (106.8 mg; 82 %) as a colorless oil. 1H NMR (DMSO-d6, 300 MHz): δ 9.94 (s, 1H); 7.64 (dd, J = 1.9 Hz, J = 8.3 Hz, 1H) ; 7.57 (ddd, J = 2.0 Hz, J = 4.8 Hz, J = 8.1 Hz, 1H) ; 7.47 (dd, J = 8.3 Hz, J = 11.1 Hz, 1H); 4.10 (t, J = 6.5 Hz, 2H) ; 1.78 (sext, J = 7.1 Hz, 2H); 1.00 (t, J = 7.4 Hz, 3H).

### Step B: N-[(E)-(4-fluoro-3-propoxy-phenyl)methyleneamino]-N-methyl-1,1-dioxo-1,2-benzothiazol-3-am ine

The compound was prepared using the same procedure as described in example 1 step C starting from 1-(1,1-dioxo-1,2-benzothiazol-3-yl)-1-methyl-hydrazine (example 1, step B, 100.0 mg; 0.47 mmol; 1.00 eq.) and 4-fluoro-3-propoxy-benzaldehyde (103.0 mg; 0.57 mmol; 1.20 eq.) giving N-[(E)-(4-fluoro-3-propoxy-phenyl)methyleneamino]-N-methyl-1,1-dioxo-1,2-benzothiazol-3-amine (122.8 mg; 69 %) as a white solid. 1H NMR (DMSO-d6, 400 MHz): δ 8.85 (m, 1H) ; 8.46 (s, 1H) ; 8.10 (m, 1H): 7.91 (m, 2H) ; 7.63 (dd, J = 2.0 Hz, J = 8.4 Hz, 1H); 7.47 (ddd, J = 1.8 Hz, J = 4.4 Hz, J = 8.1 Hz, 1H); 7.41 (dd, J = 8.6 Hz, J = 11.0 Hz, 1H); 4.14 (t, J = 6.6 Hz, 2H); 3.79 (s, 3H); 1.82 (sext, J = 7.1 Hz, 2H) ; 1.02 (t, J = 7.4 Hz, 3H). mp: 178-191 °C.

### Example 13: N-[(E)-(4-fluoro-3-isopropoxy-phenyl)methyleneamino]-N-methyl-1,1-dioxo-1,2-benzothiazol-3-amine

### Step A: 4-fluoro-3-isopropoxy-benzaldehyde

The compound was prepared using the same procedure as described in example 11 step A starting from 4-fluoro-3-hydroxybenzaldehyde (100.00 mg; 0.71 mmol; 1.00 eq.) and 2-bromopropane (268 µl; 2.85 mmol; 4.00 eq.) giving 4-fluoro-3-isopropoxy-benzaldehyde (84.6 mg; 65 %) as a colorless oil. 1H NMR (DMSO-d6, 300 MHz): δ 9.93 (s, 1H); 7.66 (dd, J = 1.9 Hz, J = 8.3 Hz, 1H) ; 7.55 (ddd, J = 2.0 Hz, J = 4.8 Hz, J = 8.4 Hz, 1H); 7.46 (dd, J = 8.4 Hz, J = 11.1 Hz, 1H) ; 4.77 (sept, J = 6.0 Hz, 1H) ; 1.32 (d, J = 6.1 Hz, 6H).

### Step B: N-[(E)-(4-fluoro-3-isopropoxy-phenyl)methyleneamino]-N-methyl-1,1-dioxo-1,2-benzothiazol-3-amine

The compound was prepared using the same procedure detailed in example 1 step C starting from 1-(1,1-dioxo-1,2-benzothiazol-3-yl)-1-methyl-hydrazine (example 1, step B, 85.0 mg; 0.40 mmol; 1.00 eq.) and 4-fluoro-3-isopropoxy-benzaldehyde (81.0 mg; 0.44 mmol; 1.10 eq.) giving N-[(E)-(4-fluoro-3-isopropoxy-phenyl)methyleneamino]-N-methyl-1,1-dioxo-1,2-benzothiazol-3-amine (40.6 mg; 27 %) as a white solid. 1H NMR (DMSO-d6, 400 MHz): δ 8.83 (m, 1H) ; 8.46 (s, 1H) ; 8.10 (m, 1H) ; 7.90 (m, 2H) ; 7.63 (dd, J = 1.8 Hz, J = 8.4 Hz, 1H); 7.47 (ddd, J = 1.8 Hz, J = 4.4 Hz, J = 8.4 Hz, 1H); 7.41 (dd, J = 8.6 Hz, J = 11.0 Hz, 1H); 4.74 (sept, J = 6.1 Hz, 1H); 3.79 (s, 3H); 1.38 (d, J = 5.9 Hz, 6H). mp: 222-235 °C.

### Example 14: 2-[5-[(E)-[(1,1-dioxo-1,2-benzothiazol-3-yl)-methyl-hydrazono]methyl]-2-fluoro-phenoxy]ethanol

### Step A: 4-fluoro-3-(2-hydroxyethoxy)benzaldehyde

The compound was prepared using the same procedure as described in example 11 step A starting from 4-fluoro-3-hydroxybenzaldehyde (100.0 mg; 0.71 mmol; 1.00 eq.) and 2-bromoethanol (203 µl; 2.85 mmol; 4.00 eq.) giving 4-fluoro-3-(2-hydroxyethoxy) benzaldehyde (116.4 mg; 89 %) as a colorless oil. 1H NMR (DMSO-d6, 300 MHz): δ 9.94 (s, 1H) ; 7.67 (dd, J = 1.8 Hz, J = 8.4 Hz, 1H) ; 7.58 (ddd, J = 1.7 Hz, J = 4.8 Hz, J = 8.4 Hz, 1H); 7.48 (dd, J = 8.1 Hz, J = 11.2 Hz, 1H); 4.96 (t, J = 5.4 Hz, 1H); 4.16 (t, J = 4.8 Hz, 2H) ; 3.76 (q, J = 5.1 Hz, 2H).

### Step B: 2-[5-[(E)-[(1,1-dioxo-1,2-benzothiazol-3-yl)-methyl-hydrazono]methyl]-2-fluorophenoxy]ethanol

The compound was prepared using the same procedure as described in example 1 step C starting from 1-(1,1-dioxo-1,2-benzothiazol-3-yl)-1-methyl-hydrazine (example 1, step B, 100.0 mg; 0.47 mmol; 1.00 eq.) and 4-fluoro-3-(2-hydroxyethoxy)benzaldehyde (110.0 mg; 0.60 mmol; 1.26 eq.) giving 2-[5-[(E)-[(1,1-dioxo-1,2-benzothiazol-3-yl)-methylhydrazono]methyl]-2-fluoro-phenoxy]ethanol (100.4 mg; 56 %) as an orange solid. 1H NMR (DMSO-d6, 400 MHz): δ 8.86 (m, 1H) ; 8.46 (s, 1H); 8.09 (m, 1H); 7.90 (m, 2H); 7.68 (dd, J = 1.9 Hz, J = 8.5 Hz, 1H) ; 7.47 (ddd, J = 2.0 Hz, J = 4.4 Hz, J = 8.1 Hz, 1H) ; 7.41 (dd, J = 8.6 Hz, J = 11.2 Hz, 1H); 5.00 (t, J = 5.4 Hz, 1H); 4.20 (t, J = 5.0 Hz, 2H) ; 3.81 (q, J = 4.8 Hz, 2H) ; 3.79 (s, 3H). mp: 209-222 °C.

### Example 15: N-[(E)-[4-fluoro-3-(2-methoxyethoxy)phenyl]methyleneamino]-N-methyl-1,1-dioxo-1 ,2-benzothiazol-3-amine

### Step A: 4-fluoro-3-(2-methoxyethoxy)benzaldehyde

The compound was prepared using the same procedure as described in example 11 step A starting from 4-fluoro-3-hydroxybenzaldehyde (100.0 mg; 0.71 mmol; 1.00 eq.) and 1-bromo-2-methoxy-ethane (272 µl; 2.85 mmol; 4.00 eq.) giving 4-fluoro-3-(2-methoxyethoxy)benzaldehyde (120.4 mg; 85 %) as a colorless oil. 1H NMR (DMSO-d6, 300 MHz): δ 9.94 (s, 1H) ; 7.67 (dd, J = 1.8 Hz, J = 8.4 Hz, 1H) ; 7.59 (ddd, J = 2.0 Hz, J = 4.8 Hz, J = 8.1 Hz, 1H); 7.49 (dd, J = 8.1 Hz, J = 11.1 Hz, 1H); 4.27 (m, 2H) ; 3.71 (m, 2H); 3.32 (s, 3H).

### Step B: N-[(E)-[4-fluoro-3-(2-methoxyethoxy)phenyl]methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 100.0 mg; 0.47 mmol; 1.00 eq.) and 4-fluoro-3-(2-methoxyethoxy)benzaldehyde (115.0 mg; 0.58 mmol; 1.23 eq.) giving N-[(E)-[4-fluoro-3-(2-methoxyethoxy)phenyl] methyleneamino]-N-methyl-1,1-dioxo-1,2-benzothiazol-3-amine (113.5 mg; 61 %) as a white solid. 1H NMR (DMSO-d6, 400 MHz): δ 8.86 (m, 1H); 8.45 (s, 1H); 8.10 (m, 1H); 7.90 (m, 2H); 7.66 (dd, J = 2.0 Hz, J = 8.4 Hz, 1H); 7.48 (ddd, J = 1.8 Hz, J = 4.4 Hz, J = 8.6 Hz, 1H) ; 7.42 (dd, J = 8.6 Hz, J = 11.0 Hz, 1H) ; 4.30 (m, 2H) ; 3.79 (s, 3H); 3.75 (m, 2H) ; 3.32 (s, 3H). mp: 187-197 °C.

### Example 16: N-[(E)-[3-fluoro-5-methoxy-4-(2-methoxyethoxy)-phenyl]-methyleneamino]-N-methyl-1, 1-dioxo-1 ,2-benzothiazol-3-amine

### Step A: 3-fluoro-5-methoxy-4-(2-methoxyethoxy)benzaldehyde

The compound was prepared using the same procedure as described in example 11 step A starting from 3-fluoro-4-hydroxy-5-methoxybenzaldehyde (100.0 mg; 0.59 mmol; 1.00 eq.) and 1-bromo-2-methoxy-ethane (224 µl; 2.35 mmol; 4.00 eq.) giving 3-fluoro-5-methoxy-4-(2-methoxyethoxy)benzaldehyde (112.3 mg; 84 %) as a colorless oil. 1H NMR (DMSO-d6, 300 MHz): δ 9.88 (d, J = 1.7 Hz, 1H); 7.44 (m, 2H); 4.24 (m, 2H); 3.91 (s, 3H); 3.60 (m, 2H) ; 3.27 (s, 3H).

### Step B: N-[(E)-[3-fluoro-5-methoxy-4-(2-methoxyethoxy)phenyl]methyleneamino]-N-methyl-1, 1-dioxo-1 ,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 100.0 mg; 0.47 mmol; 1.00 eq.) and 3-fluoro-5-methoxy-4-(2-methoxyethoxy) benzaldehyde (110.0 mg; 0.48 mmol; 1.02 eq.) giving N-[(E)-[3-fluoro-5-methoxy-4-(2-methoxyethoxy)phenyl]methyleneamino]-N-methyl-1, **1-dioxo-1** ,2-benzothiazol-3-amine (114.4 mg; 57 %) as a white solid. 1H NMR (DMSO-d6, 400 MHz): δ 8.83 (m, 1H) ; 8.42 (s, 1H) ; 8.09 (m, 1H) ; 7.92 (m, 2H) ; 7.38 (t, J = 1.5 Hz, 1H) ; 7.33 (dd, J = 1.9 Hz, J = 10.9 Hz, 1H); 4.19 (m, 2H); 3.94 (s, 3H); 3.78 (s, 3H); 3.62 (m, 2H); 3.29 (s, 3H). mp: 135-144 °C.

### Example 17: 5-[(E)-[(1,1-dioxo-1,2-benzothiazol-3-yl)-methyl-hydrazono]methyl]-2-fluoro-benzamide

### Step A: 2-fluoro-5-formyl-benzamide

2-fluoro-5-formylbenzonitrile (1.00 g; 6.71 mmol; 1.00 eq.) in sulfuric acid (10.00 ml) was heated 1h at 100°C. After being cooling to rt, the mixture was diluted with ice-cold water, filtered and the solid washed with water before being dried under vacuum at 50°C. The filtrate was then extracted with AcOEt and the organic layer was washed with NaHC0₃ sat. and brine, dried over MgSO₄ and concentrated to dryness to give a second solid. The two solids were combined to give 2-fluoro-5-formyl-benzamide (1.01 g; 90 %) as a beige solid. 1H NMR (DMSO-d6, 300 MHz): δ 10.02 (s, 1H); 8.21 (dd, J = 2.2 Hz, J = 7.0 Hz, 1H) ; 8.07 (ddd, J = 2.2 Hz, J = 5.0 Hz, J = 8.5 Hz, 1H) ; 7.91 (br s, 1H) ; 7.80 (br s, 1H) ; 7.53 (dd, J = 8.5 Hz, J = 10.2 Hz, 1H).

### Step B: 5-[(E)-[(1,1-dioxo-1,2-benzothiazol-3-yl)-methyl-hydrazono]methyl]-2-fluorobenzamide

The compound was prepared using the same procedure as described in example 1 step C starting from 1-(1,1-dioxo-1,2-benzothiazol-3-yl)-1-methyl-hydrazine (example 1 , step B, 1.05 g; 4.97 mmol; 1.00 eq.) and 3-fluoro-5-methoxy-4-(2-methoxyethoxy)benzaldehyde (1.00 g; 5.96 mmol; 1.20 eq.) giving 5-[(E)-[(1,1-dioxo-1,2-benzothiazol-3-yl)-methylhydrazono]methyl]-2-fluoro-benzamide (1.56 g; 87 %) as a white solid. 1H NMR (DMSO-d6, 400 MHz): δ 8.84 (m, 1H); 8.55 (s, 1H); 8.16 (dd, J = 2.2 Hz, J = 7.0 Hz, 1H); 8.11 (m, 1H); 8.06 (ddd, J = 2.3 Hz, J = 4.8 Hz, J = 8.7 Hz, 1H); 7.89 (m, 3H); 7.78 (br s, 1H); 7.49 (dd, J = 8.6 Hz, J = 10.1 Hz, 1H); 3.80 (s, 3H).

### Example 18: 2-(dimethylamino)-5-[(E)-[(1,1-dioxo-1,2-benzothiazol-3-yl)-methyl-hydrazono]methyl]benzamide

### Step A: 2-(dimethylamino)-5-formyl-benzamide

The compound was prepared using the same procedure as described in example 17 step A starting from 2-(dimethylamino)-5-formylbenzonitrile (200.0 mg; 1.15 mmol; 1.00 eq.) giving 2-(dimethylamino)-5-formyl-benzamide (106.6 mg; 48 %) as a beige solid. 1H NMR (DMSO-d6, 300 MHz): δ 9.73 (s, 1H) ; 7.88 (br s, 1H) ; 7.77 (m, 1H) ; 7.73 (m, 1H) ; 7.43 (br s, 1H) ; 6.96 (d, J = 8.8 Hz, 1H) ; 2.97 (s, 6H).

### Step B: 2-(dimethylamino)-5-[(E)-[(1,1-dioxo-1,2-benzothiazol-3-yl)-methyl-hydrazono] methyl]benzamide

The compound was prepared using the same procedure as described in example 1 step C starting from 1-(1,1-dioxo-1,2-benzothiazol-3-yl)-1-methyl-hydrazine (example 1, step B, 97.1 mg; 0.46 mmol; 1.00 eq.) and 2-(dimethylamino)-5-formyl-benzamide (106.0 mg; 0.55 mmol; 1.20 eq.) giving 2-(dimethylamino)-5-[(E)-[(1,1-dioxo-1,2-benzothiazol-3-yl)-methyl-hydrazono]methyl]benzamide (154.0 mg; 87 %) as an orange solid. 1H NMR (DMSO-d6, 400 MHz): δ 8.91 (m, 1H) ; 8.42 (s, 1H) ; 8.07 (m, 1H) ; 8.01(br s, 1H) ; 7.88 (m, 3H); 7.82 (dd, J = 2.2 Hz, J = 8.6 Hz, 1H) ; 7.47 (br s, 1H) ; 7.09 (d, J = 8.8 Hz, 1H) ; 3.78 (s, 3H); 2.91 (s, 6H).

### Example 19: [4-[(E)-[(1,1-dioxo-1,2-benzothiazol-3-yl)-methyl-hydrazono]methyl]-2-methoxy-phenyl]methanol

### Step A: methyl 2-methoxy-4-methyl-benzoate

Potassium carbonate (817.5 mg; 5.92 mmol; 3.00 eq.) was added to a solution of 4-methylsalicylic acid (300.0 mg; 1.97 mmol; 1.00 eq.) in DMF (6.00 ml) and the mixture was stirred 10 min at rt. Then, methyl iodide (368 µl; 5.92 mmol; 3.00 eq.) was added and the mixture was stirred 19h at rt. The mixture was then extracted with EtOAc and the organic layer washed with brine, dried over MgSO₄, filtered and concentrated under vacuum to give methyl 2-methoxy-4-methyl-benzoate (330.3 mg; 93 %) as an orange oil. 1H NMR (DMSO-d6, 300 MHz): δ 7.57 (d, J = 7.9 Hz, 1H) ; 6.97 (s, 1H); 6.83 (m, 1H); 3.80 (s, 3H); 3.75 (s, 3H); 2.35 (s, 3H).

### Step B: methyl 4-(dibromomethyl)-2-methoxy-benzoate

N-Bromosuccinimide (738.3 mg; 4.15 mmol; 2.30 eq.) and benzoyl peroxide (21.8 mg; 0.09 mmol; 0.05 eq.) were added to a solution of methyl 2-methoxy-4-methyl-benzoate (325.0 mg; 1.80 mmol; 1.00 eq.) in carbon tetrachloride (18.20 ml). The reaction was stirred 20h30 at 80°C, then cooled to rt, filtered and solid washed with DCM. The filtrate was diluted with water and extracted with DCM (3x). Combined organic layers were washed with saturated sodium thiosulfate, water, and brine, dried over MgSO₄, filtered and concentrated under vacuum to give methyl 4-(dibromomethyl)-2-methoxy-benzoate (604.7 mg; 99 %) as an orange resin, used directly in next step without purification. 1H NMR (DMSO-d6, 300 MHz): δ 7.70 (d, J = 8.1 Hz, 1H); 7.38 (s, 1H); 7.35 (d, J = 1.7 Hz, 1H) ; 7.29 (dd, J = 1.7 Hz, J = 8.0 Hz, 1H) ; 3.86 (s, 3H); 3.80 (s, 3H).

### Step C: methyl 4-formyl-2-methoxy-benzoate

Silver nitrate (607.1 mg; 3.57 mmol; 2.00 eq.) was added to a solution of methyl 4-(dibromomethyl)-2-methoxy-benzoate (604.0 mg; 1.79 mmol; 1.00 eq.) in acetone (4.00 ml) and water (1.00 ml). The mixture was then stirred 2h30 at rt under dark. It was filtered and solids were washed with EtOAc. The filtrate was washed twice with saturated NaHCO₃, water and brine, dried over MgSO₄ and concentrated. The residue was purified by chromatography (column SiO₂ 25g, flow rate 25ml/min, gradient from 100% cyclohexane to 80/20 cyclohexane / EtOAc) to give methyl 4-formyl-2-methoxy-benzoate (200.1 mg; 52 %) as a colorless oil. 1H NMR (DMSO-d6, 300 MHz): δ 10.05 (s, 1H) ; 7.81 (d, J = 7.6 Hz, 1H); 7.63 (d, J = 1.2 Hz, 1H); 7.57 (dd, J = 1.4 Hz, J = 7.7 Hz, 1H); 3.91 (s, 3H); 3.83 (s, 3H).

### Step D: methyl 4-(1,3-dioxolan-2-yl)-2-methoxy-benzoate

Triethylorthoformate (165 µl; 0.99 mmol; 1.10 eq.) and phenyltrimethylammonium tribromide (16.9 mg; 0.05 mmol; 0.05 eq.) were added to a suspension of methyl 4-formyl-2-methoxy-benzoate (175.0 mg; 0.90 mmol; 1.00 eq.) in ethylene glycol (201 µl; 3.60 mmol; 4.00 eq.). The mixture was then stirred 4h at rt. Saturated NaHC0₃ and EtOAc were added and the mixture was extracted with EtOAc. Combined organic layers were washed with water and brine, dried over MgSO₄, filtered and concentrated under vacuum to give methyl 4-(1,3-dioxolan-2-yl)-2-methoxy-benzoate (202.0 mg; 94 %) as a pale yellow oil, used directly in the next step without purification

### Step E: [4-(1,3-dioxolan-2-yl)-2-methoxy-phenyl]methanol

LiAlH₄ (63.7 mg; 1.68 mmol; 2.00 eq.) was added by portion to a solution of methyl 4-(1,3-dioxolan-2-yl)-2-methoxy-benzoate (200.0 mg; 0.84 mmol; 1.00 eq.) in THF (3.00 ml) at 0°C. The mixture was stirred 1h at 0°C. Glauber's salt (500.0 mg) was added at 0°C and the mixture was stirred at rt overnight. It was filtered and solids were washed successively with THF and EtOAc. The filtrate was concentrated and the residue was purified by chromatography (column SiO₂ 10g, flow rate 15ml/min, gradient from 100% cyclohexane to 60/40 cyclohexane / EtOAc) to give [4-(1,3-dioxolan-2-yl)-2-methoxy-phenyl]methanol (79.3 mg; 45 %) as a colorless resin. 1H NMR (DMSO-d6, 300 MHz): δ 7.37 (d, J = 7.6 Hz, 1H); 7.01 (dd, J = 1.4 Hz, J = 7.7 Hz, 1H); 6.96 (d, J = 1.5 Hz, 1H); 5.70 (s, 1H); 5.01 (t, J = 5.6 Hz, 1H); 4.48 (d, J = 5.4 Hz, 2H) ; 4.05 (m, 2H) ; 3.95 (m, 2H) ; 3.78 (s, 3H).

### Step F: 4-(hydroxymethyl)-3-methoxy-benzaldehyde

A solution of [4-(1,3-dioxolan-2-yl)-2-methoxy-phenyl]methanol (75.0 mg; 0.36 mmol; 1.00 eq.) in a mixture of MeOH (4.00 ml) and HCl (1.00 ml) was stirred 40 min at rt. MeOH was evaporated and the residue extracted with EtOAc. The organic layer was washed with brine, dried over MgSO₄, filtered and concentrated under vacuum to give 4-(hydroxymethyl)-3-methoxy-benzaldehyde (49.3 mg; 83 %) as a white solid. 1H NMR (DMSO-d6, 300 MHz): δ 9.97 (s, 1H); 7.63 (m, 1H); 7.56 (dd, J = 1.3 Hz, J = 7.6 Hz, 1H); 7.40 (d, J = 1.3 Hz, 1H); 5.27 (t, J = 5.6 Hz, 1H); 4.56 (d, J = 5.3 Hz, 2H); 3.86 (s, 3H).

### Step G: [4-[(E)-[(1,1-dioxo-1,2-benzothiazol-3-yl)-methyl-hydrazono]methyl]-2-methoxyphenyl]methanol

The compound was prepared using the same procedure as described in example 1 step C starting from 1-(1,1-dioxo-1,2-benzothiazol-3-yl)-1-methyl-hydrazine (example 1, step B, 47.7 mg; 0.23 mmol; 1.00 eq.) and 4-(hydroxymethyl)-3-methoxy-benzaldehyde (45.0 mg; 0.27 mmol; 1.20 eq.) giving [4-[(E)-[(1,1-dioxo-1,2-benzothiazol-3-yl)-methyl-hydrazono]methyl]-2-methoxy-phenyl]methanol (61.6 mg; 76 %) as a yellow solid. 1H NMR (DMSO-d6, 400 MHz): δ 8.91 (d, J = 7.5 Hz, 1H); 8.47 (s, 1H); 8.09 (m, 1H); 7.92 (m, 2H); 7.56 (d, J = 7.5 Hz, 1H); 7.48 (dd, J = 1.3 Hz, J = 7.7 Hz, 1H); 7.43 (t, J = 1.1 Hz, 1H); 5.18 (t, J = 5.6 Hz, 1H); 4.56 (d, J = 5.7 Hz, 2H); 3.90 (s, 3H); 3.80 (s, 3H).

### Example 20: 2-[4-[(E)-[(1,1-dioxo-1,2-benzothiazol-3-yl)-methyl-hydrazono]methyl]-2-methoxy-phenyl]-N-methyl-acetamide

### Step A: 2-(4-bromo-2-methoxy-phenyl)-N-methyl-acetamide

2-(4-bromo-2-methoxy-phenyl)acetic acid (200.0 mg; 0.78 mmol; 1.0 eq.) was dissolved in NMP (3.00 ml) then MeNH₂ solution (2M solution in MeOH, 3.88 ml; 7.75 mmol; 9.93 eq.) and DIPEA (276 µL; 1.55 mmol; 2.00 eq.) were added followed by the addition of propylphosphonic anhydride (50% solution in THF, 1.19 ml; 1.94 mmol; 2.49 eq.) by portion. The mixture was stirred 5h at rt, then poured into water and extracted with EtOAc (3x). Combined organic layers were washed with brine, dried over MgSO₄ and concentrated. The residue was triturated in water and filtered to give 2-(4-bromo-2-methoxy-phenyl)-N-methyl-acetamide (99.0 mg; 50 %) as a white solid, used directly in the next step without purification. LC/MS (Method B): Rt = 2.24 min ; MS : m/z = 258-260 [M+H]⁺

### Step B: 2-(4-formyl-2-methoxy-phenyl)-N-methyl-acetamide

To a solution of 2-(4-bromo-2-methoxy-phenyl)-N-methyl-acetamide (95.0 mg; 0.37 mmol; 1.00 eq.) in dry THF (1.00 ml), was added methylmagnesium bromide (3 M solution in diethyl ether, 132 µL; 1.10 mmol; 2.97 eq.) at -78 °C. After 1h, anhydrous THF (4.00 ml) was added at a rate that maintained the internal reaction temperature below a threshold of -50 °C. Reaction was then cooled to -78 °C and *tert*-butyl lithium (1.7 M solution in pentane, 864 µL; 1.47 mmol; 3.97 eq.) was added dropwise. After 30 min, DMF (143 µL; 1.84 mmol; 4.97 eq.) was added and the reaction was stirred 12h at rt. It was quenched with water and after removal of volatiles, the aqueous residue was extracted with EtOAc (3x). Combined organic layers were washed with brine, dried over MgSO₄ and concentrated under vacuum to give 2-(4-formyl-2-methoxy-phenyl)-N-methyl-acetamide (76.0 mg; quant.) as a yellow solid, used directly in the next step without purification. LC/MS (Method B): Rt = 1.60 min ; MS : m/z = 208 [M+H]⁺

### Step C: 2-[4-[(E)-[(1,1-dioxo-1,2-benzothiazol-3-yl)-methyl-hydrazono]methyl]-2-methoxy-phenyl]-N-methyl-acetamide

The compound was prepared using the same procedure as described in example 1 step C starting from 1-(1,1-dioxo-1,2-benzothiazol-3-yl)-1-methyl-hydrazine (example 1, step B, 41.0 mg; 0.19 mmol; 1.00 eq.) and 2-(4-formyl-2-methoxy-phenyl)-N-methyl-acetamide (40.0 mg; 0.19 mmol; 1.00 eq.) giving 2-[4-[(E)-[(1,1-dioxo-1,2-benzothiazol-3-yl)-methyl-hydrazono]methyl]-2-methoxy-phenyl]-N-methyl-acetamide (28.0 mg; 36 %) as a pale yellow solid. 1H NMR (DMSO-d6, 400 MHz): δ 8.90 (d, J = 7.5 Hz, 1H); 8.46 (s, 1H); 8.09 (d, J = 7.0 Hz, 1H) ; 7.87 (m, 3H); 7.39 (m, 3H); 3.88 (s, 3H); 3.80 (s, 3H); 3.45 (s, 2H) ; 2.59 (d, J = 4.6 Hz, 3H). mp: 228-231°C.

### Example 21: N-[(E)-[4-(2-methoxyethoxy)phenyl]methyleneamino]-N-methyl-1,1-dioxo-1,2-benzothiazol-3-amine

### Step A: N-[(E)-[4-(2-methoxyethoxy)phenyl]methyleneamino]methanamine

Sodium acetate (22.8 mg; 0.28 mmol; 1.00 eq.) and methyl hydrazine (15 µL; 0.28 mmol; 1.00 eq.) were added to a solution of 4-(2-methoxyethoxy)benzaldehyde (50.0 mg; 0.28 mmol; 1.00 eq.) in ethanol (1.00 ml). The mixture was heated 5 minutes at 80°C under microwave irradiation. The mixture was then concentrated under vacuum to give N-[(E)-[4-(2-methoxyethoxy)phenyl]methyleneamino]methanamine (57.0 mg; 99 %) as a white solid, used directly in the next step without purification. LC/MS (Method A): Rt = 1.13min ; MS : m/z = 209 [M+H]⁺

### Step B: N-[(E)-[4-(2-methoxyethoxy)phenyl]methyleneamino]-N-methyl-1,1-dioxo-1,2-benzothiazol-3-amine

N-[(E)-[4-(2-methoxyethoxy)phenyl]methyleneamino]methanamine (57.0 mg; 0.27 mmol; 1.00 eq.) was suspended into THF (3.00 ml). 3-chloro-1,2-benzothiazole 1,1-dioxide (example 1, step A, 55.2 mg; 0.27 mmol; 1.00 eq.) was added and the mixture was stirred 2h under reflux. The mixture was then cooled to rt and the solid was filtered. The solid was washed with THF and water, and dried under vacuum to give N-[(E)-[4-(2-methoxyethoxy)phenyt]methyleneamino]-N-methyl-1,1-dioxo-1,2-benzothiazo)-3-amine (68.0 mg; 67 %) as a white solid. 1H NMR (DMSO-d6, 400 MHz): δ 8.89 (m, 1H); 8.43 (s, 1H); 8.07 (m, 1H); 7.92 (m, 2H) ; 7.83 (m, 2H) ; 7.13 (m, 2H) ; 4.19 (m, 2H) ; 3.79 (s, 3H); 3.70 (m, 2H); 3.33 (s, 3H). mp: 206°C.

### Example 22: N-[(E)-[3-(2-methoxyethoxy)phenyl]methyleneamino]-N-methyl-1,1-dioxo-1,2-benzothiazol-3-amine

### Step A: N-[(E)-[3-(2-methoxyethoxy)phenyl]methyleneamino]methanamine

The compound was prepared using the same procedure as described in example 21 step A starting from 3-(2-methoxyethoxy)benzaldehyde (50.0 mg; 0.28 mmol; 1.00 eq.) giving N-[(E)-[3-(2-methoxyethoxy)phenyl]methyleneamino]methanamine (57.0 mg; 99 %) as a white solid, used directly in next step without purification. LC/MS (Method A): Rt = 1.40min ; MS : m/z = 209 [M+H]⁺

### Step B: N-[(E)-[3-(2-methoxyethoxy)phenyl]methyleneamino]-N-methyl-1,1-dioxo-1,2-benzothiazol-3-a mine

The compound was prepared using the same procedure as described in example 21 step B starting from 3-chloro-1,2-benzothiazole 1,1-dioxide (example 1, step A, 55.2 mg; 0.27 mmol; 1.00 eq.) and N-[(E)-[3-(2-methoxyethoxy)phenyl]methyleneamino]methanamine (57.0 mg; 0.27 mmol; 1.00 eq.) giving N-[(E)-[3-(2-methoxyethoxy)phenyl]methyl eneamino]-N-methyl-1,1-dioxo-1,2-benzothiazol-3-amine (56.0 mg; 55 %) as a white solid. 1H NMR (DMSO-d6, 400 MHz): δ 8.87 (m, 1H); 8.44 (s, 1H); 8.09 (m, 1H); 7.92 (m, 2H) ; 7.48 (m, 2H) ; 7.41 (d, J = 2.2 Hz, 1H); 7.13 (m, 1H); 4.20 (m, 2H) ; 3.80 (s, 3H); 3.72 (m, 2H) ; 3.33 (s, 3H). mp: 170°C.

### Example 23: 5-[(E)-[(1,1-dioxo-1,2-benzothiazol-3-yl)-isobutyl-hydrazono]methyl]-3-methyl-1 H-benzimidazol-2-one

### Step A: methyl 3-(methylamino)-4-nitro-benzoate

Methylamine (2.00 mol/l in THF, 99.43 ml; 198.86 mmol; 2.00 eq.) was then added dropwise to a solution of methyl 3-fluoro-4-nitrobenzoate (19.80 g; 99.43 mmol; 1.00 eq.) in THF (100.00 ml). The mixture was stirred 2h at rt. Saturated NaHC0₃ solution was added to the mixture and THF was concentrated. The residue was diluted with EtOAc. Organic layer was recovered, washed with saturated NaHC0₃ solution, brine, dried over Na₂SO₄, filtered and concentrated under vacuum to give methyl 3-(methylamino)-4-nitrobenzoate (20.80 g; quant.) as an orange powder. 1H NMR (DMSO-d6, 300 MHz): δ 8.22 (d, J = 8.8 Hz, 1H); 7.56 (d, J = 1.7 Hz, 1H); 7.25 (dd, J = 1.6 Hz, J = 8.7 Hz, 1H); 3.95 (s, 3H); 3.09 (d, J = 5.1 Hz, 3H).

### Step B: methyl 4-amino-3-(methylamino)benzoate

Methyl 3-(methylamino)-4-nitro-benzoate (20.80 g; 98.96 mmol; 1.00 eq.) was dissolved into EtOAc (350.00 ml) and THF (350.00 ml). Then, Pd/C 10% (4.21 g; 39.58 mmol; 0.40 eq.) was added and mixture was stirred 13h at rt under hydrogen atmosphere (1atm). The mixture was filtered, and Pd residues was washed by EtOAc and THF, concentrated under vacuum to give methyl 4-amino-3-(methylamino)benzoate (17.80 g; quant.) as a brown powder, used directly in the next step without purification. 1H NMR (DMSO-d6, 300 MHz): δ 7.16 (dd, J = 1.9 Hz, J = 8.0 Hz, 1H); 6.92 (d, J = 2.0 Hz, 1H); 6.53 (d, J = 8.1 Hz, 1H); 5.34 (s, 2H); 4.79 (q, J = 4.8 Hz, 1H); 3.73 (s, 3H), 2.74 (d, J = 5.0 Hz, 3H).

### Step C: methyl 3-methyl-2-oxo-1H-benzimidazole-5-carboxylate

1,1'-Carbonyldiimidazole (19.22 g; 0.12 mol; 1.20 eq.) was added to a solution of methyl 4-amino-3-(methylamino)benzoate (17.80 g; 0.10 mol; 1.00 eq.) in THF (750.00 ml) and the mixture was stirred 8h under reflux. The mixture was concentrated to dryness. The residue was taken up into HCl 1N, filtered and the solid washed by water until pH of filtrate was neutral. The solid was dried under vacuum, washed again with diethyl ether and dried again under vacuum to give methyl 3-methyl-2-oxo-1H-benzimidazole-5-carboxylate (15.60 g; 77 %) as a white solid. 1H NMR (DMSO-d6, 300 MHz): δ 11.27 (br s, 1H); 7.69 (dd, J = 1.6 Hz, J = 8.2 Hz, 1H); 7.63 (d, J = 1.5 Hz, 1H); 7.07 (d, J = 8.3 Hz, 1H); 3.84 (s, 3H); 3.33 (s, 3H).

### Step D: 5-(hydroxymethyl)-3-methyl-1H-benzimidazol-2-one

Methyl 3-methyl-2-oxo-1H-benzimidazole-5-carboxylate (16.00 g; 0.08 mol; 1.00 eq.) was added by portions to a suspension of LiAlH₄ (4.42 g; 116.39 mmol; 1.50 eq.) in THF (300.00 ml). The mixture was stirred 15h at rt. The mixture was quenched slowly by adding portion-wise Glauber's salt (20.00 g). Then, the mixture was diluted with water and extracted with EtOAc (3x). Combined organics layers were washed with brine, dried over Na₂SO₄, filtered and concentrated under vacuum to give 5-(hydroxymethyl)-3-methyl-1H-benzimidazol-2-one (3.00 g; 22 %) as a grey powder. 1H NMR (DMSO-d6, 300 MHz): δ 10.75 (br s, 1H); 7.03 (m, 1H); 6.92 (m, 2H); 5.10 (br t, J = 5.0 Hz, 1H); 4.49 (d, J = 3.6 Hz, 2H) ; 3.26 (s, 3H).

### Step E: 3-methyl-2-oxo-1H-benzimidazole-5-carbaldehyde

5-(hydroxymethyl)-3-methyl-1H-benzimidazol-2-one (3.00 g; 16.84 mmol; 1.00 eq.) was dissolved in DCM (75.00 ml) and dess-martin periodinane (15% w/w in DCM, 70.00 ml; 33.67 mmol; 2.00 eq.) was added dropwise. The mixture was stirred 16h at rt. Saturated NaHC0₃ solution was added dropwise and the aqueous layer was extracted with DCM. Combined organic layers were washed with brine, dried over Na₂SO₄, filtered and concentrated. The residue was purified by chromatography (column SiO₂ 340g, flowrate 100ml/min, gradient from 100% DCM to 90/10 DCM / MeOH) to give 3-methyl-2-oxo-1H-benzimidazole-5-carbaldehyde (1.88 g; 63 %) as a pink solid. 1H NMR (DMSO-d6, 300 MHz): δ 11.36 (br s, 1H) ; 9.91 (s, 1H) ; 7.63 (dd, J = 1.5 Hz, J = 7.9 Hz, 1H) ; 7.59 (d, J = 1.3 Hz, 1H); 7.16 (d, J = 7.9 Hz, 1H); 3.34 (s, 3H).

### Step F: tert-butyl N-[(E)-(3-methyl-2-oxo-1H-benzimidazol-5-yl)methyleneamino] carbamate

*tert*-Butyl carbazate (7.17 g; 54.25 mmol; 1.00 eq.) was added to a suspension of 3-methyl-2-oxo-1H-benzimidazole-5-carbaldehyde (10.18 g; 54.89 mmol; 1.02 eq.) in EtOH (179.00 ml). The mixture was stirred 44h at rt and then concentrated under vacuum to give tert-butyl N-[(E)-(3-methyl-2-oxo-1H-benzimidazol-5-yl)methyleneamino]carbamate (16.06 g; quant.) as an orange solid, used directly in the next step without purification. 1H NMR (DMSO-d6, 300 MHz): δ 11.01 (s, 1H); 10.78 (br s, 1H); 8.00 (s, 1H); 7.32 (s, 1H); 7.21 (dd, J = 1.2 Hz, J = 8.0 Hz, 1H); 6.99 (d, J = 8.1 Hz, 1H); 3.30 (s, 3H); 1.47 (s, 9H). Step G: tert-butyl 5-[(E)-(tert-butoxycarbonylhydrazono)methyl]-3-methyl-2-oxobenzimidazole-1-carboxylate

Cesium carbonate (27.00 g; 82.87 mmol; 1.50 eq.) followed by di-*tert*-butyl dicarbonate (17.00 ml; 79.45 mmol; 1.44 eq.) were added to a solution of tert-butyl N-[(E)-(3-methyl-2-oxo-1H-benzimidazol-5-yl)methyleneamino]carbamate (16.06 g; 55.31 mmol; 1.00 eq.) in DMF (240.00 ml). The reaction was then stirred 2h at rt. Water was added and the resulting aqueous layer was extracted twice with EtOAc. Combined organic layers were dried over MgSO₄, filtered and concentrated under vacuum to give tert-butyl 5-[(E)-(tert-butoxycarbonylhydrazono)methyl]-3-methyl-2-oxo-benzimidazole-1-carboxylate (41.47 g; quant.) as an orange oil. 1H NMR (DMSO-d6, 300 MHz): δ 10.92 (br s, 1H); 8.03 (s, 1H); 7.71 (d, J = 8.3 Hz, 1H) ; 7.36 (m, 2H) ; 3.33 (s, 3H); 1.60 (s, 9H) ; 1.48 (s, 9H).

### Step H: tert-butyl 5-[(E)-[tert-butoxycarbonyl(isobutyl)hydrazono]methyl]-3-methyl-2-oxo-benzimidazole-1 -carboxylate

Cesium carbonate (54.08 g; 165.97 mmol; 3.00 eq.) followed by 1-bromo-2-methylpropane (9.02 ml; 82.98 mmol; 1.50 eq.) were added to a solution of tert-butyl 5-[(E)-(tert-butoxycarbonylhydrazono)methyl]-3-methyl-2-oxo-benzimidazole-1-carboxylate (21.60 g; 55.32 mmol; 1.00 eq.) in DMF (300.00 ml). The reaction was then stirred 3h at 80°C and cooled to rt. Water was added and the aqueous layer was extracted once with EtOAc. The organic layer was dried over MgSO₄, filtered and concentrated. The residue was purified by chromatography (column SiO₂ 750g, flow rate 200ml/min, gradient from 100% cyclohexane to 60/40 cyclohexane / EtOAc) to give tert-butyl 5-[(E)-[tert-butoxycarbonyl(isobutyl)hydrazono]methyl]-3-methyl-2-oxo-benzimidazole-1-carboxylate (9.50 g; 39 %) as a white solid. 1H NMR (DMSO-d6, 300 MHz): δ 8.01 (s, 1H); 7.72 (m, 1H); 7.47 (m, 2H) ; 3.70 (d, J = 7.4 Hz, 2H); 3.31 (s, 3H); 2.00 (m, 1H) ; 1.60 (s, 9H); 1.50 (s, 9H) ; 0.89 (d, J = 6.8 Hz, 6H).

### Step I: 5-[(E)-(isobutylhydrazono)methyl]-3-methyl-1H-benzimidazol-2-one

A solution of tert-butyl 5-[(E)-[tert-butoxycarbonyl(isobutyl)hydrazono]methyl]-3-methyl-2-oxo-benzimidazole-1-carboxylate (9.50 g; 21.27 mmol; 1.00 eq.) in HCl 4.0M solution in 1,4-dioxane (114.00 ml) was stirred 15 min at rt. The reaction was concentrated under vacuum to give 5-[(E)-(isobutylhydrazono)methyl]-3-methyl-1H-benzimidazol-2-one (5.24 g; quant.) as an orange paste, used directly in the next step without purification. 1H NMR (DMSO-d6, 300 MHz): δ 11.25 (s, 1H); 8.62 (s, 1H); 7.63 (m, 1H); 7.46 (s, 1H); 7.39 (dd, J = 1.5 Hz, J = 8.1 Hz, 1H); 7.08 (d, J = 8.1 Hz, 1H); 3.32 (s, 3H); 3.09 (d, J = 6.9 Hz, 2H) ; 2.10 (sept, J = 6.8 Hz, J = 13.5 Hz, 1H); 1.01 (d, J = 6.8 Hz, 6H).

### Step J: 5-[(E)-[(1,1-dioxo-1,2-benzothiazol-3-yl)-isobutyl-hydrazono]methyl]-3-methyl-1H-benzimidazol-2-one

The compound was prepared using the same procedure as described in example 21 step B starting from 3-chloro-1,2-benzothiazole 1,1-dioxide (example 1, step A, 4.29 g; 21.27 mmol; 1.00 eq.) and 5-[(E)-(isobutylhydrazono)methyl]-3-methyl-1H-benzimidazol-2-one (5.24 g; 21.27 mmol; 1.00 eq.) giving 5-[(E)-[(1,1-dioxo-1,2-benzothiazol-3-yl)-isobutyl-hydrazono]methyl]-3-methyl-1H-benzimidazol-2-one (6.45 g; 72 %) as a yellow solid. 1H NMR (DMSO-d6, 400 MHz): δ 11.21 (s, 1H); 8.88 (d, J = 7.5 Hz, 1H); 8.58 (s, 1H); 8.08 (m, 1H); 7.91 (m, 2H) ; 7.58 (dd, J = 1.5 Hz, J = 7.9 Hz, 1H); 7.57 (s, 1H); 7.16 (d, J = 7.9 Hz, 1H) ; 4.29 (d, J = 7.7 Hz, 2H) ; 3.37 (s, 7H) ; 2.27 (sept, J = 7.3 Hz, 1H) ; 0.99 (d, J = 6.6 Hz, 6H). mp: 263-287°C.

### Example 24: 5-[(E)-[(1,1-dioxo-1,2-benzothiazol-3-yl)-methyl-hydrazono]methyl]-3-ethyl-1H-benzimidazol-2-one

### Step A: methyl 3-ethyl-2-oxo-1H-benzimidazole-5-carboxylate

Sulfuric acid (24 µl; 0.44 mmol; 0.30 eq.) was added to a suspension of 3-ethyl-2-xo-2,3-dihydro-1H-1,3-benzodiazole-5-carboxylic acid (300.0 mg; 1.45 mmol; 1.00 eq.) in MeOH (3.00 ml). The mixture was stirred 24h under reflux and cooled to rt. The precipitate formed was filtered, washed with water and dried under vacuum to give methyl 3-ethyl-2-oxo-1H-benzimidazole-5-carboxylate (231.6 mg; 72 %) as a white solid. 1H NMR (DMSO-d6, 300 MHz): δ 11.27 (s, 1H) ; 7.69 (dd, J = 1.6 Hz, J = 8.1 Hz, 1H); 7.66 (m, 1H); 7.08 (d, J = 8.1 Hz, 1H) ; 3.88 (q, J = 7.1 Hz, 2H) ; 3.84 (s, 3H); 1.20 (t, J = 7.1 Hz, 3H).

### Step B: 3-ethyl-5-(hydroxymethyl)-1H-benzimidazol-2-one

The compound was prepared using the same procedure as described in example 23 step D starting from methyl 3-ethyl-2-oxo-1H-benzimidazole-5-carboxylate (300.0 mg; 1.36 mmol; 1.00 eq.) giving 3-ethyl-5-(hydroxymethyl)-1H-benzimidazol-2-one (234.0 mg; 89 %) as a beige solid. 1H NMR (DMSO-d6, 300 MHz): δ 10.72 (s, 1H); 7.07 (s, 1H); 6.92 (m, 2H); 5.09 (t, J = 5.7 Hz, 1H); 4.49 (d, J = 5.7 Hz, 2H); 3.80 (q, J = 7.2 Hz, 2H); 1.19 (t, J = 7.2 Hz, 3H).

### Step C: 3-ethyl-2-oxo-1H-benzimidazole-5-carbaldehyde

The compound was prepared using the same procedure as described in example 23 step E starting from 3-ethyl-5-(hydroxymethyl)-1H-benzimidazol-2-one (234.0 mg; 1.22 mmol; 1.00 eq.) giving 3-ethyl-2-oxo-1H-benzimidazole-5-carbaldehyde (170.0 mg; 73 %) as a white solid. 1H NMR (DMSO-d6, 300 MHz): δ 11.39 (s, 1H) ; 9.91 (s, 1H) ; 7.64 (s, 1H) ; 7.62 (dd, J =1.5 Hz, J = 7.8 Hz, 1H); 7.17 (d, J = 7.8 Hz, 1H); 3.90 (q, J = 7.1 Hz, 2H); 1.22 (t, J = 7.1 Hz, 3H).

### Step D: 5-[(E)-[(1,1-dioxo-1,2-benzothiazol-3-yl)-methyl-hydrazono]methyi]-3-ethyl-1H-benzimidazol-2-one

The compound was prepared using the same procedure as described in example 1 step C starting from 1-(1,1-dioxo-1,2-benzothiazol-3-yl)-1-methyl-hydrazine (example 1, step B, 50.0 mg; 0.26 mmol; 1.00 eq.) and 3-ethyl-2-oxo-1H-benzimidazole-5-carbaldehyde (55.5 mg; 0.26 mmol; 1.00 eq.) giving 5-[(E)-[(1,1-dioxo-1,2-benzothiazol-3-yl)-methyl-hydrazono]methyl]-3-ethyl-1H-benzimidazol-2-one (65.8 mg; 65 %) as a yellow solid. 1H NMR (DMSO-d6, 400 MHz): δ 11.19 (s, 1H); 8.92 (m, 1H); 8.50 (s, 1H); 8.09 (m, 1H); 7.90 (m, 2H); 7.59 (s, 1H); 7.56 (dd, J = 1.3 Hz, J = 8.1 Hz, 1H); 7.16 (d, J = 8.1 Hz, 1H) ; 3.92 (t, J = 7.3 Hz, 2H) ; 3.81 (s, 3H); 1.29 (t, J = 7.3 Hz, 3H). mp: 258-294°C.

### Example 25: 5-[(E)-[(1,1-dioxo-1,2-benzothiazol-3-yl)-methyl-hydrazono]methyl]-3-propyl-1H-benzimidazol-2-one

### Step A: (3-fluoro-4-nitro-phenyl)methanol

Diisobutyl aluminium hydride (1M in DCM, 6.54 ml; 56.59 mmol; 2.30 eq) was added dropwise to a solution of methyl 3-fluoro-4-nitro-benzoate (4.90 g; 24.61 mmol; 1.00eq) in DCM (100.00 ml) at -78°C. The reaction mixture was allowed to warm to rt and stirred for 2h. Additional DIBAL (1M in DCM, 6.54 ml; 56.59 mmol; 2.30 eq) was added at 0°C and the reaction was stirred 18h at rt. New addition of DIBAL (1M in DCM, 6.54 ml; 56.59 mmol; 2.30 eq) was done at 0°C and the reaction was again stirred 44h at rt. The reaction was diluted with diethyl ether, poured into 1M solution of Rochelle's salt and the mixture stirred until two clear layers appeared. The organic layer was recovered and aqueous layer was extracted twice with diethyl ether. Combined organic layers were dried over MgSO₄, filtered and concentrated under vacuum to give (3-fluoro-4-nitro-phenyl)methanol (4.16 g; 99 %) as an orange oil. 1H NMR (DMSO-d6, 300 MHz): δ 8.14 (t, J = 8.2 Hz, 1H) ; 7.47 (m, 1H); 7.38 (m, 1H); 5.62 (t, J = 5.7 Hz, 1H); 4.62 (d, J = 5.7 Hz, 2H).

### Step B: tert-butyl-[(3-fluoro-4-nitro-phenyl)methoxy]-dimethyl-silane

Imidazole (1.99 g, 29.00 mmol, 1.20 eq) and tert-butyldimethylsilyl chloride (5.05 ml, 29.00 mmol, 1.20 eq) were added to a solution of (3-fluoro-4-nitro-phenyl)methanol (4.16 g, 24.30 mmol, 1.00 eq) in DMF (20.00 ml). The reaction was stirred 1h at rt, then diluted in water and extracted with EtOAc (3x). Combined organic layers were washed with brine, dried over MgSO₄, filtered and concentrated under vacuum to give tert-butyl-[(3-fluoro-4-nitro-phenyl)methoxy]-dimethyl-silane (5.97 g, 86 %) as a brown oil. 1H NMR (DMSO-d6, 300 MHz): δ 8.06 (t, J = 8.2 Hz, 1H); 7.34 (m, 1H) ; 7.27 (m, 1H) ; 4.73 (s, 2H) ; 0.81 (m, 9H) ; 0.11 (s, 6H).

### Step C: [4-nitro-3-(propylamino)phenyl]methanol

Propylamine (860 µL, 10.40 mmol, 1.50 eq) and triethylamine (1.89 ml, 14.03 mmol, 2.00 eq) were added to a solution of tert-butyl-[(3-fluoro-4-nitro-phenyl)methoxy]-dimethylsilane (2.00 g, 7.01 mmol, 1.00 eq) in DMF (25.00 ml). The reaction was stirred 1h at 130°C under microwave irradiation. It was then diluted with water and extracted with DCM. The organic layer was washed with HCl 1M, water and brine, dried over MgSO₄ and concentrated under vacuum to give [4-nitro-3-(propylamino)phenyl]methanol (1.96 g, quant.) as an orange oil, used directly in the next step without purification. 1H NMR (DMSO-d6, 300 MHz): δ 8.20 (t, J = 5.9 Hz, 1H) ; 8.02 (m, 1H) ; 6.98 (s, 1H) ; 6.60 (d, J = 8.9 Hz, 1H) ; 5.42 (t, J = 5.7 Hz, 1H) ; 4.51 (d, J = 5.7 Hz, 2H) ; 2.25 (m, 2H) ; 1.65 (m, 2H) ; 0.96 (t, J = 7.43 Hz, 3H).

### Step D: 5-[[tert-butyl(dimethyl)silyl]oxymethyl]-2-nitro-N-propyl-aniline

The compound was prepared using the same procedure as described in example 25 step B starting from [4-nitro-3-(propylamino)phenyl]methanol (1.96 g; 9.32 mmol; 1.00 eq.) giving 5-[[tert-butyl(dimethyl)silyl]oxymethyl]-2-nitro-N-propyl-aniline (1.95 g; 55 %) as an orange oil. 1H NMR (DMSO-d6, 300 MHz): δ 8.26 (t, J = 5.53 Hz, 1H); 8.03 (d, J = 8.9 Hz, 1H) ; 6.98 (d, J = 1.6 Hz, 1H); 6.58 (dd, J = 1.6 Hz, J = 8.9 Hz, 1H) ; 4.73 (s, 2H) ; 3.29 (m, 2H) ; 1.64 (m, 2H) ; 0.94 (t, J = 7.43 Hz, 3H); 0.93 (s, 9H) ; 0.10 (s, 6H).

### Step E: 4-[[tert-butyl(dimethyl)silyl]oxymethyl]-N2-propyl-benzene-1,2-diamine

The compound was prepared using the same procedure as described in example 23 step B starting from 5-[[tert-butyl(dimethyl)silyl]oxymethyl]-2-nitro-N-propyl-aniline (1.95 g; 6.01 mmol; 1.00 eq.) giving 4-[[tert-butyl(dimethyl)silyl]oxymethyl]-N2-propyl-benzene-1,2-diamine (1.65 g; 89 %) as an brown oil. 1H NMR (DMSO-d6, 300 MHz): δ 6.47 (d, J = 7.6 Hz, 1H) ; 6.38 (d, J = 1.7 Hz, 1H); 6.33 (dd, J = 1.7 Hz, J = 7.6 Hz, 1H) ; 4.50 (s, 2H) ; 4.43 (br s, 2H) ; 4.38 (br s, 1H) ; 2.97 (m, 2H) ; 1.60 (sext, J = 7.3 Hz, 2H) ; 0.95 (t, J = 7.3 Hz, 3H); 0.88 (s, 9H) ; 0.03 (s, 6H).

### Step F: 5-[[tert-butyl(dimethyl)silyl]oxymethyl]-3-propyl-1H-benzimidazol-2-one

The compound was prepared using the same procedure as in example 23 step C starting from 4-[[tert-butyl(dimethyl)silyl]oxymethyl]-N2-propyl-benzene-1,2-diamine (1.65 g; 5.60 mmol; 1. eq.) giving 5-[[tert-butyl(dimethyl)silyl]oxymethyl]-3-propyl-1H-benzimidazol-2-one (1.76 g; 78 %) as an black oil. 1H NMR (DMSO-d6, 300 MHz): δ 10.77 (s, 1H) ; 7.30 (d, J = 1.0 Hz, 1H); 7.04 (s, 1H); 6.93 (d, J = 1.0 Hz, 1H); 4.70 (s, 2H); 3.72 (t, J = 7.1 Hz, 2H) ; 1.64 (sext, J = 7.3 Hz, 2H); 0.85 (t, J = 7.3 Hz, 3H); 0.83 (s, 9H) ; 0.06 (s, 6H).

### Step G: 5-(hydroxymethyl)-3-propyl-1H-benzimidazol-2-one

5-[[tert-butyl(dimethyl)silyl]oxymethyl]-3-propyl-1H-benzimidazol-2-one (1.76 g; 5.49 mmol; 1.00 eq) was dissolved in TBAF (1M in THF, 21ml; 71.00 mmol; 13.00 eq) and the reaction mixture was stirred 1h at 50°C. The mixture was diluted with EtOAc and washed with saturated NaHC0₃ solution. The aqueous layer was extracted with EtOAc (3x). Combined organic layers were dried over MgSO₄, filtered and concentrated under vacuum. The residue was purified by chromatography (column SiO₂ 50g, flow rate 40ml/min, gradient from 100% cyclohexane to 100% EtOAc) to give 5-(hydroxymethyl)-3-propyl-1H-benzimidazol-2-one (519.7 mg; 46 %) as a brown resin. 1H NMR (DMSO-d6, 300 MHz): δ 10.72 (s, 1H) ; 7.06 (s, 1H) ; 6.91 (m, 2H) ; 5.08 (t, J = 5.7 Hz, 1H) ; 4.48 (d, J = 5.7 Hz, 2H) ; 3.73 (t, J = 7.1 Hz, 2H) ; 1.65 (sext, J = 7.3 Hz, 2H) ; 0.87 (t, J = 7.3 Hz, 3H).

### Step H: 2-oxo-3-propyl-1H-benzimidazole-5-carbaldehyde

The compound was prepared using the same procedure as described in example 23 step E starting from 5-(hydroxymethyl)-3-propyl-1H-benzimidazol-2-one (50.0 mg; 0.24 mmol; 1.00 eq.) giving 2-oxo-3-propyl-1H-benzimidazole-5-carbaldehyde (32.1 mg; 65 %) as a brown powder. 1H NMR (DMSO-d6, 300 MHz): δ 11.39 (s, 1H); 9.90(s, 1H); 7.64 (s, 1H) ; 7.63 (dd, J = 1.5 Hz, J = 7.8 Hz, 1H); 7.17 (d, J = 7.8 Hz, 1H); 3.82 (t, J = 7.1 Hz, 2H); 1.68 (sext, J = 7.3 Hz, 2H) ; 0.88 (t, J = 7.4 Hz, 3H).

### Step I: 5-[(E)-[(1,1-dioxo-1,2-benzothiazol-3-yl)-methyl-hydrazono]methyl]-3-propyl-1H-benzimidazol-2-one

The compound was prepared using the same procedure as in example 1 step C starting from 1-(1,1-dioxo-1,2-benzothiazol-3-yl)-1-methyl-hydrazine (example 1, step B, 28.0 mg; 0.13 mmol; 1 eq.) and 2-oxo-3-propyl-1H-benzimidazole-5-carbaldehyde (29.8 mg; 0.15 mmol; 1.1 eq.) giving 5-[(E)-[(1,1-dioxo-1,2-benzothiazol-3-yl)-methyl-hydrazono]methyl]-3-propyl-1H-benzimidazol-2-one (24.0 mg; 46 %) as a beige solid. 1H NMR (DMSO-d6, 400 MHz): δ 11.20 (s, 1H) ; 8.92 (m, 1H); 8.50 (m, 1H) ; 8.09 (m, 1H) ; 7.89 (m, 2H) ; 7.59 (br s, 1H); 7.55 (dd, J = 1.3 Hz, J = 8.0 Hz, 1H); 7.16 (d, J = 8.0 Hz, 1H); 3.85 (t, J = 7.0 Hz, 2H) ; 3.81 (s, 3H) ; 1.74 (sext, J = 7.2 Hz, 2H) ; 0.93 (t, J = 7.4 Hz, 3H). mp: 224-303°C.

### Example 26: 5-[(E)-[(1,1-dioxo-1,2-benzothiazol-3-yl)-methyl-hydrazono]methyl]-3-isopropyl-1H-benzimidazol-2-one

### Step A: methyl 3-isopropyl-2-oxo-1H-benzimidazole-5-carboxylate

The compound was prepared using the same procedure as described in example 24 step A starting from 3-isopropyl-2-oxo-1H-benzimidazole-5-carboxylic acid (1.00 g; 4.54 mmol; 1.00 eq.) giving methyl 3-isopropyl-2-oxo-1H-benzimidazole-5-carboxylate (700.0 mg; 66 %) as a brown solid. 1H NMR (DMSO-d6, 300 MHz): δ 11.27 (s, 1H) ; 7.71 (m, 1H) ; 7.68 (dd, J = 1.5 Hz, J = 8.1 Hz, 1H); 7.07 (d, J = 8.1 Hz, 1H) ; 4.60 (sept, J = 7.0 Hz, 1H); 3.84 (s, 3H); 1.45 (d, J = 6.9 Hz, 6H).

### Step B: 5-(hydroxymethyl)-3-isopropyl-1H-benzimidazol-2-one

The compound was prepared using the same procedure as described in example 23 step D starting from methyl 3-ethyl-2-oxo-1H-benzimidazole-5-carboxylate (700.0 mg; 2.99 mmol; 1.00 eq.) giving 5-(hydroxymethyl)-3-isopropyl-1H-benzimidazol-2-one (616.0 mg; quant.) as a brown oil. 1H NMR (DMSO-d6, 300 MHz): δ 11.25 (br s, 1H); 7.17 (s, 1H); 6.90 (s, 2H) ; 4.55 (sept, J = 7.1 Hz, 1H); 4.48 (s, 2H) ; 1.43 (d, J = 7.1 Hz, 6H).

### Step C: 3-isopropyl-2-oxo-1H-benzimidazole-5-carbaldehyde

The compound was prepared using the same procedure as described in example 23 step E starting from 5-(hydroxymethyl)-3-isopropyl-1H-benzimidazol-2-one (620.0 mg; 3.01 mmol; 1.00 eq.) giving 3-isopropyl-2-oxo-1H-benzimidazole-5-carbaldehyde (366.0 mg; 60 %) as a beige solid. 1H NMR (DMSO-d6, 300 MHz): δ 11.38 (br s, 1H) ; 9.91 (s, 1H) ; 7.72 (d, J = 1.3 Hz, 1H); 7.61 (dd, J = 1.3 Hz, J = 8.0 Hz, 1H); 7.16 (d, J = 8.0 Hz, 1H); 4.62 (sept, J = 7.0 Hz, 1H) ; 1.47 (d, J = 7.0 Hz, 6H).

### Step D: 3-isopropyl-5-[(E)-(methylhydrazono)methyl]-1H-benzimidazol-2-one

The compound was prepared using the same procedure as described in example 21 step A starting from 3-isopropyl-2-oxo-1H-benzimidazole-5-carbaldehyde (100.0 mg; 0.49 mmol; 1.00 eq.) giving 3-isopropyl-5-[(E)-(methylhydrazono)methyl]-1H-benzimidazol-2-one (113.0 mg; 99 %) as a beige solid, used directly in next step without purification. LC/MS (Method a): Rt = 0.84min ; MS : m/z = 233 [M+H] ⁺

### Step E: 5-[(E)-[(1,1-dioxo-1,2-benzothiazol-3-yl)-methyl-hydrazono]methyl]-3-isopropyl-1H-benzimidazol-2-one

The compound was prepared using the same procedure as described in example 21 step B starting from 3-chloro-1,2-benzothiazole 1,1-dioxide (example 1, step A, 98.1 mg; 0.49 mmol; 1.00 eq.) and 3-isopropyl-5-[(E)-(methylhydrazono)methyl]-1H-benzimidazol-2-one (113.0 mg; 0.49 mmol; 1.00 eq.) giving 5-[(E)-[(1,1-dioxo-1,2-benzothiazol-3-yl)-methylhydrazono]methyl]-3-isopropyl-1H-benzimidazol-2-one (126.0 mg; 65 %) as a yellow solid. 1H NMR (DMSO-d6, 400 MHz): δ 11.19 (s, 1H); 8.92 (m, 1H); 8.52 (s, 1H); 8.10 (m, 1H); 7.88 (m, 2H); 7.73 (d, J = 1.1 Hz, 1H); 7.56 (dd, J = 1.3 Hz, J = 8.1 Hz, 1H); 7.16 (d, J = 8.1 Hz, 1H); 4.66 (sept, J = 7.0 Hz, 1H); 3.81 (s, 3H); 1.51 (d, J = 7.0 Hz, 6H).

### Example 27: 3-cyclopropyl-5-[(E)-[(1,1-dioxo-1,2-benzothiazol-3-yl)-methyl-hydrazono] methyl]-1H-benzimidazol-2-one

### Step A: methyl 3-cyclopropyl-2-oxo-1H-benzimidazole-5-carboxylate

The compound was prepared using the same procedure as described in example 24 step A starting from 3-cyclopropyl-2-oxo-1H-benzimidazole-5-carboxylic acid (1.00 g; 4.58 mmol; 1.00 eq.) giving methyl 3-cyclopropyl-2-oxo-1H-benzimidazole-5-carboxylate (800.0 mg; 75 %) as a white solid. 1H NMR (DMSO-d6, 300 MHz): δ 11.17 (s, 1H); 7.69 (dd, J = 1.7 Hz, J = 8.1 Hz, 1H); 7.66 (m, 1H); 7.04 (dt, J = 0.7 Hz, J = 8.3 Hz, 1H); 3.85 (s, 3H); 2.90 (quint J = 3.6 Hz, 1H) ; 1.05 (m, 2H) ; 0.88 (m, 2H).

### Step B: 3-cyclopropyl-5-(hydroxymethyl)-1H-benzimidazol-2-one

The compound was prepared using the same procedure as described in example 23 step D starting from methyl 3-cyclopropyl-2-oxo-1H-benzimidazole-5-carboxylate (800.0 mg; 3.44 mmol; 1.00 eq.) giving 3-cyclopropyl-5-(hydroxymethyl)-1H-benzimidazol-2-one (700.0 mg; quant.) as a white solid, used directly in the next step without purification.

### Step C: 3-cyclopropyl-2-oxo-1H-benzimidazole-5-carbaldehyde

The compound was prepared using the same procedure as described in example 23 step E starting from 3-cyclopropyl-5-(hydroxymethyl)-1H-benzimidazol-2-one (700.0 mg; 3.43 mmol; 1.00 eq.) giving 3-cyclopropyl-2-oxo-1H-benzimidazole-5-carbaldehyde (400.0 mg; 58 %) as a white solid. 1H NMR (DMSO-d6, 300 MHz): δ 11.29 (br s, 1H); 9.92 (s, 1H) ; 7.62 (dd, J = 1.5 Hz, J = 7.8 Hz, 1H); 7.60 (m, 1H); 7.14 (dd, J = 0.3 Hz, J = 7.8 Hz, 1H) ; 2.91 (quint, J = 3.6 Hz, 1H); 1.04 (m, 2H) ; 0.89 (m, 2H).

### Step D: 3-cyclopropyl-5-[(E)-(methylhydrazono)methyl]-1H-benzimidazol-2-one

The compound was prepared using the same procedure as described in example 21 step A starting from 3-cyclopropyl-2-oxo-1H-benzimidazole-5-carbaldehyde (100.0 mg; 0.49 mmol; 1.00 eq.) giving 3-cyclopropyl-5-[(E)-(methylhydrazono)methyl]-1H-benzimidazol-2-one (113.0 mg; 99 %) as a white solid, used directly in the next step without purification.

### Step E: 3-cyclopropyl-5-[(E)-[(1,1-dioxo-1,2-benzothiazol-3-yl)-methyl-hydrazono] methyl]-1H-benzimidazol-2-one

The compound was prepared using the same procedure as described in example 21 step B starting from 3-chloro-1,2-benzothiazole 1,1-dioxide (example 1, step A, 99.0 mg; 0.49 mmol; 1.00 eq.) and 3-cyclopropyl-5-[(E)-(methylhydrazono)methyl]-1H-benzimidazol-2-one (113.0 mg; 0.49 mmol; 1.00 eq.) giving 3-cyclopropyl-5-[(E)-[(1,1-dioxo-1,2-benzothiazol-3-yl)-methyl-hydrazono]methyl]-1H-benzimidazol-2-one (84.0 mg; 41 %) as a yellow solid. 1H NMR (DMSO-d6, 400 MHz): δ 11.09 (s, 1H); 8.95 (m, 1H); 8.51 (s, 1H); 8.09 (m, 1H); 7.92 (m, 2H) ; 7.68 (d, J = 1.3 Hz, 1H); 7.53 (dd, J = 1.4 Hz, J = 8.0 Hz, 1H) ; 7.11 (d, J = 7.9 Hz, 1H) ; 3.81 (s, 3H); 2.96 (sept, J = 4.0 Hz, 1H) ; 1.08 (m, 2H); 0.96 (m, 2H).

### Example 28: 3-cyclobutyl-5-[(E)-[(1,1-dioxo-1,2-benzothiazol-3-yl)-methylhydrazono] methyl]-1H-benzimidazol-2-one

### Step A: methyl 3-(cyclobutylamino)-4-nitro-benzoate

The compound was prepared using the same procedure as described in example 23 step A starting from methyl 3-fluoro-4-nitro-benzoate (500.0 mg; 2.51 mmol; 1.00 eq.) and cyclobutylamine (398 µL; 4.65 mmol; 1.85 eq) giving methyl 3-(cyclobutylamino)-4-nitrobenzoate (590.0 mg; 74 %) as an orange oil. 1H NMR (DMSO-d6, 300 MHz): δ 8.18 (d, J =8.8 Hz, 1H); 7.99(d, J = 5.9 Hz, 1H); 7.39(d, J = 1.8 Hz, 1H); 7.18(dd, J = 1.8 Hz, J= 8.9 Hz, 1H); 4.18 (m, 1H); 3.89 (s, 3H); 2.43 (m, 2H) ; 2.06 (m, 2H) ; 1.82 (m, 2H).

### Step B: methyl 4-amino-3-(cyclobutylamino)benzoate

The compound was prepared using the same procedure as described in example 23 step B starting from methyl 3-(cyclobutylamino)-4-nitro-benzoate (590.0 mg; 2.36 mmol; 1.00 eq.) giving methyl 4-amino-3-(cyclobutylamino)benzoate (507.0 mg; 96 %) as a beige solid. 1H NMR (DMSO-d6, 300 MHz): δ 7.13 (dd, J = 2.0 Hz, J = 8.1 Hz, 1H); 6.86 (d, J = 2.0 Hz, 1H); 6.52 (d, J = 8.3 Hz, 1H); 5.42 (s, 2H); 4.86 (d, J = 6.6 Hz, 1H); 3.82 (m, 1H); 2.36 (m, 2H); 1.82 (m, 4H).

### Step C: methyl 3-cyclobutyl-2-oxo-1H-benzimidazole-5-carboxylate

The compound was prepared using the same procedure as described in example 23 step C starting from methyl 4-amino-3-(cyclobutylamino)benzoate (507.0 mg; 2.30 mmol; 1.00 eq.) giving methyl 3-cyclobutyl-2-oxo-1H-benzimidazole-5-carboxylate (539.0 mg; 91 %) as a pink solid. 1H NMR (DMSO-d6, 300 MHz): δ 11.28 (s, 1H); 7.77 (m, 1H); 7.69 (dd, J = 1.6 Hz, J = 8.2 Hz, 1H); 7.07 (dd, J = 0.5 Hz, J = 8.3 Hz, 1H) ; 4.84 (m, 1H) ; 3.84 (s, 3H); 2.83 (m, 2H) ; 2.30 (m, 2H) ; 1.84 (m, 2H).

### Step D: 3-cyclobutyl-5-(hydroxymethyl)-1H-benzimidazol-2-one

The compound was prepared using the same procedure as described in example 23 step D starting from methyl 3-cyclobutyl-2-oxo-1H-benzimidazole-5-carboxylate (539.0 mg; 2.19 mmol; 1.00 eq.) giving 3-cyclobutyl-5-(hydroxymethyl)-1H-benzimidazol-2-one (417.0 mg; 55 %) as a brown oil, used directly in next step without purification. 1H NMR (DMSO-d6, 300 MHz): δ 7.24 (s, 1H); 6.88 (m, 2H) ; 4.81 (m, 1H); 4.48 (s, 2H) ; 2.83 (m, 2H); 2.23 (m, 2H) ; 1.83 (m, 2H).

### Step E: 3-cyclobutyl-2-oxo-1H-benzimidazole-5-carbaldehyde

The compound was prepared using the same procedure as described in example 23 step E starting from 3-cyclobutyl-5-(hydroxymethyl)-1H-benzimidazol-2-one (417.0 mg; 1.91 mmol; 1.00 eq.) giving 3-cyclobutyl-2-oxo-1H-benzimidazole-5-carbaldehyde (193.0 mg; 47 %) as a brown solid. 1H NMR (DMSO-d6, 300 MHz): δ 11.40 (br s, 1H) ; 9.92 (s, 1H) ; 7.77 (d, J = 1.3 Hz, 1H); 7.62 (dd, J = 1.4 Hz, J = 8.0 Hz, 1H); 7.16 (d, J = 7.9 Hz, 1H); 4.86 (m, 1H) ; 2.82 (m, 2H) ; 2.29 (m, 2H) ; 1.84 (m, 2H).

### Step F: 3-cyclobutyl-5-[(E)-[(1,1-dioxo-1,2-benzothiazol-3-yi)-methyl-hydrazono]methyl]-1H-benzimidazol-2-one

The compound was prepared using the same procedure as described in example 1 step C starting from 1-(1,1-dioxo-1,2-benzothiazol-3-yl)-1-methyl-hydrazine (example 1, step B, 50.0 mg; 0.24 mmol; 1.00 eq.) and 3-cyclobutyl-2-oxo-1H-benzimidazole-5-carbaldehyde (61.4 mg; 0.28 mmol; 1.20 eq.) giving 3-cyclobutyl-5-[(E)-[(1,1-dioxo-1,2-benzothiazol-3-yl)-methyl-hydrazono]methyl]-1H-benzimidazol-2-one (58.0 mg; 60 %) as a yellow solid. 1H NMR (DMSO-d6, 400 MHz): δ 11.21 (s, 1H); 8.93 (d, J = 7.5 Hz, 1H); 8.53 (s, 1H); 8.10 (m, 1H); 7.83 (m, 2H); 7.82 (m, 1H); 7.57 (dd, J = 1.4 Hz, J = 8.3 Hz, 1H) ; 7.16 (d, J = 8.1 Hz, 1H) ; 4.90 (quin, J = 9.0 Hz, 1H) ; 3.82 (s, 3H); 2.86 (dquin, J = 2.6 Hz, J = 9.5 Hz, 2H) ; 2.37 (m, 2H) ; 1.90 (m, 2H). mp: 323-336°C.

### Example 29: 5-[(E)-[(1,1-dioxo-1,2-benzothiazol-3-yl)-methyl-hydrazono]methyl]-3-isobutyl-1H-benzimidazol-2-one

### Step A: methyl 3-(isobutylamino)-4-nitro-benzoate

The compound was prepared using the same procedure as described in example 23 step A starting from methyl 3-fluoro-4-nitro-benzoate (2.00 g; 10.04 mmol; 1.00 eq.) and isobutylamine (1.86 ml; 18.58 mmol; 1.85 eq) giving methyl 3-(isobutylamino)-4-nitrobenzoate (2.54 g; quant.) as an orange solid. 1H NMR (DMSO-d6, 300 MHz): δ 8.18 (d, J =8.7 Hz, 1H); 8.17(br s, 1H); 7.52(d, J = 1.4Hz, 1H); 7.14(dd, J = 1.4 Hz, J = 8.7 Hz, 1H); 3.89 (s, 3H); 3.23 (t, J = 6.2 Hz, 2H) ; 1.97 (m, 1H) ; 0.98 (d, J = 6.7 Hz, 6H).

### Step B: methyl 4-amino-3-(isobutylamino)benzoate

The compound was prepared using the same procedure as described in example 23 step B starting from methyl 3-(isobutylamino)-4-nitro-benzoate (2.54 g; 10.07 mmol; 1.00 eq.) giving methyl 4-amino-3-(isobutylamino)benzoate (2.40 g; quant.) as a grey solid. 1H NMR (DMSO-d6, 300 MHz): δ 7.13 (dd, J = 2.0 Hz, J = 8.1 Hz, 1H) ; 6.94 (d, J = 2.0 Hz, 1H) ; 6.53 (d, J = 8.1 Hz, 1H) ; 5.45 (br s, 2H); 4.56 (t, J = 4.6 Hz, 1H) ; 3.72 (s, 3H); 2.85 (dd, J = 4.8 Hz, J = 6.4 Hz, 2H) ; 1.90 (sept, J = 6.7 Hz, 1H) ; 0.98 (d, J = 6.6 Hz, 6H).

### Step C: methyl 3-isobutyl-2-oxo-1H-benzimidazole-5-carboxylate

The compound was prepared using the same procedure as described in example 23 step C starting from methyl 4-amino-3-(isobutylamino)benzoate (2.40 g; 10.80 mmol; 1.00 eq.) giving methyl 3-isobutyl-2-oxo-1H-benzimidazole-5-carboxylate (2.35 g; 87 %) as a beige solid. 1H NMR (DMSO-d6, 300 MHz): δ 11.29 (s, 1H); 7.69 (dd, J = 1.6 Hz, J = 8.2 Hz, 1H) ; 7.64 (d, J = 1.5 Hz, 1H) ; 7.08 (d, J = 8.1 Hz, 1H) ; 3.84 (s, 3H); 3.66 (d, J = 7.6 Hz, 2H) ; 2.09 (sept, J = 6.8 Hz, 1H) ; 0.88 (d, J = 6.6 Hz, 6H).

### Step D: 5-(hydroxymethyl)-3-isobutyl-1H-benzimidazol-2-one

The compound was prepared using the same procedure as described in example 23 step D starting from methyl 3-isobutyl-2-oxo-1H-benzimidazole-5-carboxylate (2.35 g; 9.45 mmol; 1.00 eq.) giving 5-(hydroxymethyl)-3-isobutyl-1H-benzimidazol-2-one (1.48 g; 71 %) as a beige solid, used directly in next step without purification. 1H NMR (DMSO-d6, 300 MHz): δ 6.71 (d, J = 7.6 Hz, 1H); 11.30 (s, 1H) ; 6.67 (d, J = 1.3 Hz, 1H) ; 6.61 (dd, J = 1.5 Hz, J = 7.8 Hz, 1H) ; 4.39 (s, 2H) ; 3.49 (d, J = 7.3 Hz, 2H) ; 2.08 (sept, J = 6.6 Hz, 1H) ; 0.84 (d, J = 6.8 Hz, 6H).

### Step E: 3-isobutyl-2-oxo-1H-benzimidazole-5-carbaldehyde

The compound was prepared using the same procedure as described in example 23 step E starting from 5-(hydroxymethyl)-3-isobutyl-1H-benzimidazol-2-one (400.0 mg; 1.82 mmol; 1.00 eq.) giving 3-isobutyl-2-oxo-1H-benzimidazole-5-carbaldehyde (262.0 mg; 66 %) as a beige solid. 1H NMR (DMSO-d6, 300 MHz): δ 11.40 (br s, 1H); 9.90 (s, 1H); 7.63 (s, 1H); 7.62 (dd, J = 1.5 Hz, J = 7.1 Hz, 1H); 7.17 (m, 1H); 3.67 (d, J = 7.4 Hz, 2H); 2.12 (sept, J = 7.0 Hz, 1H) ; 0.89 (d, J = 6.8 Hz, 6H).

### Step F: 5-[(E)-[(1,1-dioxo-1,2-benzothiazol-3-yl)-methyl-hydrazono]methyl]-3-isobutyl-1H-benzimidazol-2-one

The compound was prepared using the same procedure as described in example 1 step C starting from 1-(1,1-dioxo-1,2-benzothiazol-3-yl)-1-methyl-hydrazine (example 1, step B, 64.5 mg; 0.31 mmol; 1.00 eq.) and 3-isobutyl-2-oxo-1H-benzimidazole-5-carbaldehyde (100.0 mg; 0.46 mmol; 1.50 eq.) giving 5-[(E)-[(1,1-dioxo-1,2-benzothiazol-3-yl)-methyl-hydrazono]methyl]-3-isobutyl-1H-benzimidazol-2-one (58.0 mg; 45 %) as a yellow solid. 1H NMR (DMSO-d6, 400 MHz): δ 11.20 (s, 1H); 8.91 (m, 1H); 8.49 (s, 1H); 8.10 (td, J = 0.9 Hz, J = 7.5 Hz, 1H); 7.89 (m, 2H); 7.59 (d, J = 1.1 Hz, 1H); 7.54 (dd, J = 1.5 Hz, J = 8.1 Hz, 1H); 7.16 (d, J = 7.9 Hz, 1H); 3.81 (s, 3H); 3.70 (d, J = 7.3 Hz, 2H); 2.18 (sept, J = 7.0 Hz, 1H) ; 0.95 (d, J = 6.8 Hz, 6H).

### Example 30: 5-[(E)-[(1,1-dioxo-1,2-berizothiazol-3-yl)-methyl-hydrazono]methyl]-3-(2,2,2-trifluoroethyl)-1H-benzimidazol-2-one

### Step A: methyl 2-oxo-3-(2,2,2-trifluoroethyl)-1H-benzimidazole-5-carboxylate

The compound was prepared using the same procedure as described in example 24 step A starting from 2-oxo-3-(2,2,2-trifluoroethyl)-1H-benzimidazole-5-carboxylic acid (1.00 g; 3.84 mmol; 1.00 eq.) giving methyl 2-oxo-3-(2,2,2-trifluoroethyl)-1H-benzimidazole-5-carboxylate (936.0 mg; 89 %) as a white solid. 1H NMR (DMSO-d6, 300 MHz): δ 11.58 (br s, 1H); 7.85 (m, 1H); 7.75 (dd, J = 1.6 Hz, J = 8.2 Hz, 1H); 7.14 (dd, J = 0.3 Hz, J = 8.3 Hz, 1H) ; 4.84 (q, J = 9.4 Hz, 2H) ; 3.85 (s, 3H).

### Step B: 5-(hydroxymethyl)-3-(2,2,2-trifluoroethyl)-1H-benzimidazol-2-one

The compound was prepared using the same procedure as described in example 23 step D starting from methyl 2-oxo-3-(2,2,2-trifluoroethyl)-1H-benzimidazole-5-carboxylate (932.0 mg; 3.40 mmol; 1.00 eq.) giving 5-(hydroxymethyl)-3-(2,2,2-trifluoroethyl)-1H-benzimidazol-2-one (776.0 mg; 93 %) as a white solid, used directly in next step without purification. 1H NMR (DMSO-d6, 300 MHz): δ 11.05 (br s, 1H); 7.19 (s, 1H); 7.00 (m, 1H), 6.96 (m, 1H); 5.16 (t, J = 5.6 Hz, 1H); 4.70 (q, J = 9.5 Hz, 2H) ; 4.49 (d, J = 5.6 Hz, 2H).

### Step C: 2-oxo-3-(2,2,2-trifluoroethyl)-1H-benzimidazole-5-carbaldehyde

The compound was prepared using the same procedure as described in example 23 step E starting from 5-(hydroxymethyl)-3-(2,2,2-trifluoroethyl)-1H-benzimidazol-2-one (775.0 mg; 3.15 mmol; 1.00 eq.) giving 2-oxo-3-(2,2,2-trifluoroethyl)-1H-benzimidazole-5-carbaldehyde (200.0 mg; 26 %) as a beige solid. 1H NMR (DMSO-d6, 300 MHz): δ 11.69 (br s, 1H); 9.91 (s, 1H); 7.78 (s, 1H); 7.70 (dd, J = 1.5 Hz, J = 8.1 Hz, 1H); 7.23 (d, J = 8.1 Hz, 1H); 4.84 (q, J = 9.4 Hz, 2H).

### Step D: 5-[(E)-[(1,1-dioxo-1,2-benzothiazol-3-yl)-methyl-hydrazono]methyl]-3-(2,2,2-trifluoroethyl)-1H-benzimidazol-2-one

The compound was prepared using the same procedure as described in example 1 step C starting from 1-(1,1-dioxo-1,2-benzothiazol-3-yl)-1-methyl-hydrazine (example 1, step B, 36.1 mg; 0.17 mmol; 1.00 eq.) and 2-oxo-3-(2,2,2-trifluoroethyl)-1H-benzimidazole-5-carbaldehyde (50.0 mg; 0.20 mmol; 1.20 eq.) giving 5-[(E)-[(1,1-dioxo-1,2-benzothiazol-3-yl)-methyl-hydrazono]methyl]-3-(2,2,2-trifluoroethyl)-1H-benzimidazol-2-one (49.0 mg; 66 %) as a pale yellow solid. 1H NMR (DMSO-d6, 400 MHz): δ 11.51 (br s, 1H); 8.91 (d, J = 7.9 Hz, 1H); 8.49 (s, 1H); 8.09 (td, J = 1.0 Hz, J = 7.3 Hz, 1H); 7.92 (dt, J = 1.0 Hz, J = 7.5 Hz, 1H); 7.81 (dt, J = 1.2 Hz, J = 7.8 Hz, 1H); 7.76 (s, 1H); 7.58 (dd, J = 1.5 Hz, J = 8.1 Hz, 1H); 7.21 (d, J = 7.9 Hz, 1H); 4.88 (q, J = 9.4 Hz, 2H); 3.81 (s, 3H). mp: 329-373°C.

### Example 31: 5-[(E)-[(1,1-dioxo-1,2-benzothiazol-3-yl)-methyl-hydrazono]methyl]-3-methyl -isoindolin-1-one

### Step A: tert-butyl 5-bromo-1-oxo-isoindoline-2-carboxylate

5-bromoisoindolin-1-one (1.00 g; 4.63 mmol; 1.00 eq) was dissolved in DCM (35.00 ml) then di-*tert*-butyl dicarbonate (1.50 g; 6.93 mmol; 1.50 eq), triethylamine (705 µl; 5.09 mmol; 1.10 eq) and 4-dimethylaminopyridine (621.0 mg; 5.08 mmol; 1.10 eq) were added. The mixture was stirred 18h at rt, then poured onto water and extracted with DCM. The organic layer was dried over MgSO₄, filtered and concentrated. The residue was purified by chromatography (gradient from 100% n-heptane to 50/50 n-heptane / EtOAc) to give tert-butyl 5-bromo-1-oxo-isoindoline-2-carboxylate (1.33 g; 92 %) as a yellow solid. LC/MS (Method E): Rt = 2.24min ; MS : m/z = 256-258 [M+H-*t*Bu]⁺

### Step B: tert-butyl 5-bromo-3-methyl-1-oxo-isoindoline-2-carboxylate

tert-butyl 5-bromo-1-oxo-isoindoline-2-carboxylate (1.33 g; 4.25 mmol; 1.00 eq) was dissolved in dry THF (20.00 ml) and cooled to -78 °C. A solution of lithium bis(trimethylsilyl)amide (1.5 M solution in THF, 4.25 ml; 6.37 mmol; 1.50 eq) was added dropwise. After stirring at -78°C for 1h, methyl iodide (793 µl; 12.74 mmol; 3.00 eq) was added and stirring was continued 5h at -78°C, prior to additionnal Mel (793 µl; 12.74 mmol; 3.00 eq). After 2h at -78 °C, the mixture was quenched with brine and extracted with EtOAc. The organic layer was dried over MgSO₄, filtered and concentrated. The residue was purified by chromatography (gradient from 100% n-heptane to 80/20 n-heptane / EtOAc) to give tert-butyl 5-bromo-3-methyl-1-oxo-isoindoline-2-carboxylate (288.0 mg; 21 %) as a white solid. 1H NMR (DMSO-d6, 300 MHz): δ 7.96 (s, 1H); 7.69 (m, 2H) ; 5.06 (q, J = 6.5 Hz, 1H) ; 1.54 (d, J = 6.5 Hz, 3H); 1.51 (s, 9H).

### Step C: 5-bromo-3-methyl-isoindolin-1-one

To a solution of tert-butyl 5-bromo-3-methyl-1-oxo-isoindoline-2-carboxylate (185.0 mg; 0.57 mmol; 1.00 eq) in 1,4-dioxane (2.50 ml), was added 1,4-dioxane saturated with HCl (2.50 ml) and the mixture stirred 1h at rt. The solvent was then removed evaporate to give 5-bromo-3-methyl-isoindolin-1-one (120.0 mg; quant) as a yellow solid. 1H NMR (DMSO-d6, 300 MHz): δ 8.72 (s, 1H) ; 7.82 (s, 1H) ; 7.65 (d, J = 7.9 Hz, 1H) ; 7.56 (d, J = 7.9 Hz, 1H) ; 4.62 (q, J = 6.7 Hz, 1H) ; 1.36 (d, J = 6.7 Hz, 3H).

### Step D: 3-methyl-1-oxo-isoindoline-5-carbaldehyde

The compound was prepared using the same procedure as described in example 20 step B starting from 5-bromo-3-methyl-isoindolin-1-one (70.0 mg; 0.31 mmol; 1.00 eq) giving 3-methyl-1-oxo-isoindoline-5-carbaldehyde (42.0 mg; quant) as a yellow solid, used in next step without purification. LC/MS (Method B): Rt = 1.68 min ; MS : m/z = 176 [M+H]⁺

### Step E: 5-[(E)-[(1,1-dioxo-1,2-benzothiazol-3-yl)-methyl-hydrazono]methyl]-3-methyl-isoindolin-1-one

The compound was prepared using the same procedure as in example 1 step C starting from 1-(1,1-dioxo-1,2-benzothiazol-3-yl)-1-methyl-hydrazine (example 1, step B, 48.0 mg; 0.23 mmol; 1.00 eq.) and 3-methyl-1-oxo-isoindoline-5-carbaldehyde (60.0 mg; 0.35 mmol; 1.50 eq.) giving 5-[(E)-[(1,1-dioxo-1,2-benzothiazol-3-yl)-methyl-hydrazono]methyl]-3-methyl-isoindolin-1-one (30.0 mg; 36 %) as a pale brown solid. 1H NMR (DMSO-d6, 300 MHz): δ 8.84 (m, 2H) ; 8.58 (s, 1H) ; 8.10 (d, J = 7.3 Hz, 1H) ; 7.95 (m, 4H) ; 7.80 (d, J = 8.0 Hz, 1H) ; 4.75 (q, J = 6.4 Hz, 1H), 3.82 (s, 3H) ; 1.43 (d, J = 6.4 Hz, 3H). mp > 298°C.

### Example 32: 5-[(E)-[(1,1-dioxo-1,2-benzothiazol-3-yl)-methyl-hydrazono]methyl]-3,3-dimethyl-isoindolin-1-one

### Step A: 5-bromo-2-[(4-methoxyphenyl)methyl]isoindolin-1-one

5-bromoisoindolin-1-one (1.00 g; 4.63 mmol; 1.00 eq) dissolved in a mixture of THF (25.00 ml) and DMF (4.50 ml) was added to a suspension of sodium hydride (60% dispersion in mineral oil, 185 mg; 4.62 mmol; 1.00 eq) and tetrabutylammonium iodide (349.0 mg; 0.92 mmol; 0.20 eq) in THF (23.00 ml). After 1.5 h stirring at rt, 4-methoxybenzyl chloride (724.0 mg; 4.63 mmol; 1.00 eq) was added and the mixture was stirred 18h at rt. The reaction mixture was used in the next step without work-up. LC/MS (Method E): Rt = 2.73 min ; MS : m/z = 332-334 [M+H]⁺

### Step B: 5-bromo-2-[(4-methoxyphenyl)methyl]-3,3-dimethyl-isoindolin-1-one

Sodium hydride (60% dispersion in mineral oil, 925.0 mg; 23.12 mmol; 5.00 eq) was added to the reaction mixture of 5-bromo-2-[(4-methoxyphenyl)methyl]isoindolin-1-one, then stirred at rt for 30 min. Then methyl iodide (1.73 ml; 27.74 mmol; 6.00 eq) was added and the mixture was stirred 1h at 66 °C. The mixture was quenched with water and extracted with EtOAc. The organic layer was dried over MgSO₄, filtered and concentrated. The residue was purified by chromatography (gradient from 100% n-heptane to 50/50 n-heptane / EtOAc) to give 5-bromo-2-[(4-methoxyphenyl)methyl]-3,3-dimethyl-isoindolin-1-one (990.0 mg; 59 %) as a white solid. LC/MS (Method D): Rt = 2.29 min ; MS : m/z = 360-362 [M+H]⁺

### Step C: 5-bromo-3,3-dimethyl-isoindolin-1-one

A solution of 5-bromo-2-[(4-methoxyphenyl)methyl]-3,3-dimethyl-isoindolin-1-one (990.0 mg; 2.75 mmol; 1.00 eq) and ammonium cerium (IV) nitrate (4.61 g; 8.24 mmol; 3.00 eq) in acetonitrile (14.00 ml) and water (7.00 ml) was stirred 1h at 0 °C and 18h at rt. Then, more ammonium cerium (IV) nitrate (1.00 g; 1.78 mmol; 0.65 eq) was added at 0 °C and stirring was continued 1h at rt. The solution was diluted with EtOAc and washed with brine. The organic layer was dried over MgSO₄, filtered and concentrated. The residue was purified by chromatography (gradient from 100% chloroform to 95/5 chloroform / MeOH) to give 5-bromo-3,3-dimethyl-isoindolin-1-one (559.0 mg; 85 %) as a brown solid. 1H NMR (DMSO-d6, 300 MHz): δ 8.72 (s, 1H) ; 7.95 (d, J = 1.4 Hz, 1H) ; 7.63 (dd, J = 8.0 Hz, J = 1.4 Hz, 1H) ; 7.53 (d, J = 8.0 Hz, 1H) ; 1.44 (s, 6H).

### Step D: 3,3-dimethyl-1-oxo-isoindoline-5-carbaldehyde

The compound was prepared using the same procedure as described in example 20 step B starting from 5-bromo-3,3-dimethyl-isoindolin-1-one (456.0 mg; 1.90 mmol; 1.00 eq) giving 3,3-dimethyl-1-oxo-isoindoline-5-carbaldehyde (212.0 mg; 59 %) as a yellow solid, used in next step without purification. LC/MS (Method B): Rt = 1.71 min ; MS : m/z = 190 [M+H]⁺

### Step E: 5-[(E)-[(1,1-dioxo-1,2-benzothiazol-3-yl)-methyl-hydrazono]methyl]-3,3-dimethyl-isoindolin-1-one

The compound was prepared using the same procedure as described in example 1 step C starting from 1-(1,1-dioxo-1,2-benzothiazol-3-yl)-1-methyl-hydrazine (example 1, step B, 56.0 mg; 0.26 mmol; 1.00 eq.) and 3-methyl-1-oxo-isoindoline-5-carbaldehyde (78.0 mg; 0.39 mmol; 1.50 eq.) giving 5-[(E)-[(1,1-dioxo-1,2-benzothiazol-3-yl)-methyl-hydrazono]methyl]-3,3-dimethyl-isoindolin-1-one (26.0 mg; 26 %) as a pale yellow solid. 1H NMR (DMSO-d6, 300 MHz): δ 8.83 (m, 2H) ; 8.58 (s, 1H) ; 8.10 (m, 1H) ; 7.89 (m, 4H) ; 7.77 (d, J =7.7 Hz, 1H) ; 3.82 (s, 3H) ; 1.51 (s, 6H). mp > 295°C.

### Example 33: 5-[(E)-[(1,1-dioxo-1,2-benzothiazol-3-yl)-methyl-hydrazono]methyl]-3,3-dimethyl-indolin-2-one

### Step A: 3,3-dimethyl-2-oxo-indoline-5-carbaldehyde

The compound was prepared using the same procedure as described in example 20 step B starting from 5-bromo-3,3-dimethyl-indolin-2-one (100.0 mg; 0.40 mmol; 1.00 eq) giving 3,3-dimethyl-2-oxo-indoline-5-carbaldehyde (39.0 mg; 52 %) as an orange solid. 1H NMR (DMSO-d6, 300 MHz): δ 10.84 (br s, 1H) ; 9.85 (s, 1H) ; 7.83 (s, 1H) ; 7.79 (d, J = 7.9 Hz, 1H) ; 7.03 (d, J = 7.9 Hz, 1H) ; 1.29 (s, 6H).

### Step B: 5-[(E)-[(1,1-dioxo-1,2-benzothiazol-3-yl)-methyl-hydrazono]methyl]-3,3-dimethyl-indolin-2-one

The compound was prepared using the same procedure as described in example 1 step C starting from 1-(1,1-dioxo-1,2-benzothiazol-3-yl)-1-methyl-hydrazine (example 1, step B, 40.0 mg; 0.19 mmol; 1.00 eq.) and 3,3-dimethyl-2-oxo-indoline-5-carbaldehyde (36.0 mg; 0.19 mmol; 1.00 eq.) giving 5-[(E)-[(1,1-dioxo-1,2-benzothiazol-3-yl)-methyl-hydrazono]methyl]-3,3-dimethyl-indolin-2-one (48.0 mg; 66 %) as a yellow solid. 1H NMR (DMSO-d6, 300 MHz): δ 10.68 (s, 1H) : 8.87 (d, J = 7.2 Hz, 1H) ; 8.44 (s, 1H) ; 8.08 (d, J = 7.2 Hz, 1H) ; 7.90 (m, 2H) ; 7.75 (m, 2H) ; 7.04 (d, J = 8.5 Hz, 1H) ; 3.79 (s, 3H) ; 1.33 (s, 6H). mp > 300°C.

### Example 34: 5-[(E)-[(1,1-dioxo-1,2-benzothiazol-3-yl)-methyl-hydrazono]methyl]-3,3-diethyl-indolin-2-one

### Step A: tert-butyl 5-bromo-2-oxo-indoline-1-carboxylate

5-bromoindolin-2-one (1.50 g; 6.93 mmol; 1.00 eq) was dissolved in THF (45.00 ml) then di-*tert*-butyl dicarbonate (2.29 g; 10.40 mmol; 1.50 eq) and NaHCO₃ (5.24 g; 62.41 mmol; 9.00 eq) were added. The mixture was stirred 3h under reflux. Solids were filtered and the filtrate was concentrated. The residue was purified by chromatography (gradient from 100% n-heptane to 80/20 n-heptane / EtOAc) to give tert-butyl 5-bromo-2-oxo-indoline-1-carboxylate (1.52 g; 71 %) as a white solid. LC/MS (Method C): Rt = 2.85 min ; MS : m/z = 212-214 [M+H-Boc]+

### Step B: 5-bromo-3,3-diethyl-indolin-2-one

tert-Butyl 5-bromo-2-oxo-indoline-1-carboxylate (1.00 g; 3.20 mmol; 1.00 eq) was dissolved in dry THF (13.00 ml) under Ar atmosphere and ethyl iodide (769 µL; 9.61 mmol; 3.00 eq) was added. The mixture was cooled to 0 °C and sodium hydride (55% in mineral oil, 308.0 mg; 7.05 mmol; 2.20 eq) was added portion-wise over 45 min maintaining the internal temperature below 10 °C. The mixture was then stirred 3 h before water was added and the resultant solution extracted with DCM. The organic layer was washed with brine, dried over MgSO₄, filtered and concentrated. The residue was purified by chromatography (gradient from 80/20 n-heptane / EtOAc to 70/30 n-heptane / EtOAc) to give 5-bromo-3,3-diethyl-indolin-2-one (128.0 mg; 15 %) as a white solid. LC/MS (Method E): Rt = 2.68 min ; MS : m/z = 269-270 [M+H]⁺

### Step C: 3,3-diethyl-2-oxo-indoline-5-carbaldehyde

The compound was prepared using the same procedure as described in example 20 step B starting from 5-bromo-3,3-diethyl-indolin-2-one (128.0 mg; 0.48 mmol; 1.00 eq) giving 3,3-diethyl-2-oxo-indoline-5-carbaldehyde (118.0 mg; quant.), used in the next step without purification. LC/MS (Method E): Rt = 2.14 min ; MS : m/z = 218 [M+H]⁺

### Step D: 5-[(E)-[(1,1-dioxo-1,2-benzothiazol-3-yl)-methyl-hydrazono]methyl]-3,3-diethyl-indolin-2-one

The compound was prepared using the same procedure as described in example 1 step C starting from 1-(1,1-dioxo-1,2-benzothiazol-3-yl)-1-methyl-hydrazine (example 1, step B, 107.0 mg; 0.51 mmol; 1.00 eq.) and 3,3-diethyl-2-oxo-indoline-5-carbaldehyde (110.0 mg; 0.51 mmol; 1.00 eq.) giving 5-[(E)-[(1,1-dioxo-1,2-benzothiazol-3-yl)-methyl-hydrazono]methyl]-3,3-diethyl-indolin-2-one (29.0 mg; 14 %) as a beige solid. 1H NMR (DMSO-d6, 300 MHz): δ 10.70 (s, 1H) ; 8.87 (m, 1H) ; 8.45 (s, 1H) ; 8.08 (m, 1H) ; 7.90 (m, 2H); 7.77 (d, J = 8.0 Hz, 1H) ; 7.67 (s, 1H) ; 7.04 (d, J = 8.0 Hz, 1H) ; 3.79 (s, 3H); 1.80 (m, 4H) ; 0.59 (t, J = 7.1 Hz, 6H). mp: 288-289°C.

### Example 35: N-[(E)-1H-benzotriazol-5-ylmethyleneamino]-N-methyl-1,1-dioxo-1,2-benzothiazol-3-amine

The compound was prepared using the same procedure as in example 1 step C starting from 1-(1,1-dioxo-1,2-benzothiazol-3-yl)-1-methyl-hydrazine (example 1, step B, 46.0 mg; 0.31 mmol; 1.10 eq.) and 1H-benzotriazole-5-carbaldehyde (prepared according known procedures in Krasavin, M. et al Syn. Commun., 2005, 35, 2587-2595), (60 mg; 0.28 mmol; 1. eq.) giving N-[(E)-1H-benzotriazol-5-ylmethyleneamino]-N-methyl-1,1-dioxo-1,2-benzothiazol-3-amine (52.0 mg; 54 %) as a beige solid. 1H NMR (DMSO-d6, 300 MHz): δ 8.91 (d, J = 7.2 Hz, 1H) ; 8.69 (s, 1H) ; 8.39 (s, 1H) ; 8.02 (m, 5H) ; 3.84 (s, 3H). mp: 313-315°C.

### Example 36: 6-[(E)-[(1,1-dioxo-1,2-benzothiazol-3-yl)-methyl-hydrazono]methyl]-4-methoxy-pyridin-3-ol

### Step A: 5-benzyloxy-2-(hydroxymethyl)pyran-4-one

Sodium hydroxide (1.00 mol/l, 7.74 ml; 7.74 mmol; 1.10 eq.) was added to a solution of kojic acid (1.00 g; 7.04 mmol; 1.00 eq.) in MeOH (7.00 ml). Then, benzyl bromide (926 µL; 7.74 mmol; 1.10 eq.) was added dropwise and the reaction was heated 18h under reflux. The mixture was cooled to rt and 15ml of HCl 1N was added. The mixture was stirred at rt for 20min and the solid was filtered then washed with water. It was put into a mixture of heptane/AcOEt 1/1, triturated, filtered again and dried under vacuum to give 5-benzyloxy-2-(hydroxymethyl)pyran-4-one (1.28 g; 74 %), as a white solid. 1H NMR (DMSO-d6, 300 MHz): δ 8.17 (s, 1H) ; 7.38 (m, 5H) ; 6.32 (s, 1H) ; 5.68 (br s, 1H) ; 4.94 (s, 2H) ; 4.29 (br d, J = 3.0 Hz, 2H).

### Step B: 5-benzyloxy-2-(hydroxymethyl)-1H-pyridin-4-one

5-benzyloxy-2-(hydroxymethyl)pyran-4-one (1.27 g; 5.47 mmol; 1.00 eq.) and ammonia (7N in MeOH, 13.28 ml; 92.97 mmol; 17.00 eq.) were combined in a pressure reactor vessel (Q-tube) and heated 24h at 90°C. The mixture was concentrated and the residue was triturated in water. The solid was filtered, washed with water and dried under vacuum at 50°C to give 5-benzyloxy-2-(hydroxymethyl)-1H-pyridin-4-one (957.8 mg; 76 %) as a brown solid, used in next step without purification 1H NMR (DMSO-d6, 300 MHz): δ 7.36 (m, 7H) ; 6.09 (brs, 1H) ; 5.54 (br s, 1H) ; 5.01 (s, 2H) ; 4.33 (s, 2H).

### Step C: (5-benzyloxy-4-methoxy-2-pyridyl)methanol

Trimethylsilyl diazomethane (2.00 mol/l in diethyl ether, 4.11 ml; 8.22 mmol; 2.00 eq.) was added to a solution of 5-benzyloxy-2-(hydroxymethyl)-1H-pyridin-4-one (950.0 mg; 4.11 mmol; 1.00 eq.) in a mixture of MeOH (9.00 ml) and DMF (30.00 ml). The mixture was stirred 18h at rt and then extracted with EtOAc. The organic layer was washed with brine, dried over MgSO₄, filtered and concentrated. The residue was purified by chromatography (column SiO₂ 50g, flow rate 40ml/min, gradient from 100% DCM to 95/5 DCM / MeOH) to give (5-benzyloxy-4-methoxy-2-pyridyl)methanol (535.2 mg; 53 %) as a beige solid. 1H NMR (DMSO-d6, 300 MHz): δ 8.08 (s, 1H) ; 7.39 (m, 5H) ; 7.09 (s, 1H) ; 5.32 (t, J = 5.7 Hz, 1H) ; 5.13 (s, 2H) ; 4.44 (d, J = 5.6 Hz, 2H) ; 3.85 (s, 3H).

### Step D: 5-benzyloxy-4-methoxy-pyridine-2-carbaldehyde

Manganese(IV) oxide (1.70 g; 19.57 mmol; 10.00 eq.) was added to a solution of (5-benzyloxy-4-methoxy-2-pyridyl)methanol (480.0 mg; 1.96 mmol; 1.00 eq.) in DCM (9.60 ml) and the reaction was stirred 48h at rt. After filtration, solids were washed with DCM and the filtrate was concentrated under vacuum to give 5-benzyloxy-4-methoxy-pyridine-2-carbaldehyde (334.4 mg; 70 %) as an orange solid. 1H NMR (DMSO-d6, 300 MHz): δ 9.83 (s, 1H) ; 8.51 (s, 1H) ; 7.52 (s, 1H) ; 7.47 (m, 5H) ; 5.34 (s, 2H) ; 3.94 (s, 3H).

### Step E: 5-hydroxy-4-methoxy-pyridine-2-carbaldehyde

5-benzyloxy-4-methoxy-pyridine-2-carbaldehyde (70.0 mg; 0.29 mmol; 1.00 eq.) was dissolved in MeOH (1.40 ml). Then Pd/C 10% (24.5 mg; 0.12 mmol; 0.40 eq.) was added and the mixture was stirred 3h30 at rt under hydrogen atmosphere (1atm). The mixture was filtered over Whatman. Solids were washed with MeOH and the filtrate concentrated. The residue was purified by chromatography (column SiO₂ 5g, flow rate 15ml/min, gradient from 100% DCM to 95/5 DCM / MeOH) to give 5-hydroxy-4-methoxy-pyridine-2-carbaldehyde (35.2 mg; 80 %) as a white solid. 1H NMR (DMSO-d6, 300 MHz): δ 10.67 (br s, 1H) ; 9.79 (s, 1H) ; 8.21 (s, 1H) ; 7.49 (s, 1H) ; 3.93 (s, 3H).

### Step F: 6-[(E)-[(1,1-dioxo-1,2-benzothiazol-3-yl)-methyl-hydrazono]methyl]-4-methoxy-pyridin-3-ol

The compound was prepared using the same procedure as described in example 1 step C starting from 1-(1,1-dioxo-1,2-benzothiazol-3-yl)-1-methyl-hydrazine (example 1, step B, 40.0 mg; 0.19 mmol; 1.00 eq.) and 5-hydroxy-4-methoxy-pyridine-2-carbaldehyde (34.8 mg; 0.23 mmol; 1.20 eq.) giving 6-[(E)-[(1,1-dioxo-1,2-benzothiazol-3-yl)-methyl-hydrazono]methyl]-4-methoxy-pyridin-3-ol (9.4 mg; 14 %) as a pale yellow solid. 1H NMR (DMSO-d6, 400 MHz): δ 10.17 (br s, 1H) ; 9.05 (m, 1H) ; 8.27 (d, J = 0.4 Hz, 1H) ; 8.16 (s, 1H) ; 8.09 (m, 1H); 7.93 (dt, J = 1.5 Hz, J = 7.7 Hz, 1H); 7.89 (dt, J = 1.3 Hz, J = 7.5 Hz, 1H) ; 7.54 (s, 1H) ; 3.98 (s, 3H) ; 3.80 (s, 3H).

### Example 37: N-[(E)-(6-chloro-5-fluoro-2-pyridyl)methyleneamino]-N-methyl-1,1-dioxo-1,2-benzothiazol-3-amine

### Step A: (6-chloro-5-fluoro-2-pyridyl)methanol

6-Chloro-5-fluoropicolinic acid (1.50 g; 8.38 mmol; 1.00 eq.) was dissolved in anhydrous THF (42.00 ml) then lithium aluminium hydride (477.0 mg; 12.57 mmol; 1.50 eq.) was added portion-wise at 0 °C. The mixture was stirred 18h at rt and diluted with EtOAc, filtered through a short pad of Celite^{™} and washed with additional EtOAc. The filtrate was concentrated under vacuum to give (6-chloro-5-fluoro-2-pyridyl)methanol (1.36 g; quant.) as a yellow oil, which was used in the next step without purification. The product was not detected by LCMS analysis.

### Step B: 6-chloro-5-fluoro-pyridine-2-carbaldehyde

A suspension of (6-chloro-5-fluoro-2-pyridyl)methanol (1.36 g; 8.43 mmol; 1.00 eq.) in acetone (42.00 ml) was purged with argon, then pyridinium dichromate (3.56 g; 9.27 mmol; 1.10 eq.) was added in small portions. The mixture was stirred 18h at rt and filtered through a short pad of Celite^{™}. Solids were washed with additional DCM and the filtrate was concentrated under vacuum to give 6-chloro-5-fluoro-pyridine-2-carbaldehyde (1.45 g, quant.) as a brown solid, which was taken into the next step without purification. The product was not detected by LCMS analysis.

### Step C: N-[(E)-(6-chloro-5-fluoro-2-pyridyl)methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 200.0 mg; 0.95 mmol; 1.00 eq.) and 6-chloro-5-fluoro-pyridine-2-carbaldehyde (302.0 mg; 1.89 mmol; 2.00 eq.) giving N-[(E)-(6-chloro-5-fluoro-2-pyridyl)methyleneamino]-N-methyl-1,1-dioxo-1,2-benzothiazol-3-amine (93.0 mg; 28 %) as a white solid. 1H NMR (DMSO-d6, 300 MHz): δ 9.10 (m, 1H) ; 8.39 (s, 1H) ; 8.09 (m, 3H) ; 7.89 (m, 2H), 3.80 (s, 3H). mp: 219-221 °C.

### Example 38: N-ethyl-N-[(E)-(4-fluoro-3-methoxy-phenyl)methyleneamino]-1,1-dioxo-1,2-benzothiazol-3-amine

### Step A: N-[(E)-(4-fluoro-3-methoxy-phenyl)methyleneamino]ethanamine

The compound was prepared using the same procedure as described in example 21 step A starting from 4-fluoro-3-methoxybenzaldehyde (558.7 mg; 3.62 mmol; 1.00 eq.) and ethylhydrazine hydrochloride (350.0 mg; 3.62 mmol; 1.00 eq.) giving N-[(E)-(4-fluoro-3-methoxy-phenyl)methyleneamino]ethanamine (711.2 mg; quant.) as a white solid, used directly in next step without purification. 1H NMR (DMSO-d6, 300 MHz): δ 8.08 (s, 1H) ; 7.41 (dd, J = 1.8 Hz, J = 8.6 Hz, 1H); 7.25 (dd, J = 8.4 Hz, J = 11.1 Hz, 1H); 7.19 (ddd, J = 1.8 Hz, J = 4.6 Hz, J = 8.4 Hz, 1H) ; 3.87 (s, 3H); 3.19 (q, J = 7.2 Hz, 2H); 1.21 (t, J = 7.2 Hz, 3H).

### Step B: N-ethyl-N-[(E)-(4-fluoro-3-methoxy-phenyl)methyleneamino]-1,1-dioxo-1,2-benzothiazol-3-amine

The compound was prepared using the same procedure as described in example 21 step B starting from 3-chloro-1,2-benzothiazole 1,1-dioxide (example 21, step A, 729.3 mg; 3.93 mmol; 1.00 eq.) and N-[(E)-(4-fluoro-3-methoxy-phenyl)methyleneamino]ethanamine (771.0 mg; 3.93 mmol; 1 eq.) giving N-ethyl-N-[(E)-(4-fluoro-3-methoxy-phenyl) methyleneamino]-1,1-dioxo-1,2-benzothiazol-3-amine (627.0 mg; 44 %) as a white solid. 1H NMR (DMSO-d6, 400 MHz): δ 8.81 (m, 1H) ; 8.09 (m, 1H) ; 7.92 (dt, J = 1.5 Hz, J = 7.5 Hz, 1H) ; 7.89 (dt, J = 1.1 Hz, J = 6.8 Hz, 1H) ; 7.67 (m, 1H) ; 7.66 (dd, J = 2.0 Hz, J = 8.4 Hz, 1H) ; 7.49 (ddd, J = 1.8 Hz, J = 4.6 Hz, J = 8.4 Hz, 1H) ; 7.42 (dd, J = 8.1 Hz, J = 10.8 Hz, 1H) ; 4.40 (q, J = 7.0 Hz, 2H) ; 3.97 (s, 3H) ; 1.31 (t, J = 7.0 Hz, 3H). mp: 199-206°C.

### Example 39: N-[(E)-(4-fluoro-3-methoxy-phenyl)methyleneamino]-N-isopropyl-1,1-dioxo-1,2-benzothiazol-3-amine

### Step A: N-[(E)-(4-fluoro-3-methoxy-phenyl)methyleneamino]propan-2-amine

The compound was prepared using the same procedure as described in example 21 step A starting from 4-fluoro-3-methoxybenzaldehyde (100.0 mg; 0.65 mmol; 1.00 eq.) and isopropylhydrazine hydrochloride (71.7 mg; 0.65 mmol; 1.00 eq.) giving N-[(E)-(4-fluoro-3-methoxy-phenyl)methyleneamino]propan-2-amine (158.0 mg; quant.) as a white solid, used directly in next step without purification. 1H NMR (DMSO-d6, 300 MHz): δ 7.60 (s, 1H); 7.26 (dd, J = 1.8 Hz, J = 8.6 Hz, 1H); 7.14 (dd, J = 8.3 Hz, J = 11.3 Hz, 1H); 7.00 (ddd, J = 2.0 Hz, J = 4.6 Hz, J = 8.4 Hz, 1H) ; 3.84 (s, 3H) ; 3.43 (m, 1H), 1.11 (d, J = 6.3 Hz, 6H).

### Step B: N-[(E)-(4-fluoro-3-methoxy-phenyl)methyleneamino]-N-isopropyl-1,1-dioxo-1,2-benzothiazol-3-amine

The compound was prepared using the same procedure as described in example 21 step B starting from 3-chloro-1,2-benzothiazole 1,1-dioxide (example 1, step A, 286.9 mg; 1.43 mmol; 2.20 eq.) and N-[(E)-(4-fluoro-3-methoxy-phenyl)methyleneamino]propan-2-amine (136.0 mg; 0.65 mmol; 1.00 eq.) giving N-[(E)-(4-fluoro-3-methoxyphenyl)methyleneamino]-N-isopropyl-1,1-dioxo-1,2-benzothiazol-3-amine (55.0 mg; 23 %) as a white solid. 1H NMR (DMSO-d6, 400 MHz): δ 8.72 (s, 1H) ; 8.47 (m, 1H) ; 8.07 (m, 1H) ; 7.85 (m, 2H) ; 7.70 (dd, J = 1.9 Hz, J = 8.5 Hz, 1H) ; 7.51 (ddd, J = 2.0 Hz, J = 4.4 Hz, J = 8.4 Hz, 1H); 7.43 (dd, J = 8.4 Hz, J = 10.8 Hz, 1H); 5.18 (sept, J = 6.9 Hz, 1H) ; 3.95 (s, 3H); 1.57 (d, J = 7.0 Hz, 6H). mp: 149-166°C.

### Example 40: N-[(E)-(4-fluoro-3-methoxy-phenyl)methyleneamino]-N-isobutyl-1,1-dioxo-1,2-benzothiazol-3-amine

### Step A: N-[(E)-(4-fluoro-3-methoxy-phenyl)methyleneamino]-2-methyl-propan-1 -amine

The compound was prepared using the same procedure as described in example 21 step A starting from 4-fluoro-3-methoxybenzaldehyde (100.0 mg; 0.65 mmol; 1.00 eq.) and 2-methylpropylhydrazine hydrochloride (80.78 mg; 0.65 mmol; 1.00 eq.) giving N-[(E)-(4-fluoro-3-methoxy-phenyl)methyleneamino]-2-methyl-propan-1-amine (146.0 mg; quant.) as a pale yellow solid, used directly in next step without purification. 1H NMR (DMSO-d6, 300 MHz): δ 7.50 (s, 1H) ; 7.26 (dd, J = 1.9 Hz, J = 8.7 Hz, 1H) ; 7.13 (dd, J = 8.3 Hz, J = 11.4 Hz, 1H) ; 6.98 (ddd, J = 2.0 Hz, J = 4.5 Hz, J = 8.4 Hz, 1H) ; 3.83 (s, 3H) ; 2.92 (d, J = 6.6 Hz, 2H) ; 1.86 (m, 1H) ; 0.92 (d, J = 6.6 Hz, 6H).

### Step B: N-[(E)-(4-fluoro-3-methoxy-phenyl)methyleneamino]-N-isobutyl-1,1-dioxo-1,2-benzothiazol-3-amine

The compound was prepared using the same procedure as described in example 21 step B starting from 3-chloro-1,2-benzothiazole 1,1-dioxide (example 1, step A, 156.4 mg; 0.78 mmol; 1.20 eq.) and N-[(E)-(4-fluoro-3-methoxy-phenyl)methyleneamino]-2-methyl-propan-1-amine (145.0 mg; 0.65 mmol; 1.00 eq.) giving N-[(E)-(4-fluoro-3-methoxyphenyl)methyleneamino]-N-isobutyl-1,1-dioxo-1,2-benzothiazol-3-amine (41.0 mg; 16 %) as a white solid. 1H NMR (DMSO-d6, 400 MHz): δ 8.83 (m, 1H) ; 8.54 (s, 1H) ; 8.09 (m, 1H) ; 7.92 (dt, J = 1.5 Hz, J = 7.3 Hz, 1H) ; 7.89 (dt, J = 1.3 Hz, J = 7.5 Hz, 1H) ; 7.66 (dd, J = 2.0 Hz, J = 8.6 Hz, 1H) ; 7.49 (ddd, J = 2.0 Hz, J = 4.6 Hz, J = 8.6 Hz, 1H) ; 7.42 (dd, J = 8.1 Hz, J = 11.4 Hz, 1H); 4.27 (d, J = 7.7 Hz, 2H); 3.97 (s, 3H); 2.28 (sept, J = 6.8 Hz, 1H) ; 0.99 (d, J = 6.8 Hz, 6H). mp: 182-184°C.

### Example 41: 2-[(1,1-dioxo-1,2-benzothiazol-3-yl)-[(E)-(4-fluoro-3-methoxy-phenyl) methyleneamino]amino]ethanol

### Step A: 2-[(2E)-2-[(4-fluoro-3-methoxy-phenyl)methylene]hydrazino]ethanol

The compound was prepared using the same procedure as described in example 21 step A starting from 4-fluoro-3-methoxybenzaldehyde (3.00 g; 19.46 mmol; 1.00 eq.) and 2-hydroxyethyl hydrazine (1.45 ml; 21.41 mmol; 1.10 eq.) giving 2-[(2E)-2-[(4-fluoro-3-methoxy-phenyl)methylene]hydrazino]ethanol (4.13 g; quant.) as a pale yellow paste, used directly in the next step without purification. 1H NMR (DMSO-d6, 300 MHz): δ 7.56 (s, 1H) ; 7.27 (dd, J = 1.8 Hz, J = 8.6 Hz, 1H) ; 7.14 (dd, J = 8.3 Hz, J = 11.4 Hz, 1H); 6.99 (ddd, J = 2.0 Hz, J = 4.8 Hz, J = 8.6 Hz, 1H) ; 3.84 (s, 3H) ; 3.56 (t, J = 5.9 Hz, 2H) ; 3.18 (m, 2H).

### Step B: 2-[(1,1-dioxo-1,2-benzothiazol-3-yl)-[(E)-(4-fluoro-3-methoxy-phenyl)methylene amino]amino]ethanol

The compound was prepared using the same procedure as described in example 21 step B starting from 3-chloro-1,2-benzothiazole 1,1-dioxide (example 1, step A, 3.57 g; 17.69 mmol; 1.00 eq.) and N-[(E)-(4-fluoro-3-methoxy-phenyl)methyleneamino]-2-methyl-propan-1-amine (4.13 g; 19.46 mmol; 1.10 eq.) giving 2-[(1,1-dioxo-1,2-benzothiazol-3-yl)-[(E)-(4-fluoro-3-methoxy-phenyl)methyleneamino]amino]ethanol (4.75 g; 71 %) as a yellow solid. 1H NMR (DMSO-d6, 400 MHz): δ 8.80 (m, 1H) ; 8.66 (s, 1H) ; 8.09 (m, 1H) ; 7.93 (dt, J = 1.3 Hz, J = 7.5 Hz, 1H) ; 7.91 (dt, J = 1.1 Hz, J = 7.5 Hz, 1H) ; 7.63 (dd, J = 1.8 Hz, J = 8.4 Hz, 1H) ; 7.43 (m, 2H) ; 5.14 (t, J = 5.6 Hz, 1H) ; 4.45 (t, J = 5.8 Hz, 2H) ; 3.96 (s, 3H) ; 3.80 (q, J = 5.9 Hz, 2H). mp: 194-237°C.

### Example 42: N-[(E)-(4-fluoro-3-methoxy-phenyl)methyleneamino]-N-(2-methoxyethyl)-1,1-dioxo-1,2-benzothiazol-3-amine

### Step A: N-[(E)-(4-fluoro-3-methoxy-phenyl)methyleneamino]-2-methoxy-ethanamine

The compound was prepared using the same procedure as described in example 21 step A starting from 4-fluoro-3-methoxybenzaldehyde (200.0 mg; 1.30 mmol; 1.00 eq.) and (2-methoxyethyl)hydrazine hydrochloride (180.7 mg; 1.43 mmol; 1.10 eq.) giving N-[(E)-(4-fluoro-3-methoxy-phenyl)methyleneamino]-2-methoxy-ethanamine (359.3 mg; quant.) as an orange paste, used directly in the next step without purification. LC/MS (Method a): Rt = 1.65 min ; MS : m/z = 227 [M+H]⁺

### Step B: N-[(E)-(4-fluoro-3-methoxy-phenyl)methyleneamino]-N-(2-methoxyethyl)-1,1-dioxo-1,2-benzothiazol-3-amine

The compound was prepared using the same procedure as described in example 21 step B starting from 3-chloro-1,2-benzothiazole 1,1-dioxide (example 1, step A, 70.0 mg; 0.35 mmol; 1.00 eq.) and N-[(E)-(4-fluoro-3-methoxy-phenyl)methyleneamino]-2-methoxy-ethanamine (94.3 mg; 0.42 mmol; 1.20 eq.) giving N-[(E)-(4-fluoro-3-methoxyphenyl)methyleneamino]-N-(2-methoxyethyl)-1,1-dioxo-1,2-benzothiazol-3-amine (34.7 mg; 25 %) as a pink solid. 1H NMR (DMSO-d6, 400 MHz): δ 8.80 (m, 1H) ; 8.61 (s, 1H) ; 8.10 (m, 1H) ; 7.91 (m, 2H) ; 7.63 (dd, J = 1.8 Hz, J = 8.4 Hz, 1H) ; 7.46 (ddd, J = 2.0 Hz, J = 4.6 Hz, J = 8.6 Hz, 1H) ; 7.41 (dd, J = 8.4 Hz, J = 11.2 Hz, 1H); 4.58 (t, J = 5.6 Hz, 2H) ; 3.97 (s, 3H) ; 3.73 (t, J = 5.7 Hz, 2H) ; 3.30 (s, 3H).

### Example 43: N-(2-ethoxyethyi)-N-[(E)-(4-fluoro-3-methoxy-phenyl)methyleneamino]-1,1-dioxo-1,2-benzothiazol-3-amine

### Step A: tert-butyl N-[(E)-(4-fluoro-3-methoxy-phenyl)methyleneamino]carbamate

The compound was prepared using the same procedure as described in example 23 step F starting from 4-fluoro-3-methoxybenzaldehyde (10.00 g; 64.88 mmol; 1.00 eq.) giving tert-butyl N-[(E)-(4-fluoro-3-methoxy-phenyl)methyleneamino]carbamate (17.06 g; 98 %) as a white solid. ¹H NMR (DMSO-d6, 300 MHz): δ 10.94 (br s, 1H) ; 7.97 (s, 1H) ; 7.38 (dd, J = 1.9 Hz, J = 8.5 Hz, 1H) ; 7.24 (dd, J = 8.8 Hz, J = 11.1 Hz, 1H) ; 7.15 (ddd, J = 1.8 Hz, J = 4.6 Hz, J = 8.4 Hz, 1H) ; 3.88 (s, 3H) ; 1.47 (s, 9H).

### Step B: tert-butyl N-(2-ethoxyethyl)-N-[(E)-(4-fluoro-3-methoxy-phenyl)methylene amino]carbamate

The compound was prepared using the same procedure as described in example 23 step H starting from tert-butyl N-[(E)-(4-fluoro-3-methoxy-phenyl)methyleneamino]carbamate (200.0 mg; 0.75 mmol; 1.00 eq.) and 2-bromoethyl ethyl ether (93 µL; 0.82 mmol; 1.10 eq.) giving tert-butyl N-(2-ethoxyethyl)-N-[(E)-(4-fluoro-3-methoxy-phenyl)-methyleneamino] carbamate (253.9 mg; quant.) as a yellow oil. ¹H NMR (DMSO-d6, 300 MHz): δ 8.04 (s, 1H) ; 7.49 (dd, J = 1.4 Hz, J = 8.5 Hz, 1H) ; 7.25 (m, 2H) ; 4.02 (m, 2H) ; 3.87 (s, 3H) ; 3.52 (t, J = 5.9 Hz, 2H) ; 3.44 (q, J = 6.9 Hz, 2H) ; 1.49 (s, 9H) ; 1.08 (t, J = 7.0 Hz, 3H).

### Step C: 2-ethoxy-N-[(E)-(4-fluoro-3-methoxy-phenyl)methyleneamino]ethanamine

The compound was prepared using the same procedure as described in example 23 step I starting from tert-butyl N-(2-ethoxyethyl)-N-[(E)-(4-fluoro-3-methoxy-phenyl)methylene amino]carbamate (253.0 mg; 0.74 mmol; 1.00 eq.) giving 2-ethoxy-N-[(E)-(4-fluoro-3-methoxy-phenyl)methyleneamino]ethanamine (178.0 mg; quant.) as a yellow oil, used in the next step without purification. ¹H NMR (DMSO-d6, 300 MHz): δ 8.02 (s, 1H) ; 7.39 (dd, J = 1.8 Hz, J = 8.6 Hz, 1H); 7.24(dd, J = 8.4 Hz, J = 11.2 Hz, 1H); 7.17 (ddd, J = 1.8 Hz, J = 4.6 Hz, J = 8.3 Hz, 1H) ; 3.87 (s, 3H); 3.60 (t, J = 5.8 Hz, 2H) ; 3.47 (q, J = 7.0 Hz, 2H); 3.34 (t, J = 5.6 Hz, 2H) ; 1.13 (t, J = 6.9 Hz, 3H).

### Step D: N-(2-ethoxyethyl)-N-[(E)-(4-fluoro-3-methoxy-phenyl)methyleneamino]-1,1-dioxo-1,2-benzothiazol-3-amine

The compound was prepared using the same procedure as described in example 21 step B starting from 3-chloro-1,2-benzothiazole 1,1-dioxide (example 1, step A, 179.8 mg; 0.89 mmol; 1.20 eq.) and 2-ethoxy-N-[(E)-(4-fluoro-3-methoxy-phenyl)methyleneamino] ethanamine (178.0 mg; 0.74 mmol; 1.00 eq.) giving N-(2-ethoxyethyl)-N-[(E)-(4-fluoro-3-methoxy-phenyl)methyleneamino]-1,1-dioxo-1,2-benzothiazol-3-amine (184.5 mg; 61 %) as a white solid. 1H NMR (DMSO-d6, 400 MHz): δ 8.81 (m, 1H) ; 8.63 (s, 1H) ; 8.10 (m, 1H) ; 7.91 (m, 2H), 7.62 (dd, J = 1.7 Hz, J = 8.5 Hz, 1H) ; 7.43 (m, 2H) ; 4.56 (t, J = 5.6 Hz, 2H) ; 3.97 (s, 3H) ; 3.77 (t, J = 5.6 Hz, 2H) ; 3.50 (q, J = 6.9 Hz, 2H) ; 1.07 (t, J = 6.9 Hz, 3H). mp: 170-172°C.

### Example 44: 3-[(1,1-dioxo-1,2-benzothiazol-3-yl)-[(E)-(4-fluoro-3-methoxy-phenyl) methyleneamino]amino]propan-1-ol

### Step A: tert-butyl N-[3-[tert-butyl(dimethyl)silyl]oxypropyl]-N-[(E)-(4-fluoro-3-methoxyphenyl)methyleneamino]carbamate

The compound was prepared using the same procedure as described in example 23 step H starting from tert-butyl N-[(E)-(4-fluoro-3-methoxy-phenyl)methyleneamino]carbamate (example 43, step A, 300 mg; 1.12 mmol; 1.0 eq.) and (3-bromopropoxy)-tert-butyl dimethylsilane (389 µL; 1.68 mmol; 1.5 eq.) giving tert-butyl N-[3-[tert-butyl (dimethyl)silyl]oxypropyl]-N-[(E)-(4-fluoro-3-methoxy-phenyl)methyleneamino] carbamate (501.2 mg; 74 %) as a colorless oil. ¹H NMR (DMSO-d6, 300 MHz): δ 7.89 (s, 1H) ; 7.42 (dd, J = 1.7 Hz, J = 8.5 Hz, 1H) ; 7.20 (m, 2H) ; 3.85 (t, J = 6.0 Hz, 2H) ; 3.82 (s, 3H); 3.60 (t, J = 6.0 Hz , 2H) ; 1.65 (quint., J = 6.0 Hz, 2H) ; 1.44 (s, 9H) ; 0.83 (s, 9H) ; 0.00 (s, 6H).

### Step B: 3-[(2E)-2-[(4-fluoro-3-methoxy-phenyl)methylene]hydrazino]propan-1-ol

The compound was prepared using the same procedure as described in example 23 step I starting from tert-butyl N-[3-[tert-butyl(dimethyl)silyl]oxypropyl]-N-[(E)-(4-fluoro-3-methoxy-phenyl)methyleneamino]carbamate (200.0 mg; 0.33 mmol; 1.00 eq.) giving 3-[(2E)-2-[(4-fluoro-3-methoxy-phenyl)methylene]hydrazino]propan-1-ol (75.0 mg; quant.) as a yellow oil, used in the next step without purification. ¹H NMR (DMSO-d₆, 300 MHz): δ 8.35 (br s, 1H) ; 7.48 (m, 1H) ; 7.32 (m, 2H) ; 3.89 (s, 3H) ; 3.49 (m, 2H); 3.36 (m, 2H) ; 1.82 (m, 2H).

### Step C: 3-[(1,1-dioxo-1,2-benzothiazol-3-yl)-[(E)-(4-fluoro-3-methoxy-phenyl)methylene amino]amino]propan-1-ol

The compound was prepared using the same procedure as described in example 21 step B starting from 3-chloro-1,2-benzothiazole 1,1-dioxide (example 1, step A, 100.2 mg; 0.50 mmol; 1.50 eq.) and 3-[(2E)-2-[(4-fluoro-3-methoxy-phenyl)methylene]hydrazino]propan-1-ol (75.0 mg; 0.33 mmol; 1.00 eq.) giving 3-[(1,1-dioxo-1,2-benzothiazol-3-yl)-[(E)-(4-fluoro-3-methoxy-phenyl)methyleneamino]amino]propan-1-ol (87.1 mg; 67 %) as a white solid. 1H NMR (DMSO-d6, 400 MHz): δ 8.81 (m, 1H) ; 8.53 (s, 1H) ; 8.09 (m, 1H) ; 7.92 (dt, J = 1.5 Hz, J = 7.5 Hz, 1H); 7.89 (dt, J = 1.3 Hz, J = 7.0 Hz, 1H); 7.64 (dd, J = 1.9 Hz, J = 8.5 Hz, 1H) ; 7.47 (ddd, J = 2.0 Hz, J = 4.8 Hz, J = 8.4 Hz, 1H) ; 7.42 (dd, J = 8.4 Hz, J = 11.2 Hz, 1H) ; 4.74 (t, J = 5.2 Hz, 1H) ; 4.40 (t, J = 7.7 Hz, 2H); 3.97 (s, 3H); 3.57 (q, J = 6.2 Hz, 2H) ; 1.89 (m, 2H). mp: 172°C.

### Example 45: 2-(chloromethyl)-3-[(1,1-dioxo-1,2-benzothiazol-3-yl)-[(E)-(4-fluoro-3-methoxy-phenyl)methyleneamino]amino]propan-1-ol

### Step A: tert-butyl N-[(E)-(4-fluoro-3-methoxy-phenyl)methyleneamino]-N-(oxetan-3-ylmethyl)carbamate

The compound was prepared using the same procedure as described in example 23 step H starting from tert-butyl N-[(E)-(4-fluoro-3-methoxy-phenyl)methyleneamino]carbamate (example 43, step A, 161.0 mg; 0.60 mmol; 1.00 eq.) and 3-bromomethyl-oxetane (99.7 mg; 0.66 mmol; 1.10 eq.) giving tert-butyl N-[(E)-(4-fluoro-3-methoxy-phenyl) methyleneamino]-N-(oxetan-3-ylmethyl)carbamate (175.0 mg; 86 %) as a colorless oil. ¹H NMR (DMSO-d₆, 300 MHz): δ 8.00 (s, 1H) ; 7.48 (dd, J = 1.6 Hz, J = 8.7 Hz, 1H) ; 7.26 (m, 2H) ; 4.62 (dd, J = 6.0 Hz, J = 7.8 Hz, 2H) ; 4.39 (t, J = 6.1 Hz, 2H); 4.17 (d, J = 7.1 Hz, 2H) ; 3.86 (s, 3H) ; 3.30 (m, 1H) ; 1.49 (s, 9H).

### Step B: 2-(chloromethyl)-3-[(2E)-2-[(4-fluoro-3-methoxy-phenyl)methylene]hydrazino] propan-1-ol

The compound was prepared using the same procedure as described in example 23 step I starting from tert-butyl N-[(E)-(4-fluoro-3-methoxy-phenyl)methyleneamino]-N-(oxetan-3-ylmethyl)carbamate (175.0 mg; 0.52 mmol; 1.00 eq.) giving 3-[(2E)-2-[(4-fluoro-3-methoxy-phenyl)methylene]hydrazino]propan-1-ol (123.2 mg; 86 %) as a yellow oil, used in the next step without purification. LC/MS (Method a): Rt = 1.77 min ; MS : m/z = 275 [M+H]⁺

### Step C: 2-(chloromethyl)-3-[(1,1-dioxo-1,2-benzothiazol-3-yl)-[(E)-(4-fluoro-3-methoxyphenyl)methyleneamino]amino]propan-1-ol

The compound was prepared using the same procedure as in example 21 step B starting from 3-chloro-1,2-benzothiazole 1,1-dioxide (example 1, step A, 125.1 mg; 0.62 mmol; 1.38 eq.) and 2-(chloromethyl)-3-[(2E)-2-[(4-fluoro-3-methoxy-phenyl) methylene] hydrazino] propan-1-ol (123.2 mg; 0.45 mmol; 1 eq.) giving 2-(chloromethyl)-3-[(1,1-dioxo-1,2-benzo thiazol-3-yl)-[(E)-(4-fluoro-3-methoxy-phenyl)methyleneamino]amino]propan-1-ol (58.4 mg; 26 %) as a pale yellow solid. 1H NMR (DMSO-d6, 400 MHz): δ 8.81 (m, 1H) ; 8.54 (s, 1H) ; 8.10 (m, 1H) ; 7.92 (ddd, J = 1.3 Hz, J = 7.5 Hz, J = 15.0 Hz, 1H) ; 7.90 (ddd, J = 1.3 Hz, J = 7.3 Hz, J = 14.5 Hz, 1H); 7.62 (dd, J = 1.4 Hz, J = 8.5 Hz, 1H); 7.43 (m, 2H) : 4.97 (br s, 1H) ; 4.47 (m, 2H) ; 3.97 (s, 3H) ; 3.84 (m, 2H) ; 3.58 (d, J = 5.3 Hz, 2H) ; 2.47 (m, 1H).

### Example 46: N-[(E)-(4-fluoro-3-methoxy-phenyl)methyleneamino]-1,1-dioxo-N-(2-phenyl ethyl)-1,2-benzothiazol-3-amine

### Step A: tert-butyl N-[(E)-(4-fluoro-3-methoxy-phenyl)methyleneamino]-N-(2-phenyl ethyl)carbamate

The compound was prepared using the same procedure as in example 23 step H starting from tert-butyl N-[(E)-(4-fluoro-3-methoxy-phenyl)methyleneamino]carbamate (example 43, step A, 100.0 mg; 0.37 mmol; 1. eq.) and (2-bromoethyl)benzene (100 µL; 0.74 mmol; 1.50 eq.) giving tert-butyl N-[(E)-(4-fluoro-3-methoxy-phenyl)methyleneamino]-N-(2-phenylethyl) carbamate (83.0 mg; 60 %). LC/MS (Method D): Rt = 2.75 min ; MS : m/z = 317 [M+H-*t*Bu]⁺

### Step B: N-[(E)-(4-fluoro-3-methoxy-phenyl)methyleneamino]-2-phenyl-ethanamine

The compound was prepared using the same procedure as described in example 23 step I starting from tert-butyl N-[(E)-(4-fluoro-3-methoxy-phenyl)methyleneamino]-N-(2-phenylethyl)carbamate (83.0 mg; 0.22 mmol; 1.00 eq.) giving N-[(E)-(4-fluoro-3-methoxyphenyl)methyleneamino]-2-phenyl-ethanamine (61.0 mg; quant.) as a yellow solid, used in the next step without purification. LC/MS (Method B): Rt = 3.07 min ; MS : m/z = 273 [M+H]⁺

### Step C: N-[(E)-(4-fluoro-3-methoxy-phenyl)methyleneamino]-1,1-dioxo-N-(2-phenyl ethyl)-1,2-benzothiazol-3-amine

The compound was prepared using the same procedure as described in example 21 step B starting from 3-chloro-1,2-benzothiazole 1,1-dioxide (example 1, step A, 99.0 mg; 0.49 mmol; 2.20 eq.) and N-[(E)-(4-fluoro-3-methoxy-phenyl)methyleneamino]-2-phenyl-ethanamine (61.0 mg; 0.22 mmol; 1.00 eq.) giving N-[(E)-(4-fluoro-3-methoxyphenyl)methyleneamino]-1,1-dioxo-N-(2-phenylethyl)-1,2-benzothiazol-3-amine (55.0 mg, 56 %) as a white solid. 1H NMR (DMSO-d6, 300 MHz): δ 8.77 (d, J = 7.2 Hz, 1H); 8.48 (s, 1H) ; 8.09 (d, J = 7.2 Hz, 1H) ; 7.90 (m, 2H) ; 7.59 (d, J = 8.3 Hz, 1H) ; 7.32 (m, 7H); 4.57 (t, J = 7.2 Hz, 2H) ; 3.96 (s, 3H) ; 3.07 (t, J =7.2 Hz, 2H). mp: 256-258 °C.

### Example 47: N-[(E)-(4-fluoro-3-methoxy-phenyl)methyleneamino]-N-methyl-5-nitro-1,1-dioxo-1,2-benzothiazol-3-amine

Step A: N-[(E)-(4-fluoro-3-methoxy-phenyl)methyleneamino]methanamine The compound was prepared using the same procedure as described in example 21 step A starting from 4-fluoro-3-methoxybenzaldehyde (1.00 g; 6.57 mmol; 1.00 eq.) N-[(E)-(4-fluoro-3-methoxy-phenyl)methyleneamino]methanamine (2.08 g; quant.) as a beige powder, used without further purification. ¹H NMR (DMSO-d6, 300 MHz): δ 7.38 (s, 1H) ; 7.34 (m, 1H) ; 7.30 (dd, J = 2.0 Hz, J = 8.6 Hz, 1H) ; 7.14 (dd, J = 8.3 Hz, J = 11.3 Hz, 1H) ; 7.02 (ddd, J = 2.0 Hz, J = 4.8 Hz, J = 8.3 Hz, 1H) ; 3.84 (s, 3H); 2.80 (d, J=1.7 Hz, 3H).

### Step B: 3-chloro-5-nitro-1,2-benzothiazole 1,1-dioxide

5-nitro-1,1-dioxo-1,2-benzothiazol-3-one (1.00 g; 4.38 mmol; 1.00 eq.) and PCl₅ (1.00 g; 4.82 mmol; 1.10 eq.) were mixed and heated at 170°C during 1h30. Diethyl ether was added and the reaction was then concentrated under vacuum to give 3-chloro-5-nitro-1,2-benzothiazole 1,1-dioxide (1.08 g; quant.) as a beige solid, used in the next step without purification. ¹H NMR (DMSO-d₆, 300 MHz): δ 8.61 (dd, J = 2.1 Hz, J = 8.3 Hz, 1H) ; 8.42 (dd, J = 0.5 Hz, J = 2.1 Hz, 1H) ; 8.27 (dd, J = 0.5 Hz, J = 8.3 Hz, 1H).

### Step C: N-[(E)-(4-fluoro-3-methoxy-phenyl)methyleneamino]-N-methyl-5-nitro-1,1-dioxo-1,2-benzothiazol-3-amine

The compound was prepared using the same procedure as described in example 21 step B starting from 3-chloro-5-nitro-1,2-benzothiazole 1,1-dioxide (1.08 g; 4.38 mmol; 1.00 eq.) and N-[(E)-(4-fluoro-3-methoxy-phenyl)methyleneamino]methanamine (1.18 g; 6.48 mmol; 1.48 eq.) giving N-[(E)-(4-fluoro-3-methoxy-phenyl)methyleneamino]-N-methyl-5-nitro-1,1-dioxo-1,2-benzothiazol-3-amine (979.2 mg; 57 %) as a yellow solid. 1H NMR (DMSO-d6, 400 MHz): δ 9.62 (dd, J = 0.4 Hz, J = 2.0 Hz, 1H) ; 8.69 (dd, J = 2.1 Hz, J = 8.3 Hz, 1H) ; 8.56 (s, 1H) ; 8.39 (dd, J = 0.4 Hz, J = 8.4 Hz, 1H) ; 7.70 (dd, J = 1.9 Hz, J = 8.3 Hz, 1H) ; 7.51 (ddd, J = 1.8 Hz, J = 4.6 Hz, J = 8.4 Hz, 1H) ; 7.45 (dd, J = 8.4 Hz, J = 11.0 Hz, 1H) ; 3.98 (s, 3H) ; 3.84 (s, 3H).

### Example 48: N-[3-[[(E)-(4-fluoro-3-methoxy-phenyl)methyleneamino]-methyl-amino]-1,1-dioxo-1,2-benzothiazol-5-yl]acetamide

### Step A: N3-[(E)-(4-fluoro-3-methoxy-phenyl)methyleneamino]-N3-methyl-1,1-dioxo-1,2-benzothiazole-3,5-diamine

To a solution of N-[(E)-(4-fluoro-3-methoxy-phenyl)methyleneamino]-N-methyl-5-nitro-1,1-dioxo-1,2-benzothiazol-3-amine (example 47) (953.0 mg; 2.43 mmol; 1.00 eq.) in THF (28.60 ml) were added iron powder (1.36 g; 24.29 mmol; 10.00 eq.), ammonium chloride (1.04 g; 19.43 mmol; 8.00 eq.) solubilized into water (4.80 ml) and concentrated HCl (203 µl; 2.43 mmol; 1.00 eq.). The reaction mixture was stirred at 80°C for 3 days and diluted with EtOAc and MeOH. Solids were filtered and washed with EtOAc and MeOH. After concentration of the filtrate, the residue was taken up into DCM and solids were filtered and washed with DCM, water and THF. Solids were combined, dissolved into DMF and concentrated under vacuum to give N3-[(E)-(4-fluoro-3-methoxy-phenyl) methyleneamino]-N3-methyl-1,1-dioxo-1,2-benzothiazole-3,5-diamine (173.0 mg; 17 %) as an orange powder. 1H NMR (DMSO-d6, 400 MHz): δ 8.39 (s, 1H) ; 7.92 (d, J = 2.0 Hz, 1H) ; 7.60 (m, 2H) ; 7.47 (ddd, J = 2.0 Hz, J = 4.6 Hz, J = 8.4 Hz, 1H) ; 7.37 (dd, J = 8.4 Hz, J = 11.2 Hz, 1H) ; 6.87 (dd, J = 2.1 Hz, J = 8.3 Hz, 1H) ; 6.30 (br s, 2H) ; 3.95 (s, 3H) ; 3.73 (s, 3H).

### Step B: N-[3-[[(E)-(4-fluoro-3-methoxy-phenyl)methyleneamino]-methyl-amino]-1,1-dioxo-1,2-benzothiazol-5-yl]acetamide

A suspension of N3-[(E)-(4-fluoro-3-methoxy-phenyl)methyleneamino]-N3-methyl-1,1-dioxo-1,2-benzothiazole-3,5-diamine (50.0 mg; 0.14 mmol; 1.00 eq.) in acetic anhydride (1.50 ml) was stirred 1h at 140°C. The reaction was then cooled to rt. The solid was filtered, washed with EtOH several times and dried under vacuum 18h at 45°C. The solid was finally triturated into water, filtered, washed with water and dried again under vacuum at 45°C to give N-[3-[[(E)-(4-fluoro-3-methoxy-phenyl)methyleneamino]-methyl-amino]-1,1-dioxo-1,2-benzothiazol-5-yl]acetamide (10.5 mg; 18 %) as a pale yellow solid. 1H NMR (DMSO-d6, 400 MHz): δ 10.58 (s, 1H) ; 9.33 (d, J = 1.8 Hz, 1H) ; 8.45 (s, 1H) ; 8.00 (d, J = 8.4 Hz, 1H) ; 7.76 (dd, J = 2.0 Hz, J = 8.4 Hz, 1H) ; 7.66 (m, 2H) ; 7.36 (dd, J = 8.6 Hz, J = 11.2 Hz, 1H) ; 3.95 (s, 3H) ; 3.79 (s, 3H) ; 2.13 (s, 3H).

### Example 49: N3-[(E)-(4-fluoro-3-methoxy-phenyl)methyleneamino]-N3-methyl-1,1-dioxo-1,2-benzothiazole-3,6-diamine

### Step A: 3-chloro-6-nitro-1,2-benzothiazole 1,1-dioxide

The compound was prepared using the same procedure as described in example 47 step B starting from 6-nitro-1,1-dioxo-1,2-benzothiazol-3-one (200.0 mg; 0.88 mmol; 1.00 eq.) giving 3-chloro-6-nitro-1,2-benzothiazole 1,1-dioxide (213.0 mg; 99 %) as a beige solid, used in next step without purification. 1H NMR (DMSO-d6, 300 MHz): δ 8.78 (dd, J = 0.7 Hz, J = 2.0 Hz, 1H) ; 8.57 (dd, J = 2.1 Hz, J = 8.3 Hz, 1H) ; 8.06 (dd, J = 0.5 Hz, J = 8.3 Hz, 1H).

### Step B: N-[(E)-(4-fluoro-3-methoxy-phenyl)methyleneamino]-N-methyl-6-nitro-1,1-dioxo-1,2-benzothiazol-3-amine

The compound was prepared using the same procedure as described in example 21 step B starting from 3-chloro-6-nitro-1,2-benzothiazole 1,1-dioxide (210.0 mg; 0.85 mmol; 1.00 eq.) and N-[(E)-(4-fluoro-3-methoxy-phenyl)methyleneamino]methanamine (example 47, step A), (294.8 mg; 1.62 mmol; 1.90 eq.) giving N-[(E)-(4-fluoro-3-methoxyphenyl)methyleneamino]-N-methyl-6-nitro-1,1-dioxo-1,2-benzothiazol-3-amine (150.1 mg; 45 %) as a yellow solid. 1H NMR (DMSO-d6, 300 MHz): δ 9.06 (d, J = 8.8 Hz, 1H) ; 8.88 (d, J = 2.1 Hz, 1H) ; 8.77 (dd, J = 2.1 Hz, J = 8.8 Hz, 1H) ; 8.54 (s, 1H) ; 7.64 (dd, J = 1.9 Hz, J = 8.3 Hz, 1H) ; 7.53 (ddd, J = 1.8 Hz, J = 4.5 Hz, J = 8.3 Hz, 1H) ; 7.41 (dd, J = 8.3 Hz, J = 11.2 Hz, 1H) ; 3.98 (s, 3H) ; 3.82 (s, 3H).

### Step C: N3-[(E)-(4-fluoro-3-methoxy-phenyl)methyleneamino]-N3-methyl-1,1-dioxo-1,2-benzothiazole-3,6-diamine

The compound was prepared using the same procedure as described in example 48 step A starting from N-[(E)-(4-fluoro-3-methoxy-phenyl)methyleneamino]-N-methyl-6-nitro-1,1-dioxo-1,2-benzothiazol-3-amine (140.0 mg; 0.36 mmol; 1.00 eq.) giving N3-[(E)-(4-fluoro-3-methoxy-phenyl)methyleneamino]-N3-methyl-1,1-dioxo-1,2-benzothiazole-3,6-diamine (37.8 mg; 28 %) as a yellow solid. 1H NMR (DMSO-d6, 400 MHz): δ 8.43 (d, J = 8.8 Hz, 1H) ; 8.35 (s, 1H) ; 7.60 (dd, J = 1.4 Hz, J = 8.5 Hz, 1H) ; 7.40 (m, 2H) ; 6.98 (d, J = 2.2 Hz, 1H) ; 6.84 (dd, J = 2.2 Hz, J = 8.8 Hz, 1H) ; 6.67 (br s, 2H) ; 3.95 (s, 3H) ; 3.70 (s, 3H).

### Example 50: [3-[[(E)-(4-fluoro-3-methoxy-phenyl)methyleneamino]-methyl-amino]-1,1-dioxo-1,2-benzothiazol-6-yl]boronic acid

### Step A: 5-bromo-2-methyl-benzenesulfonamide

Ammonia in MeOH (25.00 ml; 7.00 mol/L; 175.00 mmol; 9.40 eq.) was added dropwise to a solution of 5-bromo-2-methylbenzenesulfonyl chloride (5.00 g; 18.55 mmol; 1.00 eq.) in THF (50 ml) and the reaction was stirred 1h at rt. Solid was filtered and washed twice with EtOAc. The filtrate was concentrated to dryness and dried under vacuum to give 5-bromo-2-methyl-benzenesulfonamide (4.94 g; 92 %) as a beige solid. ¹H NMR (DMSO-d6, 300 MHz): δ 7.95 (d, J = 2.1 Hz, 1H) ; 7.69 (dd, J = 2.1 Hz, J = 8.1 Hz, 1H) ; 7.47 (br s, 2H) ; 7.35 (d, J = 8.1 Hz, 1H), 2.54 (s, 3H)

### Step B: 4-bromo-2-sulfamoyl-benzoic acid

Potassium permanganate (6.72 g; 42.50 mmol; 2.50 eq.) was added to a suspension of 5-bromo-2-methyl-benzenesulfonamide (4.94 g; 17.00 mmol; 1.00 eq.) in 5 % aqueous NaOH (85 ml). The reaction was then stirred 5h at 100°C and cooled to rt. After filtration, the filtrate was acidified to pH 1 with HCl conc. and the solid formed was filtered. It was dissolved in EtOAc and the resulting organic layer was dried over MgSO₄, filtered and concentrated to dryness to give 4-bromo-2-sulfamoyl-benzoic acid (4.84 g; 88 %) as a beige solid. ¹H NMR (DMSO-d6, 300 MHz): δ 13.82 (br s, 1H); 8.09 (d, J = 2.0 Hz, 1H); 7.91 (dd, J = 2.0 Hz, J = 8.3 Hz, 1H) ; 7.67 (d, J = 8.3 Hz, 1H) ; 7.35 (s, 2H).

### Step C: 6-bromo-1,1-dioxo-1,2-benzothiazol-3-one

4-bromo-2-sulfamoyl-benzoic acid (4.83 g; 17.24 mmol; 1.00 eq.) was dissolved in H₂SO₄ (18 ml) and the reaction was stirred 5h at rt. The reaction was then poured onto ice (250 ml) and the solid was filtered. It was washed with water (3x) and dissolved in EtOAc. The resulting organic layer was dried over MgSO₄, filtered and concentrated to dryness to give 6-bromo-1,1-dioxo-1,2-benzothiazol-3-one (4.07 g; 89 %) as a white solid. ¹H NMR (DMSO-d6, 300 MHz): δ 8.48 (d, J = 1.7 Hz, 1H) ; 8.08 (dd, J = 1.7 Hz, J = 8.1 Hz, 1H) ; 7.85 (d, J = 8.1 Hz, 1H).

### Step D: 6-bromo-3-chloro-1,2-benzothiazole 1,1-dioxide

6-bromo-1,1-dioxo-1,2-benzothiazol-3-one (2.18 g; 8.25 mmol; 1.00 eq.) was suspended into phosphorus(V) oxychloride (19.00 ml; 201.36 mmol; 24.40 eq.) and the mixture was stirred 18h under reflux. The reaction was then concentrated to dryness and coevaporated 3 times with toluen to give 6-bromo-3-chloro-1,2-benzothiazole 1,1-dioxide (2.89 g; quant.) as a beige solid, used without further purification. ¹H NMR (DMSO-d6, 300 MHz): δ 8.57 (d, J = 1.7 Hz, 1H) ; 8.13 (dd, J = 1.7 Hz, J = 8.1 Hz, 1H) ; 7.90 (d, J = 8.1 Hz, 1H).

### Step E: 6-bromo-N-[(E)-(4-fluoro-3-methoxy-phenyl)methyleneamino]-N-methyl-1,1-dioxo-1,2-benzothiazol-3-amine

The compound was prepared using the same procedure as in example 21 step B starting from 6-bromo-3-chloro-1,2-benzothiazole 1,1-dioxide (2.12 g; 7.56 mmol; 1. eq.) and N-[(E)-(4-fluoro-3-methoxy-phenyl)methyleneamino]methanamine (example 47, step A), (1.42 g; 7.79 mmol; 1.03 eq.) giving 6-bromo-N-[(E)-(4-fluoro-3-methoxyphenyl)methylene amino]-N-methyl-1,1-dioxo-1,2-benzothiazol-3-amine (1.00 g; 31 %) as an orange powder. ¹H NMR (DMSO-d₆, 300 MHz): δ 8.73 (d, J = 8.4 Hz, 1H) ; 8.46 (m, 2H) ; 8.17 (dd, J = 1.9 Hz, J = 8.5 Hz, 1H); 7.60 (dd, J = 1.7 Hz, J = 8.4 Hz, 1H); 7.43 (m, 2H) ; 3.97 (s, 3H); 3.78 (s, 3H).

### Step F: N-[(E)-(4-fluoro-3-methoxy-phenyl)methyleneamino]-N-methyl-1,1-dioxo-6-(4,4,5,5-tetramethyl-1,3,2-dioxaborolan-2-yl)-1,2-benzothiazol-3-amine

Bis(pinacolato)diboron (1.13 g; 4.46 mmol; 2.00 eq.), potassium acetate (656.2 mg; 6.69 mmol; 3.00 eq.) and 1,1'-Bis(diphenylphosphino)ferrocene-palladium(II)dichloride dichloromethane complex (364.00 mg; 0.45 mmol; 0.20 eq.) were added to a solution of 6-bromo-N-[(E)-(4-fluoro-3-methoxy-phenyl)methyleneamino]-N-methyl-1,1-dioxo-1,2-benzo thiazol-3-amine (950.00 mg; 2.23 mmol; 1.00 eq.) in 1,4-dioxane (19 ml) under nitrogen atmosphere. The reaction was then stirred 2h under reflux. The reaction was then cooled to rt and the solid was filtered. It was washed with 1,4-dioxane, EtOAc and twice with DCM. The filtrate was then concentrated to dryness and the residue taken up into diethyl ether. The solid formed was filtered, washed twice with diethyl ether and dried under vacuum to give N-[(E)-(4-fluoro-3-methoxy-phenyl)methyleneamino]-N-methyl-1,1-dioxo-6-(4,4,5,5-tetramethyl-1,3,2-dioxaborolan-2-yl)-1,2-benzothiazol-3-amine (1.21 g; quant.) as a brown solid, used without further purification. ¹H NMR (DMSO-d6, 300 MHz): δ 8.74 (d, J = 7.9 Hz, 1H) ; 8.44 (s, 1H) ; 8.06 (d, J = 9.0 Hz, 1H) ; 8.00 (s, 1H) ; 7.63 (d, J = 8.3 Hz, 1H) ; 7.44 (m, 2H) ; 3.97 (s, 3H) ; 3.78 (s, 3H) ; 1.21 (s, 12H).

### Step G: [3-[[(E)-(4-fluoro-3-methoxy-phenyl)methyleneamino]-methyl-amino]-1,1-dioxo-1,2-benzothiazol-6-yl]boronic acid

Sodium metaperiodate (1.43 g; 6.69 mmol; 3.00 eq.) and ammonium acetate (378.0 mg; 4.90 mmol; 2.20 eq.) were added to a solution of N-[(E)-(4-fluoro-3-methoxyphenyl)methyleneamino]-N-methyl-1,1-dioxo-6-(4,4,5,5-tetramethyl-1,3,2-dioxaborolan-2-yl)-1,2-benzothiazol-3-amine (1.05 g; 2.23 mmol; 1.00 eq.) in acetone (40 ml). Water (40 ml) was then added and the reaction was stirred 3h at rt. The solid was filtered and washed once with water, EtOH and DCM. It was finally dried overnight under vacuum at 50°C to give [3-[[(E)-(4-fluoro-3-methoxy-phenyl)methyleneamino]-methyl-amino]-1,1-dioxo-1,2-benzothiazol-6-yl]boronic acid (357.80 mg; 39 %) as a beige powder. ¹H NMR (DMSO-d6, 400 MHz): δ 8.82 (d, J = 7.7 Hz, 1H); 8.66 (s, 2H); 8.47 (s, 1H); 8.34 (s, 1H); 8.25 (d, J = 7.5 Hz, 1H) ; 7.65 (d, J = 8.4 Hz, 1H) ; 7.48 (s, 1H) ; 7.42 (m, 1H) ; 3.98 (s, 3H) ; 3.80 (s, 3H).

### Example 51: 3-[[(E)-(4-fluoro-3-methoxy-phenyl)methyleneamino]-methyl-amino]-1,1-dioxo-1,2-benzothiazol-6-ol

N-[(E)-(4-fluoro-3-methoxy-phenyl)methyleneamino]-N-methyl-1,1-dioxo-6-(4,4,5,5-tetramethyl-1,3,2-dioxaborolan-2-yl)-1,2-benzothiazol-3-amine (example 50, step F), (1.60 g; 3.38 mmol; 1.00 eq.) was suspended in THF (64 ml). Hydrogen peroxide (479 µL; 8.45 mmol; 2.50 eq.) and sodium hydroxide (5.07 ml; 1M in water; 5.07 mmol; 1.50 eq.) were added and the mixture was stirred 5 minutes at rt. The reaction was then acidified to pH 1 with HCl 1N and was concentrated to dryness. The residue obtained was taken up into water and the solid was filtered, washed with water and dried to furnish 3-[[(E)-(4-fluoro-3-methoxy-phenyl)methyleneamino]-methyl-amino]-1,1-dioxo-1,2-benzothiazol-6-ol (1.07 g; 78 %) as a beige solid. ¹H NMR (DMSO-d6, 400 MHz): δ 11.25 (s, 1H) ; 8.64 (d, J = 8.8 Hz, 1H) ; 8.41 (s, 1H) ; 7.60 (d, J = 8.4 Hz, 1H) ; 7.43 (m, 2H) ; 7.29 (d, J = 2.1 Hz, 1H) ; 7.21 (dd, J = 2.3 Hz, J = 8.9 Hz, 1H) ; 3.96 (s, 3H) ; 3.74 (s, 3H). mp: 258°C.

### Example 52: 6-benzyloxy-N-[(E)-(4-fluoro-3-methoxy-phenyl)methyleneamino]-N-methyl-1,1-dioxo-1,2-benzothiazol-3-amine

Cesium carbonate (134.5 mg; 0.41 mmol; 1.50 eq.) was added to a suspension of 3-[[(E)-(4-fluoro-3-methoxy-phenyl)methyleneamino]-methyl-amino]-1,1-dioxo-1,2-benzothiazol-6-ol (example 51), (100.0 mg; 0.28 mmol; 1.00 eq.) in DMF (2.00 ml) and the mixture was stirred 10 min at rt. Then, benzyl bromide (50 µL; 0.41 mmol; 1.50 eq.) was added and the reaction was stirred 1h at 50°C. The reaction was cooled to rt and 10ml of water was added. The solid was filtered and washed once with water, EtOH and dried under vacuum 3h at 50°C to give 6-benzyloxy-N-[(E)-(4-fluoro-3-methoxy-phenyl)methyleneamino]-N-methyl-1,1-dioxo-1,2-benzothiazol-3-amine (73.8 mg; 57 %) as a beige solid. 1H NMR (400MHz, DMSO-d6) δ 8.72 (d, J = 8.9 Hz, 1H) ; 8.43 (s, 1H) ; 7.76 (d, J = 2.3 Hz, 1H) ; 7.60 (d, J = 8.6 Hz, 1H) ; 7.44 (m, 8H) ; 5.33 (s, 2H) ; 3.95 (s, 3H) ; 3.76 (s, 3H). mp : 141-220°C.

### Example 53: 5-ethoxy-N-[(E)-(4-fluoro-3-methoxy-phenyl)methyleneamino]-N-methyl-1,1-dioxo-1,2-benzothiazol-3-amine

### Step A: 5-bromo-1,1-dioxo-1,2-benzothiazol-3-one

NH₃ (50.00 ml) 32% aq. was added dropwise to a solution of methyl 5-bromo-2-(chlorosulfonyl)benzoate (10.00 g; 31.89 mmol; 1.00 eq.) in THF (100.00 ml) at 0°C. The reaction was allowed to warm to rt and stirred 6h at rt, before being concentrated under vacuum. The residue was taken up into a saturated solution of NaHCO₃ and extracted once with diethyl ether. The aqueous layer was then acidified to pH 1 with concentrated HCl and the resulting precipitate was filtered and dried under vacuum to give 5-bromo-1,1-dioxo-1,2-benzothiazol-3-one (7.70 g; 92 %), as a white solid, used in the next step without purification. ¹H NMR (DMSO-d6, 300 MHz): δ 8.04 (m, 3H).

### Step B: 5-bromo-3-chloro-1,2-benzothiazole 1,1-dioxide

The compound was prepared using the same procedure as described in example 1 step A starting from 5-bromo-1,1-dioxo-1,2-benzothiazol-3-one (1.00 g; 3.82 mmol; 1.00 eq.) giving 5-bromo-3-chloro-1,2-benzothiazole 1,1-dioxide (1.07 g; quant.) as a beige solid, used in the next step without purification.

### Step C: 5-bromo-N-[(E)-(4-fluoro-3-methoxy-phenyl)methyleneamino]-N-methyl-1,1-dioxo-1,2-benzothiazol-3-amine

The compound was prepared using the same procedure as described in example 21 step B starting from 5-bromo-3-chloro-1,2-benzothiazole 1,1-dioxide (1.07 g; 3.81 mmol; 1.00 eq.) and N-[(E)-(4-fluoro-3-methoxy-phenyl)methyleneamino]methanamine (example 47, step A), (690.0 mg; 3.81 mmol; 1.00 eq.) giving 5-bromo-N-[(E)-(4-fluoro-3-methoxyphenyl)methyleneamino]-N-methyl-1,1-dioxo-1,2-benzothiazol-3-amine (1.34 g; 77 %) as an orange powder. ¹H NMR (DMSO-d6, 400 MHz): δ 9.04 (dd, J = 0.4 Hz, J = 1.8 Hz, 1H) ; 8.49 (s, 1H) ; 8.11 (dd, J = 1.8 Hz, J = 8.1 Hz, 1H) ; 8.08 (dd, J = 0.7 Hz, J = 8.1 Hz, 1H) ; 7.62 (dd, J = 1.8 Hz, J = 8.4 Hz, 1H) ; 7.43 (m, 2H); 4.02 (s, 3H) ; 3.79 (s, 3H). mp: 318°C.

### Step D: N-[(E)-(4-fluoro-3-methoxy-phenyl)methyleneamino]-N-methyl-1,1-dioxo-5-(4,4,5,5-tetramethyl-1,3,2-dioxaborolan-2-yl)-1,2-benzothiazol-3-amine

The compound was prepared using the same procedure as described in example 50 step F starting from 5-bromo-N-[(E)-(4-fluoro-3-methoxy-phenyl)methyleneamino]-N-methyl-1,1-dioxo-1,2-benzothiazol-3-amine (600.0 mg; 1.41 mmol; 1.00 eq.) giving N-[(E)-(4-fluoro-3-methoxy-phenyl)methyleneamino]-N-methyl-1,1-dioxo-5-(4,4,5,5-tetramethyl-1,3,2-dioxaborolan-2-yl)-1,2-benzothiazol-3-amine (500.0 mg; 75 %) as a yellow powder. ¹H NMR (DMSO-d6, 300 MHz): δ 9.22 (s, 1H) ; 8.49 (s, 1H) ; 8.08 (m, 2H) ; 7.65 (dd, J = 2.0 Hz, J = 8.4 Hz, 1H) ; 7.60 (ddd, J = 1.8 Hz, J = 4.4 Hz, J = 8.3 Hz, 1H) ; 7.34 (dd, J = 8.4 Hz, J = 11.1 Hz, 1H) ; 3.95 (s, 3H) ; 3.80 (s, 3H) ; 1.34 (s, 12H).

### Step E: 3-[[(E)-(4-fluoro-3-methoxy-phenyl)methyleneamino]-methyl-amino]-1,1 -dioxo-1,2-benzothiazol-5-ol

The compound was prepared using the same procedure as described in example 51 starting from N-[(E)-(4-fluoro-3-methoxy-phenyl)methyleneamino]-N-methyl-1,1-dioxo-5-(4,4,5,5-tetramethyl-1,3,2-dioxaborolan-2-yl)-1,2-benzothiazol-3-amine (305.0 mg; 0.64 mmol; 1.00 eq.) giving 3-[[(E)-(4-fluoro-3-methoxy-phenyl)methyleneamino]-methyl-amino]-1,1-dioxo-1,2-benzothiazol-5-ol (216.6 mg; 89 %) as a beige powder. ¹H NMR (DMSO-d₆, 300 MHz): δ 10.89 (br s, 1H) ; 8.43 (s, 1H) ; 8.23 (d, J = 2.1 Hz, 1H) ; 7.86 (d, J = 8.4 Hz, 1H); 7.63(dd, J = 1.7 Hz, J = 8.4Hz, 1H) ; 7.44(m, 2H); 7.18(dd, J = 2.2 Hz, J = 8.3 Hz, 1H) ; 3.96 (s, 3H) ; 3.76 (s, 3H).

### Step F: 5-ethoxy-N-[(E)-(4-fluoro-3-methoxy-phenyl)methyleneamino]-N-methyl-1,1-dioxo-1,2-benzothiazol-3-amine

The compound was prepared using the same procedure as described in example 52 starting from 3-[[(E)-(4-fluoro-3-methoxy-phenyl)methyleneamino]-methyl-amino]-1,1-dioxo-1,2-benzothiazol-5-ol (75.0 mg; 0.21 mmol; 1.00 eq.) and ethyl iodide (25 µl; 0.32 mmol; 1.50 eq.) giving 5-ethoxy-N-[(E)-(4-fluoro-3-methoxy-phenyl)methyleneamino]-N-methyl-1,1-dioxo-1,2-benzothiazol-3-amine (70.0 mg; 83 %) as a white powder. ¹H NMR (DMSO-d6, 400 MHz): δ 8.44 (s, 1H) ; 8.39 (d, J = 2.4 Hz, 1H) ; 8.00 (d, J = 8.4 Hz, 1H) ; 7.63 (m, 1H) ; 7.41 (m, 3H) ; 4.18 (q, J = 6.9 Hz, 2H) ; 3.97 (s, 3H) ; 3.77 (s, 3H) ; 1.36 (t, J = 6.9 Hz, 3H).

### Example 54: N-[(E)-(4-fluoro-3-methoxy-phenyl)methyleneamino]-5-isopropoxy-N-methyl -1,1-dioxo-1,2-benzothiazol-3-amine

Tributylphosphine (129 µl 0.52 mmol; 2.50 eq.) and diisopropyl azodicarboxylate (102 µl; 0.52 mmol; 2.50 eq.) were dissolved in dry THF (1.90 ml) at 0°C. Then, 3-[[(E)-(4-fluoro-3-methoxy-phenyl)methyleneamino]-methyl-amino]-1,1-dioxo-1,2-benzothiazol-5-ol (example 53, step E), (75.0 mg; 0.21 mmol; 1.00 eq.) was added. After 10 min stirring, propan-2-ol (40 µl; 0.52 mmol; 2.50 eq.) was added. The mixture was stirred 18h at rt and then concentrated under vacuum. The residue was purified by chromatography (column SiO₂ 5g, flow rate 15ml/min, gradient from 100% cyclohexane to 50/50 cyclohexane / EtOAc) to give N-[(E)-(4-fluoro-3-methoxy-phenyl)methyleneamino]-5-isopropoxy-N-methyl-1,1-dioxo-1,2-benzothiazol-3-amine (37.0 mg; 42 %) as a white solid. 1H NMR (DMSO-d6, 400 MHz): δ 8.44 (s, 1H) ; 8.35 (d, J = 2.4 Hz, 1H) ; 7.98 (d, J = 8.4 Hz, 1H) ; 7.63 (dd, J = 1.7 Hz, J = 8.5 Hz, 1H) ; 7.41 (m, 3H) ; 4.81 (sept, J = 5.7 Hz, 1H) ; 3.98 (s, 3H); 3.77 (s, 3H); 1.30 (d, J = 5.9 Hz, 6H). mp: 180-287°C.

### Example 55: N-[(E)-(4-fluoro-3-methoxy-phenyl)methyleneamino]-N-methyl-1,1-dioxo-5-phenoxy-1,2-benzothiazol-3-amine

5-bromo-N-[(E)-(4-fluoro-3-methoxy-phenyl)methyleneamino ]-N-methyl-1,1-dioxo-1,2-benzothiazol-3-amine (example 53, step C), (50.0 mg; 0.12 mmol; 1.00 eq.) was suspended in toluene (2.00 ml) under inert atmosphere. Then, phenol (11 µL; 0.12 mmol; 1.00 eq.) was added followed by potassium phosphate, tribasic (49.8 mg; 0.23 mmol; 2.00 eq.), palladium(II) acetate (2.6 mg; 0.01 mmol; 0.10 eq.) and 2-(di-tert-butylphosphino)biphenyl (5.25 mg; 0.02 mmol; 0.15 eq.). The resulting mixture was stirred 24h under reflux, then filtered and washed with toluene. The filtrate was concentrated under vacuum and the residue purified by chromatography (column SiO₂ 5g, flow rate 15ml/min, gradient from 100% cyclohexane to 70/30 cyclohexane / EtOAc) to give N-[(E)-(4-fluoro-3-methoxy-phenyl)methyleneamino]-N-methyl-1,1-dioxo-5-phenoxy-1,2-benzothiazol-3-amine (13.4 mg, 25%) as a pale yellow powder. 1H NMR (DMSO-d6, 400 MHz): δ 8.47 (d, J = 2.0 Hz, 1H) ; 8.41 (s, 1H) ; 8.09 (d, J = 8.4 Hz, 1H) ; 7.54 (dd, J = 2.0 Hz, J = 8.4 Hz, 1H); 7.46 (m, 3H); 7.22 (m, 4H); 6.97 (ddd, J = 2.0 Hz, J = 4.2 Hz, J = 8.1 Hz, 1H) ; 3.84 (s, 3H) ; 3.76 (s, 3H).

### Example 56: N-[(E)-(4-fluoro-3-methoxy-phenyl)methyleneamino]-5-(2-methoxyethoxy)-N-methyl-1,1-dioxo-1,2-benzothiazol-3-amine

The compound was prepared using the same procedure as described in example 52 starting from 3-[[(E)-(4-fluoro-3-methoxy-phenyl)methyleneamino]-methyl-amino]-1,1-dioxo-1,2-benzothiazol-5-ol (example 53, step E), (75.0 mg; 0.21 mmol; 1.00 eq.) and 1-bromo-2-methoxy-ethane (30 µl; 0.31 mmol; 1.50 eq.) giving N-[(E)-(4-fluoro-3-methoxyphenyl)methyleneamino]-5-(2-methoxyethoxy)-N-methyl-1,1-dioxo-1,2-benzothiazol-3-amine (63.0 mg; 71 %) as a white powder. ¹H NMR (DMSO-d6, 400 MHz): δ 8.44 (s, 1H) ; 8.40 (d, J = 2.4 Hz, 1H) ; 8.01 (d, J = 8.4 Hz, 1H) ; 7.63 (m, 1H) ; 7.42 (m, 3H) ; 4.26 (m, 2H) ; 3.97 (s, 3H); 3.77 (s, 3H) ; 3.74 (m, 2H) ; 3.32 (s, 3H). mp : 200°C.

### Example 57: 5-[2-(dimethylamino)ethoxy]-N-[(E)-(4-fluoro-3-methoxyphenyl)methylene amino]-N-methyl-1,1-dioxo-1,2-benzothiazol-3-amine

The compound was prepared using the same procedure as described in example 54 starting from 3-[[(E)-(4-fluoro-3-methoxy-phenyl)methyleneamino]-methyl-amino]-1,1-dioxo-1,2-benzothiazol-5-ol (example 53, step E), (75.0 mg; 0.21 mmol; 1.00 eq.) and dimethylamino ethanol (52 µl; 0.52 mmol; 2.50 eq.) giving 5-[2-(dimethylamino)ethoxy]-N-[(E)-(4-fluoro-3-methoxy-phenyl)methyleneamino]-N-methyl-1,1-dioxo-1,2-benzothiazol-3-amine (19.0 mg; 21 %) as a beige powder. ¹H NMR (DMSO-d6, 400 MHz): δ 8.45 (s, 1H) ; 8.37 (d, J = 2.2 Hz, 1H) ; 8.00 (d, J = 8.6 Hz, 1H) ; 7.63 (dd, J = 1.8 Hz, J = 8.4 Hz, 1H) ; 7.42 (m, 3H) ; 4.21 (t, J = 5.8 Hz, 2H) ; 3.97 (s, 3H) ; 3.77 (s, 3H) ; 2.64 (t, J = 5.8 Hz, 2H) ; 2.21 (s, 6H).

### Example 58: 2-[[3-[[(E)-(4-fluoro-3-methoxy-phenyl)methyleneamino]-methyl-amino]-1,1-dioxo-1,2-benzothiazol-5-yl]oxy]-N,N-dimethyl-acetamide

### Step A: tert-butyl 2-[[3-[[(E)-(4-fluoro-3-methoxy-phenyl)methyleneamino]-methyl-amino]-1,1-dioxo-1,2-benzothiazol-5-yl]oxy]acetate

The compound was prepared using the same procedure as described in example 52 starting from 3-[[(E)-(4-fluoro-3-methoxy-phenyl)methyleneamino]-methyl-amino]-1,1-dioxo-1,2-benzothiazol-5-ol (example 53, step E), (300.0 mg; 0.83 mmol; 1.00 eq.) and tert-butyl bromoacetate (240 µl; 1.65 mmol; 2.00 eq.) giving tert-butyl 2-[[3-[[(E)-(4-fluoro-3-methoxy-phenyl)methyleneamino]-methyl-amino]-1,1-dioxo-1,2-benzothiazol-5-yl]oxy] acetate (300.0 mg; 76 %) as a beige powder, used in next step without purification. LC/MS (Method a): Rt = 2.46 min ; MS : m/z = 478 [M+H]⁺

### Step B: 2-[[3-[[(E)-(4-fluoro-3-methoxy-phenyl)methyleneamino]-methyl-amino]-1,1-dioxo-1,2-benzothiazol-5-yl]oxy]acetic acid

Trifluoroacetic acid (2.00 ml) was added to a solution of tert-butyl 2-[[3-[[(E)-(4-fluoro-3-methoxy-phenyl)methyleneamino]-methyl-amino]-1,1-dioxo-1,2-benzothiazol-5-yl]oxy] acetate (300.00 mg; 0.63 mmol; 1.00 eq.) in DCM (3.00 ml) and the mixture was stirred 4h at rt. After concentration under vacuum, the resulting solid was filtered washed twice with water and dried under vacuum to give 2-[[3-[[(E)-(4-fluoro-3-methoxyphenyl)methyleneamino]-methyl-amino]-1,1-dioxo-1,2-benzothiazol-5-yl]oxy]acetic acid (230.0 mg; 87 %), as a grey solid. LC/MS (Method a): Rt = 1.90 min ; MS : m/z= 422 [M+H]⁺

### Step C: 2-[[3-[[(E)-(4-fluoro-3-methoxy-phenyl)methyleneamino]-methyl-amino]-1,1-dioxo-1,2-benzothiazol-5-yl]oxy]-N,N-dimethyl-acetamide

N-methylmorpholine (21.6 mg; 0.21 mmol; 1.20 eq.) and HATU (81.2 mg; 0.21 mmol; 1.20 eq.) were added to a solution of 2-[[3-[[(E)-(4-fluoro-3-methoxy-phenyl)methyleneamino]-methyl-amino]-1,1-dioxo-1,2-benzothiazol-5-yl]oxy]acetic acid (75.0 mg; 0.18 mmol; 1.00 eq.) in DMF (1.50 ml). After 5 min stirring, dimethylamine (2M in THF, 267 µl; 0.53 mmol; 3.00 eq.) was added and the mixture was stirred 1h30 at rt. A saturated solution of aqueous NaHCO₃ was then added and the resulting precipitate was filtered, washed with water and dried under vacuum to give 2-[[3-[[(E)-(4-fluoro-3-methoxyphenyl)methyleneamino]-methyl-amino]-1,1-dioxo-1,2-benzothiazol-5-yl]oxy]-N,N-dimethyl-acetamide (74.0 mg; 91 %) as a beige solid. ¹H NMR (DMSO-d6, 400 MHz): δ 8.46 (s, 1H) ; 8.39 (d, J = 2.4 Hz, 1H) ; 8.00 (d, J = 8.6 Hz, 1H) ; 7.64 (d, J = 8.6 Hz, 1H) ; 7.43 (m, 1H) ; 7.40 (m, 1H) ; 7.36 (dd, J = 2.4 Hz, J = 8.6 Hz, 1H) ; 5.05 (s, 2H) ; 3.95 (s, 3H) ; 3.77 (s, 3H) ; 2.96 (s, 3H); 2.82 (s, 3H). mp: 220°C.

### Example 59: 6-(5,5-dimethyl-1,3,2-dioxaborinan-2-yl)-N-[(E)-(4-fluoro-3-methoxyphenyl) methyleneamino]-N-methyl-1,1-dioxo-1,2-benzothiazol-3-amine

[3-[[(E)-(4-fluoro-3-methoxy-phenyl)methyleneamino]-methyl-amino]-1,1-dioxo-1,2-benzothiazol-6-yl]boronic acid (example 50), (100.0 mg; 0.26 mmol; 1.00 eq.) was suspended in toluene (3.00 ml). Isopropylene glycol (39.9 mg; 0.38 mmol; 1.50 eq.) was added and the mixture was stirred 6h under reflux. The mixture was cooled to rt and the precipitate was filtered, washed 3 times with toluene and dried under vacuum to give 6-(5,5-dimethyl-1,3,2-dioxaborinan-2-yl)-N-[(E)-(4-fluoro-3-methoxy-phenyl)methylene amino]-N-methyl-1,1-dioxo-1,2-benzothiazol-3-amine (70.0 mg; 59 %), as a beige solid. ¹H NMR (DMSO-d6, 400 MHz): δ 8.83 (dd, J = 0.4 Hz, J = 7.9 Hz, 1H) ; 8.45 (s, 1H) ; 8.17 (dd, J = 1.1 Hz, J = 8.1 Hz, 1H) ; 8.10 (m, 1H) ; 7.62 (dd, J = 1.8 Hz, J = 8.4 Hz, 1H) ; 7.47 (ddd, J = 1.8 Hz, J = 4.6 Hz, J = 8.4 Hz, 1H); 7.40 (dd, J = 8.4 Hz, J = 11.2 Hz, 1H); 3.97 (s, 3H) ; 3.82 (s, 4H) ; 3.77 (s, 3H), 0.99 (s, 6H).

### Example 60: 6-diethoxyphosphoryl-N-[(E)-(4-fluoro-3-methoxy-phenyl)methyleneamino]-N-methyl-1,1-dioxo-1,2-benzothiazol-3-amine

A solution of 6-bromo-N-[(E)-(4-fluoro-3-methoxy-phenyl)methyleneamino]-N-methyl-1,1-dioxo-1,2-benzothiazol-3-amine (example 50, step E), (200.00 mg; 0.47 mmol; 1.00 eq.), triethylphosphite (111 µL; 0.66 mmol; 1.40 eq.) and palladium(II) acetate (10.5 mg; 0.05 mmol; 0.10 eq.) in acetonitrile (1.60 ml) was stirred 45 minutes at 160°C under microwave irradiation. The reaction was then diluted with ACN and precipitation occurred. The solid was filtered, washed extensively with ACN and the filtrate was concentrated under vacuum. The residue was taken up into EtOH, filtered, washed once with EtOH and dried under vacuum at 50°C to give 6-diethoxyphosphoryl-N-[(E)-(4-fluoro-3-methoxy-phenyl)methyleneamino]-N-methyl-1,1-dioxo-1,2-benzothiazol-3-amine (104.4 mg; 46 %) as a green solid. 1H NMR (DMSO-d6, 400 MHz): δ 9.00 (dd, J = 2.6 Hz, J = 8.3 Hz, 1H) ; 8.50 (s, 1H) ; 8.27 (m, 2H) ; 7.64 (dd, J = 1.9 Hz, J = 8.5 Hz, 1H) ; 7.52 (m, 1H) ; 7.40 (dd, J = 8.3 Hz, J = 11.1 Hz, 1H) ; 4.13 (m, 4H) ; 3.98 (s, 3H) ; 3.81 (s, 3H) ; 1.28 (t, J = 7.0 Hz, 6H).

### Example 61: N-[(E)-[2-(azetidin-1-yl)thiazol-5-yl]methyleneamino]-N-methyl-1,1-dioxo-1,2-benzothiazol-3-amine

### Step A: N-[(E)-(2-chlorothiazol-5-yl)methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B, 100.0 mg; 0.47 mmol; 1.00 eq.) and 2-chlorothiazole-5-carbaldehyde (70.0 mg; 0.47 mmol; 1.00 eq.) giving N-[(E)-(2-chlorothiazol-5-yl)methyleneamino]-N-methyl-1,1-dioxo-1,2-benzothiazol-3-amine (136.0 mg; 84 %) as a yellow solid. 1H NMR (DMSO-d6, 300 MHz): δ 8.68 (s, 1H) ; 8.60 (d, J = 7.6 Hz, 1H) ; 8.16 (s, 1H) ; 8.10 (d, J = 7.2 Hz, 1H) ; 7.97 (td, J = 1.0 Hz, J = 7.6 Hz, 1H); 7.89 (td, J = 1.0 Hz, J = 7.6 Hz, 1H) ; 3.76 (s, 3H).

### Step B: N-[(E)-[2-(azetidin-1-yl)thiazol-5-yl]methyleneamino]-N-methyl-1,1-dioxo-1,2-benzothiazol-3-amine

Azetidine hydrochloride (16.0 mg; 0.18 mmol; 1.20 eq.) was dissolved in dry acetonitrile (0.75 ml) then N,N-diisopropylethylamine (51 µL; 0.29 mmol; 2.00 eq.) and N-[(E)-(2-chlorothiazol-5-yl)methyleneamino]-N-methyl-1,1-dioxo-1,2-benzothiazol-3-amine (50.0 mg; 0.15 mmol; 1.00 eq.) were added. The reaction mixture was stirred 12h at 50 °C. More azetidine hydrochloride (8.0 mg; 0.09 mmol; 0.60 eq.) and N,N-diisopropylethylamine (51 µL; 0.29 mmol; 2.00 eq.) were added and after 2 days, the mixture was diluted with EtOH and the precipitate was filtered off and washed with EtOH then acetonitrile. The solid was then dissolved in EtOAc and washed with water twice. The organic layer was dried over MgSO₄, filtered, concentrated and dried under vacuum at 70 °C to give N-[(E)-[2-(azetidin-1-yl)thiazol-5-yl]methyleneamino]-N-methyl-1,1-dioxo-1,2-benzothiazol-3-amine (29.0 mg, 55 %) as a yellow solid. 1H NMR (DMSO-d6, 300 MHz): δ 8.78 (dd, J = 1.6 Hz, J = 7.3Hz, 1H); 8.63 (s, 1H) ; 8.12 (dd, J = 1.6 Hz, J = 7.3 Hz, 1H); 7.93 (m, 2H) ; 7.77 (s, 1H) ; 4.22 (t, J = 7.5 Hz, 4H) ; 3.79 (s, 3H) ; 2.51 (m, 2H). mp: 256-258 °C.

### Example 62: [3-[ethyl-[(E)-(4-fluoro-3-methoxy-phenyl)methyleneamino]amino]-1,1-dioxo-1,2-benzothiazol-6-yl]boronic acid

### Step A: 6-bromo-N-ethyl-N-[(E)-(4-fluoro-3-methoxy-phenyl)methyleneamino]-1,1-dioxo-1,2-benzothiazol-3-amine

The compound was prepared using the same procedure as described in example 21 step B starting from 6-bromo-3-chloro-1,2-benzothiazole 1,1-dioxide (example 50, step D), (228 mg; 0.81 mmol; 1.2 eq.) and N-[(E)-(4-fluoro-3-methoxy-phenyl)methyleneamino]etha namine (example 38, step A), (191.0 mg; 0.97 mmol; 1.20 eq.) giving 6-bromo-N-ethyl-N-[(E)-(4-fluoro-3-methoxy-phenyl)methyleneamino]-1,1-dioxo-1,2-benzothiazol-3-amine (85.2 mg; 23 %) as a pale yellow solid. ¹H NMR (DMSO-d₆, 300 MHz): δ 8.68 (d, J = 8.6 Hz, 1H) ; 8.55 (s, 1H) ; 8.46 (d, J = 1.7 Hz, 1H) ; 8.16 (dd, J = 1.8 Hz, J = 8.6 Hz, 1H) ; 7.63 (dd, J = 2.0 Hz, J = 8.4 Hz, 1H) ; 7.48 (ddd, J = 1.8 Hz, J = 5.1 Hz, J = 8.4 Hz, 1H); 7.40 (dd, J = 8.3 Hz, J = 10.9 Hz, 1H); 4.39 (q, J = 6.9 Hz, 2H) ; 3.97 (s, 3H) ; 1.30 (t, J = 7.1 Hz, 3H).

### Step B: N-ethyl-N-[(E)-(4-fluoro-3-methoxy-phenyl)methyleneamino]-1,1-dioxo-6-(4,4,5,5-tetramethyl-1,3,2-dioxaborolan-2-yl)-1,2-benzothiazol-3-amine

The compound was prepared using the same procedure as described in example 50 step F starting from 6-bromo-N-ethyl-N-[(E)-(4-fluoro-3-methoxy-phenyl)methyleneamino]-1,1-dioxo-1,2-benzothiazol-3-amine (80.0 mg; 0.18 mmol; 1.00 eq.) giving N-ethyl-N-[(E)-(4-fluoro-3-methoxy-phenyl)methyleneamino]-1,1-dioxo-6-(4,4,5,5-tetramethyl-1,3,2-dioxaborolan-2-yl)-1,2-benzothiazol-3-amine (65.9 mg; 77 %) as a brown solid. ¹H NMR (DMSO-d₆, 300 MHz): δ 8.83 (d, J = 7.9 Hz, 1H) ; 8.55 (s, 1H) ; 8.17 (dd, J = 1.0 Hz, J = 7.9 Hz, 1H) ; 8.09 (s, 1H) ; 7.66 (dd, J = 2.0 Hz, J = 8.4 Hz, 1H) ; 7.51 (ddd, J = 2.1 Hz, J = 4.6 Hz, J = 8.4 Hz, 1H); 7.40 (dd, J = 8.3 Hz, J = 11.1 Hz, 1H); 4.40 (q, J = 6.8 Hz, 2H) ; 3.98 (s, 3H) ; 1.35 (s, 12H) ; 1.30 (t, J = 7.0 Hz, 3H).

### Step C: [3-[ethyl-[(E)-(4-fluoro-3-methoxy-phenyl)methyleneamino]amino]-1,1-dioxo-1,2-benzothiazol-6-yl]boronic acid

N-ethyl-N-[(E)-(4-fluoro-3-methoxy-phenyl)methyleneamino]-1,1-dioxo-6-(4,4,5,5-tetramethyl-1,3,2-dioxaborolan-2-yl)-1,2-benzothiazol-3-amine (60.0 mg; 0.12 mmol; 1.00 eq.) was suspended in a mixture of acetonitrile (1.50 ml) and HCl (1M in water, 1.23 ml; 1.23 mmol; 10.00 eq.). Then, the mixture was heated 5h30 under reflux, cooled to rt and concentrated. The residue was taken up into water and the solid was filtered, washed with water (3x) and dried under vacuum at 50°C. The obtained solid was triturated in a mixture 1:1 DMSO/ACN, filtered and washed with ACN. The filtrate was concentrated and the residue was purified by LCMS-preparative (Column: C18, 21,2 x 150mm 5µm (phenomenex); Flowrate: 25 ml/min; Elution: H2O, 0.1% HCOOH / ACN, 0.1% HCOOH) to give [3-[ethyl-[(E)-(4-fluoro-3-methoxy-phenyl)methyleneamino]amino]-1,1-dioxo-1,2-benzothiazol-6-yl]boronic acid (14.80 mg; 29 %), as a beige solid. ¹H NMR (DMSO-d₆, 400 MHz): δ 8.77 (dd, J = 0.7 Hz, J = 8.1 Hz, 1H); 8.64 (s, 2H); 8.54 (s, 1H); 8.34 (d, J = 0.7 Hz, 1H) ; 8.24 (dd, J = 1.1 Hz, J = 7.9 Hz, 1H) ; 7.68 (dd, J = 2.0 Hz, J = 8.4 Hz, 1H), 7.50 (ddd, J = 2.0 Hz, J = 4.6 Hz, J = 8.4 Hz, 1H) ; 7.42 (dd, J = 8.4 Hz, J = 11.2 Hz, 1H) ; 4.40 (q, J = 7.0 Hz, 2H) ; 3.98 (s, 3H) ; 1.30 (t, J = 6.8 Hz, 3H).

### Example 63: [3-[[(E)-(4-fluoro-3-methoxy-phenyl)methyleneamino]-isobutyl-amino]-1,1-dioxo-1,2-benzothiazol-6-yl]boronic acid

### Step A: 6-bromo-N-[(E)-(4-fluoro-3-methoxy-phenyl)methyleneamino]-N-isobutyl-1,1-dioxo-1,2-benzothiazol-3-amine

The compound was prepared using the same procedure as described in example 21 step B starting from 6-bromo-3-chloro-1,2-benzothiazole 1,1-dioxide (example 50, step D), (2.32 g; 8.27 mmol; 1.00 eq.) and N-[(E)-(4-fluoro-3-methoxy-phenyl)methyleneamino]-2-methyl-propan-1-amine (example 40, step A), (2.77 g; 8.53 mmol; 1.03 eq.) giving 6-bromo-N-[(E)-(4-fluoro-3-methoxy-phenyl)methyleneamino]-N-isobutyl-1,1-dioxo-1,2-benzothiazol-3-amine (700.0 mg; 18 %) as a white solid. ¹H NMR (DMSO-d₆, 400 MHz): δ 8.69 (d, J = 8.6 Hz, 1H) ; 8.54 (s, 1H); 8.46 (d, J = 1.8 Hz, 1H) ; 8.16 (dd, J = 1.9 Hz, J = 8.5 Hz, 1H) ; 7.63 (dd, J = 1.9 Hz, J = 8.5 Hz, 1H) ; 7.48 (ddd, J = 2.0 Hz, J = 4.6 Hz, J = 8.3 Hz, 1H); 7.40 (dd, J = 8.4 Hz, J = 11.1 Hz, 1H); 4.26 (d, J = 7.8 Hz, 2H); 3.97 (s, 3H) ; 2.26 (sept, J = 6.4 Hz, 1H) ; 0.98 (d, J = 6.8 Hz, 6H).

### Step B: N-[(E)-(4-fluoro-3-methoxy-phenyl)methyleneamino]-N-isobutyl-1,1-dioxo-6-(4,4,5,5-tetramethyl-1,3,2-dioxaborolan-2-yl)-1,2-benzothiazol-3-amine

The compound was prepared using the same procedure as described in example 50 step F starting from 6-bromo-N-[(E)-(4-fluoro-3-methoxy-phenyl)methyleneamino]-N-isobutyl-1,1-dioxo-1,2-benzothiazol-3-amine (600.0 mg; 1.24 mmol; 1.00 eq.) giving N-[(E)-(4-fluoro-3-methoxy-phenyl)methyleneamino]-N-isobutyl-1,1-dioxo-6-(4,4,5,5-tetramethyl-1,3,2-dioxaborolan-2-yl)-1,2-benzothiazol-3-amine (495.7 mg; 77 %) as a brown solid. ¹H NMR (DMSO-d₆, 300 MHz): δ 8.85 (d, J = 7.9 Hz, 1H) ; 8.54 (s, 1H) ; 8.17 (dd, J = 1.1 Hz, J = 8.0 Hz, 1H) ; 8.09 (s, 1H) ; 7.66 (dd, J = 2.0 Hz, J = 8.4 Hz, 1H) ; 7.51 (ddd, J = 1.8 Hz, J = 4.8 Hz, J = 8.6 Hz, 1H); 7.40 (dd, J = 8.4 Hz, J = 11.1 Hz, 1H); 4.28 (d, J = 7.9 Hz, 2H) ; 3.98 (s, 3H) ; 2.27 (m, 1H) ; 1.35 (s, 12H) ; 0.99 (d, J = 6.6 Hz, 6H).

### Step C: [3-[[(E)-(4-fluoro-3-methoxy-phenyl)methyleneamino]-isobutyl-amino]-1,1-dioxo-1,2-benzothiazol-6-yl]boronic acid

The compound was prepared using the same procedure as described in example 50 step G starting from N-[(E)-(4-fluoro-3-methoxy-phenyl)methyleneamino]-N-isobutyl-1,1-dioxo-6-(4,4,5,5-tetramethyl-1,3,2-dioxaborolan-2-yl)-1,2-benzothiazol-3-amine (495.0 mg; 0.96 mmol; 1.00 eq.) giving [3-[[(E)-(4-fluoro-3-methoxy-phenyl)methyleneamino]-isobutyl-amino]-1,1-dioxo-1,2-benzothiazol-6-yl]boronic acid (151.0 mg; 36 %) as a pale yellow solid. ¹H NMR (DMSO-d₆, 400 MHz): δ 8.79 (d, J = 8.4 Hz, 1H) ; 8.64 (s, 2H) ; 8.54 (s, 1H) ; 8.34 (s, 1H) ; 8.24 (dd, J = 1.1 Hz, J = 8.1 Hz, 1H); 7.68 (dd, J = 1.9 Hz, J = 8.5 Hz, 1H) ; 7.50 (ddd, J = 1.9 Hz, J = 4.6 Hz, J = 8.3 Hz, 1H) ; 7.42 (dd, J = 8.4 Hz, J = 11.2 Hz, 1H) ; 4.27 (d, J = 7.7 Hz, 2H) ; 3.98 (s, 3H) ; 2.28 (sept, J = 6.8 Hz, 1H) ; 0.99 (d, J = 6.6 Hz, 6H). mp: 203-236°C.

### Example 64: [3-[[(E)-(4-fluorophenyl)methyleneamino]-methyl-amino]-1,1-dioxo-1,2-benzothiazol-6-yl]boronic acid

### Step A: N-[(E)-(4-fluorophenyl)methyleneamino]methanamine

The compound was prepared using the same procedure as described in example 21 step A starting from 4-fluorobenzaldehyde (234.6 mg; 1.89 mmol; 1.00 eq.) giving N-[(E)-(4-fluorophenyl)methyleneamino]methanamine (287.0 mg; quant.) as a white solid, used without further purification.

### Step B: 6-bromo-N-[(E)-(4-fluorophenyl)methyleneamino]-N-methyl-1,1-dioxo-1,2-benzothiazol-3-amine

The compound was prepared using the same procedure as described in example 21 step B starting from 6-bromo-3-chloro-1,2-benzothiazole 1,1-dioxide (example 50, step D), (530.2 mg; 1.89 mmol; 1.00 eq.) and N-[(E)-(4-fluorophenyl)methyleneamino]methanamine (287.0 mg; 1.89 mmol; 1.00 eq.) giving 6-bromo-N-[(E)-(4-fluorophenyl)methyleneamino]-N-methyl-1,1-dioxo-1,2-benzothiazol-3-amine (220.0 mg; 29 %) as a yellow solid, used in next step without purification. LC/MS (Method a): Rt = 2.27 min ; MS : m/z = 396-398 [M+H]⁺

### Step C: N-[(E)-(4-fluorophenyl)methyleneamino]-N-methyl-1,1-dioxo-6-(4,4,5,5-tetramethyl-1,3,2-dioxaborolan-2-yl)-1,2-benzothiazol-3-amine

The compound was prepared using the same procedure as described in example 50 step F starting from 6-bromo-N-[(E)-(4-fluorophenyl)methyleneamino]-N-methyl-1,1-dioxo-1,2-benzothiazol-3-amine (220.0 mg; 0.56 mmol; 1.00 eq.) giving N-[(E)-(4-fluorophenyl) methyleneamino]-N-methyl-1,1-dioxo-6-(4,4,5,5-tetramethyl-1,3,2-dioxaborolan-2-yl)-1,2-benzothiazol-3-amine (140.0 mg; 57 %) as a grey solid. ¹H NMR (DMSO-d₆, 300 MHz): δ 8.89 (d, J = 7.9 Hz, 1H) ; 8.50 (s, 1H) ; 8.23 (dd, J = 0.9 Hz, J = 8.0 Hz, 1H) ; 8.08 (s, 1H) ; 7.97 (dd, J = 5.5 Hz, J = 8.8 Hz, 2H) ; 7.39 (t, J = 8.8 Hz, 2H) ; 3.80 (s, 3H) ; 1.35 (s, 12H). Step D: [3-[[(E)-(4-fluorophenyl)methyleneamino]-methyl-amino]-1,1-dioxo-1,2-benzothiazol-6-yl]boronic acid

The compound was prepared using the same procedure as described in example 62 step C starting from N-[(E)-(4-fluorophenyl)methyleneamino]-N-methyl-1,1-dioxo-6-(4,4,5,5-tetramethyl-1,3,2-dioxaborolan-2-yl)-1,2-benzothiazol-3-amine (140.0 mg; 0.32 mmol; 1.00 eq.) giving [3-[[(E)-(4-fluorophenyl)methyleneamino]-methyl-amino]-1,1-dioxo-1,2-benzothiazol-6-yl]boronic acid (100.0 mg; 88 %) as a beige solid. ¹H NMR (DMSO-d₆, 400 MHz): δ 8.80 (d, J = 8.4 Hz, 1H) ; 8.63 (s, 2H) ; 8.49 (s, 1H) ; 8.34 (s, 1H) ; 8.27 (dd, J = 1.1 Hz, J = 8.1 Hz, 1H) ; 7.96 (m, 2H) ; 7.42 (m, 2H) ; 3.80 (s, 3H).

### Example 65: [3-[isobutyl-[(E)-(3-methyl-2-oxo-1H-benzimidazol-5-yl)methyleneamino] amino]-1,1-dioxo-1,2-benzothiazol-6-yl]boronic acid

### Step A: 5-[(E)-[(6-bromo-1,1-dioxo-1,2-benzothiazol-3-yl)-isobutyl-hydrazono]methyl]-3-methyl-1H-benzimidazol-2-one

The compound was prepared using the same procedure as described in example 21 step B starting from 6-bromo-3-chloro-1,2-benzothiazole 1,1-dioxide (example 50, step D), (527.4 mg; 1.88 mmol; 1.00 eq.) and 5-[(E)-(isobutylhydrazono)methyl]-3-methyl-1H-benzimidazol-2-one (example 23, step I, 839.5 mg; 1.88 mmol; 1.00 eq.) giving 5-[(E)-[(6-bromo-1,1-dioxo-1,2-benzothiazol-3-yl)-isobutyl-hydrazono]methyl]-3-methyl-1H-benzimi dazol-2-one (430.0 mg; 47 %) as a yellow solid, used in next step without purification. LC/MS (Method a): Rt = 2.10 min ; MS : m/z = 490-492 [M+H]⁺

### Step B: [3-[isobutyl-[(E)-(3-methyl-2-oxo-1H-benzimidazol-5-yl)methyleneamino]amino]-1,1-dioxo-1,2-benzothiazol-6-yl]boronic acid

The compound was prepared using the same procedure as described in example 50 step F starting from 5-[(E)-[(6-bromo-1,1-dioxo-1,2-benzothiazol-3-yl)-isobutyl-hydrazono] methyl] -3-methyl-1H-benzimidazol-2-one (430.0 mg; 0.88 mmol; 1.00 eq.) giving 5-[(E)-[[1,1-dioxo-6-(4,4,5,5-tetramethyl-1,3,2-dioxaborolan-2-yl)-1,2-benzothiazol-3-yl]-isobutyl-hydrazono] methyl]-3-methyl-1H-benzimidazol-2-one. The latter was directly purified by LCMS-Preparative (Column: Kinetex C18,30 x 150mm 5µm (phenomenex); Flow rate: 42 ml/min; Elution: H2O, 0.1% HCOOH / ACN, 0.1% HCOOH; Gradient: 10 to 100% ACN over 12 minutes) to give [3-[isobutyl-[(E)-(3-methyl-2-oxo-1H-benzimidazol-5-yl)methyleneamino] amino]-1,1-dioxo-1,2-benzothiazol-6-yl]boronic acid (80.0 mg; 19 %) as a yellow solid. ¹H NMR (DMSO-d₆ + 10% v/v D₂O, 400 MHz): δ 8.85 (d, J = 8.1 Hz, 1H) ; 8.55 (s, 1H) ; 8.32 (s, 1H) ; 8.27 (dd, J = 0.8 Hz, J = 8.0 Hz, 1H) ; 7.62 (d, J = 7.9 Hz, 1H) ; 7.58 (s, 1H) ; 7.21 (d, J = 7.9 Hz, 1H) ; 4.30 (d, J = 7.5 Hz, 2H) ; 3.39 (s, 3H) ; 2.27 (sept, J = 7.0 Hz, 1H) ; 0.99 (d, J = 6.6 Hz, 6H).

### Example 66: [3-[[(E)-(3-ethyl-2-oxo-1H-benzimidazol-5-yl)methyleneamino]-methyl-amino]-1,1-dioxo-1,2-benzothiazol-6-yl]boronic acid

### Step A: 3-ethyl-5-[(E)-(methylhydrazono)methyl]-1H-benzimidazol-2-one

The compound was prepared using the same procedure as described in example 21 step A starting from 3-ethyl-2-oxo-1H-benzimidazole-5-carbaldehyde (example 24, step E), (200.0 mg; 1.05 mmol; 1.00 eq.) giving 3-ethyl-5-[(E)-(methylhydrazono)methyl]-1H-benzimidazol-2-one (252.0 mg; quant.) as a white solid, used without further purification. ¹H NMR (DMSO-d₆, 300 MHz): δ 11.00 (br s, 1H) ; 7.47 (s, 1H) ; 7.27 (d, J = 1.3 Hz, 1H) ; 7.11 (dd, J = 1.5 Hz, J = 8.1 Hz, 1H) ; 7.05 (br s, 1H) ; 6.93 (d, J = 8.1 Hz, 1H) ; 3.81 (q, J = 7.0 Hz, 2H) ; 2.79 (s, 3H) ; 1.19 (t, J = 7.1 Hz, 3H).

### Step B: 5-[(E)-[(6-bromo-1,1-dioxo-1,2-benzothiazol-3-yl)-methyl-hydrazono]methyl]-3-ethyl-1H-benzimidazol-2-one

The compound was prepared using the same procedure as described in example 21 step B starting from 6-bromo-3-chloro-1,2-benzothiazole 1,1-dioxide (example 50, step D), (245.3 mg; 0.87 mmol; 1.00 eq.) and 3-ethyl-5-[(E)-(methylhydrazono)methyl]-1H-benzimidazol-2-one (229.0 mg; 1.05 mmol; 1.20 eq.) giving 5-[(E)-[(6-bromo-1,1-dioxo-1,2-benzothiazol-3-yl)-methyl-hydrazono]methyl]-3-ethyl-1H-benzimidazol-2-one (130.0 mg; 32 %) as a yellow solid, used in next step without purification. ¹H NMR (DMSO-d₆, 300 MHz): δ 11.26 (s, 1H) ; 8.79 (d, J = 8.6 Hz, 1H) ; 8.51 (s, 1H) ; 8.45 (d, J = 1.7 Hz, 1H) ; 8.12 (dd, J = 1.6 Hz, J = 8.5 Hz, 1H) ; 7.55 (m, 2H) ; 7.16 (d, J = 8.3 Hz, 1H) ; 3.92 (q, J = 7.2 Hz, 2H) ; 3.79 (s, 3H) ; 1.27 (t, J = 7.0 Hz, 3H).

### Step C: 5-[(E)-[[1,1-dioxo-6-(4,4,5,5-tetramethyl-1,3,2-dioxaborolan-2-yl)-1,2-benzothiazol-3-yl]-methyl-hydrazono]methyl]-3-ethyl-1H-benzimidazol-2-one

The compound was prepared using the same procedure as described in example 50 step F starting from 5-[(E)-[(6-bromo-1,1-dioxo-1,2-benzothiazol-3-yl)-methyl-hydrazono]methyl] -3-ethyl-1H-benzimidazol-2-one (130.0 mg; 0.28 mmol; 1.00 eq.) giving 5-[(E)-[[1,1-dioxo-6-(4,4,5,5-tetramethyl-1,3,2-dioxaborolan-2-yl)-1,2-benzothiazol-3-yl]-methyl-hydrazono] methyl]-3-ethyl-1H-benzimidazol-2-one (65.0 mg; 45 %) as a brown solid. ¹H NMR (DMSO-d₆, 300 MHz): δ 11.19 (s, 1H) ; 8.94 (d, J = 8.1 Hz, 1H) ; 8.49 (s, 1H) ; 8.17 (d, J = 8.3 Hz, 1H) ; 8.09 (s, 1H) ; 7.59 (m, 2H) ; 7.16 (d, J = 8.3 Hz, 1H) ; 3.93 (q, J = 7.4 Hz, 2H); 3.81 (s, 3H); 1.35 (s, 12H); 1.28 (t, J = 7.0 Hz, 3H).

### Step D: [3-[[(E)-(3-ethyl-2-oxo-1H-benzimidazol-5-yl)methyleneamino]-methyl-amino]-1,1-dioxo-1,2-benzothiazol-6-yl]boronic acid

The compound was prepared using the same procedure as described in example 62 step C starting from 5-[(E)-[[1,1-dioxo-6-(4,4,5,5-tetramethyl-1,3,2-dioxaborolan-2-yl)-1,2-benzothiazol -3-yl]-methyl-hydrazono]methyl]-3-ethyl-1H-benzimidazol-2-one (65.0 mg; 0.13 mmol; 1.00 eq.) giving [3-[[(E)-(3-ethyl-2-oxo-1H-benzimidazol-5-yl)methyleneamino]-methyl-amino]-1,1-dioxo-1,2-benzothiazol-6-yl]boronic acid (35.0 mg; 64 %) as a yellow solid. ¹H NMR (DMSO-d₆, 400 MHz): δ 11.19 (s, 1H) ; 8.87 (d, J = 7.9 Hz, 1H) ; 8.64 (s, 2H) ; 8.50 (s, 1H) ; 8.35 (s, 1H) ; 8.24 (dd, J = 1.0 Hz, J = 8.0 Hz, 1H) ; 7.60 (d, J = 0.9 Hz, 1H) ; 7.56 (dd, J = 1.3 Hz, J = 8.1 Hz, 1H) ; 7.17 (d, J = 7.9 Hz, 1H) ; 3.92 (q, J = 7.1 Hz, 2H) ; 3.81 (s, 3H) ; 1.30 (t, J = 7.2 Hz, 3H).

### Example 67: [3-[[(E)-(3-isopropyl-2-oxo-1H-benzimidazol-5-yl)methyleneamino]-methyl-amino]-1,1-dioxo-1,2-benzothiazol-6-yl]boronic acid

### Step A: 5-[(E)-[(6-bromo-1,1-dioxo-1,2-benzothiazol-3-yl)-methyl-hydrazono]methyl]-3-isopropyl-1H-benzimidazol-2-one

The compound was prepared using the same procedure as described in example 21 step B starting from 6-bromo-3-chloro-1,2-benzothiazole 1,1-dioxide (example 50, step D), (212.6 mg; 0.76 mmol; 1.00 eq.) and 3-isopropyl-5-[(E)-(methylhydrazono)methyl]-1H-benzimidazol-2-one (example 26, step D), (176.0 mg; 0.76 mmol; 1.00 eq.) giving 5-[(E)-[(6-bromo-1,1-dioxo-1,2-benzothiazol-3-yl)-methyl-hydrazono]methyl]-3-isopropyl-1H-benzimidazol-2-one (267.0 mg; 69 %) as a yellow solid, used in next step without purification. ¹H NMR (DMSO-d₆, 400 MHz): δ 11.19 (s, 1H) ; 8.78 (d, J = 8.6 Hz, 1H) ; 8.52 (s, 1H) ; 8.46 (d, J = 2.0 Hz, 1H) ; 8.06 (dd, J = 2.0 Hz, J = 8.6 Hz, 1H) ; 7.70 (s, 1H) ; 7.57 (dd, J = 1.4 Hz, J = 8.3 Hz, 1H) ; 7.14 (d, J = 8.1 Hz, 1H) ; 4.64 (sept, J = 6.9 Hz, 1H) ; 3.79 (s, 3H) ; 1.51 (d, J = 6.8 Hz, 6H).

### Step B: 5-[(E)-[[1,1-dioxo-6-(4,4,5,5-tetramethyl-1,3,2-dioxaborolan-2-yl)-1,2-benzothiazol-3-yl]-methyl-hydrazono]methyl]-3-isopropyl-1H-benzimidazol-2-one

The compound was prepared using the same procedure as described in example 50 step F starting from 5-[(E)-[(6-bromo-1,1-dioxo-1,2-benzothiazol-3-yl)-methyl-hydrazono]methyl] -3-isopropyl-1H-benzimidazol-2-one (240.0 mg; 0.50 mmol; 1.00 eq.) giving 5-[(E)-[[1,1-dioxo-6-(4,4,5,5-tetramethyl-1,3,2-dioxaborolan-2-yl)-1,2-benzothiazol-3-yl]-methyl-hydrazono]methyl]-3-isopropyl-1H-benzimidazol-2-one (179.0 mg; 68 %) as a green solid. ¹H NMR (DMSO-d₆, 400 MHz): δ 11.18 (s, 1H) ; 8.93 (dd, J = 0.7 Hz, J = 8.1 Hz, 1H); 8.52 (s, 1H); 8.12 (dd, J = 1.1 Hz, J = 7.9 Hz, 1H); 8.09 (t, J = 0.8 Hz, 1H); 7.72 (s, 1H) ; 7.60 (dd, J = 1.3 Hz, J = 8.1 Hz, 1H) ; 7.15 (d, J = 8.1 Hz, 1H) ; 4.66 (sept, J = 7.0 Hz, 1H) ; 3.81 (s, 3H) ; 1.51 (d, J = 6.8 Hz, 6H) ; 1.35 (s, 12H).

### Step C: [3-[[(E)-(3-isopropyl-2-oxo-1H-benzimidazol-5-yl)methyleneamino]-methyl-amino]-1,1-dioxo-1,2-benzothiazol-6-yl]boronic acid

The compound was prepared using the same procedure as described in example 62 step C starting from 5-[(E)-[[1,1-dioxo-6-(4,4,5,5-tetramethyl-1,3,2-dioxaborolan-2-yl)-1,2-benzothiazol-3-yl]-methyl-hydrazono]methyl]-3-isopropyl-1H-benzimidazol-2-one (150.0 mg; 0.29 mmol; 1.00 eq.) giving [3-[[(E)-(3-isopropyl-2-oxo-1H-benzimidazol-5-yl)methyleneamino]-methyl-amino]-1,1-dioxo-1,2-benzothiazol-6-yl]boronic acid (18.2 mg; 14 %) as a yellow solid. ¹H NMR (DMSO-d₆ + 10% v/v D₂O, 400 MHz): δ 8.88 (d, J = 7.9 Hz, 1H) ; 8.50 (s, 1H) ; 8.34 (s, 1H) ; 8.20 (dd, J = 1.1 Hz, J = 8.1 Hz, 1H) ; 7.75 (d, J = 1.1 Hz, 1H) ; 7.58 (dd, J = 1.2 Hz, J = 8.3 Hz, 1H) ; 7.19 (d, J = 8.1 Hz, 1H); 4.67 (quint, J = 6.8 Hz, 1H) ; 3.81 (s, 3H) ; 1.53 (d, J = 6.8 Hz, 6H).

### Example 68: 2-[3-[[(E)-(4-fluoro-3-methoxy-phenyl)methyleneamino]-methyl-amino]-1,1-dioxo-1,2-benzothiazol-6-yl]-6-methyl-1,3,6,2-dioxazaborocane-4,8-dione

[3-[[(E)-(4-fluoro-3-methoxy-phenyl)methyleneamino]-methyl-amino]-1,1-dioxo-1,2-benzo thiazol-6-yl]boronic acid (example 50), (200.0 mg; 0.51 mmol; 1.00 eq.) was suspended in a mixture of toluene (3.60 ml) and DMSO (400 µl). N-Methyliminodiacetic acid (112.8 mg; 0.77 mmol; 1.50 eq.) and MgSO₄ (310.0 mg; 2.58 mmol; 5.00 eq.) were added and the mixture was stirred 1h under reflux. The mixture was filtered and the solid washed with toluen, EtOH (3x) and dried to give as a clear grey solid. The latter was washed with an aqueous solution of Na₂CO₃ (2x), water (5x) and EtOH, dried under vaccum to give 2-[3-[[(E)-(4-fluoro-3-methoxy-phenyl)methyleneamino]-methyl-amino]-1,1-dioxo-1,2-benzo thiazol-6-yl]-6-methyl-1,3,6,2-dioxazaborocane-4,8-dione (85.00 mg; 32 %) as a white solid. ¹H NMR (DMSO-d₆, 400 MHz): δ 8.82 (dd, J = 0.7 Hz, J = 8.1 Hz, 1H) ; 8.47 (s, 1H) ; 8.11 (m, 1H) ; 7.99 (dd, J = 1.2 Hz, J = 8.0 Hz, 1H); 7.64 (dd, J = 2.0 Hz, J = 8.6 Hz, 1H) ; 7.52 (ddd, J = 1.9 Hz, J = 4.5 Hz, J = 8.4 Hz, 1H) ; 7.41 (dd, J = 8.4 Hz, J = 11.2 Hz, 1H) ; 4.42 (d, J = 17.2 Hz, 2H) ; 4.23 (d, J = 17.2 Hz, 2H) ; 3.96 (s, 3H) ; 3.80 (s, 3H) ; 2.63 (s, 3H).

### Example 69: 2-[3-[[(E)-(4-fluoro-3-methoxy-phenyl)methyleneamino]-isobutyl-amino]-1,1-dioxo-1,2-benzothiazol-6-yl]-6-methyl-1,3,6,2-dioxazaborocane-4,8-dione

The compound was prepared using the same procedure as described in example 68 starting from [3-[[(E)-(4-fluoro-3-methoxy-phenyl)methyleneamino]-isobutyl-amino]-1,1-dioxo-1,2-benzothiazol-6-yl]boronic acid (example 63), (70.0 mg; 0.16 mmol; 1.00 eq.) giving 2-[3-[[(E)-(4-fluoro-3-methoxy-phenyl)methyleneamino]-isobutyl-amino]-1,1-dioxo-1,2-benzothiazol-6-yl]-6-methyl-1,3,6,2-dioxazaborocane-4,8-dione (70.0 mg; 80 %) as a white solid. ¹H NMR (DMSO-d₆, 400 MHz): δ 8.79 (d, J = 8.1 Hz, 1H) ; 8.54 (s, 1H) ; 8.10 (s, 1H) ; 7.98 (dd, J = 1.1 Hz, J = 7.9 Hz, 1H) ; 7.67 (dd, J = 2.1 Hz, J = 8.5 Hz, 1H) ; 7.54 (ddd, J = 1.9 Hz, J = 4.5 Hz, J = 8.4 Hz, 1H) ; 7.42 (dd, J = 8.4 Hz, J = 11.2 Hz, 1H) ; 4.42 (d, J = 17.4 Hz, 2H) ; 4.28 (d, J = 7.7 Hz, 2H) ; 4.22 (d, J = 17.2 Hz, 2H) ; 3.97 (s, 3H) ; 2.63 (s, 3H) ; 2.28 (m, 1H) ; 0.99 (d, J = 6.8 Hz, 6H).

### Example 70: 3-methyl-5-[(E)-[methyl-(5-methyl-1,1-dioxo-1,2-benzothiazol-3-yl) hydrazono]methyl]-1H-benzimidazol-2-one

### Step A: 5-methyl-1,1-dioxo-1,2-benzothiazol-3-one

The compound was prepared using the same procedure as described in example 53 step A starting from methyl 2-chlorosulfonyl-5-methyl-benzoate (4.90 g; 19.70 mmol; 1.00 eq.) giving 5-methyl-1,1-dioxo-1,2-benzothiazol-3-one (3.13 g; 81 %) as a white solid. 1H NMR (DMSO-d6, 300 MHz): δ 8.03 (d, J = 7.9 Hz, 1H) ; 7.82 (br s, 1H) ; 7.78 (m, 1H) ; 2.50 (s, 3H).

### Step B: 3-chloro-5-methyl-1,2-benzothiazole 1,1-dioxide

The compound was prepared using the same procedure as described in example 1 step A starting from 5-methyl-1,1-dioxo-1,2-benzothiazol-3-one (300.0 mg; 1.52 mmol; 1.00 eq.) giving 3-chloro-5-methyl-1,2-benzothiazole 1,1-dioxide (330.0 mg; quant.) as a pale yellow solid, used in next step without purification. 1H NMR (DMSO-d6, 300 MHz): δ 8.05 (d, J = 7.9 Hz, 1H) ; 7.85 (brs, 1H) ; 7.81 (m, 1H) ; 2.51 (s, 3H).

### Step C: 1-methyl-1-(5-methyl-1,1-dioxo-1,2-benzothiazol-3-yl)hydrazine

The compound was prepared using the same procedure as described in example 1 step B starting from 3-chloro-5-methyl-1,2-benzothiazole 1,1-dioxide (326.0 mg; 1.51 mmol; 1.00 eq.) giving 1-methyl-1-(5-methyl-1,1-dioxo-1,2-benzothiazol-3-yl)hydrazine (189.0 mg; 56 %) as a white solid. 1H NMR (DMSO-d6, 300 MHz): δ 8.81 (s, 1H) ; 7.81 (d, J = 7.8 Hz, 1H) ; 7.59 (d, J = 7.8 Hz, 1H) ; 5.58 (s, 2H) ; 3.42 (s, 3H) ; 2.45 (s, 3H).

### Step D: 3-methyl-5-[(E)-[methyl-(5-methyl-1,1-dioxo-1,2-benzothiazol-3-yl)hydrazono] methyl]-1H-benzimidazol-2-one

The compound was prepared using the same procedure as described in example 1 step C starting from 1-methyl-1-(5-methyl-1,1-dioxo-1,2-benzothiazol-3-yl)hydrazine (100.0 mg; 0.44 mmol; 1.00 eq.) and 3-methyl-2-oxo-1H-benzimidazole-5-carbaldehyde (example 23 step E), (82.1 mg; 0.47 mmol; 1.05 eq.) giving 3-methyl-5-[(E)-[methyl-(5-methyl-1,1-dioxo-1,2-benzothiazol-3-yl)hydrazono]methyl]-1H-benzimidazol-2-one (110.0 mg; 62 %) as a pale yellow solid. 1H NMR (DMSO-d6, 400 MHz): δ 8.82 (s, 1H) ; 8.47 (s, 1H) ; 7.95 (d, J = 7.7 Hz, 1H) ; 7.70 (d, J = 7.9 Hz, 1H) ; 7.56 (s, 1H) ; 7.49 (d, J = 8.1 Hz, 1H) ; 7.14 (d, J = 7.9 Hz, 1H) ; 3.80 (s, 3H) ; 3.38 (s, 3H) ; 2.58 (s, 3H).

### Example 71: [3-[isobutyl-[(E)-(3-isopropyl-2-oxo-1H-benzimidazol-5-yl)methylene amino]amino]-1,1-dioxo-1,2-benzothiazol-6-yl]boronic acid

### Step A: 5-[(E)-(isobutylhydrazono)methyl]-3-isopropyl-1H-benzimidazol-2-one

The compound was prepared using the same procedure as described in example 21 step A starting from 3-isopropyl-2-oxo-1H-benzimidazole-5-carbaldehyde (example 26, step C), (295.0 mg; 1.44 mmol; 1.00 eq.) and 2-methylpropylhydrazine hydrochloride (216.0 mg; 1.73 mmol; 1.20 eq.) giving 5-[(E)-(isobutylhydrazono)methyl]-3-isopropyl-1H-benzimidazol-2-one (524.9 mg; quant.) as a yellow resin, used directly in next step without purification. ¹H NMR (DMSO-d₆, 300 MHz): δ 10.92 (s, 1H) ; 7.86 (br s, 1H) ; 7.40 (s, 1H) ; 7.18 (d, J = 7.9 Hz, 1H); 6.95 (d, J = 8.1 Hz, 1H) ; 4.56 (m, 1H); 2.96 (d, J = 6.9 Hz, 2H) ; 1.91 (m, 1H) ; 1.44 (d, J = 6.9 Hz, 6H) ; 0.95 (d, J = 6.8 Hz, 6H).

### Step B: 5-[(E)-[(6-bromo-1,1-dioxo-1,2-benzothiazol-3-yl)-isobutyl-hydrazono] methyl]-3-isopropyl-1H-benzimidazol-2-one

The compound was prepared using the same procedure as described in example 21 step B starting from 6-bromo-3-chloro-1,2-benzothiazole 1,1-dioxide (example 50, step D), (530.0 mg; 1.89 mmol; 1.33 eq.) and 5-[(E)-(isobutylhydrazono)methyl]-3-isopropyl-1H-benzimidazol-2-one (390.0 mg; 1.42 mmol; 1.00 eq.) giving 5-[(E)-[(6-bromo-1,1-dioxo-1,2-benzothiazol-3-yl)-isobutyl-hydrazono]methyl]-3-isopropyl-1H-benzimidazol-2-one (420.4 mg; 55 %) as a yellow solid. ¹H NMR (DMSO-d₆, 300 MHz): δ 11.18 (s, 1H) ; 8.73 (d, J = 8.6 Hz, 1H) ; 8.57 (s, 1H) ; 8.45 (d, J = 1.8 Hz, 1H) ; 8.03 (dd, J = 1.7 Hz, J = 8.5 Hz, 1H) ; 7.71 (s, 1H) ; 7.57 (dd, J = 1.2 Hz, J = 8.3 Hz, 1H) ; 7.13 (d, J = 8.1 Hz, 1H) ; 4.63 (m, 1H) ; 4.26 (d, J = 7.6 Hz, 2H) ; 2.26 (m, 1H) ; 1.50 (d, J = 6.9 Hz, 6H) ; 0.98 (d, J = 6.8 Hz, 6H).

### Step C: [3-[isobutyl-[(E)-(3-isopropyl-2-oxo-1H-benzimidazol-5-yl)methylene amino]amino]-1,1-dioxo-1,2-benzothiazol-6-yl]boronic acid

The compound was prepared using the same procedure as described in example 50 step F starting from 5-[(E)-[(6-bromo-1,1-dioxo-1,2-benzothiazol-3-yl)-isobutyl-hydrazono]methyl]-3-isopropyl-1H-benzimidazol-2-one (400.0 mg; 0.75 mmol; 1.00 eq.) giving 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3-isopropyl-1H-benzimidazol-2-one. The latter was directly purified by LC/MS-Preparative (Column: Kinetex C18 21,20 x 150mm 5µm (phenomenex); Flow rate: 25 ml/min; Elution: H2O, 0.1% HCOOH / ACN, 0.1% HCOOH; Gradient: 10 to 100% ACN over 12 minutes) to give [3-[isobutyl-[(E)-(3-isopropyl-2-oxo-1H-benzimidazol-5-yl)methyleneamino]amino]-1,1-dioxo-1,2-benzothiazol-6-yl]boronic acid (93.6 mg; 25 %) as a beige solid. ¹H NMR (DMSO-d₆ + 10% v/v D₂O, 400 MHz): δ 8.84 (d, J = 8.1 Hz, 1H) ; 8.56 (s, 1H) ; 8.35 (s, 1H) ; 8.19 (d, J = 8.1 Hz, 1H) ; 7.76 (s, 1H) ; 7.59 (dd, J = 1.1 Hz, J = 8.4 Hz, 1H) ; 7.18 (d, J = 8.1 Hz, 1H) ; 4.66 (m, 1H) ; 4.29 (d, J = 7.7 Hz, 2H) ; 2.29 (m, 1H) ; 1.53 (d, J = 7.0 Hz, 6H) ; 1.00 (d, J = 6.8 Hz, 6H). mp: 268-282 °C.

### Example 72: [3-[[(E)-(4-fluoro-3-methoxy-phenyl)methyleneamino]-(2-methoxy ethyl)amino]-1,1-dioxo-1,2-benzothiazol-6-yl]boronic acid

### Step A: 6-bromo-N-[(E)-(4-fluoro-3-methoxy-phenyl)methyleneamino]-N-(2-methoxyethyl)-1,1-dioxo-1,2-benzothiazol-3-amine

The compound was prepared using the same procedure as described in example 21 step B starting from 6-bromo-3-chloro-1,2-benzothiazole 1,1-dioxide (example 50, step D), (499.2 mg; 1.78 mmol; 1.10 eq.) and N-[(E)-(4-fluoro-3-methoxy-phenyl)methyleneamino]-2-methoxy-ethanamine (example 42, step A), (366.0 mg; 1.62 mmol; 1.00 eq.) giving 6-bromo-N-[(E)-(4-fluoro-3-methoxy-phenyl)methyleneamino]-N-(2-methoxyethyl)-1,1-dioxo-1,2-benzothiazol-3-amine (580.0 mg; 76 %) as a yellow solid. ¹H NMR (DMSO-d₆, 300 MHz): δ 8.67 (d, J = 8.6 Hz, 1H) ; 8.61 (s, 1H) ; 8.47 (d, J = 1.8 Hz, 1H) ; 8.17 (dd, J = 1.9 Hz, J = 8.5 Hz, 1H) ; 7.60 (dd, J = 1.7 Hz, J = 8.3 Hz, 1H) ; 7.42 (m, 2H) ; 4.57 (t, J = 5.6 Hz, 2H) ; 3.97 (s, 3H) ; 3.72 (t, J = 5.6 Hz, 2H) ; 3.29 (s, 3H).

### Step B: N-[(E)-(4-fluoro-3-methoxy-phenyl)methyleneamino]-N-(2-methoxyethyl)-1,1-dioxo-6-(4,4,5,5-tetramethyl-1,3,2-dioxaborolan-2-yl)-1,2-benzothiazol-3-amine

The compound was prepared using the same procedure as described in example 50 step F starting from 6-bromo-N-[(E)-(4-fluoro-3-methoxy-phenyl)methyleneamino]-N-(2-methoxyethyl)-1,1-dioxo-1,2-benzothiazol-3-amine (580.0 mg; 1.23 mmol; 1.00 eq.) giving N-[(E)-(4-fluoro-3-methoxy-phenyl)methyleneamino]-N-(2-methoxyethyl)-1,1-dioxo-6-(4,4,5,5-tetramethyl-1,3,2-dioxaborolan-2-yl)-1,2-benzothiazol-3-amine (523.0 mg; 82 %) as a yellow oil. ¹H NMR (DMSO-d₆, 300 MHz): δ 8.83 (d, J = 8.4 Hz, 1H) ; 8.60 (s, 1H) ; 8.17 (dd, J = 1.0 Hz, J = 7.9 Hz, 1H) ; 8.10 (s, 1H) ; 7.64 (m, 1H) ; 7.39 (m, 2H) ; 4.58 (t, J = 5.4 Hz, 2H) ; 3.98 (s, 3H) ; 3.73 (t, J = 5.5 Hz, 2H) ; 3.30 (s, 3H) ; 1.35 (s, 12H).

### Step C: [3-[[(E)-(4-fluoro-3-methoxy-phenyl)methyleneamino]-(2-methoxyethyl) amino]-1,1-dioxo-1,2-benzothiazol-6-yl]boronic acid

The compound was prepared using the same procedure as described in example 62 step C starting from N-[(E)-(4-fluoro-3-methoxy-phenyl)methyleneamino]-N-(2-methoxy ethyl)-1,1-dioxo-6-(4,4,5,5-tetramethyl-1,3,2-dioxaborolan-2-yl)-1,2-benzothiazol-3-amine (523.0 mg; 1.01 mmol; 1.00 eq.) giving [3-[[(E)-(4-fluoro-3-methoxy-phenyl)methyleneamino]-(2-methoxyethyl)amino]-1,1-dioxo-1,2-benzothiazol-6-yl] boronic acid (78.0 mg; 17 %) as a beige solid. ¹H NMR (DMSO-d₆, 400 MHz): δ 8.77 (d, J = 8.1 Hz, 1H) ; 8.65 (s, 2H) ; 8.59 (s, 1H) ; 8.35 (s, 1H) ; 8.24 (dd, J = 1.0 Hz, J = 8.0 Hz, 1H) ; 7.64 (dd, J = 1.9 Hz, J = 8.5 Hz, 1H) ; 7.43 (m, 2H) ; 4.58 (t, J = 5.6 Hz, 2H) ; 3.98 (s, 3H) ; 3.73 (t, J = 5.6 Hz, 2H) ; 3.30 (s, 3H). mp: 106-222 °C.

### Example 73: [3-[[(E)-(3-isopropyl-2-oxo-1H-benzimidazol-5-yl)methyleneamino]-(2-methoxyethyl)amino]-1,1-dioxo-1,2-benzothiazol-6-yl]boronic acid

### Step A: tert-butyl N-[(E)-(3-isopropyl-2-oxo-1H-benzimidazol-5-yl)methylene amino]carbamate

The compound was prepared using the same procedure as described in example 23 step F starting from 3-isopropyl-2-oxo-1H-benzimidazole-5-carbaldehyde (example 26, step C), (500.0 mg; 2.45 mmol; 1.00 eq.) giving tert-butyl N-[(E)-(3-isopropyl-2-oxo-1H-benzimidazol-5-yl)methyleneamino]carbamate (829.4 mg; quantitative) as a beige solid. ¹H NMR (DMSO-d₆, 300 MHz): δ 10.98 (s, 1H); 10.77 (br s, 1H) ; 7.99 (s, 1H); 7.44 (d, J = 1.2 Hz, 1H) ; 7.21 (dd, J = 1.4 Hz, J = 8.2 Hz, 1H) ; 6.98 (d, J = 7.9 Hz, 1H) ; 4.57 (sept, J = 7.0 Hz, 1H) ; 1.47 (s, 9H) ; 1.45 (d, J = 7.1 Hz, 6H).

### Step B: tert-butyl 5-[(E)-(tert-butoxycarbonylhydrazono)methyl]-3-isopropyl-2-oxo-benzimidazole-1-carboxylate

The compound was prepared using the same procedure as described in example 23 step G starting from tert-butyl N-[(E)-(3-isopropyl-2-oxo-1H-benzimidazol-5-yl)methyleneamino]carbamate (780.0 mg; 2.38 mmol; 1.00 eq.) giving tert-butyl 5-[(E)-(tert-butoxycarbonylhydrazono)methyl]-3-isopropyl-2-oxo-benzimidazole-1-carboxylate (663.0 mg; 67%) as an orange resin. ¹H NMR (DMSO-d₆, 300 MHz): δ 10.92 (br s, 1H) ; 8.03 (s, 1H) ; 7.75 (d, J = 8.4 Hz, 1H) ; 7.52 (d, J = 1.3 Hz, 1H) ; 7.35 (dd, J = 1.4 Hz, J = 8.5 Hz, 1H) ; 4.60 (sept, J = 6.6 Hz, 1H) ; 1.59 (s, 9H) ; 1.47 (m, 15H).

### Step C: tert-butyl 5-[(E)-[tert-butoxycarbonyl(2-methoxyethyl)hydrazono]methyl]-3-isopropyl-2-oxo-benzimidazole-1-carboxylate

The compound was prepared using the same procedure as described in example 23 step H starting from tert-butyl 5-[(E)-(tert-butoxycarbonylhydrazono)methyl]-3-isopropyl-2-oxo-benzimidazole-1-carboxylate (560.0 mg; 1.34 mmol; 1.00 eq.) and 1-bromo-2-methoxyethane (191 µL; 2.01 mmol; 1.50 eq.) giving tert-butyl 5-[(E)-[tert-butoxycarbonyl(2-methoxyethyl)hydrazono]methyl]-3-isopropyl-2-oxo-benzimidazole-1-carboxylate (472.1 mg; 73%) as a white solid. ¹H NMR (DMSO-d₆, 300 MHz): δ 8.08 (s, 1H) ; 7.76 (d, J = 8.4 Hz, 1H); 7.65 (d, J = 1.2 Hz, 1H) ; 7.43 (dd, J = 1.3 Hz, J = 8.4 Hz, 1H) ; 4.62 (sept, J = 7.0 Hz, 1H) ; 4.03 (m, 2H) ; 3.50 (t, J = 6.3 Hz, 2H) ; 3.27 (s, 3H) ; 1.59 (s, 9H) ; 1.50 (s, 9H) ; 1.47 (d, J = 6.9 Hz, 6H).

### Step D: 3-isopropyl-5-[(E)-(2-methoxyethylhydrazono)methyl]-1H-benzimidazol-2-one

The compound was prepared using the same procedure as described in example 23 step I starting from tert-butyl 5-[(E)-[tert-butoxycarbonyl(2-methoxyethyl)hydrazono] methyl]-3-isopropyl-2-oxo-benzimidazole-1-carboxylate (470.0 mg; 0.97 mmol; 1.00 eq.) giving tert-butyl 3-isopropyl-5-[(E)-(2-methoxyethylhydrazono)methyl]-1H-benzimidazol-2-one as an orange gum, used in the next step without further purification. ¹H NMR (DMSO-d₆, 300 MHz): δ 11.19 (s, 1H) ; 8.44 (s, 1H) ; 7.57 (s, 1H) ; 7.37 (dd, J = 1.4 Hz, J = 8.2 Hz, 1H) ; 7.06 (d, J = 8.1 Hz, 1H) ; 4.59 (sept, J = 6.9 Hz, 1H) ; 3.65 (t, J = 6.0 Hz, 2H) ; 3.41 (t, J = 5.3 Hz, 2H) ; 3.32 (s, 3H) ; 1.45 (d, J = 6.9 Hz, 6H).

### Step E: 5-[(E)-[(6-bromo-1,1-dioxo-1,2-benzothiazol-3-yl)-(2-methoxyethyl) hydrazono]methyl]-3-isopropyl-1H-benzimidazol-2-one

The compound was prepared using the same procedure as described in example 21 step B starting from 6-bromo-3-chloro-1,2-benzothiazole 1,1-dioxide (example 50, step D), (530.0 mg; 1.89 mmol; 1.95 eq.) and 3-isopropyl-5-[(E)-(2-methoxyethylhydrazono)methyl]-1H-benzimidazol-2-one (267.0 mg; 0.97 mmol; 1.00 eq.) giving 5-[(E)-[(6-bromo-1,1-dioxo-1,2-benzothiazol-3-yl)-(2-methoxyethyl)-hydrazono]methyl]-3-isopropyl-1H-benzimidazol-2-one (489.4 mg; 95 %) as a pale yellow solid. ¹H NMR (DMSO-d₆, 300 MHz): δ 11.19 (s, 1H) ; 8.71 (d, J = 8.6 Hz, 1H) ; 8.63 (s, 1H) ; 8.46 (d, J = 1.8 Hz, 1H) ; 8.04 (dd, J = 2.0 Hz, J = 8.6 Hz, 1H) ; 7.69 (s, 1H) ; 7.54 (dd, J = 1.4 Hz, J = 8.2 Hz, 1H) ; 7.14 (d, J = 8.1 Hz, 1H) ; 4.65 (m, 1H) ; 4.57 (t, J = 5.7 Hz, 2H) ; 3.73 (t, J = 5.6 Hz, 2H) ; 3.31 (s, 3H) ; 1.51 (d, J = 6.9 Hz, 6H).

### Step F: 5-[(E)-[[1,1-dioxo-6-(4,4,5,5-tetramethyl-1,3,2-dioxaborolan-2-yl)-1,2-benzothiazol-3-yl]-(2-methoxyethyl)hydrazono]methyl]-3-isopropyl-1H-benzimidazol-2-one

The compound was prepared using the same procedure as described in example 50 step F starting from 5-[(E)-[(6-bromo-1,1-dioxo-1,2-benzothiazol-3-yl)-(2-methoxyethyl)-hydrazono]methyl]-3-isopropyl-1H-benzimidazol-2-one (485.0 mg; 0.91 mmol; 1.00 eq.) giving 5-[(E)-[[1,1-dioxo-6-(4,4,5,5-tetramethyl-1,3,2-dioxaborolan-2-yl)-1,2-benzothiazol-3-yl]-(2-methoxyethyl)hydrazono]methyl]-3-isopropyl-1H-benzimidazol-2-one (288.6 mg; 56 %) as a beige solid. ¹H NMR (DMSO-d₆, 300 MHz): δ 11.19 (s, 1H) ; 8.87 (d, J = 8.1 Hz, 1H) ; 8.63 (s, 1H) ; 8.10 (m, 2H) ; 7.71 (d, J = 0.7 Hz, 1H) ; 7.58 (dd, J = 1.5 Hz, J = 8.3 Hz, 1H); 7.15 (d, J = 8.1 Hz, 1H) ; 4.67 (m, 1H) ; 4.58 (t, J = 5.4 Hz, 2H); 3.74 (t, J = 5.5 Hz, 2H) ; 3.31 (s, 3H) ; 1.51 (d, J = 6.9 Hz, 6H) ; 1.35 (s, 12H).

### Step G: 3-[[(E)-(3-isopropyl-2-oxo-1H-benzimidazol-5-yl)methyleneamino]-(2-methoxyethyl)amino]-1,1-dioxo-1,2-benzothiazol-6-yl]boronic acid

The compound was prepared using the same procedure as described in example 62 step C starting from 5-[(E)-[[1,1-dioxo-6-(4,4,5,5-tetramethyl-1,3,2-dioxaborolan-2-yl)-1,2-benzothiazol-3-yl]-(2-methoxyethyl)hydrazono]methyl]-3-isopropyl-1H-benzimi dazol-2-one (285.0 mg; 0.50 mmol; 1.00 eq.) giving 3-[[(E)-(3-isopropyl-2-oxo-1H-benzimidazol-5-yl)methyleneamino]-(2-methoxyethyl)amino]-1,1-dioxo-1,2-benzo thiazol-6-yl]boronic acid (129.2 mg; 53 %) as a beige solid. ¹H NMR (DMSO-d₆, 400 MHz): δ 11.20 (s, 1H) ; 8.81 (d, J = 7.9 Hz, 1H); 8.66 (s, 2H); 8.62 (s, 1H); 8.36 (s, 1H); 8.18 (dd, J = 1.1 Hz, J = 7.9 Hz, 1H) ; 7.73 (s, 1H) ; 7.53 (dd, J = 1.3 Hz, J = 8.1 Hz, 1H) ; 7.16 (d, J = 7.9 Hz, 1H) ; 4.67 (sept, J = 7.0 Hz, 1H) ; 4.58 (t, J = 5.6 Hz, 2H) ; 3.74 (t, J = 5.7 Hz, 2H) ; 3.31 (s, 3H); 1.52 (d, J = 6.8 Hz, 6H). mp: 243-266 °C.

### Example 74: 3-cyclobutyl-5-[(E)-[(1,1-dioxo-1,2-benzothiazol-3-yl)-methyl-hydrazorio]methyl]-1H-benzimidazole-2-thione

### Step A: 5-bromo-N-cyclobutyl-2-nitro-aniline

4-bromo-2-fluoro-1-nitrobenzene (600 mg; 2.73 mmol; 1.00 eq.) was dissolved in dry DCM (12.00 mL). Cyclobutylamine (351 µL; 4.09 mmol; 1.50 eq.) and K₂CO₃ (565 mg; 4.09 mmol; 1.50 eq.) were added and the mixture was stirred at rt. After completion, the mixture was poured onto water and extracted with DCM twice. Combined organic phases were dried over MgSO₄, filtered and concentrated to give 5-bromo-N-cyclobutyl-2-nitroaniline (719.0 mg; 97 %), used in the next step without purification. LC/MS (Method b): Rt = 3.07 min ; MS : m/z = 271-273 [M+H]⁺

### Step B: 4-bromo-N2-cyclobutyl-benzene-1,2-diamine

5-bromo-N-cyclobutyl-2-nitro-aniline (719 mg; 2.65 mmol; 1.00 eq.) was dissolved in a mixture of EtOH (10.00 mL) and HCl conc. (2.40 mL). Then SnCl₂.H₂O (2.99 g; 13.26 mmol; 5.00 eq.) was added and the mixture was stirred at 70 °C overnight. The reaction was allowed to cool to rt and after dilution with water, the pH was adjusted to 8 by the addition of aq. KOH solution. It was then extracted with EtOAc, dried over MgSO₄, filtered and concentrated to give 4-bromo-N2-cyclobutyl-benzene-1,2-diamine (500.0 mg, 78 %), which was used in the next step without further purification. LC/MS (Method b): Rt = 2.76 min ; MS : m/z = 241-243 [M+H]⁺

### Step C: 5-bromo-3-cyclobutyl-1H-benzimidazole-2-thione

4-bromo-N2-cyclobutyl-benzene-1,2-diamine (600.0 mg; 2.48 mmol; 1.00 eq.) and 1,1'-thiocarbonyldiimidazole (1.10 g; 6.19 mmol; 2.50 eq.) were combined in dry THF (12.00 mL) and stirred under reflux for 12 h. Then, the solvent was evaporated and the residue purified by chromatography (column SiO₂ 25g, flow rate 25ml/min, gradient from 100% n-heptane to 80/20 n-heptane / EtOAc) to give 5-bromo-3-cyclobutyl-1H-benzimidazole-2-thione (206.0 mg; 30 %). 1H NMR (CDCl₃, 300 MHz): δ 11.21 (s, 1H) ; 7.71 (s, 1H) ; 7.33 (d, J = 8.4 Hz, 1H) ; 7.13 (d, J = 8.4 Hz, 1H) ; 5.73 (m, 1H) ; 2.90 (m, 2H) ; 2.51 (m, 2H) ; 2.02 (m, 2H).

### Step D: 3-cyclobutyl-2-thioxo-1H-benzimidazole-5-carbaldehyde

The compound was prepared using the same procedure as described in example 20 step B starting from 5-bromo-3-cyclobutyl-1H-benzimidazole-2-thione (87.0 mg; 0.31 mmol; 1.00 eq) giving 3-cyclobutyl-2-thioxo-1H-benzimidazole-5-carbaldehyde (27.0 mg; 38 %) as a yellow solid. LC/MS (Method B): Rt = 2.24 min ; MS : m/z = 233 [M+H]⁺

### Step E: 3-cyclobutyl-5-[(E)-[(1,1-dioxo-1,2-benzothiazol-3-yl)-methyl-hydrazono]methyl]-1H-benzimidazole-2-thione

The compound was prepared using the same procedure as described in example 1 step C starting from 1-(1,1-dioxo-1,2-benzothiazol-3-yl)-1-methyl-hydrazine (example 1, step B), (24.0 mg; 0.12 mmol; 1.00 eq.) and 3-cyclobutyl-2-thioxo-1H-benzimidazole-5-carbaldehyde (27.0 mg; 0.12 mmol; 1.10 eq.) giving 3-cyclobutyl-5-[(E)-[(1,1-dioxo-1,2-benzothiazol-3-yl)-methyl-hydrazono]methyl]-1H-benzimidazole-2-thione (14.0 mg; 28 %) as a pale brown solid. 1H NMR (DMSO-d6, 300 MHz): δ 13.08 (s, 1H) ; 8.92 (d, J = 7.9 Hz, 1H) ; 8.63 (s, 1H) ; 8.19 (s, 1H) ; 8.10 (d, J = 7.9 Hz, 1H) ; 7.84 (m, 3H) ; 7.36 (d, J = 8.4 Hz, 1H) ; 5.72 (m, 1H) ; 3.83 (s, 3H) ; 2.97 (m, 2H) ; 2.41 (m, 2H) ; 1.97 (m, 2H). mp: > 270°C.

### Example 75: [3-[ethyl-[(E)-(3-isopropyl-2-oxo-1H-benzimidazol-5-yl)methylene amino]amino]-1,1-dioxo-1,2-benzothiazol-6-yl]boronic acid

### Step A: tert-butyl 5-[(E)-[tert-butoxycarbonyl(ethyl)hydrazono]methyl]-3-isopropyl-2-oxo-benzimidazole-1-carboxylate

The compound was prepared using the same procedure as described in example 23 step H starting from tert-butyl 5-[(E)-(tert-butoxycarbonylhydrazono)methyl]-3-isopropyl-2-oxo-benzimidazole-1-carboxylate (example 43, step B), (640.0 mg; 1.53 mmol; 1.00 eq.) and iodoethane (171 µL; 2.29 mmol; 1.50 eq.) giving tert-butyl tert-butyl 5-[(E)-[tert-butoxycarbonyl(ethyl)hydrazono]methyl]-3-isopropyl-2-oxo-benzimidazole-1-carboxylate (470.4 mg; 68%) as a white solid. ¹H NMR (DMSO-d₆, 300 MHz): δ 8.00 (s, 1H) ; 7.76 (d, J = 8.3 Hz, 1H) ; 7.67 (d, J = 1.3 Hz, 1H) ; 7.45 (dd, J = 1.4 Hz, J = 8.5 Hz, 1H) ; 4.62 (sept, J = 6.9 Hz, 1H) ; 3.89 (q, J = 6.8 Hz, 2H) ; 1.59 (s, 9H) ; 1.50 (s, 9H) ; 1.47 (d, J = 7.1 Hz, 6H) ; 1.10 (t, J = 6.9 Hz, 3H).

### Step B: 5-[(E)-(ethylhydrazono)methyl]-3-isopropyl-1H-benzimidazol-2-one

The compound was prepared using the same procedure as described in example 23 step I starting from tert-butyl 5-[(E)-[tert-butoxycarbonyl(ethyl)hydrazono]methyl]-3-isopropyl-2-oxo-benzimidazole-1-carboxylate (470.0 mg; 1.03 mmol; 1.00 eq.) giving 5-[(E)-(ethylhydrazono)methyl]-3-isopropyl-1H-benzimidazol-2-one as a yellow solild, used in the next step without further purification. ¹H NMR (DMSO-d₆, 300 MHz): δ 11.27 (s, 1H) ; 8.65 (s, 1H) ; 7.62 (s, 1H) ; 7.45 (dd, J = 1.3 Hz, J = 8.1 Hz, 1H) ; 7.09 (d, J = 8.1 Hz, 1H) ; 4.59 (sept, J = 6.9 Hz, 1H) ; 3.29 (q, J = 7.2 Hz, 2H) ; 1.45 (d, J = 6.9 Hz, 6H) ; 1.28 (t, J = 7.2 Hz, 3H).

### Step C: 5-[(E)-[(6-bromo-1,1-dioxo-1,2-benzothiazol-3-yl)-ethyl-hydrazono]methyl]-3-isopropyl-1H-benzimidazol-2-one

The compound was prepared using the same procedure as described in example 21 step B starting from 6-bromo-3-chloro-1,2-benzothiazole 1,1-dioxide (example 50, step D), (530.0 mg; 1.89 mmol; 1.83 eq.) and 5-[(E)-(ethylhydrazono)methyl]-3-isopropyl-1H-benzimidazol-2-one (254.1 mg; 1.03 mmol; 1.00 eq.) giving 5-[(E)-[(6-bromo-1,1-dioxo-1,2-benzothiazol-3-yl)-ethyl-hydrazono]methyl]-3-isopropyl-1H-benzimi dazol-2-one (357.7 mg; 71%) as a yellow solid. ¹H NMR (DMSO-d₆, 300 MHz): δ 11.19 (s, 1H) ; 8.72 (d, J = 8.6 Hz, 1H) ; 8.58 (s, 1H) ; 8.45 (d, J = 1.8 Hz, 1H) ; 8.05 (dd, J = 1.9 Hz, J = 8.5 Hz, 1H) ; 7.73 (s, 1H) ; 7.58 (dd, J = 1.2 Hz, J = 8.1 Hz, 1H) ; 7.14 (d, J = 8.1 Hz, 1H) ; 4.64 (sept, J = 7.1 Hz, 1H) ; 4.39 (q, J = 6.7 Hz, 2H) ; 1.51 (d, J = 6.9 Hz, 6H) ; 1.31 (t, J = 7.1 Hz, 3H). Step D: 5-[(E)-[[1,1-dioxo-6-(4,4,5,5-tetramethyl-1,3,2-dioxaborolan-2-yl)-1,2-benzothiazol-3-yl]-ethyl-hydrazono]methyl]-3-isopropyl-1H-benzimidazol-2-one

The compound was prepared using the same procedure as described in example 50 step F starting from 5-[(E)-[(6-bromo-1,1-dioxo-1,2-benzothiazol-3-yl)-ethyl-hydrazono] methyl]-3-isopropyl-1H-benzimidazol-2-one (350.0 mg; 0.71 mmol; 1.00 eq.) giving 5-[(E)-[[1,1-dioxo-6-(4,4,5,5-tetramethyl-1,3,2-dioxaborolan-2-yl)-1,2-benzothiazol-3-yl]-ethyl-hydrazono]methyl]-3-isopropyl-1H-benzimidazol-2-one (113.0 mg; 29 %) as a beige solid. ¹H NMR (DMSO-d₆, 300 MHz): δ 11.19 (s, 1H) ; 8.88 (d, J = 7.9 Hz, 1H) ; 8.59 (s, 1H) ; 8.11 (m, 2H); 7.75 (s, 1H); 7.63 (dd, J = 1.0 Hz, J = 8.3 Hz, 1H); 7.16 (d, J = 8.1 Hz, 1H) ; 4.65 (sept, J = 6.9 Hz, 1H) ; 4.41 (q, J = 6.9 Hz, 2H) ; 1.51 (d, J = 6.9 Hz, 6H) ; 1.35 (m, 15H).

### Step E: [3-[ethyl-[(E)-(3-isopropyl-2-oxo-1H-benzimidazol-5-yl)methyleneamino] amino]-1,1-dioxo-1,2-benzothiazol-6-yl]boronic acid

The compound was prepared using the same procedure as described in example 62 step C starting from 5-[(E)-[[1,1-dioxo-6-(4,4,5,5-tetramethyl-1,3,2-dioxaborolan-2-yl)-1,2-benzothiazol-3-yl]-ethyl-hydrazono]methyl]-3-isopropyl-1H-benzimidazol-2-one (110.0 mg; 0.20 mmol; 1.00 eq.) giving [3-[ethyl-[(E)-(3-isopropyl-2-oxo-1H-benzimidazol-5-yl)methyleneamino]amino]-1,1-dioxo-1,2-benzothiazol-6-yl]boronic acid (67.0 mg; 72 %) as a beige solid. ¹H NMR (DMSO-d₆ + 10% v/v D₂O, 400 MHz): δ 8.83 (d, J = 8.1 Hz, 1H); 8.56 (s, 1H); 8.33 (s, 1H); 8.19 (dd, J = 1.1 Hz, J = 7.9 Hz, 1H); 7.78 (d, J = 1.1 Hz, 1H); 7.60 (dd, J = 1.4 Hz, J = 8.3 Hz, 1H) ; 7.21 (d, J = 8.1 Hz, 1H) ; 4.67 (sept, J = 6.9 Hz, 1H) ; 4.41 (q, J = 6.9 Hz, 2H); 1.53 (d, J = 7.0 Hz, 6H); 1.33 (t, J = 7.0 Hz, 3H). mp: 123-152 °C.

### Example 76: [3-[[(E)-(3-cyclobutyl-2-oxo-1H-benzimidazol-5-yl)methyleneamino]-methyl-amino]-1,1-dioxo-1,2-benzothiazol-6-yl]boronic acid

### Step A: 3-cyclobutyl-5-[(E)-(methylhydrazono)methyl]-1H-benzimidazol-2-one

The compound was prepared using the same procedure as described in example 21 step A starting from 3-cyclobutyl-2-oxo-1H-benzimidazole-5-carbaldehyde (example 28, step E), (614.1 mg; 2.84 mmol; 1.00 eq.) giving 3-cyclobutyl-5-[(E)-(methylhydrazono)methyl]-1H-benzimidazol-2-one (693.0 mg; quant.) as a solid, used directly in the next step without purification.

### Step B: 5-[(E)-[(6-bromo-1,1-dioxo-1,2-benzothiazol-3-yl)-methyl-hydrazono] methyl]-3-cyclobutyl-1H-benzimidazol-2-one

The compound was prepared using the same procedure as described in example 21 step B starting from 6-bromo-3-chloro-1,2-benzothiazole 1,1-dioxide (example 50, step D), (796.7 mg; 2.84 mmol; 1.00 eq.) and 3-cyclobutyl-5-[(E)-(methylhydrazono)methyl]-1H-benzimidazol-2-one (693.8 mg; 2.84 mmol; 1.00 eq.) giving 5-[(E)-[(6-bromo-1,1-dioxo-1,2-benzothiazol-3-yl)-methyl-hydrazono]methyl]-3-cyclobutyl-1H-benzimidazol-2-one (637.0 mg; 46 %) as a yellow solid. LC/MS (Method a): Rt = 2.05 min ; MS : m/z = 488-491 [M+H]⁺

### Step C: 3-cyclobutyl-5-[(E)-[[1,1-dioxo-6-(4,4,5,5-tetramethyl-1,3,2-dioxaborolan-2-yl)-1,2-benzothiazol-3-yl]-methyl-hydrazono]methyl]-1H-benzimidazol-2-one

The compound was prepared using the same procedure as described in example 50 step F starting from 5-[(E)-[(6-bromo-1,1-dioxo-1,2-benzothiazol-3-yl)-methyl-hydrazono]methyl]-3-cyclobutyl-1H-benzimidazol-2-one (637.0 mg; 1.30 mmol; 1.00 eq.) giving 3-cyclobutyl-5-[(E)-[[1,1-dioxo-6-(4,4,5,5-tetramethyl-1,3,2-dioxaborolan-2-yl)-1,2-benzothiazol-3-yl]-methyl-hydrazono]methyl]-1H-benzimidazol-2-one (610.0 mg; 87 %) as a grey solid. ¹H NMR (DMSO-d₆, 300 MHz): δ 11.22 (br s, 1H) ; 8.84 (br d, J = 6.8 Hz, 1H) ; 8.50 (s, 1H) ; 8.03 (m, 2H) ; 7.82 (s, 1H) ; 7.57 (br d, J = 8.3 Hz, 1H) ; 7.16 (br d, J = 7.3 Hz, 1H) ; 4.90 (quint., J = 9.1 Hz, 1H) ; 3.80 (s, 3H) ; 2.86 (m, 2H) ; 2.35 (m, 2H) ; 1.90 (m, 2H) ; 1.23 (s, 12H).

### Step D: [3-[[(E)-(3-cyclobutyl-2-oxo-1H-benzimidazol-5-yl)methyleneamino]-methyl-amino]-1,1-dioxo-1,2-benzothiazol-6-yl]boronic acid

The compound was prepared using the same procedure as described in example 62 step C starting from 3-cyclobutyl-5-[(E)-[[1,1-dioxo-6-(4,4,5,5-tetramethyl-1,3,2-dioxaborolan-2-yl)-1,2-benzothiazol-3-yl]-methyl-hydrazono]methyl]-1H-benzimi-dazol-2-one (620.0 mg; 1.16 mmol; 1.00 eq.) giving [3-[[(E)-(3-cyclobutyl-2-oxo-1H-benzimidazol-5-yl)methyleneamino]-methyl-amino]-1,1-dioxo-1,2-benzothiazol-6-yl]boronic acid (310.0 mg; 58 %) as a green solid. ¹H NMR (DMSO-d₆ + 10% v/v D₂O, 400 MHz): δ 8.88 (d, J = 8.0 Hz, 1H) ; 8.50 (s, 1H) ; 8.34 (s, 1H) ; 8.19 (d, J = 8.0 Hz, 1H) ; 7.83 (s, 1H) ; 7.58 (d, J = 8.0 Hz, 1H) ; 7.19 (d, J = 8.0 Hz, 1H); 4.90 (quint., J = 8.8 Hz, 1H) ; 3.82 (s, 3H) ; 2.86 (m, 2H) ; 2.38 (m, 2H) ; 1.89 (m, 2H).

### Example 77: [3-[[(E)-(3-cyclobutyl-2-oxo-1H-benzimidazol-5-yl)methyleneamino]-ethyl-amino]-1,1-dioxo-1,2-benzothiazol-6-yl]boronic acid

### Step A: 3-cyclobutyl-5-[(E)-(ethylhydrazono)methyl]-1H-benzimidazol-2-one

The compound was prepared using the same procedure as described in example 21 step A starting from 3-cyclobutyl-2-oxo-1H-benzimidazole-5-carbaldehyde (example 28, step E), (307.1 mg; 1.42 mmol; 1.00 eq.) and 1-ethylhydrazine hydrochloride (137.1 mg; 1.42 mmol; 1.00 eq.) giving 3-cyclobutyl-5-[(E)-(ethylhydrazono)methyl]-1H-benzimidazol-2-one (366.0 mg; quant.) as a solid, used directly in next step without purification. LC/MS (Method a): Rt = 1.36 min ; MS : m/z = 259 [M+H]⁺

### Step B: 5-[(E)-[(6-bromo-1,1-dioxo-1,2-benzothiazol-3-yl)-ethyl-hydrazono]methyl]-3-cyclobutyl-1H-benzimidazol-2-one

The compound was prepared using the same procedure as described in example 21 step B starting from 6-bromo-3-chloro-1,2-benzothiazole 1,1-dioxide (example 50, step D), (398.4 mg; 1.42 mmol; 1.00 eq.) and 3-cyclobutyl-5-[(E)-(ethylhydrazono)methyl]-1H-benzimidazol-2-one (366.8 mg; 1.42 mmol; 1.00 eq.) giving 5-[(E)-[(6-bromo-1,1-dioxo-1,2-benzothiazol-3-yl)-ethyl-hydrazono]methyl]-3-cyclobutyl-1H-benzimidazol-2-one (280.0 mg; 39 %) as an orange solid. LC/MS (Method a): Rt = 2.13 min ; MS : m/z = 502-504 [M+H]⁺

### Step C: 3-cyclobutyl-5-[(E)-[[1,1-dioxo-6-(4,4,5,5-tetramethyl-1,3,2-dioxaborolan-2-yl)-1,2-benzothiazol-3-yl]-ethyl-hydrazono]methyl]-1H-benzimidazol-2-one

The compound was prepared using the same procedure as described in example 50 step F starting from 5-[(E)-[(6-bromo-1,1-dioxo-1,2-benzothiazol-3-yl)-ethyl-hydrazono] methyl]-3-cyclobutyl-1H-benzimidazol-2-one (280.0 mg; 0.56 mmol; 1.00 eq.) giving 3-cyclobutyl-5-[(E)-[[1,1-dioxo-6-(4,4,5,5-tetramethyl-1,3,2-dioxaborolan-2-yl)-1,2-benzothiazol-3-yl]-ethyl-hydrazono]methyl]-1H-benzimidazol-2-one (200.0 mg; 65 %) as a yellow solid. ¹H NMR (DMSO-d₆, 300 MHz): δ 11.21 (br s, 1H) ; 8.78 (br d, J = 7.6 Hz, 1H) ; 8.57 (m, 1H) ; 8.01 (m, 2H) ; 7.83 (s, 1H) ; 7.59 (br d, J = 8.3 Hz, 1H) ; 7.16 (br d, J = 7.3 Hz, 1H) ; 4.89 (quint., J = 8.9 Hz, 1H) ; 4.40 (q, J = 6.6 Hz, 2H) ; 2.86 (m, 2H) ; 2.34 (m, 2H); 1.89 (m, 2H) ; 1.31 (br t, J = 7.0 Hz, 3H) ; 1.22 (s, 12H).

### Step D: [3-[[(E)-(3-cyclobutyl-2-oxo-1H-benzimidazol-5-yl)methyleneamino]-ethyl-amino]-1,1-dioxo-1,2-benzothiazol-6-yl]boronic acid

The compound was prepared using the same procedure as described in example 62 step C starting from 3-cyclobutyl-5-[(E)-[[1,1-dioxo-6-(4,4,5,5-tetramethyl-1,3,2-dioxaborolan-2-yl)-1,2-benzothiazol-3-yl]-ethyl-hydrazono]methyl]-1H-benzimidazol-2-one (200.0 mg; 0.36 mmol; 1.00 eq.) giving [3-[[(E)-(3-cyclobutyl-2-oxo-1H-benzimidazol-5-yl)methyleneamino]-ethyl-amino]-1,1-dioxo-1,2-benzothiazol-6-yl]boronic acid (105.0 mg; 60 %) as a green solid. ¹H NMR (DMSO-d₆ + 10% v/v D₂O, 400 MHz): δ 8.84 (d, J = 7.9 Hz, 1H); 8.57 (s, 1H); 8.34 (s, 1H); 8.19 (dd, J = 1.1 Hz, J = 7.9 Hz, 1H); 7.86 (d, J = 1.3 Hz, 1H) ; 7.61 (dd, J = 1.3 Hz, J = 8.1 Hz, 1H) ; 7.21 (d, J = 8.1 Hz, 1H) ; 4.90 (quint., J = 8.9 Hz, 1H) ; 4.42 (q, J = 7.5 Hz, 2H) ; 2.86 (dquint., J = 2.9 Hz, J = 9.7 Hz, 2H) ; 2.38 (m, 2H) ; 1.90 (m, 2H) ; 1.33 (t, J = 7.0 Hz, 3H).

### Example 78: N-[(E)-[4-fluoro-3-(trifluoromethoxy)phenyl]methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B), (100.0 mg; 0.47 mmol; 1.00 eq.) and 4-fluoro-3-(trifluoromethoxy)benzaldehyde (108.4 mg; 0.52 mmol; 1.10 eq.) giving N-[(E)-[4-fluoro-3-(trifluoromethoxy)phenyl]methyleneamino]-N-methyl-1,1-dioxo-1,2-benzothiazol-3-amine (118.4 mg; 57 %) as a white solid. 1H NMR (DMSO-d₆, 400 MHz): δ 8.78 (m, 1H) ; 8.53 (s, 1H) ; 8.11 (m, 1H) ; 8.01 (m, 2H) ; 7.89 (m, 2H); 7.73 (dd, J = 8.6 Hz, J = 10.1 Hz, 1H) ; 3.79 (s, 3H). mp: 230-280 °C.

### Example 79: [3-[[(E)-(3-cyclobutyl-2-oxo-1H-benzimidazol-5-yl)methyleneamino]-(2-methoxyethyl)amino]-1,1-dioxo-1,2-benzothiazol-6-yl]boronic acid

### Step A: 3-cyclobutyl-5-[(E)-(2-methoxyethylhydrazono)methyl]-1H-benzimidazol-2-one

The compound was prepared using the same procedure as described in example 21 step A starting from 3-cyclobutyl-2-oxo-1H-benzimidazole-5-carbaldehyde (example 28, step E), (307.1 mg; 1.42 mmol; 1.00 eq.) and (2-methoxyethyl)hydrazine hydrochloride (179.8 mg; 1.42 mmol; 1.00 eq.) giving 3-cyclobutyl-5-[(E)-(2-methoxyethylhydrazono)methyl]-1H-benzimidazol-2-one (409.0 mg; quant.) as a solid, used directly in the next step without purification. LC/MS (Method a): Rt = 1.42 min ; MS : m/z = 289 [M+H]⁺

### Step B: 5-[(E)-[(6-bromo-1,1-dioxo-1,2-benzothiazol-3-yl)-(2-methoxyethyl) hydrazono]methyl]-3-cyclobutyl-1H-benzimidazol-2-one

The compound was prepared using the same procedure as described in example 21 step B starting from 6-bromo-3-chloro-1,2-benzothiazole 1,1-dioxide (example 50, step D), (398.4 mg; 1.42 mmol; 1.00 eq.) and 3-cyclobutyl-5-[(E)-(2-methoxyethyl hydrazono)methyl]-1H-benzimidazol-2-one (409.0 mg; 1.42 mmol; 1.00 eq.) giving 5-[(E)-[(6-bromo-1,1-dioxo-1,2-benzothiazol-3-yl)-(2-methoxyethyl)hydrazono] methyl]-3-cyclobutyl-1H-benzimidazol-2-one (260.0 mg; 34 %) as a yellow solid. LC/MS (Method a): Rt = 2.11 min ; MS : m/z = 532-534 [M+H]⁺

### Step C: 3-cyclobutyl-5-[(E)-[[1,1-dioxo-6-(4,4,5,5-tetramethyl-1,3,2-dioxaborolan-2-yl)-1,2-benzothiazol-3-yl]-(2-methoxyethyl)hydrazono]methyl]-1H-benzimidazol-2-one

The compound was prepared using the same procedure as described in example 50 step F starting from -[(E)-[(6-bromo-1,1-dioxo-1,2-benzothiazol-3-yl)-(2-methoxyethyl) hydrazono]methyl]-3-cyclobutyl-1H-benzimidazol-2-one (260.0 mg; 0.49 mmol; 1.00 eq.) giving 3-cyclobutyl-5-[(E)-[[1,1-dioxo-6-(4,4,5,5-tetramethyl-1,3,2-dioxaborolan -2-yl)-1,2-benzothiazol-3-yl]-(2-methoxyethyl)hydrazono]methyl]-1H-benzimidazol-2-one (220.0 mg; 78 %) as a green solid. ¹H NMR (DMSO-d₆, 300 MHz): δ 11.21 (br s, 1H); 8.77 (m, 1H); 8.61 (s, 1H) ; 8.02 (m, 2H); 7.79 (s, 1H); 7.55 (m, 1H); 7.16 (m, 1H); 4.88 (quint., J = 8.9 Hz, 1H); 4.58 (br s, 2H); 3.74 (br t, J = 5.1 Hz, 2H) ; 3.31 (s, 3H); 2.87 (m, 2H); 2.34 (m, 2H); 1.89 (m, 2H); 1.23 (s, 12H).

### Step D: [3-[[(E)-(3-cyclobutyl-2-oxo-1H-benzimidazol-5-yl)methyleneamino]-(2-methoxyethyl)amino]-1,1-dioxo-1,2-benzothiazol-6-yl]boronic acid

The compound was prepared using the same procedure as in example 62 step C starting from 3-cyclobutyl-5-[(E)-[[1,1-dioxo-6-(4,4,5,5-tetramethyl-1,3,2-dioxaboro lan-2-yl)-1,2-benzothiazol-3-yl]-(2-methoxyethyl)hydrazono]methyl]-1H-benzimida zol-2-one (220.0 mg; 0.38 mmol; 1.00 eq.) giving [3-[[(E)-(3-cyclobutyl-2-oxo-1H-benzimidazol-5-yl)methyleneamino]-(2-methoxyethyl)amino]-1,1-dioxo-1,2-benzo thiazol-6-yl]boronic acid (123.0 mg; 65 %) as a green solid. ¹H NMR (DMSO-d₆, 400 MHz): δ 11.22 (s, 1H) ; 8.82 (d, J = 7.9 Hz, 1H) ; 8.64 (m, 3H) ; 8.36 (s, 1H); 8.19 (dd, J = 1.0 Hz, J = 8.0 Hz, 1H) ; 7.81 (s, 1H); 7.54 (dd, J = 1.3 Hz, J = 8.1 Hz, 1H); 7.16 (d, J = 7.9 Hz, 1H); 4.90 (quint., J = 8.9 Hz, 1H) ; 4.59 (t, J = 5.7 Hz, 2H) ; 3.75 (t, J = 5.6 Hz, 2H) ; 3.31 (s, 3H) ; 2.87 (dquint., J = 2.5 Hz, J = 9.7 Hz, 2H); 2.37 (m, 2H); 1.90 (m, 2H).

### Example 80: [3-[cyclobutylmethyl-[(E)-(4-fluoro-3-methoxy-phenyl)methylene amino]amino]-1,1-dioxo-1,2-benzothiazol-6-yl]boronic acid

### Step A: tert-butyl N-(cyclobutylmethyl)-N-[(E)-(4-fluoro-3-methoxy-phenyl) methyleneamino]carbamate

The compound was prepared using the same procedure as described in example 23 step H starting from tert-butyl N-[(E)-(4-fluoro-3-methoxy-phenyl)methyleneamino] carbamate (example 43, step A), (500.0 mg; 1.86 mmol; 1.00 eq.) and bromomethylcyclobutane (416.6 mg; 2.80 mmol; 1.50 eq.) giving tert-butyl N-(cyclobutylmethyl)-N-[(E)-(4-fluoro-3-methoxy-phenyl)methyleneamino]carbamate (592.0 mg; 94 %) as a colorless resin. ¹H NMR (DMSO-d₆, 300 MHz): δ 7.92 (s, 1H); 7.49 (m, 1H); 7.25 (m, 2H); 3.91 (d, J = 7.3 Hz, 2H); 3.87 (s, 3H); 2.62 (quint., J = 7.4 Hz, 1H); 1.95 (m, 2H); 1.78 (m, 4H); 1.49 (s, 9H).

### Step B: 1-cyclobutyl-N-[(E)-(4-fluoro-3-methoxy-phenyl)methyleneamino] methanamine

The compound was prepared using the same procedure as described in example 23 step I starting from tert-butyl N-(cyclobutylmethyl)-N-[(E)-(4-fluoro-3-methoxy-phenyl)methyleneamino]carbamate (585.0 mg; 1.74 mmol; 1.00 eq.) giving 1-cyclobutyl-N-[(E)-(4-fluoro-3-methoxy-phenyl)methyleneamino]methanamine (410.0 mg; quant.) as a yellow resin, used in the next step without purification. ¹H NMR (DMSO-d₆, 300 MHz): δ 8.27 (s, 1H); 7.45 (dd, J = 1.7 Hz, J = 8.3 Hz, 1H); 7.31 (dd, J = 8.4 Hz, J = 11.1 Hz, 1H); 7.24 (m, 1H); 3.88 (s, 3H); 3.25 (d, J = 7.1 Hz, 2H); 2.68 (quint., J = 7.3 Hz, 1H); 2.05 (m, 2H); 1.84 (m, 4H).

### Step C: 6-bromo-N-(cyclobutylmethyl)-N-[(E)-(4-fluoro-3-methoxy-phenyl) methyleneamino]-1,1-dioxo-1,2-benzothiazol-3-amine

The compound was prepared using the same procedure as described in example 21 step B starting from 6-bromo-3-chloro-1,2-benzothiazole 1,1-dioxide (example 50, step D), (530.0 mg; 1.89 mmol; 1.09 eq.) and 1-cyclobutyl-N-[(E)-(4-fluoro-3-methoxyphenyl)methyleneamino]methanamine (410.0 mg; 1.74 mmol; 1.00 eq.) giving 6-bromo-N-(cyclobutylmethyl)-N-[(E)-(4-fluoro-3-methoxy-phenyl)methyleneamino]-1,1-dioxo-1,2-benzothiazol-3-amine (552.0 mg; 66 %) as a pale yellow solid. ¹H NMR (DMSO-d₆, 300 MHz): δ 8.67 (d, J = 8.6 Hz, 1H); 8.52 (s, 1H); 8.46 (d, J = 1.8 Hz, 1H); 8.15 (dd, J = 1.9 Hz, J = 8.5 Hz, 1H); 7.62 (dd, J = 1.8 Hz, J = 8.4 Hz, 1H); 7.46 (ddd, J = 2.0 Hz, J = 4.8 Hz, J = 8.4 Hz, 1H); 7.40 (dd, J = 8.4 Hz, J = 10.9 Hz, 1H); 4.48 (d, J = 7.3 Hz, 2H); 3.97 (s, 3H); 2.84 (quint., J = 7.6 Hz, 1H) ; 1.91 (m, 6H).

### Step D: [3-[cyclobutylmethyl-[(E)-(4-fluoro-3-methoxy-phenyl)methyleneamino] amino]-1,1-dioxo-1,2-benzothiazol-6-yl]boronic acid

The compound was prepared using the same procedure as described in example 50 step F starting from 6-bromo-N-(cyclobutylmethyl)-N-[(E)-(4-fluoro-3-methoxyphenyl)methyleneamino]-1,1-dioxo-1,2-benzothiazol-3-amine (550.0 mg; 1.14 mmol; 1.00 eq.) giving N-(cyclobutylmethyl)-N-[(E)-(4-fluoro-3-methoxy-phenyl)methyleneamino]-1,1-dioxo-6-(4,4,5,5-tetramethyl-1,3,2-dioxaborolan-2-yl)-1,2-benzothiazol-3-amine. The latter was directly purified by LC/MS-Preparative (Column: Kinetex C18 21,20 x 150mm 5µm (phenomenex); Flow rate: 25 ml/min; Elution: H2O, 0.1% HCOOH / ACN, 0.1% HCOOH; Gradient: 5 to 95% ACN over 36 minutes) to give [3-[cyclobutylmethyl-[(E)-(4-fluoro-3-methoxy-phenyl) methyleneamino]amino]-1,1-dioxo-1,2-benzothiazol-6-yl]boronic acid (73.9 mg; 14 %) as a beige solid. ¹H NMR (DMSO-d₆ + 10% v/v D₂O, 400 MHz): δ 8.78 (d, J = 8.1 Hz, 1H); 8.49 (s, 1H); 8.32 (s, 1H); 8.24 (d, J = 7.9 Hz, 1H); 7.65 (d, J = 8.6 Hz, 1H); 7.50 (ddd, J = 1.8 Hz, J = 4.4 Hz, J = 8.4 Hz, 1H); 7.41 (dd, J = 8.3 Hz, J = 11.1 Hz, 1H); 4.49 (d, J = 7.0 Hz, 2H); 3.98 (s, 3H); 2.86 (quint., J = 7.6 Hz, 1H); 2.02 (m, 2H); 1.88 (m, 4H). mp: 199-242 °C.

### Example 81: [5-fluoro-3-[[(E)-(4-fluoro-3-methoxy-phenyl)methyleneamino]-N-isobutylamino]-1,1-dioxo-1,2-benzothiazol-6-yl]boronic acid

### Step A: 5-bromo-4-fluoro-2-methyl-benzenesulfonamide

The compound was prepared using the same procedure as described in example 50 step A starting from 5-bromo-4-fluoro-2-methyl-benzenesulfonyl chloride (1.00 g; 3.48 mmol; 1.00 eq.) giving 5-bromo-4-fluoro-2-methyl-benzenesulfonamide (907.6 mg; 97 %) as a white solid. ¹H NMR (DMSO-d₆, 300 MHz): δ 8.06 (d, J = 6.9 Hz, 1H); 7.57 (s, 2H); 7.48 (d, J = 9.9 Hz, 1H); 2.56 (s, 3H).

### Step B: 4-bromo-5-fluoro-2-sulfamoyl-benzoic acid

The compound was prepared using the same procedure as described in example 50 step B starting from 5-bromo-4-fluoro-2-methyl-benzenesulfonamide (900.0 mg; 3.36 mmol; 1.00 eq.) giving 4-bromo-5-fluoro-2-sulfamoyl-benzoic acid (495.1 mg; 50 %) as a white solid, used in the next step without purification. ¹H NMR (DMSO-d₆, 300 MHz): δ 8.21 (d, J = 6.6 Hz, 1H); 7.76 (d, J = 8.8 Hz, 1H); 7.38 (s, 2H).

### Step C: 6-bromo-5-fluoro-1,1-dioxo-1,2-benzothiazol-3-one

The compound was prepared using the same procedure as described in example 50 step C starting from 4-bromo-5-fluoro-2-sulfamoyl-benzoic acid (495.0 mg; 1.66 mmol; 1.00 eq.) giving 6-bromo-5-fluoro-1,1-dioxo-1,2-benzothiazol-3-one (328.8 mg; 70 %) as a white solid. ¹H NMR (DMSO-d₆, 300 MHz): δ 8.60 (d, J = 5.6 Hz, 1H); 7.85 (d, J = 7.6 Hz, 1H).

### Step D: 6-bromo-3-chloro-5-fluoro-1,2-benzothiazole 1,1-dioxide

The compound was prepared using the same procedure as described in example 50 step D starting from 6-bromo-5-fluoro-1,1-dioxo-1,2-benzothiazol-3-one (325.0 mg; 1.15 mmol; 1.00 eq.) giving 6-bromo-3-chloro-5-fluoro-1,2-benzothiazole 1,1-dioxide (334.9 mg; 97 %) as a white solid, used in the next step without purification. ¹H NMR (DMSO-d₆, 300 MHz): δ 8.72 (d, J = 5.8 Hz, 1H); 7.95 (d, J = 7.4 Hz, 1H).

### Step E: 6-bromo-5-fluoro-N-[(E)-(4-fluoro-3-methoxy-phenyl)methyleneamino]-N-isobutyl-1,1-dioxo-1,2-benzothiazol-3-amine

The compound was prepared using the same procedure as described in example 21 step B starting from 6-bromo-3-chloro-5-fluoro-1,2-benzothiazole 1,1-dioxide (334.9 mg; 1.12 mmol; 1.00 eq.) and N-[(E)-(4-fluoro-3-methoxy-phenyl)methyleneamino]-2-methyl-propan-1-amine (example 40, step A), (436.0 mg; 1.94 mmol; 1.73 eq.) giving 6-bromo-5-fluoro-N-[(E)-(4-fluoro-3-methoxy-phenyl)methyleneamino]-N-isobutyl-1,1-dioxo-1,2-benzothiazol-3-amine (272.0 mg; 50 %) as a pale yellow solid. ¹H NMR (DMSO-d₆, 300 MHz): δ 8.69 (d, J = 6.3 Hz, 1H); 8.64 (d, J = 9.7 Hz, 1H); 8.56 (s, 1H); 7.66 (m, 1H); 7.43 (m, 2H); 4.25 (d, J = 7.8 Hz, 2H); 3.97 (s, 3H); 2.25 (m, J = 7.0 Hz, 1H) ; 0.98 (d, J = 6.8 Hz, 6H).

### Step F: [5-fluoro-3-[[(E)-(4-fluoro-3-methoxy-phenyl)methyleneamino]-isobutyl-amino]-1,1-dioxo-1,2-benzothiazol-6-yl]boronic acid

The compound was prepared using the same procedure as in example 50 step F starting from 6-bromo-5-fluoro-N-[(E)-(4-fluoro-3-methoxy-phenyl)methyleneamino] -N-isobutyl-1,1-dioxo-1,2-benzothiazol-3-amine (270.0 mg; 0.56 mmol; 1.00 eq.) giving 5-fluoro-N-[(E)-(4-fluoro-3-methoxy-phenyl)methyleneamino]-N-isobutyl-1,1-dioxo-6-(4,4,5,5-tetramethyl-1,3,2-dioxaborolan-2-yl)-1,2-benzothiazol-3-amine. The latter was directly purified by LC/MS-Preparative (Column: Kinetex C18 21,20 x 150mm 5µm (phenomenex); Flow rate: 25 ml/min; Elution: H2O, 0.1% HCOOH / ACN, 0.1% HCOOH; Gradient: 5 to 95% ACN over 36 minutes) to give [5-fluoro-3-[[(E)-(4-fluoro-3-methoxyphenyl)methyleneamino]-isobutyl-amino]-1,1-dioxo-1,2-benzothiazol-6-yl]boronic acid (110.9 mg; 43 %) as a beige solid. ¹H NMR (DMSO-d₆ + 10% v/v D₂O, 400 MHz): δ 8.53 (s, 1H); 8.47 (d, J = 10.1 Hz, 1H); 8.15 (d, J = 5.3 Hz, 1H); 7.66 (d, J = 8.6 Hz, 1H); 7.43 (m, 2H); 4.26 (d, J = 7.7 Hz, 2H); 3.97 (s, 3H) ; 2.26 (m, J = 6.7 Hz, 1H); 0.99 (d, J = 6.6 Hz, 6H). mp: 212-250 °C.

### Example 82: 5-[(E)-[(1,1-dioxo-1,2-benzothiazol-3-yl)-ethyl-hydrazono]methyl]-3,3-diethyl-indolin-2-one

### Step A: tert-butyl N-[(E)-(3,3-diethyl-2-oxo-indolin-5-yl)methyleneamino]carbamate

The compound was prepared using the same procedure as in example 23 step F starting from 3,3-diethyl-2-oxo-indoline-5-carbaldehyde (example 34, step C), (1.80 g; 8.28 mmol; 1.00 eq.) giving tert-butyl N-[(E)-(3,3-diethyl-2-oxo-indolin-5-yl)methyl eneamino]carbamate (2.42 g; 88 %) as a white solid. ¹H NMR (DMSO-d₆, 300 MHz): δ 10.76 (br s, 1H); 10.54 (s, 1H); 7.95 (s, 1H); 7.47 (s, 1H); 7.37 (d, J = 7.9 Hz, 1H); 6.86 (d, J = 7.9 Hz, 1H); 1.75 (m, 4H); 1.46 (s, 9H); 0.51 (t, J = 7.1 Hz, 6H).

### Step B: tert-butyl 5-[(E)-(tert-butoxycarbonylhydrazono)methyl]-3,3-diethyl-2-oxo-indoline-1-carboxylate

The compound was prepared using the same procedure as described in example 23 step G starting from tert-butyl N-[(E)-(3,3-diethyl-2-oxo-indolin-5-yl)methyleneamino] carbamate (2.22 g; 6.70 mmol; 1.00 eq.) giving tert-butyl 5-[(E)-(tert-butoxy carbonylhydrazono)methyl]-3,3-diethyl-2-oxo-indoline-1-carboxylate (2.88 g; quant) as a white solid. LC/MS (Method d): Rt = 2.32 min ; MS : m/z = 376 [M+H-tBu]⁺

### Step C: tert-butyl 5-[(E)-[tert-butoxycarbonyl(ethyl)hydrazono]methyl]-3,3-diethyl-2-oxo-indoline-1-carboxylate

The compound was prepared using the same procedure as described in example 23 step H starting from tert-butyl 5-[(E)-(tert-butoxycarbonylhydrazono)methyl]-3,3-diethyl-2-oxo-indoline-1-carboxylate (400.0 mg; 0.93 mmol; 1.00 eq.) and bromoethane (104 µL; 1.39 mmol; 1.50 eq.) giving tert-butyl 5-[(E)-[tert-butoxycarbonyl(ethyl)hydrazono]methyl]-3,3-diethyl-2-oxo-indoline-1-carboxylate (320.0 mg; 75 %) as a beige solid. ¹H NMR (CDCl₃, 300 MHz): δ 7.85 (m, 2H); 7.63 (d, J = 1.6 Hz, 1H); 7.50 (dd, J = 6.8 Hz, J = 1.6 Hz, 1H); 3.93 (q, J = 6.9 Hz, 2H); 2.00 (m, 2H); 1.81 (m, 2H); 1.64 (s, 9H); 1.57 (s, 6H); 1.55 (s, 3H); 1.21 (t, J = 6.9 Hz, 3H); 0.61 (t, J = 7.3 Hz, 6H).

### Step D: 3,3-diethyl-5-[(E)-(ethylhydrazono)methyl]indolin-2-one

The compound was prepared using the same procedure as described in example 23 step I starting from tert-butyl 5-[(E)-[tert-butoxycarbonyl(ethyl)hydrazono]methyl]-3,3-diethyl-2-oxo-indoline-1-carboxylate (317.0 mg; 0.69 mmol; 1.00 eq.) giving 3,3-diethyl-5-[(E)-(ethylhydrazono)methyl]indolin-2-one (241.0 mg; quantitative) as a brown solid, used in the next step without purification. LC/MS (Method b): Rt = 2.23 min ; MS : m/z = 260 [M+H]⁺

### Step E: 5-[(E)-[(1,1-dioxo-1,2-benzothiazol-3-yl)-ethyl-hydrazono]methyl]-3,3-diethyl-indolin-2-one

The compound was prepared using the same procedure as described in example 21 step B starting from 3-chloro-1,2-benzothiazole 1,1-dioxide (example 1, step A), (62.0 mg; 0.31 mmol; 1.00 eq.) and 3,3-diethyl-5-[(E)-(ethylhydrazono)methyl]indolin-2-one (80.0 mg; 0.31 mmol; 1.00 eq.) giving 5-[(E)-[(1,1-dioxo-1,2-benzothiazol-3-yl)-ethylhydrazono]methyl]-3,3-diethyl-indolin-2-one (17.0 mg; 13 %) as a beige solid. 1H NMR (DMSO-d6, 300 MHz): δ 10.71 (s, 1H); 8.82 (m, 1H); 8.52 (s, 1H); 8.08 (m, 1H); 7.87 (m, 2H) ; 7.79 (d, J = 8.0 Hz, 1H); 7.70 (s, 1H); 7.03 (d, J = 8.0 Hz, 1H); 4.39 (m, 2H) ; 1.80 (m, 4H); 1.30 (t, J = 6.9 Hz, 3H); 0.59 (t, J = 7.1 Hz, 6H). mp: 284-286°C.

### Example 83: [3-[ethyl-[(E)-(3-ethyl-2-oxo-1H-benzimidazol-5-yl)methyleneamino] amino]-1,1-dioxo-1,2-benzothiazol-6-yl]boronic acid

### Step A: tert-butyl N-[(E)-(3-ethyl-2-oxo-1H-benzimidazol-5-yl)methyleneamino] carbamate

The compound was prepared using the same procedure as described in example 23 step F starting from 3-ethyl-2-oxo-1H-benzimidazole-5-carbaldehyde (example 24, step C), (4.31 g; 13.60 mmol; 1.00 eq.) giving crude tert-butyl N-[(E)-(3-ethyl-2-oxo-1H-benzimidazol-5-yl)methyleneamino]carbamate (4.50 g; quantitative) as a pale yellow solid, used in the next step without further purification. ¹H NMR (DMSO-d₆, 300 MHz): δ 11.00 (s, 1H); 10.79 (br s, 1H); 7.99 (s, 1H); 7.35 (s, 1H); 7.21 (dd, J = 1.5 Hz, J = 8.1 Hz, 1H) ; 7.00 (d, J = 7.9 Hz, 1H); 3.84 (q, J = 7.2 Hz, 2H); 1.47 (s, 9H); 1.20 (m, 3H).

### Step B: tert-butyl 5-[(E)-(tert-butoxycarbonylhydrazono)methyl]-3-ethyl-2-oxo-benzimidazole-1-carboxylate

The compound was prepared using the same procedure as described in example 23 step G starting from tert-butyl N-[(E)-(3-ethyl-2-oxo-1H-benzimidazol-5-yl)methylene amino]carbamate (4.00 g; 13.14 mmol; 1.00 eq.) giving tert-butyl 5-[(E)-(tert-butoxycarbonylhydrazono)methyl]-3-ethyl-2-oxo-benzimidazole-1-carboxylate (4.46 g; 84%) as a beige solid. ¹H NMR (DMSO-d₆, 300 MHz): δ 10.93 (br s, 1H) ; 8.03 (s, 1H) ; 7.73 (d, J = 8.3 Hz, 1H); 7.44 (d, J = 1.3 Hz, 1H); 7.35 (dd, J = 1.5 Hz, J = 8.4 Hz, 1H); 3.88 (q, J = 7.2 Hz, 2H); 1.59 (s, 9H); 1.48 (s, 9H); 1.22 (t, J = 7.2 Hz, 3H).

### Step C: tert-butyl 5-[(E)-[tert-butoxycarbonyl(ethyl)hydrazono]methyl]-3-ethyl-2-oxo-benzimidazole-1-carboxylate

The compound was prepared using the same procedure as described in example 23 step H starting from tert-butyl 5-[(E)-(tert-butoxycarbonylhydrazono)methyl]-3-ethyl-2-oxo-benzimidazole-1-carboxylate (250.0 mg; 0.62 mmol; 1.00 eq.) and bromoethane (69 µL; 0.93 mmol; 1.50 eq.) giving tert-butyl 5-[(E)-[tert-butoxycarbonyl(ethyl)hydrazono]methyl]-3-ethyl-2-oxo-benzimidazole-1-carboxylate (200.0 mg; 75%) as a beige resin. ¹H NMR (DMSO-d₆, 300 MHz): δ 8.14 (s, 1H); 7.74 (d, J = 8.4 Hz, 1H); 7.52 (d, J = 1.3 Hz, 1H); 7.46 (dd, J = 1.6 Hz, J = 8.3 Hz, 1H); 3.87 (m, 4H); 1.60 (s, 9H); 1.50 (s, 9H); 1.24 (t, J = 7.1 Hz, 3H); 1.10 (t, J = 6.9 Hz, 3H).

### Step D: 3-ethyl-5-[(E)-(ethylhydrazono)methyl]-1H-benzimidazol-2-one

The compound was prepared using the same procedure as described in example 23 step I starting from tert-butyl 5-[(E)-[tert-butoxycarbonyl(ethyl)hydrazono]methyl]-3-ethyl-2-oxo-benzimidazole-1-carboxylate (320.0 mg; 0.74 mmol; 1.00 eq.) giving crude 3-ethyl-5-[(E)-(ethylhydrazono)methyl]-1H-benzimidazol-2-one as a yellow resin, used in the next step without further purification. ¹H NMR (DMSO-d₆, 300 MHz): δ 11.33 (s, 1H); 8.76 (s, 1H) ; 7.56 (d, J = 1.2 Hz, 1H); 7.46 (dd, J = 1.5 Hz, J = 8.1 Hz, 1H); 7.12 (d, J = 7.9 Hz, 1H); 3.88 (q, J = 7.1 Hz, 2H); 3.30 (q, J = 7.3 Hz, 2H); 1.30 (t, J = 7.2 Hz, 3H); 1.21 (t, J = 7.1 Hz, 3H).

### Step E: 5-[(E)-[(6-bromo-1,1-dioxo-1,2-benzothiazol-3-yl)-ethyl-hydrazono]methyl]-3-ethyl-1H-benzimidazol-2-one

The compound was prepared using the same procedure as described in example 21 step B starting from 6-bromo-3-chloro-1,2-benzothiazole 1,1-dioxide (example 50, step D), (249.1 mg; 0.89 mmol; 1.20 eq.) and 3-ethyl-5-[(E)-(ethylhydrazono)methyl]-1H-benzimidazol-2-one (171.9 mg; 0.74 mmol; 1.00 eq.) giving 5-[(E)-[(6-bromo-1,1-dioxo-1,2-benzothiazol-3-yl)-ethyl-hydrazono]methyl]-3-ethyl-1H-benzimidazol-2-one (280.0 mg; 79 %) as an orange solid. ¹H NMR (DMSO-d₆, 300 MHz): δ 11.19 (s, 1H); 8.73 (d, J = 8.6 Hz, 1H); 8.57 (s, 1H) ; 8.44 (d, J = 1.8 Hz, 1H) ; 8.10 (dd, J = 1.9 Hz, J = 8.5 Hz, 1H) ; 7.57 (m, 2H); 7.15 (d, J = 8.6 Hz, 1H); 4.39 (q, J = 7.2 Hz, 2H); 3.91 (q, J = 7.2 Hz, 2H); 1.29 (m, 6H).

### Step F: 5-[(E)-[[1,1-dioxo-6-(4,4,5,5-tetramethyl-1,3,2-dioxaborolan-2-yl)-1,2-benzothiazol-3-yl]-ethyl-hydrazono]methyl]-3-ethyl-1H-benzimidazol-2-one

The compound was prepared using the same procedure as described in example 50 step F starting from 5-[(E)-[(6-bromo-1,1-dioxo-1,2-benzothiazol-3-yl)-ethyl-hydrazono] methyl]-3-ethyl-1H-benzimidazol-2-one (280.0 mg; 0.59 mmol; 1.00 eq.) giving crude 5-[(E)-[[1,1-dioxo-6-(4,4,5,5-tetramethyl-1,3,2-dioxaborolan-2-yl)-1,2-benzo thiazol-3-yl]-ethyl-hydrazono]methyl]-3-ethyl-1H-benzimidazol-2-one (333.0 mg; quantitative) as a brown solid, used in the next step without further purification.

### Step G: [3-[ethyl-[(E)-(3-ethyl-2-oxo-1H-benzimidazol-5-yl)methyleneamino]amino]-1,1-dioxo-1,2-benzothiazol-6-yl]boronic acid

The compound was prepared using the same procedure as described in example 62 step C starting from 5-[(E)-[[1,1-dioxo-6-(4,4,5,5-tetramethyl-1,3,2-dioxaborolan-2-yl)-1,2-benzothiazol-3-yl]-ethyl-hydrazono]methyl]-3-ethyl-1H-benzimidazol-2-one (307.0 mg; 0.59 mmol; 1.00 eq.) giving [3-[ethyl-[(E)-(3-ethyl-2-oxo-1H-benzimidazol-5-yl)methyleneamino]amino]-1,1-dioxo-1,2-benzothiazol-6-yl]boronic acid (37.3 mg; 14 %) as a beige solid. ¹H NMR (DMSO-d₆ + 10% v/v D₂O, 400 MHz): δ 8.83 (d, J = 8.1 Hz, 1H) ; 8.55 (s, 1H); 8.32 (s, 1H); 8.23 (d, J = 7.9 Hz, 1H); 7.63 (m, 2H); 7.21 (d, J = 8.1 Hz, 1H); 4.41 (br q, J = 7.0 Hz, 2H); 3.93 (br q, J = 7.3 Hz, 2H); 1.31 (m, 6H). mp: 221-342°C.

### Example 84: [3-[[(E)-(3-ethyl-2-oxo-1H-benzimidazol-5-yl)methyleneamino]-isobutyl-amino]-1,1-dioxo-1,2-benzothiazol-6-yl]boronic acid

### Step A: tert-butyl 5-[(E)-[tert-butoxycarbonyl(isobutyl)hydrazono]methyl]-3-ethyl-2-oxo-benzimidazole-1-carboxylate

The compound was prepared using the same procedure as described in example 23 step H starting from tert-butyl 5-[(E)-(tert-butoxycarbonylhydrazono)methyl]-3-ethyl-2-oxo-benzimidazole-1-carboxylate (example 83, step B), (300 mg; 0.74 mmol; 1 eq.) and 1-bromo-2-methylpropane (121 µL; 1.11 mmol; 1.50 eq.) giving tert-butyl 5-[(E)-[tert-butoxycarbonyl(isobutyl)hydrazono]methyl]-3-ethyl-2-oxo-benzimidazole-1-carboxylate (220 mg; 64%) as a beige resin. ¹H NMR (DMSO-d₆, 300 MHz): δ 8.00 (s, 1H); 7.73 (d, J = 8.4 Hz, 1H); 7.53 (d, J = 1.3 Hz, 1H); 7.47 (dd, J = 1.5 Hz, J = 8.4 Hz, 1H); 3.86 (q, J = 7.1 Hz, 2H); 3.70 (d, J = 7.4 Hz, 2H); 2.00 (m, J = 6.9 Hz, 1H); 1.60 (s, 9H); 1.50 (s, 9H) ; 1.24 (t, J = 7.2 Hz, 3H); 0.90 (d, J = 6.6 Hz, 6H).

### Step B: 3-ethyl-5-[(E)-(isobutylhydrazono)methyl]-1H-benzimidazol-2-one

The compound was prepared using the same procedure as described in example 23 step I starting from tert-butyl 5-[(E)-[tert-butoxycarbonyl(isobutyl)hydrazono]methyl]-3-ethyl-2-oxo-benzimidazole-1-carboxylate (220.0 mg; 0.48 mmol; 1.00 eq.) giving crude 3-ethyl-5-[(E)-(isobutylhydrazono)methyl]-1H-benzimidazol-2-one as a yellow resin, used in the next step without further purification. ¹H NMR (DMSO-d₆, 300 MHz): δ 11.25 (s, 1H); 8.61 (s, 1H); 7.50 (s, 1H); 7.40 (dd, J = 1.3 Hz, J = 8.1 Hz, 1H); 7.09 (d, J = 8.1 Hz, 1H); 3.87 (q, J = 7.2 Hz, 2H); 3.09 (d, J = 6.9 Hz, 2H); 2.10 (m, J = 6.9 Hz, 1H); 1.21 (t, J = 7.1 Hz, 3H); 1.01 (d, J = 6.6 Hz, 6H).

### Step C: 5-[(E)-[(6-bromo-1,1-dioxo-1,2-benzothiazol-3-yl)-isobutyl-hydrazono] methyl]-3-ethyl-1H-benzimidazol-2-one

The compound was prepared using the same procedure as described in example 21 step B starting from 6-bromo-3-chloro-1,2-benzothiazole 1,1-dioxide (example 50, step D), (160.8 mg; 0.57 mmol; 1.20 eq.) and 3-ethyl-5-[(E)-(isobutylhydrazono)methyl]-1H-benzimidazol-2-one (124.4 mg; 0.48 mmol; 1.00 eq.) giving 5-[(E)-[(6-bromo-1,1-dioxo-1,2-benzothiazol-3-yl)-isobutyl-hydrazono]methyl]-3-ethyl-1H-benzimida zol-2-one (172.0 mg; 71 %) as a yellow solid. ¹H NMR (DMSO-d₆, 300 MHz): δ 11.20 (s, 1H) ; 8.74 (d, J = 8.4Hz, 1H); 8.57 (s, 1H); 8.44 (d, J = 1.7 Hz, 1H) ; 8.10 (dd, J = 1.9 Hz, J = 8.5 Hz, 1H); 7.58 (m, 2H); 7.15 (d, J = 7.9 Hz, 1H); 4.27 (d, J = 7.8 Hz, 2H); 3.92 (q, J = 7.2 Hz, 2H) ; 2.26 (m, J = 6.9 Hz, 1H); 1.28 (t, J = 7.3 Hz, 3H); 0.99 (d, J = 6.8 Hz, 6H).

### Step D: 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3-ethyl-1H-benzimidazol-2-one

The compound was prepared using the same procedure as described in example 50 step F starting from 5-[(E)-[(6-bromo-1,1-dioxo-1,2-benzothiazol-3-yl)-isobutyl-hydrazono]methyl]-3-ethyl-1H-benzimidazol-2-one (172.0 mg; 0.34 mmol; 1.00 eq.) giving crude 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3-ethyl-1H-benzimidazol-2-one (230 mg; quantitative) as an orange oil, used in the next step without further purification. ¹H NMR (DMSO-d₆, 300 MHz): δ 11.20 (s, 1H); 8.90 (d, J = 8.1 Hz, 1H); 8.57 (s, 1H); 8.15 (dd, J = 0.9 Hz, J = 8.0 Hz, 1H); 8.09 (s, 1H); 7.61 (m, 2H); 7.16 (d, J = 8.4 Hz, 1H); 4.29 (br d, J = 7.8 Hz, 2H) ; 3.93 (q, J = 7.2 Hz, 2H); 2.27 (m, 1H); 1.35 (s, 12H); 1.28 (t, J = 7.2 Hz, 3H); 0.99 (d, J = 6.6 Hz, 6H).

### Step E: [3-[[(E)-(3-ethyl-2-oxo-1H-benzimidazol-5-yl)methyleneamino]-isobutyl-amino]-1,1-dioxo-1,2-benzothiazol-6-yl]boronic acid

The compound was prepared using the same procedure as described in example 62 step C starting from 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3-ethyl-1H-benzimidazol-2-one (188.0 mg; 0.34 mmol; 1.00 eq.) giving [3-[[(E)-(3-ethyl-2-oxo-1H-benzimidazol-5-yl)methyleneamino]-isobutyl-amino]-1,1-dioxo-1,2-benzothiazol-6-yl]boronic acid (49.2 mg; 31 %) as a beige solid. ¹H NMR (DMSO-d₆, 400 MHz): δ 11.20 (s, 1H) ; 8.83 (d, J = 7.9 Hz, 1H) ; 8.65 (s, 2H); 8.57 (s, 1H); 8.34 (s, 1H); 8.22 (dd, J = 0.9 Hz, J = 7.9 Hz, 1H); 7.63 (s, 1H); 7.58 (dd, J = 1.3 Hz, J = 8.1 Hz, 1H); 7.17 (d, J = 8.1 Hz, 1H); 4.29 (d, J = 7.7 Hz, 2H); 3.92 (q, J = 7.0 Hz, 2H); 2.27 (m, J = 7.0 Hz, 1H); 1.30 (t, J = 7.2 Hz, 3H); 0.99 (d, J = 6.6 Hz, 6H). mp: 267-286 °C

### Example 85: 5-[(E)-[(1,1-dioxo-1,2-benzothiazol-3-yl)-ethyl-hydrazono]methyl]-3,3-dimethyl-isoindolin-1-one

### Step A: tert-butyl N-[(E)-(3,3-dimethyl-1-oxo-isoindolin-5-yl)methyleneamino] carbamate

The compound was prepared using the same procedure as described in example 23 step F starting from 3,3-dimethyl-1-oxo-isoindoline-5-carbaldehyde (example 32, step D), (359.0 mg; 1.90 mmol; 1.00 eq.) giving tert-butyl N-[(E)-(3,3-dimethyl-1-oxo-isoindolin-5-yl)methyleneamino]carbamate (482.0 mg; 84 %), used in the next step without further purification. ¹H NMR (DMSO-d₆, 300 MHz): δ 11.06 (br s, 1H); 8.68 (s, 1H); 8.08 (s, 1H); 7.80 (s, 1H); 7.63 (m, 2H); 1.47 (s, 9H); 1.45 (s, 6H).

### Step B: tert-butyl N-[(E)-(3,3-dimethyl-1-oxo-isoindolin-5-yl)methyleneamino]-N-ethylcarbamate

The compound was prepared using the same procedure as described in example 23 step H starting from tert-butyl N-[(E)-(3,3-dimethyl-1-oxo-isoindolin-5-yl)methyl eneamino]carbamate (250.0 mg; 0.82 mmol; 1.00 eq.) and bromoethane (92 µL; 1.24 mmol; 1.50 eq.) giving tert-butyl N-[(E)-(3,3-dimethyl-1-oxo-isoindolin-5-yl)methyleneamino]-N-ethyl-carbamate (217.0 mg; 80%) as a white solid. ¹H NMR (DMSO-d₆, 300 MHz): δ 8.68 (s, 1H); 8.05 (s, 1H); 7.85 (s, 1H); 7.77 (d, J = 7.8 Hz, 1H) ; 7.62 (d, J = 7.8 Hz, 1H); 3.91 (q, J = 7.0 Hz, 2H); 1.50 (s, 9H); 1.45 (s, 6H) ; 1.10 (t, J = 7.0 Hz, 3H).

### Step C: 5-[(E)-(ethylhydrazono)methyl]-3,3-dimethyl-isoindolin-1-one

The compound was prepared using the same procedure as described in example 23 step I starting from tert-butyl N-[(E)-(3,3-dimethyl-1-oxo-isoindolin-5-yl)methylene amino]-N-ethyl-carbamate (200.0 mg; 0.60 mmol; 1.00 eq.) giving 5-[(E)-(ethylhydrazono)methyl]-3,3-dimethyl-isoindolin-1-one (170.0 mg; quantitative) as a yellow solid, used in the next step without further purification.

### Step D: 5-[(E)-[(1,1-dioxo-1,2-benzothiazol-3-yl)-ethyl-hydrazono]methyl]-3,3-dimethyl-isoindolin-1-one

The compound was prepared using the same procedure as described in example 21 step B starting from 3-chloro-1,2-benzothiazole 1,1-dioxide (example 1, step A), (18.0 mg; 0.09 mmol; 1.00 eq.) and 5-[(E)-(ethylhydrazono)methyl]-3,3-dimethyl-isoindolin-1-one (70.0 mg; 0.09 mmol; 1.00 eq.) giving 5-[(E)-[(1,1-dioxo-1,2-benzothiazol-3-yl)-ethyl-hydrazono]methyl]-3,3-dimethyl-isoindolin-1-one (20.0 mg; 17 %) as a white solid. 1H NMR (DMSO-d6, 300 MHz): δ 8.80 (m, 2H) ; 8.65 (s, 1H) ; 8.10 (m, 1H) ; 8.04 (s, 1H) ; 8.00 (d, J = 7.7 Hz, 1H) ; 7.90 (m, 2H); 7.77 (d, J = 7.7 Hz, 1H); 4.43 (q, J = 7.0 Hz, 2H); 1.51 (s, 6H); 1.32 (t, J = 7.0 Hz, 3H). mp: 289-291°C.

### Example 86: N-[(E)-(4-fluoro-3-methylsulfanyl-phenyl)methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B), (60.0 mg; 0.28 mmol; 1.00 eq.) and 4-fluoro-3-(methyl sulfanyl)benzaldehyde (53.2 mg; 0.31 mmol; 1.10 eq.) giving N-[(E)-(4-fluoro-3-methylsulfanyl-phenyl)methyleneamino]-N-methyl-1,1-dioxo-1,2-benzothiazol-3-amine (54.0 mg; 52 %) as a beige solid. 1H NMR (DMSO-d6, 400 MHz): δ 8.85 (m, 1H) ; 8.50 (s, 1H) ; 8.10 (m, 1H) ; 7.91 (m, 2H) ; 7.82 (dd, J = 2.1 Hz, J = 7.6 Hz, 1H) ; 7.72 (ddd, J = 2.2 Hz, J = 5.0 Hz, J = 8.5 Hz, 1H) ; 7.40 (dd, J = 8.4 Hz, J = 10.1 Hz, 1H); 3.80 (s, 3H) ; 2.61 (s, 3H).

### Example 87: [3-[[(E)-(3,3-diethyl-2-oxo-indolin-5-yl)methyleneamino]-(2-methoxy ethyl)amino]-1,1-dioxo-1,2-benzothiazol-6-yl]boronic acid

### Step A: tert-butyl 5-[(E)-[tert-butoxycarbonyl(2-methoxyethyl)hydrazono]methyl]-3,3-diethyl-2-oxo-indoline-1-carboxylate

The compound was prepared using the same procedure as described in example 23 step H starting from tert-butyl 5-[(E)-(tert-butoxycarbonylhydrazono)methyl]-3,3-diethyl-2-oxo-indoline-1-carboxylate (example 82, step B), (400.0 mg; 0.93 mmol; 1.00 eq.) and 1-bromo-2-methoxyethane (130 µL; 1.39 mmol; 1.50 eq.) giving tert-butyl 5-[(E)-[tert-butoxycarbonyl(2-methoxyethyl)hydrazono]methyl]-3,3-diethyl-2-oxo-indoline-1-carboxylate (295.0 mg; 65 %) as a pale yellow solid. ¹H NMR (CDCl₃, 300 MHz): δ 8.07 (s, 1H); 7.84 (d, J = 8.3 Hz, 1H); 7.61 (s, 1H); 7.50 (dd, J = 8.3 Hz, J = 1.6 Hz, 1H); 4.05 (t, J = 6.0 Hz, 2H) ; 3.59 (t, J = 6.0 Hz, 2H) ; 3.37 (s, 3H) ; 1.97 (m, 2H); 1.81 (m, 2H) ; 1.64 (s, 9H); 1.57 (s, 9H); 0.61 (t, J = 7.3 Hz, 6H).

### Step B: 3,3-diethyl-5-[(E)-(2-methoxyethylhydrazono)methyl]indolin-2-one

The compound was prepared using the same procedure as described in example 23 step I starting from tert-butyl 5-[(E)-[tert-butoxycarbonyl(2-methoxyethyl)hydrazono] methyl]-3,3-diethyl-2-oxo-indoline-1-carboxylate (292.0 mg; 0.60 mmol; 1.00 eq.) giving 3,3-diethyl-5-[(E)-(2-methoxyethylhydrazono)methyl]indolin-2-one (167.0 mg; quantitative) as a brown oil, used in the next step without purification. LC/MS (Method B): Rt = 2.11 min ; MS : m/z = 290 [M+H]⁺

### Step C: 5-[(E)-[(6-bromo-1,1-dioxo-1,2-benzothiazol-3-yl)-(2-methoxyethyl) hydrazono]methyl]-3,3-diethyl-indolin-2-one

The compound was prepared using the same procedure as described in example 21 step B starting from 6-bromo-3-chloro-1,2-benzothiazole 1,1-dioxide (example 50, step D), (113.0 mg; 0.40 mmol; 1.00 eq.) and 3,3-diethyl-5-[(E)-(2-methoxyethylhydrazono)methyl]indolin-2-one (117.0 mg; 0.40 mmol; 1.00 eq.) giving 5-[(E)-[(6-bromo-1,1-dioxo-1,2-benzothiazol-3-yl)-(2-methoxyethyl) hydra zono]methyl]-3,3-diethyl-indolin-2-one (137.0 mg; 64 %) as a yellow solid. ¹H NMR (DMSO-d₆, 300 MHz): δ 10.71 (s, 1H); 8.68 (d, J = 8.5 Hz, 1H) ; 8.57 (s, 1H); 8.45 (s, 1H); 8.10 (d, J = 8.5 Hz, 1H) ; 7.73 (d, J = 8.1 Hz, 1H); 7.65 (s, 1H); 7.02 (d, J = 8.1 Hz, 1H); 4.55 (t, J = 5.6 Hz, 2H); 3.72 (t, J = 5.6 Hz, 2H); 3.03 (s, 3H) ; 1.81 (m, 4H); 0.58 (t, J = 7.3 Hz, 6H).

### Step D: 5-[(E)-[[1,1-dioxo-6-(4,4,5,5-tetramethyl-1,3,2-dioxaborolan-2-yl)-1,2-benzothiazol-3-yl]-(2-methoxyethyl)hydrazono]methyl]-3,3-diethyl-indolin-2-one

The compound was prepared using the same procedure as described in example 50 step F starting from 5-[(E)-[(6-bromo-1,1-dioxo-1,2-benzothiazol-3-yl)-(2-methoxyethyl) hydrazono]methyl]-3,3-diethyl-indolin-2-one (55.0 mg; 0.10 mmol; 1.00 eq.) giving crude 5-[(E)-[[1,1-dioxo-6-(4,4,5,5-tetramethyl-1,3,2-dioxaborolan-2-yl)-1,2-benzo-thiazol-3-yl]-(2-methoxyethyl)hydrazono]methyl]-3,3-diethyl-indolin-2-one (103 mg; quantitative) as a brown oil, used in the next step without further purification. LC/MS (Method C): Rt = 2.30 min ; MS : m/z = 499 [M+H-pinacolyl ester]⁺

### Step E: [3-[[(E)-(3,3-diethyl-2-oxo-indolin-5-yl)methyleneamino]-(2-methoxyethyl) amino]-1,1-dioxo-1,2-benzothiazol-6-yl]boronic acid

The compound was prepared using the same procedure as described in example 62 step C starting from 5-[(E)-[[1,1-dioxo-6-(4,4,5,5-tetramethyl-1,3,2-dioxaborolan-2-yl)-1,2-benzothiazol-3-yl]-(2-methoxyethyl)hydrazono]methyl]-3,3-diethyl-indolin-2-one (103.0 mg; 0.09 mmol; 1.00 eq.) giving [3-[[(E)-(3,3-diethyl-2-oxo-indolin-5-yl)methyleneamino]-(2-methoxyethyl)amino]-1,1-dioxo-1,2-benzothiazol-6-yl] boronic acid (25.0 mg; 57 %) as a pale yellow solid. ¹H NMR (DMSO-d₆, 300 MHz): δ 10.72 (s, 1H); 8.75 (d, J = 8.0 Hz, 1H) ; 8.65 (s, 2H) ; 8.57 (s, 1H) ; 8.34 (s, 1H); 8.20 (d, J = 8.0 Hz, 1H) ; 7.73 (d, J = 8.0 Hz, 1H); 7.69 (s, 1H); 7.03 (d, J = 8.0 Hz, 1H); 4.56 (t, J = 5.3 Hz, 2H) ; 3.73 (t, J = 5.3 Hz, 2H); 3.30 (s, 3H) ; 1.80 (m, 4H); 0.59 (t, J = 7.3 Hz, 6H). mp: 260-263 °C.

### Example 88: [3-[[(E)-(3,3-diethyl-2-oxo-indolin-5-yl)methyleneamino]-isobutylamino]-1,1-dioxo-1,2-benzothiazol-6-yl]boronic acid

### Step A: tert-butyl 5-[(E)-[tert-butoxycarbonyl(isobutyl)hydrazono]methyl]-3,3-diethyl-2-oxo-indoline-1-carboxylate

The compound was prepared using the same procedure as described in example 23 step H starting from tert-butyl 5-[(E)-(tert-butoxycarbonylhydrazono)methyl]-3,3-diethyl-2-oxo-indoline-1-carboxylate (example 82, step B), (400.0 mg; 0.93 mmol; 1.00 eq.) and isobutyl bromide (151 µL; 1.39 mmol; 1.50 eq.) giving tert-butyl 5-[(E)-[tert-butoxycarbonyl(isobutyl)hydrazono]methyl]-3,3-diethyl-2-oxo-indoline-1-carboxylate (271.0 mg; 60 %) as a pale yellow oil. ¹H NMR (CDCl₃, 300 MHz): δ 7.85 (m, 2H); 7.61 (s, 1H); 7.50 (dd, J = 8.3 Hz, J = 1.6 Hz, 1H); 3.71 (d, J = 7.3 Hz, 2H); 2.06 (m, 1H); 1.96 (m, 2H); 1.81 (m, 2H); 1.64 (s, 9H) ; 1.57 (s, 9H); 0.97 (d, J = 6.7 Hz, 6H) ; 0.61 (t, J = 7.3 Hz, 6H).

### Step B: 3,3-diethyl-5-[(E)-(isobutylhydrazono)methyl]indolin-2-one

The compound was prepared using the same procedure as described in example 23 step I starting from tert-butyl 5-[(E)-[tert-butoxycarbonyl(isobutyl)hydrazono]methyl]-3,3-diethyl-2-oxo-indoline-1-carboxylate (268.0 mg; 0.55 mmol; 1.00 eq.) giving 3,3-diethyl-5-[(E)-(isobutylhydrazono)methyl]indolin-2-one (154.0 mg; 98 %) as a yellow solid, used in the next step without purification. LC/MS (Method b): Rt = 3.37 min ; MS : m/z = 288 [M+H]⁺ Step C: 5-[(E)-[(6-bromo-1,1-dioxo-1,2-benzothiazol-3-yl)-isobutyl-hydrazono] methyl]-3,3-diethyl-indolin-2-one

The compound was prepared using the same procedure as described in example 21 step B starting from 6-bromo-3-chloro-1,2-benzothiazole 1,1-dioxide (example 50, step D), (92.0 mg; 0.33 mmol; 1.00 eq.) and 3,3-diethyl-5-[(E)-(isobutylhydrazono)methyl]indolin-2-one (94.0 mg; 0.33 mmol; 1.00 eq.) giving 5-[(E)-[(6-bromo-1,1-dioxo-1,2-benzothiazol-3-yl)-isobutyl-hydrazono]methyl]-3,3-diethyl-indolin-2-one (110.0 mg; 63 %) as a pale yellow solid. ¹H NMR (DMSO-d₆, 300 MHz): δ 10.72 (s, 1H); 8.70 (d, J = 8.6 Hz, 1H) ; 8.51 (s, 1H); 8.44 (s, 1H); 8.10 (d, J = 8.6 Hz, 1H); 7.78 (d, J = 7.8 Hz, 1H); 7.67 (s, 1H); 7.02 (d, J = 8.1 Hz, 1H); 4.25 (d, J = 7.5 Hz, 2H) ; 2.25 (m, 1H); 1.81 (m, 4H); 0.97 (d, J = 6.6 Hz, 6H); 0.57 (t, J = 7.3 Hz, 6H).

### Step D: 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3,3-diethyl-indolin-2-one

The compound was prepared using the same procedure as described in example 50 step F starting from 5-[(E)-[(6-bromo-1,1-dioxo-1,2-benzothiazol-3-yl)-isobutyl-hydrazono]methyl]-3,3-diethyl-indolin-2-one (48.0 mg; 0.09 mmol; 1.00 eq.) giving crude 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3,3-diethyl-indolin-2-one (95.0 mg; quantitative) as a brown oil, used in the next step without further purification. LC/MS (Method C): Rt = 2.08 min ; MS : m/z = 579 [M+H]⁺

### Step E: [3-[[(E)-(3,3-diethyl-2-oxo-indolin-5-yl)methyleneamino]-isobutyl-amino]-1,1-dioxo-1,2-benzothiazol-6-yl]boronic acid

The compound was prepared using the same procedure as in example 62 step C starting from 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3,3-diethyl-indolin-2-one (95mg; 0.16 mmol; 1 eq.) giving [3-[[(E)-(3,3-diethyl-2-oxo-indolin-5-yl)methyleneamino]-iso butyl-amino]-1,1-dioxo-1,2-benzothiazol-6-yl]boronic acid (19 mg; 23 %) as a yellow solid. ¹H NMR (DMSO-d₆, 300 MHz): δ 10.72 (s, 1H); 8.78 (d, J = 8.0 Hz, 1H); 8.64 (s, 2H); 8.51 (s, 1H); 8.33 (s, 1H) ; 8.20 (d, J = 8.0 Hz, 1H); 7.78 (d, J = 8.0 Hz, 1H); 7.71 (s, 1H); 7.03 (d, J = 8.0 Hz, 1H) ; 4.27 (d, J = 7.7 Hz, 2H); 2.27(m, 1H); 1.80 (m, 4H); 0.98 (d, J = 6.6 Hz, 6H); 0.59 (t, J = 7.2 Hz, 6H). mp: 267-270 °C

### Example 89: 5'-[(E)-[(1,1-dioxo-1,2-benzothiazol-3-yl)-methyl-hydrazono]methyl] spiro[cyclopropane-1,3'-isoindoline]-1'-one

### Step A: 5'-bromospiro[cyclopropane-1,3'-isoindoline]-1'-one

Methyl 4-bromo-2-cyanobenzoate (800.0 mg; 3.33 mmol; 1.00 eq.) was combined with titanium(IV) isopropoxide (1.10 mL; 3.67 mmol; 1.10 eq.) in dry diethyl ether (16 mL) under argon atmosphere and ethylmagnesium bromide (2.22 mL; 3 M solution in diethyl ether; 6.67 mmol; 2.00 eq.) was added at rt. After 2h stirring, the reaction was quenched with 1N HCl (2 mL) and extracted with DCM. The organic extract was washed with brine, dried over MgSO₄ and concentrated under reduced pressure. The residue was recrystallized from a mixture of diethyl ether and n-heptane giving 5'-bromospiro[cyclopropane-1,3'-isoindoline]-1'-one (385.0 mg; 49 %). ¹H NMR (CDCl₃, 300 MHz): δ 7.92 (s, 1H) ; 7.74 (d, J = 7.7 Hz, 1H) ; 7.58 (d, J = 7.7 Hz, 1H) ; 7.23 (s, 1H) ; 1.65 (t, J = 6.6 Hz, 2H); 1.47 (t, J = 6.6 Hz, 2H).

### Step B: 1'-oxospiro[cyclopropane-1,3'-isoindoline]-5'-carbaldehyde

The compound was prepared using the same procedure as described in example 20 step B starting from 5'-bromospiro[cyclopropane-1,3'-isoindoline]-1'-one (120.0 mg; 0.50 mmol; 1.00 eq) giving 1'-oxospiro[cyclopropane-1,3'-isoindoline]-5'-carbaldehyde (100.0 mg; quantitative) as a yellow oil. LC/MS (Method B): Rt = 1.64 min ; MS : m/z = 188 [M+H]⁺

### Step C: 5'-[(E)-[(1,1-dioxo-1,2-benzothiazol-3-yl)-methyl-hydrazono]methyl]spiro [cyclopropane-1,3'-isoindoline]-1'-one

The compound was prepared using the same procedure as described in example 1 step C starting from 1-(1,1-dioxo-1,2-benzothiazol-3-yl)-1-methyl-hydrazine (example 1, step B), (45.0 mg; 0.21 mmol; 1.00 eq.) and 1'-oxospiro[cyclopropane-1,3'-isoindoline]-5'-carbaldehyde (100.0 mg; 0.27 mmol; 1.28 eq.) giving 5'-[(E)-[(1,1-dioxo-1,2-benzothiazol-3-yl)-methyl-hydrazono]methyl]spiro[cyclopropane-1,3'-isoindoline]-1'-one (5.0 mg; 5 %) as a yellow solid. 1H NMR (DMSO-d6, 300 MHz): δ 8.89 (s, 1H); 8.82 (d, J = 7.6 Hz, 1H); 8.54 (s, 1H); 8.10 (d, J = 7.6 Hz, 1H); 7.93 (m, 4H); 7.69 (s, 1H); 3.81 (s, 3H); 1.53 (br s, 4H). mp: > 300 °C.

### Example 90: N-[(E)-[3-(dimethylamino)-4-fluoro-phenyl]methyleneamino]-N-methyl-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B), (150.0 mg; 0.71 mmol; 1.00 eq.) and commercially available 3-(dimethylamino)-4-fluorobenzaldehyde (131.8 mg; 0.79 mmol; 1.11 eq.) giving N-[(E)-[3-(dimethylamino)-4-fluorophenyl]methyleneamino]-N-methyl-1,1-dioxo-1,2-benzothiazol-3-amine (28.0 mg; 11 %) as a yellow solid. 1H NMR (DMSO-d6, 400 MHz): δ 8.87 (m, 1H); 8.44 (s, 1H); 8.09 (m, 1H); 7.90 (m, 2H); 7.46 (dd, J = 2.0 Hz, J = 9.0 Hz, 1H); 7.39 (ddd, J = 2.0 Hz, J = 4.5, J = 8.3 Hz, 1H); 7.29 (dd, J = 8.3 Hz, J = 13.1 Hz, 1H); 3.78 (s, 3H); 2.89 (s, 3H); 2.88 (s, 3H). mp: 181-187 °C.

### Example 91: 3-[[(E)-(4-fluoro-3-methoxy-phenyl)methyleneamino]-isobutyl-amino]-1,1-dioxo-1,2-benzothiazol-6-ol

The compound was prepared using the same procedure as described in example 51 starting from N-[(E)-(4-fluoro-3-methoxy-phenyl)methyleneamino]-N-isobutyl-1,1-dioxo-6-(4,4,5,5-tetramethyl-1,3,2-dioxaborolan-2-yl)-1,2-benzothiazol-3-amine (example 63, step B), (3.00 g; 5.82 mmol; 1.00 eq.) giving 3-[[(E)-(4-fluoro-3-methoxyphenyl)methyleneamino]-isobutyl-amino]-1,1-dioxo-1,2-benzothiazol-6-ol (360.0 mg; 15 %) as a beige solid. ¹H NMR (DMSO-d₆, 400 MHz): δ 11.25 (br s, 1H) ; 8.61 (d, J = 8.8 Hz, 1H) ; 8.48 (s, 1H) ; 7.63 (dd, J = 1.9 Hz, J = 8.5 Hz, 1H); 7.46 (ddd, J = 2.0 Hz, J = 4.6 Hz, J = 8.4 Hz, 1H); 7.40 (dd, J = 8.4 Hz, J = 11.0 Hz, 1H); 7.29 (d, J = 2.2 Hz, 1H) ; 7.20 (dd, J = 2.4 Hz, J = 8.8 Hz, 1H); 4.22 (d, J = 7.7 Hz, 2H); 3.96 (s, 3H) ; 2.24 (m, J = 6.6 Hz, 1H); 0.97 (d, J = 6.8 Hz, 6H). mp: 245°C.

### Example 92: [3-[[(E)-(4-fluoro-3-methoxy-phenyl)methyleneamino]-isobutyl-amino]-1,1-dioxo-1,2-benzothiazol-6-yl]oxymethylboronic acid

Potassium carbonate (475.0 mg; 3.44 mmol; 5.57 eq.) was added to a suspension of 3-[[(E)-(4-fluoro-3-methoxy-phenyl)methyleneamino]-isobutyl-amino]-1,1-dioxo-1,2-benzothiazol-6-ol (example 91), (250.0 mg; 0.62 mmol; 1.00 eq.) in ACN (5.00 ml) and the mixture was stirred 5 min at rt. Then, (bromomethyl)boronic acid bis(isopropyl) ester (613.00 mg; 2.75 mmol; 4.46 eq.) was added and the reaction was stirred at rt until completion. The reaction was concentrated to dryness and the solid obtained was filtered and washed with water until neutral pH. The solid was then purified by LC/MS-Preparative (Column: Kinetex C18 21,20 x 150mm 5µm (phenomenex); Flow rate: 42 ml/min; Elution: H2O, 0.1% HCOOH / ACN, 0.1% HCOOH; Gradient: 10 to 100% ACN) to give [3-[[(E)-(4-fluoro-3-methoxy-phenyl)methyleneamino]-isobutyl-amino]-1,1-dioxo-1,2-benzothiazol-6-yl]oxy methylboronic acid (42.0 mg; 14 %) as a white solid. ¹H NMR (DMSO-d₆, 400 MHz): δ 8.66 (d, J = 9.0 Hz, 1H) ; 8.49 (s, 1H) ; 8.17 (s, 2H); 7.64 (dd, J = 1.9 Hz, J = 8.5 Hz, 1H); 7.47 (m, 2H); 7.41 (dd, J = 8.1 Hz, J = 11.0 Hz, 1H); 7.34 (dd, J = 2.4 Hz, J = 9.0 Hz, 1H); 4.23 (d, J = 7.7 Hz, 2H) ; 3.96 (s, 3H); 3.87 (s, 2H) ; 2.25 (m, J = 6.8 Hz, 1H) ; 0.97 (d, J = 6.6 Hz, 6H). mp: 206°C.

### Example 93: 5-ethoxy-N-[(E)-(4-fluoro-3-methoxy-phenyl)methyleneamino]-N-isobutyl-1,1-dioxo-1,2-benzothiazol-3-amine

### Step A: 5-bromo-N-[(E)-(4-fluoro-3-methoxy-phenyl)methyleneamino]-N-isobutyl-1,1-dioxo-1,2-benzothiazol-3-amine

The compound was prepared using the same procedure as described in example 21 step B starting from 5-bromo-3-chloro-1,2-benzothiazole 1,1-dioxide (example 53, step B), (1.70 g; 6.06 mmol; 1.00 eq.) and N-[(E)-(4-fluoro-3-methoxy-phenyl)methyleneamino]-2-methyl-propan-1-amine (example 40, step A), (1.36 g; 6.06 mmol; 1.00 eq.) giving 5-bromo-N-[(E)-(4-fluoro-3-methoxy-phenyl)methyleneamino]-N-isobutyl-1,1-dioxo-1,2-benzothiazol-3-amine (1.30 g; 46 %) as a beige solid. ¹H NMR (DMSO-d₆, 300 MHz): δ 9.01 (d, J = 1.5 Hz, 1H); 8.61 (s, 1H); 8.11 (dd, J = 1.7 Hz, J = 8.1 Hz, 1H); 8.08 (dd, J = 0.5 Hz, J = 8.1 Hz, 1H); 7.67 (m, 1H); 7.45 (m, 2H); 4.28 (d, J = 7.8 Hz, 2H); 4.01 (s, 3H); 2.26 (m, 1H); 0.98 (d, J = 6.6 Hz, 6H).

### Step B: N-[(E)-(4-fluoro-3-methoxy-phenyl)methyleneamino]-N-isobutyl-1,1-dioxo-5-(4,4,5,5-tetramethyl-1,3,2-dioxaborolan-2-yl)-1,2-benzothiazol-3-amine

The compound was prepared using the same procedure as described in example 50 step F starting from 5-bromo-N-[(E)-(4-fluoro-3-methoxy-phenyl)methyleneamino]-N-isobutyl-1,1-dioxo-1,2-benzothiazol-3-amine (1.1 g; 2.35 mmol; 1 eq.) giving N-[(E)-(4-fluoro-3-methoxy-phenyl)methyleneamino]-N-isobutyl-1,1-dioxo-5-(4,4,5,5-tetramethyl-1,3,2-dioxaborolan-2-yl)-1,2-benzothiazol-3-amine (1.2 g; quantitative) as a brown foam. ¹H NMR (DMSO-d₆, 300 MHz): δ 8.85 (d, J = 7.9 Hz, 1H); 8.54 (s, 1H); 8.17 (dd, J = 1.1 Hz, J = 8.0 Hz, 1H); 8.09 (s, 1H); 7.66 (dd, J = 2.0 Hz, J = 8.4 Hz, 1H); 7.51 (ddd, J = 1.8 Hz, J = 4.8 Hz, J = 8.6 Hz, 1H); 7.40 (dd, J = 8.4 Hz, J = 11.1 Hz, 1H); 4.28 (d, J = 7.9 Hz, 2H); 3.98 (s, 3H); 2.27 (m, 1H); 1.35 (s, 12H); 0.99 (d, J = 6.6 Hz, 6H).

### Step C: 3-[[(E)-(4-fluoro-3-methoxy-phenyl)methyleneamino]-isobutyl-amino]-1,1-dioxo-1,2-benzothiazol-5-ol

The compound was prepared using the same procedure as described in example 51 starting from N-[(E)-(4-fluoro-3-methoxy-phenyl)methyleneamino]-N-isobutyl-1,1-dioxo-5-(4,4,5,5-tetramethyl-1,3,2-dioxaborolan-2-yl)-1,2-benzothiazol-3-amine (1.00 g; 1.94 mmol; 1.00 eq.) giving 3-[[(E)-(4-fluoro-3-methoxy-phenyl)methylene amino]-isobutyl-amino]-1,1-dioxo-1,2-benzothiazol-5-ol (500.0 mg; 64 %) as a brown solid. ¹H NMR (DMSO-d₆, 400 MHz): δ 10.87 (br s, 1H); 8.50 (s, 1H); 8.19 (d, J = 2.0 Hz, 1H); 7.86 (d, J = 8.4 Hz, 1H); 7.65 (dd, J = 1.7 Hz, J = 8.5 Hz, 1H); 7.42 (m, 2H); 7.18 (dd, J = 2.1 Hz, J = 8.3 Hz, 1H); 4.23 (d, J = 7.7 Hz, 2H); 3.97 (s, 3H); 2.26 (m, J = 7.1 Hz, 1H); 0.98 (d, J = 6.8 Hz, 6H). mp: 235°C.

### Step D: 5-ethoxy-N-[(E)-(4-fluoro-3-methoxy-phenyl)methyleneamino]-N-isobutyl-1,1-dioxo-1,2-benzothiazol-3-amine

The compound was prepared using the same procedure as described in example 52 starting from 3-[[(E)-(4-fluoro-3-methoxy-phenyl)methyleneamino]-isobutyl-amino]-1,1-dioxo-1,2-benzothiazol-5-ol (50.0 mg; 0.12 mmol; 1.00 eq.) and ethyl iodide (45 µl; 0.18 mmol; 1.50 eq.) giving 5-ethoxy-N-[(E)-(4-fluoro-3-methoxy-phenyl)methyleneamino]-N-isobutyl-1,1-dioxo-1,2-benzothiazol-3-amine (33.0 mg; 61 %) as a white solid. ¹H NMR (DMSO-d₆, 400 MHz): δ 8.52 (s, 1H); 8.36 (d, J = 2.4 Hz, 1H); 8.00 (d, J = 8.4 Hz, 1H) ; 7.66 (dd, J = 1.7 Hz, J = 8.5 Hz, 1H); 7.43 (m, 3H); 4.25 (d, J = 7.7 Hz, 2H); 4.18 (q, J = 6.9 Hz, 2H); 3.98 (s, 3H); 2.26 (m, J = 7.3 Hz, 1H); 1.35 (t, J = 6.9 Hz, 3H); 0.98 (d, J = 6.6 Hz, 6H). mp: 164°C.

### Example 94: [3-[[(E)-(4-fluoro-3-methoxy-phenyl)methyleneamino]-isobutyl-amino]-1,1-dioxo-1,2-benzothiazol-5-yl]boronic acid

The compound was prepared using the same procedure as described in example 62 step C starting from N-[(E)-(4-fluoro-3-methoxy-phenyl)methyleneamino]-N-isobutyl-1,1-dioxo-5-(4,4,5,5-tetramethyl-1,3,2-dioxaborolan-2-yl)-1,2-benzothiazol-3-amine (example 93, step B), (100.0 mg; 0.19 mmol; 1.00 eq.) giving [3-[[(E)-(4-fluoro-3-methoxyphenyl)methyleneamino]-isobutyl-amino]-1,1-dioxo-1,2-benzothiazol-5-yl] boronic acid (32.0 mg; 37 %) as a beige solid. ¹H NMR (DMSO-d₆, 400 MHz): δ 9.31 (s, 1H); 8.58 (s, 2H) ; 8.53 (s, 1H) ; 8.24 (dd, J = 0.9 Hz, J = 7.5 Hz, 1H); 8.05 (d, J = 7.7 Hz, 1H); 7.68 (m, 2H) ; 7.37 (dd, J = 8.9 Hz, J = 11.1 Hz, 1H) ; 4.28 (d, J = 7.9 Hz, 2H) ; 3.95 (s, 3H), 2.27 (m J = 7.1 Hz, 1H); 0.99 (d, J = 6.8 Hz, 6H).

### Example 95: [3-[[(E)-(4-fluoro-3-methoxy-phenyl)methyleneamino]-isobutyl-amino]-1,1-dioxo-1,2-benzothiazol-5-yl]oxymethylboronic acid

The compound was prepared using the same procedure as described in example 92 starting from 3-[[(E)-(4-fluoro-3-methoxy-phenyl)methyleneamino]-isobutyl-amino]-1,1-dioxo-1,2-benzothiazol-5-ol (example 93, step C), (100.0 mg; 0.25 mmol; 1.00 eq.) giving [3-[[(E)-(4-fluoro-3-methoxy-phenyl)methyleneamino]-isobutyl-amino]-1,1-dioxo-1,2-benzothiazol-5-yl]oxymethylboronic acid (60.0 mg; 51 %) as a white solid. ¹H NMR (DMSO-d₆, 400 MHz): δ 8.52 (s, 1H); 8.36 (d, J = 2.4 Hz, 1H); 8.16 (s, 2H); 7.97 (d, J = 8.4 Hz, 1H); 7.66 (dd, J = 1.8 Hz, J = 8.4 Hz, 1H); 7.48 (ddd, J = 1.8 Hz, J = 4.4 Hz, J = 8.4 Hz, 1H); 7.39 (dd, J = 8.4 Hz, J = 11.2 Hz, 1H); 7.32 (dd, J = 2.3 Hz, J = 8.5 Hz, 1H); 4.25 (d, J = 7.7 Hz, 2H); 3.96 (s, 3H); 3.79 (s, 2H); 2.26 (m, J = 7.0 Hz, 1H); 0.98 (d, J = 6.6 Hz, 6H). mp: 210°C.

### Example 96: 3,3-diethyl-5-[(E)-[(6-hydroxy-1,1-dioxo-1,2-benzothiazol-3-yl)-isobutyl-hydrazono]methyl]indolin-2-one

### Step A: tert-butyl 3,3-diethyl-5-formyl-2-oxo-indoline-1-carboxylate

The compound was prepared using the same procedure as described in example 31 step B starting from 3,3-diethyl-2-oxo-indoline-5-carbaldehyde (example 34, step C), (3.29 g; 15.18 mmol; 1.00 eq.) giving tert-butyl 3,3-diethyl-5-formyl-2-oxo-indoline-1-carboxylate (4.24 g; 88 %) as a pale yellow oil. ¹H NMR (DMSO-d₆, 300 MHz): δ 9.96 (s, 1H) ; 7.91 (m, 3H); 1.86 (m, 4H); 1.57 (s, 9H); 0.53 (m, 6H).

### Step B: tert-butyl 3,3-diethyl-5-[(E)-(isobutylhydrazono)methyl]-2-oxo-indoline-1-carboxylate

Isobutylhydrazine hydrochloride (785.0 mg, 6.30 mmol, 1.00 eq.) was added to a suspension of tert-butyl 3,3-diethyl-5-formyl-2-oxo-indoline-1-carboxylate (2.00 g, 6.30 mmol, 1.00 eq.) in EtOH (20.00 mL). The mixture was stirred at rt overnight, then concentrated under vacuum to give tert-butyl 3,3-diethyl-5-[(E)-(isobutylhydrazono)methyl]-2-oxo-indoline-1-carboxylate (3.33 g; quantitative), used in next step without further purification. LC/MS (Method C): Rt = 1.91 min ; MS : m/z = 388 [M+H]⁺

### Step C: tert-butyl 5-[(E)-[(6-bromo-1,1-dioxo-1,2-benzothiazol-3-yl)-isobutyl-hydrazono]methyl]-3,3-diethyl-2-oxo-indoline-1-carboxylate

The compound was prepared using the same procedure as described in example 21 step B starting from 6-bromo-3-chloro-1,2-benzothiazole 1,1-dioxide (example 50, step D), (507.0 mg; 1.81 mmol; 1.00 eq.) and tert-butyl 3,3-diethyl-5-[(E)-(isobutylhydrazono)methyl]-2-oxo-indoline-1-carboxylate (1.40 g; 1.81 mmol; 1.00 eq.) giving tert-butyl 5-[(E)-[(6-bromo-1,1-dioxo-1,2-benzothiazol-3-yl)-isobutyl-hydrazono]methyl]-3,3-diethyl-2-oxo-indoline-1-carboxylate (391.0 mg; 34 %) as a yellow solid. ¹H NMR (DMSO-d₆, 300 MHz): δ 8.70 (d, J = 8.7 Hz, 1H); 8.56 (s, 1H); 8.45 (s, 1H) ; 8.12 (d, J = 8.7 Hz, 1H); 7.92 (s, 2H) ; 7.79 (s, 1H); 4.27 (d, J = 7.4 Hz, 2H) ; 2.28 (m, 1H); 1.87 (m, 4H); 1.59 (s, 9H); 0.99 (d, J = 6.5 Hz, 6H); 0.58 (t, J = 7.1 Hz, 6H).

### Step D: tert-butyl 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3,3-diethyl-2-oxo-indoline-1-carboxylate

The compound was prepared using the same procedure as described in example 50 step F starting from tert-butyl 5-[(E)-[(6-bromo-1,1-dioxo-1,2-benzothiazol-3-yl)-isobutyl-hydrazono]methyl]-3,3-diethyl-2-oxo-indoline-1-carboxylate (387.0 mg; 0.61 mmol; 1.00 eq.) giving tert-butyl 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3,3-diethyl-2-oxo-indoline-1-carboxylate (185.0 mg; 44%) as a yellow solid. LC/MS (Method F): Rt = 2.18 min ; MS : m/z = 679 [M+H]⁺

### Step E: tert-butyl 3,3-diethyl-5-[(E)-[(6-hydroxy-1,1-dioxo-1,2-benzothiazol-3-yl)-isobutyl-hydrazono]methyl]-2-oxo-indoline-1-carboxylate

The compound was prepared using the same procedure as described in example 51 starting from tert-butyl 5-[(E)-[[1,1-dioxo-6-(4,4,5,5-tetramethyl-1,3,2-dioxaborolan-2-yl)-1,2-benzothiazol-3-yl]-isobutyl-hydrazono]methyl]-3,3-diethyl-2-oxo-indoline-1-carboxylate (690.0 mg; 1.02 mmol; 1.00 eq.) to give tert-butyl 3,3-diethyl-5-[(E)-[(6-hydroxy-1,1-dioxo-1,2-benzothiazol-3-yl)-isobutyl-hydrazono]methyl]-2-oxo-indoline-1-carboxylate (399.0 mg ; 69 %). ¹H NMR (DMSO-d₆, 300 MHz): δ 8.59 (d, J = 8.6 Hz, 1H); 8.08 (s, 1H); 7.99 (d, J = 8.6 Hz, 1H); 7.66 (d, J = 8.6 Hz, 1H); 7.54 (s, 1H); 7.44 (s, 1H) ; 7.07 (d, J = 8.6 Hz, 1H) ; 4.24 (d, J = 7.5 Hz, 2H) ; 2.34 (m, 1H); 2.05 (m, 2H); 1.86 (m, 2H) ; 1.26 (s, 9H) ; 1.08 (d, J = 6.6 Hz, 6H) ; 0.71 (t, J = 7.3 Hz, 6H).

### Step F: 3,3-diethyl-5-[(E)-[(6-hydroxy-1,1-dioxo-1,2-benzothiazol-3-yl)-isobutyl-hydrazono]methyl]indolin-2-one

The compound was prepared using the same procedure as described in example 23 step I starting from tert-butyl 3,3-diethyl-5-[(E)-[(6-hydroxy-1,1-dioxo-1,2-benzothiazol-3-yl)-isobutyl-hydrazono]methyl]-2-oxo-indoline-1-carboxylate (85.0 mg; 0.15 mmol; 1.00 eq.) to give 3,3-diethyl-5-[(E)-[(6-hydroxy-1,1-dioxo-1,2-benzothiazol-3-yl)-isobutyl-hydrazono]methyl]indolin-2-one (28.0 mg; 41 %) as a white solid. ¹H NMR (DMSO-d₆, 300 MHz): δ 11.34 (br s, 1H); 10.74 (s, 1H); 8.60 (d, J = 8.7 Hz, 1H); 8.46 (s, 1H) ; 7.75 (d, J = 8.1 Hz, 1H); 7.67 (s, 1H); 7.31 (d, J = 2.1 Hz, 1H); 7.15 (dd, J = 8.7 Hz, J =2.1 Hz, 1H) ; 7.03 (d, J = 8.1 Hz, 1H); 4.22 (d, J = 7.5 Hz, 2H); 2.23 (m, 1H); 1.78 (m, 4H) ; 0.97 (d, J = 6.6 Hz, 6H); 0.59 (t, J = 7.2 Hz, 6H). mp: > 300 °C.

### Example 97: 5-[(E)-[(6-ethoxy-1,1-dioxo-1,2-benzothiazol-3-yl)-isobutyl-hydrazono] methyl]-3,3-diethyl-indolin-2-one

### Step A: tert-butyl 5-[(E)-[(6-ethoxy-1,1-dioxo-1,2-benzothiazol-3-yl)-isobutyl-hydrazono]methyl]-3,3-diethyl-2-oxo-indoline-1-carboxylate

The compound was prepared using the same procedure as described in example 52 starting from tert-butyl 3,3-diethyl-5-[(E)-[(6-hydroxy-1,1-dioxo-1,2-benzothiazol-3-yl)-isobutyl-hydrazono]methyl]-2-oxo-indoline-1-carboxylate (example 96, step E), (100.0 mg; 0.18 mmol; 1.00 eq.) and ethyl iodide (21 µl; 0.26 mmol; 1.50 eq.) giving tert-butyl 5-[(E)-[(6-ethoxy-1,1-dioxo-1,2-benzothiazol-3-yl)-isobutyl-hydrazono] methyl]-3,3-diethyl-2-oxo-indoline-1-carboxylate (57.0 mg; 54 %). ¹H NMR (DMSO-d₆, 300 MHz): δ 8.63 (d, J = 8.6 Hz, 1H); 8.48 (s, 1H); 7.91 (s, 1H); 7.87 (s, 1H); 7.74 (s, 1H); 7.59 (d, J = 2.1 Hz, 1H); 7.29 (dd, J = 8.6 Hz, J = 2.1 Hz, 1H); 4.20 (m, 4H); 2.17 (m, 1H); 1.83 (m, 4H); 1.55 (s, 9H); 1.34 (t, J = 6.9 Hz, 3H); 0.93 (d, J = 6.6 Hz, 6H); 0.55 (t, J = 7.3 Hz, 6H).

### Step B: 5-[(E)-[(6-ethoxy-1,1-dioxo-1,2-benzothiazol-3-yl)-isobutyl-hydrazono] methyl]-3,3-diethyl-indolin-2-one

The compound was prepared using the same procedure as described in example 23 step I starting from tert-butyl 5-[(E)-[(6-ethoxy-1,1-dioxo-1,2-benzothiazol-3-yl)-isobutyl-hydrazono]methyl]-3,3-diethyl-2-oxo-indoline-1-carboxylate (55.0 mg; 0.09 mmol; 1.00 eq.) giving 5-[(E)-[(6-ethoxy-1,1-dioxo-1,2-benzothiazol-3-yl)-isobutyl-hydrazono]methyl]-3,3-diethyl-indolin-2-one (5.0 mg; 11 %) as a yellow solid. ¹H NMR (DMSO-d₆, 300 MHz): δ 8.64 (d, J = 8.6 Hz, 1H); 8.07 (s, 1H); 7.72 (s, 1H); 7.60 (d, J = 8.6 Hz, 1H); 7.55 (s, 1H); 7.41 (d, J = 2.1 Hz, 1H); 7.08 (dd, J = 8.6 Hz, J = 2.1 Hz, 1H); 7.02 (d, J = 8.6 Hz, 1H); 4.20 (m, 4H); 2.34 (m, 1H); 1.94 (m, 4H); 1.50 (t, J = 6.9 Hz, 3H); 1.09 (d, J = 6.6 Hz, 6H); 0.74 (t, J = 7.3 Hz, 6H). mp: 280-281 °C.

### Example 98: 3,3-diethyl-5-[(E)-[isobutyl-(6-methoxy-1,1-dioxo-1,2-benzothiazol-3-yl)hydrazono]methyl]indolin-2-one

### Step A: tert-butyl 3,3-diethyl-5-[(E)-[isobutyl-(6-methoxy-1,1-dioxo-1,2-benzothiazol-3-yl)hydrazono]methyl]-2-oxo-indoline-1-carboxylate

The compound was prepared using the same procedure as described in example 52 starting from tert-butyl 3,3-diethyl-5-[(E)-[(6-hydroxy-1,1-dioxo-1,2-benzothiazol-3-yl)-isobutyl-hydrazono]methyl]-2-oxo-indoline-1-carboxylate (example 96, step E), (100.0 mg; 0.18 mmol; 1.00 eq.) and methyl iodide (16 µl; 0.26 mmol; 1.50 eq.) giving tert-butyl 3,3-diethyl-5-[(E)-[isobutyl-(6-methoxy-1,1-dioxo-1,2-benzothiazol-3-yl)hydrazono]methyl]-2-oxo-indoline-1-carboxylate (102.0 mg; 99 %). LC/MS (Method D): Rt = 2.91 min; MS : m/z = 583 [M+H]⁺

### Step B: 3,3-diethyl-5-[(E)-[isobutyl-(6-methoxy-1,1-dioxo-1,2-benzothiazol-3-yl)hydrazono]methyl]indolin-2-one

The compound was prepared using the same procedure as described in example 23 step I starting from tert-butyl 3,3-diethyl-5-[(E)-[isobutyl-(6-methoxy-1,1-dioxo-1,2-benzothiazol-3-yl)hydrazono]methyl]-2-oxo-indoline-1-carboxylate (102.0 mg; 0.14 mmol; 1.00 eq.) giving 3,3-diethyl-5-[(E)-[isobutyl-(6-methoxy-1,1-dioxo-1,2-benzothiazol-3-yl)hydrazono]methyl]indolin-2-one (5.0 mg; 7 %) as a yellow solid. ¹H NMR (DMSO-d₆, 300 MHz): δ 10.70 (s, 1H); 8.70 (d, J = 8.8 Hz, 1H); 8.47 (s, 1H); 7.77 (d, J = 7.7 Hz, 1H) ; 7.66 (m, 2H); 7.34 (dd, J = 8.8 Hz, J = 2.3 Hz, 1H); 7.02 (d, J = 8.0 Hz, 1H); 4.24 (d, J = 7.3 Hz, 2H); 3.96 (s, 3H); 2.24 (m, 1H); 1.80 (m, 4H); 0.97 (d, J = 6.9 Hz, 6H); 0.58 (t, J = 7.2 Hz, 6H). mp: 242-243 °C.

### Example 99: 3,3-diethyl-5-[(E)-[isobutyl-(6-isopropoxy-1,1-dioxo-1,2-benzothiazol-3-yl)hydrazono]methyl]indolin-2-one

### Step A: tert-butyl 3,3-diethyl-5-[(E)-[isobutyl-(6-isopropoxy-1,1-dioxo-1,2-benzothiazol-3-yl)hydrazono]methyl]-2-oxo-indoline-1-carboxylate

The compound was prepared using the same procedure as described in example 52 starting from tert-butyl 3,3-diethyl-5-[(E)-[(6-hydroxy-1,1-dioxo-1,2-benzothiazol-3-yl)-isobutyl-hydrazono]methyl]-2-oxo-indoline-1-carboxylate (example 96, step E), (100.0 mg; 0.18 mmol; 1.00 eq.) and *iso-*propyl iodide (26 µl; 0.26 mmol; 1.50 eq.) giving tert-butyl 3,3-diethyl-5-[(E)-[isobutyl-(6-isopropoxy-1,1-dioxo-1,2-benzothiazol-3-yl)hydrazono]methyl]-2-oxo-indoline-1-carboxylate (59.0 mg; 55 %). LC/MS (Method F): Rt = 3.45 min ; MS : m/z = 611 [M+H]⁺

### Step B: 3,3-diethyl-5-[(E)-[isobutyl-(6-isopropoxy-1,1-dioxo-1,2-benzothiazol-3-yl)hydrazono]methyl]indolin-2-one

The compound was prepared using the same procedure as described in example 23 step I starting from tert-butyl 3,3-diethyl-5-[(E)-[isobutyl-(6-isopropoxy-1,1-dioxo-1,2-benzothiazol-3-yl)hydrazono]methyl]-2-oxo-indoline-1-carboxylate (56.0 mg; 0.09 mmol; 1.00 eq.) giving 3,3-diethyl-5-[(E)-[isobutyl-(6-isopropoxy-1,1-dioxo-1,2-benzothiazol-3-yl)hydrazono]methyl]indolin-2-one (5.0 mg; 11 %) as a yellow solid. ¹H NMR (DMSO-d₆, 300 MHz): δ 8.63 (d, J = 8.9 Hz, 1H); 8.06 (s, 1H); 7.85 (s, 1H); 7.59 (d, J = 8.1 Hz, 1H); 7.55 (s, 1H); 7.39 (d, J = 2.1 Hz, 1H); 7.04 (m, 2H) ; 4.71 (m, 1H); 4.25 (d, J = 7.6 Hz, 2H); 2.34 (m, 1H); 1.94 (m, 4H); 1.42 (d, J = 5.9 Hz, 6H); 1.09 (d, J = 6.4 Hz, 6H); 0.74 (t, J = 7.3 Hz, 6H). mp: 293-294 °C.

### Example 100: 4-[[3-[[(E)-(4-fluoro-3-methoxy-phenyl)methyleneamino]-isobutyl-amino]-1,1-dioxo-1,2-benzothiazol-6-yl]oxy]butan-1-ol

### Step A: 4-[[3-[[(E)-(4-fluoro-3-methoxy-phenyl)methyleneamino]-isobutyl-amino]-1,1-dioxo-1,2-benzothiazol-6-yl]oxy]butyl acetate

The compound was prepared using the same procedure as described in example 52 starting from (4-fluoro-3-methoxy-phenyl)methyleneamino]-isobutyl-amino]-1,1-dioxo-1,2-benzothiazol-6-ol (example 91), (100.0 mg; 0.25 mmol; 1.00 eq.) and 4-bromobutyl acetate (54 µl; 0.37 mmol; 1.50 eq.) giving 4-[[3-[[(E)-(4-fluoro-3-methoxyphenyl)methyleneamino]-isobutyl-amino]-1,1-dioxo-1,2-benzothiazol-6-yl]oxy]butyl acetate (85.1 mg; 66 %) as a pale yellow solid. ¹H NMR (DMSO-d₆, 300 MHz): δ 8.67 (d, J = 8.9 Hz, 1H); 8.50 (s, 1H); 7.64 (m, 2H) ; 7.46 (ddd, J = 2.0 Hz, J = 4.8 Hz, J = 8.4 Hz, 1H); 7.40 (m, 2H); 4.23 (m, 4H); 4.08 (t, J = 6.1 Hz, 2H); 3.96 (s, 3H); 2.26 (m, 1H); 2.00 (s, 3H); 1.78 (m, 4H); 0.97 (d, J = 6.8 Hz, 6H).

### Step B: 4-[[3-[[(E)-(4-fluoro-3-methoxy-phenyl)methyleneamino]-isobutyl-amino]-1,1-dioxo-1,2-benzothiazol-6-yl]oxy]butan-1-ol

A solution of LiOH (20.6 mg; 0.49 mmol; 3.06 eq.) in water (3.00 mL) was added to a suspension of 4-[[3-[[(E)-(4-fluoro-3-methoxy-phenyl)methyleneamino]-isobutyl-amino]-1,1-dioxo-1,2-benzothiazol-6-yl]oxy]butyl acetate (85.1 mg; 0.16 mmol; 1.00 eq.) in THF (3.00 mL) and the reaction was stirred 1h30 at rt. Then, it was acidified with an aqueous 1M HCI solution and concentrated. The residue was taken up into water and extracted with EtOAc. The resulting organic phase was dried over MgSO₄, filtered and concentrated to dryness. The residue was purified by LC/MS-Preparative (Column: Kinetex C18 30 x 150mm 5µm (phenomenex); Flow rate: 42 ml/min; Elution: H2O, 0.1% HCOOH / ACN, 0.1% HCOOH; Gradient: 10 to 100% ACN) to give 4-[[3-[[(E)-(4-fluoro-3-methoxyphenyl)methyleneamino]-isobutyl-amino]-1,1-dioxo-1,2-benzothiazol-6-yl]oxy]butan-1-ol (12.6 mg; 16 %) as a white solid. ¹H NMR (DMSO-d₆, 400 MHz): δ 8.67 (d, J = 8.8 Hz, 1H) ; 8.50 (s, 1H); 7.64 (m, 2H); 7.47 (ddd, J = 2.0 Hz, J = 4.6 Hz, J = 8.4 Hz, 1H); 7.41 (m, 2H); 4.48 (t, J = 5.2 Hz, 1H); 4.22 (m, 4H); 3.96 (s, 3H) ; 3.46 (m, 2H); 2.25 (m, J = 7.0 Hz, 1H); 1.79 (m, 2H); 1.58 (m, 2H); 0.97 (d, J = 6.8 Hz, 6H).

### Example 101: 3,3-diethyl-5-[(E)-[isobutyl-(5-methoxy-1,1-dioxo-1,2-benzothiazol-3-yl)hydrazono]methyl]indolin-2-one

### Step A: tert-butyl 5-[(E)-[(5-bromo-1,1-dioxo-1,2-benzothiazol-3-yl)-isobutyl-hydrazono]methyl]-3,3-diethyl-2-oxo-indoline-1-carboxylate

The compound was prepared using the same procedure as described in example 21 step B starting from 5-bromo-3-chloro-1,2-benzothiazole 1,1-dioxide (example 53, step B), (1.7 g; 6.06 mmol; 1.00 eq.) and tert-butyl 3,3-diethyl-5-[(E)-(isobutyl hydrazono)methyl]-2-oxo-indoline-1-carboxylate (example 96, step B), (1.40 g; 1.81 mmol; 1.00 eq.) giving tert-butyl 5-[(E)-[(5-bromo-1,1-dioxo-1,2-benzothiazol-3-yl)-isobutyl-hydrazono]methyl]-3,3-diethyl-2-oxo-indoline-1-carboxylate (884 mg; 54 %) as a yellow solid. ¹H NMR (DMSO-d₆, 300 MHz): δ 9.02 (s, 1H); 8.58 (s, 1H); 8.09 (m, 2H); 7.87 (m, 3H); 4.27 (d, J = 7.5 Hz, 2H); 3.58 (m, 1H); 1.92 (m, 4H); 1.59 (s, 9H) ; 0.99 (d, J = 6.6 Hz, 6H); 0.56 (t, J = 7.3 Hz, 6H).

### Step B: tert-butyl 5-[(E)-[[1,1-dioxo-5-(4,4,5,5-tetramethyl-1,3,2-dioxaborolan-2-yl)-1,2-benzothiazol-3-yl]-isobutyl-hydrazono]methyl]-3,3-diethyl-2-oxo-indoline-1-carboxylate

The compound was prepared using the same procedure as described in example 50 step F starting from tert-butyl 5-[(E)-[(5-bromo-1,1-dioxo-1,2-benzothiazol-3-yl)-isobutyl-hydrazono]methyl]-3,3-diethyl-2-oxo-indoline-1-carboxylate (640.0 mg; 1.01 mmol; 1.00 eq.) giving tert-butyl 5-[(E)-[[1,1-dioxo-5-(4,4,5,5-tetramethyl-1,3,2-dioxaborolan-2-yl)-1,2-benzothiazol-3-yl]-isobutyl-hydrazono]methyl]-3,3-diethyl-2-oxo-indoline-1-carboxylate carboxylate (478.0 mg; 70%) as a white solid. LC/MS (Method C): Rt = 2.26 min ; MS : m/z = 679 [M+H]⁺

### Step C: tert-butyl 3,3-diethyl-5-[(E)-[(5-hydroxy-1,1-dioxo-1,2-benzothiazol-3-yl)-isobutyl-hydrazono]methyl]-2-oxo-indoline-1-carboxylate

The compound was prepared using the same procedure as described in example 51 starting from tert-butyl 5-[(E)-[[1,1-dioxo-5-(4,4,5,5-tetramethyl-1,3,2-dioxaborolan-2-yl)-1,2-benzothiazol-3-yl]-isobutyl-hydrazono]methyl]-3,3-diethyl-2-oxo-indoline-1-carboxylate (500.0 mg; 0.74 mmol; 1.00 eq.) to give tert-butyl 3,3-diethyl-5-[(E)-[(5-hydroxy-1,1-dioxo-1,2-benzothiazol-3-yl)-isobutyl-hydrazono]methyl]-2-oxo-indoline-1-carboxylate (164.0 mg ; 59 %). LC/MS (Method F): Rt = 2.15 min; MS : m/z = 569 [M+H]⁺

### Step D: 5-[(E)-[(5-ethoxy-1,1-dioxo-1,2-benzothiazol-3-yl)-isobutyl-hydrazono] methyl]-3,3-diethyl-indolin-2-one

The compound was prepared using the same procedure as described in example 52 and followed by procedure in example 23 step I on the crude, starting from tert-butyl 3,3-diethyl-5-[(E)-[(5-hydroxy-1,1-dioxo-1,2-benzothiazol-3-yl)-isobutyl-hydrazono]methyl]-2-oxo-indoline-1-carboxylate (81.0 mg; 0.14 mmol; 1.00 eq.) and ethyl iodide (10 µl; 0.13 mmol; 0.9 eq.) giving 5-[(E)-[(5-ethoxy-1,1-dioxo-1,2-benzothiazol-3-yl)-isobutyl-hydrazono]methyl]-3,3-diethyl-indolin-2-one (21.0 mg; 30 %), as a pale yellow solid. ¹H NMR (DMSO-d₆, 300 MHz): δ 10.73 (s, 1H); 8.49 (s, 1H); 8.35 (d, J = 2.3 Hz, 1H); 7.97 (d, J = 8.1 Hz, 1H); 7.72 (m, 2H); 7.38 (dd, J = 8.1 Hz, J = 2.3 Hz, 1H); 7.00 (d, J = 8.5 Hz, 1H); 4.21 (m, 4H); 2.25 (m, 1H); 1.77 (m, 4H); 1.37 (t, J = 6.8 Hz, 3H); 0.98 (d, J = 6.5 Hz, 6H); 0.56 (t, J = 7.3 Hz, 6H). mp: 228-230 °C.

### Example 102: [3-[[(E)-(4-fluoro-3-methoxy-phenyl)methyleneamino]-isopropyl-amino]-1,1-dioxo-1,2-benzothiazol-6-yl]boronic acid

### Step A: 6-bromo-N-[(E)-(4-fluoro-3-methoxy-phenyl)methyleneamino]-N-isopropyl-1,1-dioxo-1,2-benzothiazol-3-amine

The intermediate hydrazone was prepared using the same procedure as described in example 21 step A starting from 4-fluoro-3-methoxybenzaldehyde (372 mg; 2.41 mmol; 1.00 eq.) and isopropylhydrazine hydrochloride (267 mg; 2.41 mmol; 1.20 eq.). The hydrazone was next mixed with 6-bromo-1,1-dioxo-1,2-benzothiazol-3-one (example 50, step C), (633 mg; 2.42 mmol; 1.00 eq.) into phosphorus(V) oxychloride (5.7 ml; 60.36 mmol; 25.00 eq.) and the mixture was stirred under reflux 3h. Then, phosphorus(V) oxychloride was evaporated and the residue taken into water (30 mL). The solid obtained was filtered, washed with water (3x), ACN (3x) and dried under vacuum at 60°C for 3h. It was then mixed with ACN filtrates, previously obtained, and the solvent was evaporated. The solid obtained was taken up into diethyl ether, filtered, washed with diethyl ether (3x) and dried under vacuum at 60°C 3h to give 6-bromo-N-[(E)-(4-fluoro-3-methoxyphenyl)methyl eneamino]-N-isopropyl-1,1-dioxo-1,2-benzothiazol-3-amine (545.0 mg; 48 %) as a yellow solid. ¹H NMR (DMSO-d₆, 300 MHz): δ 8.71 (s, 1H) ; 8.43 (d, J = 1.7 Hz, 1H); 8.36 (d, J = 8.6 Hz, 1H); 8.09 (dd, J = 1.8 Hz, J = 8.6 Hz, 1H); 7.67 (dd, J = 1.9 Hz, J = 8.5 Hz, 1H); 7.50 (ddd, J = 2.0 Hz, J = 4.5, J = 8.4 Hz, 1H); 7.42 (dd, J = 8.4 Hz, J = 11.1 Hz, 1H); 5.16 (sept., J = 6.9 Hz, 1H); 3.95 (s, 3H); 1.57 (d, J = 6.9 Hz, 6H).

### Step B: N-[(E)-(4-fluoro-3-methoxy-phenyl)methyleneamino]-N-isopropyl-1,1-dioxo-6-(4,4,5,5-tetramethyl-1,3,2-dioxaborolan-2-yl)-1,2-benzothiazol-3-amine

The compound was prepared using the same procedure as in example 50 step F starting from 6-bromo-N-[(E)-(4-fluoro-3-methoxy-phenyl)methyleneamino]-N-isopropyl-1,1-dioxo-1,2-benzothiazol-3-amine (220 mg; 0.48 mmol; 1.0 eq.) giving N-[(E)-(4-fluoro-3-methoxyphenyl)methyleneamino]-N-isopropyl-1,1-dioxo-6-(4,4, 5,5-tetramethyl-1,3,2-dioxaborolan-2-yl)-1,2-benzothiazol-3-amine (96 mg; 40%) as a pale yellow solid. ¹H NMR (DMSO-d₆, 300 MHz): δ 8.71 (s, 1H); 8.52 (dd, J = 0.8 Hz, J = 7.9 Hz, 1H); 8.09 (m, 2H); 7.69 (dd, J = 2.0 Hz, J = 8.4 Hz, 1H); 7.52 (ddd, J = 2.0 Hz, J = 4.6 Hz, J = 8.4 Hz, 1H); 7.42 (dd, J = 8.4 Hz, J = 11.1 Hz, 1H); 5.18 (sept., J = 6.9 Hz, 1H); 3.96 (s, 3H); 1.58 (d, J = 6.8 Hz, 6H); 1.34 (s, 12H).

### Step C: [3-[[(E)-(4-fluoro-3-methoxy-phenyl)methyleneamino]-isopropyl-amino]-1,1-dioxo-1,2-benzothiazol-6-yl]boronic acid

The compound was prepared using the same procedure as described in example 62 step C starting from N-[(E)-(4-fluoro-3-methoxy-phenyl)methyleneamino]-N-isopropyl-1,1-dioxo-6-(4,4,5,5-tetramethyl-1,3,2-dioxaborolan-2-yl)-1,2-benzothiazol-3-amine (92.0 mg; 0.18 mmol; 1.00 eq.) giving [3-[[(E)-(4-fluoro-3-methoxy-phenyl)methyleneamino]-isopropyl-amino]-1,1-dioxo-1,2-benzothiazol-6-yl]boronic acid (20.0 mg; 24 %) as a white solid. ¹H NMR (DMSO-d₆, 400 MHz): δ 8.71 (s, 1H) ; 8.63 (s, 2H) ; 8.45 (d, J = 8.1 Hz, 1H); 8.32 (s, 1H); 8.16 (dd, J = 1.0 Hz, J = 8.0 Hz, 1H); 7.71 (dd, J = 1.9 Hz, J = 8.5 Hz, 1H); 7.52 (ddd, J = 1.8 Hz, J = 4.6 Hz, J = 8.4 Hz, 1H); 7.43 (dd, J = 8.4 Hz, J = 11.2 Hz, 1H); 5.18 (sept., J = 6.9 Hz, 1H); 3.96 (s, 3H); 1.57 (d, J = 6.8 Hz, 6H).

### Example 103: 1-(azetidin-1-yl)-2-[[3-[[(E)-(4-fluoro-3-methoxy-phenyl)methylene amino]-isobutyl-amino]-1,1-dioxo-1,2-benzothiazol-5-yl]oxy]ethanone

### Step A: tert-butyl 2-[[3-[[(E)-(4-fluoro-3-methoxy-phenyl)methyleneamino]-isobutyl-amino]-1,1-dioxo-1,2-benzothiazol-5-yl]oxy]acetate

The compound was prepared using the same procedure as described in example 52 starting from 3-[[(E)-(4-fluoro-3-methoxy-phenyl)methyleneamino]-isobutyl-amino]-1,1-dioxo-1,2-benzothiazol-5-ol (example 93, step C), (200.0 mg; 0.49 mmol; 1.00 eq.) and tert-butyl bromoacetate (144 µl; 0.98 mmol; 2.00 eq.) giving tert-butyl 2-[[3-[[(E)-(4-fluoro-3-methoxy-phenyl)methyleneamino]-isobutyl-amino]-1,1-dioxo-1,2-benzothiazol-5-yl]oxy]acetate (220.0 mg; 86 %) as a yellow solid. LC/MS (Method A): Rt = 2.61 min; MS : m/z = 520 [M+H]⁺

### Step B: 2-[[3-[[(E)-(4-fluoro-3-methoxy-phenyl)methyleneamino]-isobutyl-amino]-1,1-dioxo-1,2-benzothiazol-5-yl]oxy]acetic acid

The compound was prepared using the same procedure as described in example 58 step B starting from tert-butyl 2-[[3-[[(E)-(4-fluoro-3-methoxy-phenyl)methyleneamino]-isobutyl-amino]-1,1-dioxo-1,2-benzothiazol-5-yl]oxy]acetate (200.0 mg; 0.38 mmol; 1.00 eq.) giving 2-[[3-[[(E)-(4-fluoro-3-methoxy-phenyl)methyleneamino]-isobutyl-amino]-1,1-dioxo-1,2-benzothiazol-5-yl]oxy]acetic acid (170.0 mg; 90 %) as a white solid. ¹H NMR (DMSO-d₆, 400 MHz): δ 8.53 (s, 1H); 8.37 (d, J = 2.4 Hz, 1H); 8.01 (d, J = 8.4 Hz, 1H); 7.66 (dd, J = 1.9 Hz, J = 8.5 Hz, 1H); 7.46 (ddd, J = 2.0 Hz, J = 4.6 Hz, J = 8.4 Hz, 1H); 7.40 (m, 2H) ; 4.90 (s, 2H) ; 4.25 (d, J = 7.9 Hz, 2H) ; 3.96 (s, 3H) ; 2.27 (m, 1H); 0.98 (d, J = 6.6 Hz, 6H).

### Step C: 1-(azetidin-1-yl)-2-[[3-[[(E)-(4-fluoro-3-methoxy-phenyl)methyleneamino]-isobutyl-amino]-1,1-dioxo-1,2-benzothiazol-5-yl]oxy]ethanone

The compound was prepared using the same procedure as described in example 58 step C starting from 2-[[3-[[(E)-(4-fluoro-3-methoxy-phenyl)methyleneamino]-isobutyl-amino]-1,1-dioxo-1,2-benzothiazol-5-yl]oxy]acetic acid (50.0 mg; 0.11 mmol; 1.00 eq.) and azetidine (11 µl; 0.16 mmol; 1.50 eq.) giving 1-(azetidin-1-yl)-2-[[3-[[(E)-(4-fluoro-3-methoxy-phenyl)methyleneamino]-isobutyl-amino]-1,1-dioxo-1,2-benzothiazol-5-yl]oxy]ethanone (35.0 mg; 61 %) as a white solid. ¹H NMR (DMSO-d₆, 400 MHz): δ 8.53 (s, 1H); 8.36 (d, J = 2.4 Hz, 1H) ; 8.03 (d, J = 8.4 Hz, 1H); 7.66 (dd, J = 1.9 Hz, J = 8.3 Hz, 1H); 7.46 (ddd, J = 2.0 Hz, J = 4.8, J = 8.4 Hz, 1H); 7.41 (dd, J = 8.4 Hz, J = 10.8 Hz, 1H); 7.36 (dd, J = 2.4 Hz, J = 8.6 Hz, 1H); 4.79 (s, 2H); 4.25 (d, J = 7.7 Hz, 2H); 4.19 (t, J = 7.7 Hz, 2H); 3.96 (s, 3H); 3.89 (t, J = 7.8 Hz, 2H); 2.24 (m, 3H); 0.98 (d, J = 6.8 Hz, 6H). mp: 211 °C.

### Example 104: 2-[[3-[[(E)-(4-fluoro-3-methoxy-phenyl)methyleneamino]-isobutyl-amino]-1,1-dioxo-1,2-benzothiazol-6-yl]oxy]acetamide

### Step A: tert-butyl 2-[[3-[[(E)-(4-fluoro-3-methoxy-phenyl)methyleneamino]-isobutyl-amino]-1,1-dioxo-1,2-benzothiazol-6-yl]oxy]acetate

The compound was prepared using the same procedure as described in example 52 starting from (4-fluoro-3-methoxy-phenyl)methyleneamino]-isobutyl-amino]-1,1-dioxo-1,2-benzothiazol-6-ol (example 91), (300.0 mg; 0.74 mmol; 1.00 eq.) and tert-butyl bromoacetate (216 µl; 1.48 mmol; 2.00 eq.) giving tert-butyl 2-[[3-[[(E)-(4-fluoro-3-methoxy-phenyl)methyleneamino]-isobutyl-amino]-1,1-dioxo-1,2-benzothiazol-6-yl]oxy]acetate (350.0 mg; 91 %) as a yellow oil. LC/MS (Method A): Rt = 2.65 min; MS : m/z = 520 [M+H]⁺

### Step B: 2-[[3-[[(E)-(4-fluoro-3-methoxy-phenyl)methyleneamino]-isobutyl-amino]-1,1-dioxo-1,2-benzothiazol-6-yl]oxy]acetic acid

The compound was prepared using the same procedure as described in example 58 step B starting from tert-butyl 2-[[3-[[(E)-(4-fluoro-3-methoxy-phenyl)methyleneamino]-isobutyl-amino]-1,1-dioxo-1,2-benzothiazol-6-yl]oxy]acetate (300.0 mg; 0.58 mmol; 1.00 eq.) giving 2-[[3-[[(E)-(4-fluoro-3-methoxy-phenyl)methyleneamino]-isobutyl-amino]-1,1-dioxo-1,2-benzothiazol-6-yl]oxy]acetic acid (266.0 mg; 94 %) as a white solid. ¹H NMR (DMSO-d₆, 400 MHz): δ 8.68 (d, J = 8.8 Hz, 1H); 8.50 (s, 1H); 7.67 (d, J = 2.4 Hz, 1H); 7.63 (dd, J = 1.8 Hz, J = 8.6 Hz, 1H); 7.48 (ddd, J = 1.8 Hz, J = 4.6 Hz, J = 8.4 Hz, 1H); 7.41 (m, 2H); 4.98 (s, 2H) ; 4.24 (d, J = 7.7 Hz, 2H) ; 3.96 (s, 3H) ; 2.26 (m, 1H) ; 0.98 (d, J = 6.6 Hz, 6H).

### Step C: 2-[[3-[[(E)-(4-fluoro-3-methoxy-phenyl)methyleneamino]-isobutyl-amino]-1,1-dioxo-1,2-benzothiazol-6-yl]oxy]acetamide

The compound was prepared using the same procedure as described in example 58 step C starting from 2-[[3-[[(E)-(4-fluoro-3-methoxy-phenyl)methyleneamino]-isobutyl-amino]-1,1-dioxo-1,2-benzothiazol-6-yl]oxy]acetic acid (50.0 mg; 0.11 mmol; 1.00 eq.) and ammonium chloride (57.7 mg; 1.08 mmol; 10.00 eq.) giving 2-[[3-[[(E)-(4-fluoro-3-methoxy-phenyl)methyleneamino]-isobutyl-amino]-1,1-dioxo-1,2-benzothiazol-6-yl]oxy]acetamide (33.0 mg; 63 %) as a white solid. ¹H NMR (DMSO-d₆, 400 MHz): δ 8.69 (d, J = 9.0 Hz, 1H); 8.51 (s, 1H); 7.65 (m, 2H); 7.61 (d, J = 2.4 Hz, 1H); 7.48 (m, 2H) ; 7.42 (m, 2H) ; 4.71 (s, 2H) ; 4.24 (d, J = 7.7 Hz, 2H) ; 3.97 (s, 3H); 2.25 (m, J = 6.9 Hz, 1H); 0.97 (d, J = 6.6 Hz, 6H). mp: 112 °C.

### Example 105: 2-[[3-[[(E)-(4-fluoro-3-methoxy-phenyl)methyleneamino]-isobutyl-amino]-1,1-dioxo-1,2-benzothiazol-5-yl]oxy]-N-methyl-acetamide

The compound was prepared using the same procedure as described in example 52 starting from 3-[[(E)-(4-fluoro-3-methoxy-phenyl)methyleneamino]-isobutyl-amino]-1,1-dioxo-1,2-benzothiazol-5-ol (example 93, step C), (150.0 mg; 0.37 mmol; 1.00 eq.) and 2-chloro-N-methylacetamide (79.6 mg; 0.74 mmol; 2.00 eq.) giving 2-[[3-[[(E)-(4-fluoro-3-methoxy-phenyl)methyleneamino]-isobutyl-amino]-1,1-dioxo-1,2-benzothiazol-5-yl]oxy]-N-methyl-acetamide (14.0 mg; 8 %) as a white solid. ¹H NMR (DMSO-d₆, 400 MHz): δ 8.54 (s, 1H); 8.43 (d, J = 2.4 Hz, 1H); 8.15 (br q, J = 4.5 Hz, 1H); 8.03 (d, J = 8.4 Hz, 1H); 7.68 (dd, J = 1.9 Hz, J = 8.5 Hz, 1H); 7.49 (ddd, J = 2.0 Hz, J = 4.4 Hz, J = 8.1 Hz, 1H) ; 7.41 (dd, J = 8.5 Hz, J = 11.1 Hz, 1H); 7.37 (dd, J = 2.4 Hz, J = 8.6 Hz, 1H); 4.69 (s, 2H) ; 4.26 (d, J = 7.9 Hz, 2H); 3.96 (s, 3H); 2.64 (d, J = 4.6 Hz, 3H); 2.27 (m, 1H); 0.98 (d, J = 6.8 Hz, 6H).

### Example 106: 2-[[3-[[(E)-(4-fluoro-3-methoxy-phenyl)methyleneamino]-isobutyl-amino]-1,1-dioxo-1,2-benzothiazol-6-yl]oxymethyl]-6-methyl-1,3,6,2-dioxaza-borocane-4,8-dione

The compound was prepared using the same procedure as described in example 68 starting from [3-[[(E)-(4-fluoro-3-methoxy-phenyl)methyleneamino]-isobutyl-amino]-1,1-dioxo-1,2-benzothiazol-6-yl]oxymethylboronic acid (example 92), (100.0 mg; 0.22 mmol; 1.00 eq.) giving 2-[[3-[[(E)-(4-fluoro-3-methoxy-phenyl)methyleneamino]-isobutyl-amino]-1,1-dioxo-1,2-benzothiazol-6-yl]oxymethyl]-6-methyl-1,3,6,2-dioxazaborocane-4,8-dione (50.0 mg; 40 %) as a white solid. ¹H NMR (DMSO-d₆, 400 MHz): δ 8.68 (d, J = 8.8 Hz, 1H) ; 8.50 (s, 1H); 7.75 (d, J = 2.2 Hz, 1H); 7.64 (dd, J = 1.9 Hz, J = 8.5 Hz, 1H) ; 7.48 (m, 2H) ; 7.40 (dd, J = 8.4 Hz, J = 11.0 Hz, 1H); 4.35 (d, J = 17.2 Hz, 2H) ; 4.24 (d, J = 7.7 Hz, 2H) ; 4.13 (d, J = 17.2 Hz, 2H) ; 3.97 (s, 3H) ; 3.84 (s, 2H) ; 3.00 (s, 3H) ; 2.25 (m, 1H); 0.97 (d, J = 6.8 Hz, 6H). mp: 169 °C.

### Example 107: N-[(E)-(4-fluoro-3-methylsulfanyl-phenyl)methyleneamino]-N-isobutyl-1,1-dioxo-1,2-benzothiazol-3-amine

4-fluoro-3-(methylsulfanyl)benzaldehyde (150.0 mg; 0.88 mmol; 1.00 eq.) and 2-methylpropylhydrazine hydrochloride(120.8 mg; 0.97 mmol; 1.10 eq.) were mixed together at rt in THF (4.00 mL) and stirred 30 minutes until complete formation of the intermediate hydrazone. Then, 3-chloro-1,2-benzothiazole 1,1-dioxide (example 1, step A), (159.9 mg; 0.79 mmol; 0.90 eq.) was added and the reaction was stirred under reflux 1h30. Next, the reaction was cooled to rt and THF was evaporated. The residue was taken up into EtOH and the solid obtained was filtered, washed twice with EtOH and dried under vacuum to give N-[(E)-(4-fluoro-3-methylsulfanyl-phenyl)methyleneamino]-N-isobutyl-1,1-dioxo-1,2-benzothiazol-3-amine (206.4 mg; 57 %) as a white solid. ¹H NMR (DMSO-d₆, 400 MHz): δ 8.81 (m, 1H); 8.56 (s, 1H); 8.09 (m, 1H); 7.88 (m, 3H); 7.73 (ddd, J = 2.1 Hz, J = 5.0 Hz, J = 8.5 Hz, 1H); 7.40 (dd, J = 8.4 Hz, J = 10.1 Hz, 1H); 4.27 (d, J = 7.8 Hz, 2H); 2.61 (s, 3H); 2.28 (td, J = 6.9 Hz, J = 13.7 Hz, 1H); 0.99 (d, J = 6.8 Hz, 6H).

### Example 108: 3-[(1,1-dioxo-1,2-benzothiazol-3-yl)-[(E)-(4-fluoro-3-methoxyphenyl)methyleneamino]amino]-2,2-dimethyl-propan-1-ol

The compound was prepared using the same procedure as described in example 107 starting from 4-fluoro-3-methoxybenzaldehyde (63.0 mg; 0.41 mmol; 1.00 eq.), 3-hydrazinyl-2,2-dimethylpropan-1-ol (76.0 mg; 0.49 mmol; 1.20 eq.) and 3-chloro-1,2-benzothiazole 1,1-dioxide (example 1, step A), (65.0 mg; 0.32 mmol; 0.78 eq.) giving 3-[(1,1-dioxo-1,2-benzothiazol-3-yl)-[(E)-(4-fluoro-3-methoxy-phenyl) methyleneamino]amino]-2,2-dimethyl-propan-1-ol (23.0 mg; 17 %) as a white solid. 1H NMR (DMSO-d6, 300 MHz): δ 8.77 (d, J = 7.8 Hz, 1H); 8.37 (s, 1H); 7.99 (d , J = 7.4 Hz, 1H); 7.71 (m, 2H) ; 7.41 (d, J = 7.8 Hz, 1H); 7.22 (m, 2H) ; 4.31 (s, 2H); 3.98 (s, 3H) ; 3.36 (d, J = 5.7 Hz, 2H); 3.19 (m, 1H); 1.08 (s, 6H). mp: 207-208°C.

### Example 109: [3-[6-(4-fluoro-3-methoxy-phenyl)-3-methyl-4,5-dihydro-3H-pyridazin - 2-yl]-1,1-dioxo-1,2-benzothiazol-6-yl]boronic acid

### Step A: 4-chloro-N-methoxy-N-methyl-pentanamide

To a suspension of commercial 4-chloropentanoyl chloride (40.0 g; 258.03 mmol; 1.00 eq.) and O,N dimethyl hydroxylamine hydrochloride (27.69 g; 283.87 mmol; 1.10 eq.) in DCM (480 mL) at 0°C, was added a solution of pyridine (48.0 mL; 593.47 mmol; 2.30 eq.) in DCM (200 mL) during 20 min keeping temperature below 10°C. This mixture was stirred 1h at 0 °C, then warmed to rt overnight. Then, the mixture was washed with aqueous 1N HCl (500 mL), aqueous saturated NaHCO3 solution (500 mL) and brine (500 mL). Organic layer was dried over MgSO₄, filtered and concentrated to dryness to give 4-chloro-N-methoxy-N-methyl-pentanamide (42.79 g; 92 %) as a colorless oil. 1H NMR (DMSO-d6, 300 MHz): δ 4.21 (m, 1H); 3.67 (s, 3H); 3.09 (s, 3H); 2.55 (t, J = 7.3 Hz, 2H); 1.99 (m, 1H); 1.79 (m, 1H); 1.48 (d, J = 6.4 Hz, 3H).

### Step B: 4-chloro-1-(4-fluoro-3-methoxy-phenyl)pentan-1-one

In a 1 I three necked round bottom flask, flame dried, under argon, 2-fluoro-5-bromoanisole (27.40 g; 133.64 mmol; 1.20 eq.) was diluted in THF (200 mL) and the mixture was cooled to -78°C. Then, n-Butyllithium 1.6M in hexane (98.0 mL; 1.6 mol/L; 156.8 mmol; 1.41 eq.) was slowly added during 20 min, keeping temperature below -65°C and the reaction was stirred at -78°C for 1h. Next, a solution of 4-chloro-N-methoxy-N-methyl-pentanamide (20.00 g; 111.33 mmol; 1.00 eq.) in THF (100 mL) was slowly added to the lithiated species during 25 min, keeping temperature below -65°C. The reaction was then stirred 40 min at -78°C and quenched by addition of aqueous 1N HCl (500 mL). The mixture was allowed to warm to rt and was extracted EtOAc (3 x 250 mL). Combined organic layer was dried over MgSO₄, filtered, and concentrated to dryness to give 4-chloro-1-(4-fluoro-3-methoxy-phenyl)pentan-1-one (32.50 g; quantitative ; 47% LC/MS purity) as a yellow liquid, used in the next step without further purification. LC/MS (Method A): Rt = 2.20 min; MS : m/z = 245 [M+H]⁺

### Step C: 6-bromo-3-[6-(4-fluoro-3-methoxy-phenyl)-3-methyl-4,5-dihydro-3H-pyridazin-2-yl]-1,2-benzothiazole 1,1-dioxide

4-chloro-1-(4-fluoro-3-methoxy-phenyl)pentan-1-one (31.44 g; 60.39 mmol; 1.00 eq.) and hydrazine hydrate (18.0 mL; 35 %w/w; 198.56 mmol; 3.29 eq.) were dissolved in EtOH (295 mL). The mixture was stirred under reflux for 1 h. Then, it was concentrated under vacuum to remove ethanol and hydrazine excess to give the intermediate 3-(4-fluoro-3-methoxy-phenyl)-6-methyl-1,4,5,6-tetrahydro pyridazine as a pale yellow gum. The latter was dissolved into phosphorus(V) oxychloride (125.0 mL; 1 324.74 mmol; 21.94 eq.) and 6-bromo-1,1-dioxo-1,2-benzothiazol-3-one (example 50, step C), (15.83 g; 60.40 mmol; 1.00 eq.) was added. The mixture was stirred under reflux for 1h, then cooled to rt and poured onto ice (400 mL) very slowly. The solid obtained was filtered, washed with water (3x) and dried under vacuum. Finally, it was washed with diethyl ether (3x) and dried under vacuum at 50°C for 2h to give 6-bromo-3-[6-(4-fluoro-3-methoxy-phenyl)-3-methyl-4,5-dihydro-3H-pyridazin-2-yl]-1,2-benzothiazole 1,1-dioxide (19.64 g; 70 %; 75% LCMS purity) as a brown solid, used in the next step without further purification. 1H NMR (DMSO-d6, 300 MHz): δ 8.61 (d, J = 8.4 Hz, 1H); 8.44 (d, J = 1.8 Hz, 1H); 8.12 (dd, J = 1.9 Hz, J = 8.5 Hz, 1H): 7.60 (dd, J = 2.0 Hz, J = 8.4 Hz, 1H); 7.44 (ddd, J = 2.1 Hz, J = 4.6 Hz, J = 8.4 Hz, 1H); 7.37 (dd, J = 8.4 Hz, J = 10.9 Hz, 1H); 4.90 (m, 1H); 3.94 (s, 3H); 3.05 (m, 1H); 2.79 (m, 1H); 2.12 (m, 2H); 1.32 (d, J =6 .8 Hz, 3H).

### Step D: 3-[6-(4-fluoro-3-methoxy-phenyl)-3-methyl-4,5-dihydro-3H-pyridazin-2-yl]-6-(4,4,5,5-tetramethyl-1,3,2-dioxaborolan-2-yi)-1,2-benzothiazole 1,1-dioxide

The compound was prepared using the same procedure as described in example 50 step F starting from 6-bromo-3-[6-(4-fluoro-3-methoxy-phenyl)-3-methyl-4,5-dihydro-3H-pyridazin-2-yl]-1,2-benzothiazole 1,1-dioxide (19.98 g; 42.85 mmol; 1.00 eq.) giving 3-[6-(4-fluoro-3-methoxy-phenyl)-3-methyl-4,5-dihydro-3H-pyridazin-2-yl]-6-(4,4,5,5-tetramethyl-1,3,2-dioxaborolan-2-yl)-1,2-benzothiazole 1,1-dioxide (10.27 g; 43%) as a beige solid. ¹H NMR (DMSO-d₆, 300 MHz): δ 8.76 (dd, J = 0.7 Hz, J = 7.8 Hz, 1H); 8.10 (m, 2H); 7.63 (dd, J = 2.1 Hz, J = 8.3 Hz, 1H); 7.47 (ddd, J = 2.1 Hz, J = 4.6 Hz, J = 8.6 Hz, 1H); 7.38 (dd, J = 8.4 Hz, J = 11.1 Hz, 1H); 4.92 (m, 1H); 3.96 (s, 3H); 3.06 (m, 1H); 2.79 (m, 1H); 2.14 (m, 2H); 1.34 (s, 12H); 1.16 (s, 3H).

### Step E: [3-[6-(4-fluoro-3-methoxy-phenyl)-3-methyl-4,5-dihydro-3H-pyridazin-2-yl]-1,1-dioxo-1,2-benzothiazol-6-yl]boronic acid

The compound was prepared using the same procedure as described in example 62 step C starting from 3-[6-(4-fluoro-3-methoxy-phenyl)-3-methyl-4,5-dihydro-3H-pyridazin-2-yl]-6-(4,4,5,5-tetramethyl-1,3,2-dioxaborolan-2-yl)-1,2-benzothiazole 1,1-dioxide (10.27 g; 20.00 mmol; 1.00 eq.) giving [3-[6-(4-fluoro-3-methoxy-phenyl)-3-methyl-4,5-dihydro-3H-pyridazin-2-yl]-1,1-dioxo-1,2-benzothiazol-6-yl]boronic acid (3.06 g; 35 %) as a pale yellow solid. ¹H NMR (DMSO-d₆+ 10% v/v D₂O, 400 MHz): δ 8.73 (d, J = 8.4 Hz, 1H); 8.32 (s, 1H); 8.19 (dd, J = 1.1 Hz, J = 7.9 Hz, 1H); 7.63 (dd, J = 2.1 Hz, J = 8.5 Hz, 1H); 7.46 (ddd, J = 2.2 Hz, J = 4.4 Hz, J = 8.6 Hz, 1H) ; 7.38 (dd, J = 8.4 Hz, J = 11.0 Hz, 1H) ; 4.93 (m, 1H); 3.96 (s, 3H); 3.02 (m, 1H); 2.82 (m, 1H); 2.13 (m, 2H); 1.34 (d, J = 6.8 Hz, 3H). mp: 315°C.

### Example 110: 3-[6-(4-fluoro-3-methoxy-phenyl)-4,4-dimethyl-3,5-dihydropyridazin-2-yl]-1,2-benzothiazole 1,1-dioxide

### Step A: 4-(4-fluoro-3-methoxy-phenyl)-2,2-dimethyl-4-oxo-butanoic acid

A solution of 2-fluoro-5-bromoanisole (1.00 g; 4.88 mmol; 1.00 eq.) and 1,2-dibromoethane (0.23 mL; 2.68 mmol; 0.55 eq.) in THF (10 mL) was added to a suspension of magensium (0.59 g; 24.39 mmol; 5.00 eq.) in THF (10mL) under argon. The mixture was heated under lightly reflux for 45min, then cooled to 0°C and 2,2-dimethylsuccinic anhydride (0.56 g; 4.39 mmol; 0.90 eq.) was added. After 10min at 0°C, the reaction was stirred at rt overnight. Then, it was quenched with aqueous NH4CI sat. and extracted with EtOAc (3x). Combined organic layer was dried over MgSO₄, filtered and concentrated. The residue was purified by chromatography (column SiO₂ 25g, flow rate 25ml/min, gradient from 85/15 Cyclohexane/EtOAc to 100% EtOAc) to give 4-(4-fluoro-3-methoxy-phenyl)-2,2-dimethyl-4-oxo-butanoic acid (50.0 mg; 3 %) as a white solid. 1H NMR (CDCl₃, 300 MHz): δ 7.61 (dd, J = 2.1 Hz, J = 8.3 Hz, 1H); 7.52 (ddd, J = 2.2 Hz, J = 4.2 Hz, J = 8.3 Hz, 1H); 7.13 (dd, J = 8.4 Hz, J = 10.6 Hz, 1H); 3.94 (s, 3H) ; 3.28 (s, 2H); 1.36 (s, 6H).

### Step B: 3-(4-fluoro-3-methoxy-phenyl)-5,5-dimethyl-1,4-dihydropyridazin-6-one

4-(4-fluoro-3-methoxy-phenyl)-2,2-dimethyl-4-oxo-butanoic acid (50.00 mg; 0.17 mmol; 1.00 eq.) was dissolved in EtOH (1.00 mL) and hydrazine monohydrate (16 µL; 0.33 mmol; 2.00 eq.) was added. The reaction was stirred 3h30 under reflux and then concentrated to dryness. The residue was purified by chromatography (column SiO₂ 5g, flow rate 15ml/min, gradient from 85/15 Cyclohexane/EtOAc to 50/50) to give 3-(4-fluoro-3-methoxy-phenyl)-5,5-dimethyl-1,4-dihydropyridazin-6-one (19.0 mg; 46 %) as a white solid. 1H NMR (DMSO-d₆, 300 MHz): δ 10.88 (s, 1H); 7.51 (dd, J = 1.9 Hz, J = 8.7 Hz, 1H); 7.29 (m, 2H); 3.88 (s, 3H); 2.84 (s, 2H); 1.08 (s, 6H).

### Step C: 3-(4-fluoro-3-methoxy-phenyl)-5,5-dimethyl-4,6-dihydro-1H-pyridazine

LiAlH₄ (8.2 mg; 0.22 mmol; 3.00 eq.) was added to a solution of 3-(4-fluoro-3-methoxyphenyl)-5,5-dimethyl-1,4-dihydropyridazin-6-one (18.0 mg; 0.07 mol; 1.00 eq.) in THF (360 µL) in Q-Tube. The reaction was heated 1h under reflux and then cooled to rt, diluted with DCM, and washed with an aqueous saturated solution of Na₂SO₄ (3x).The organic layer was dried over MgSO₄, filtered and concentrated to dryness to give 3-(4-fluoro-3-methoxy-phenyl)-5,5-dimethyl-4,6-dihydro-1H-pyridazine (11.0 mg; 65 %) as a colorless oil, used in the next step without further purification. 1H NMR (CDCl₃, 300 MHz): δ 7.43 (dd, J = 1.5 Hz, J = 8.9 Hz, 1H); 7.02 (m, 2H); 3.92 (s, 3H); 2.82 (t, J = 1.2 Hz, 2H); 2.27 (t, J = 1.2 Hz, 2H); 1.05 (s, 6H).

### Step D: 3-[6-(4-fluoro-3-methoxy-phenyl)-4,4-dimethyl-3,5-dihydropyridazin-2-yl]-1,2-benzothiazole 1,1-dioxide

The compound was prepared using the same procedure as described in example 21 step B starting from 3-chloro-1,2-benzothiazole 1,1-dioxide (example 1, step A), (9.4 mg; 0.05 mmol; 1.00 eq.) and 3-(4-fluoro-3-methoxy-phenyl)-5,5-dimethyl-4,6-dihydro-1H-pyridazine (11.0 mg; 0.05 mmol; 1.00 eq.). The crude was purified by chromatography (column SiO₂ 5g, flow rate 15ml/min, gradient from 85/15 Cyclohexane/EtOAc to 50/50) to give 3-[6-(4-fluoro-3-methoxy-phenyl)-4,4-dimethyl-3,5-dihydropyridazin-2-yl]-1,2-benzothiazole 1,1-dioxide (13.0 mg; 64 %) as a pink solid. 1H NMR (CDCl₃, 500 MHz): δ 8.78 (dd, J = 1.1 Hz, J = 8.0 Hz, 1H); 7.92 (m, 1H); 7.73 (m, 1H); 7.65 (dd, J = 1.1 Hz, J = 8.0 Hz, 1H); 7.45 (dd, J = 2.1 Hz, J = 8.1 Hz, 1H); 7.25 (tdd, J = 2.2 Hz, J = 4.2 Hz, J = 8.5 Hz, 1H); 7.19 (dd, J = 8.5 Hz, J = 10.5 Hz, 1H); 3.97 (s, 3H); 3.90 (s, 2H); 2.56 (s, 2H); 1.16 (s, 6H).

### Example 111: 3-cyclobutyl-5-[(E)-[(1,1-dioxo-1,2-benzothiazol-3-yl)-methyl-hydrazono]methyl]-1-(methoxymethyl)benzimidazol-2-one

Sodium hydride (dispersion 60% in oil, 7.33 mg; 0.18 mmol; 1.50 eq.) was added to a solution of 3-cyclobutyl-5-[(E)-[(1,1-dioxo-1,2-benzothiazol-3-yl)-methyl-hydrazono]methyl]-1H-benzimidazol-2-one (example 28, step F), (50.0 mg; 0.12 mmol; 1.00 eq.) in DMF (1.00 mL). The mixture was stirred 5 minutes and chloromethyl methyl ether (10 µL; 0.13 mmol; 1.10 eq.) was added. The mixture was stirred 24h at rt and then quenched by addition of aqueous NH₄Cl sat. The resulting precipitate was filtered, washed with water (3x) and dried under vacuum at 50°C. The solid was then purified by LC/MS-Preparative (Column: Kinetex C18 21,20 x 150mm 5µm (phenomenex); Flow rate: 25 ml/min; Elution: H2O, 0.1% HCOOH / ACN, 0.1% HCOOH; Gradient: 10 to 100% ACN) to give 3-cyclobutyl-5-[(E)-[(1,1-dioxo-1,2-benzothiazol-3-yl)-methyl-hydrazono]methyl]-1-(methoxymethyl)benzimidazol-2-one (24.1 mg; 44 %) as a beige solid. ¹H NMR (DMSO-d₆, 400 MHz): δ 8.92 (dd, J = 0.9 Hz, J = 7.9 Hz, 1H); 8.57 (s, 1H); 8.11 (m, 1H) ; 7.91 (dt, J = 1.1 Hz, J = 7.3 Hz, 1H); 7.88 (d, J = 1.1 Hz, 1H); 7.86 (dt, J = 1.3 Hz, J = 7.9 Hz, 1H); 7.66 (dd, J = 1.4 Hz, J = 8.3 Hz, 1H) ; 7.43 (d, J = 8.1 Hz, 1H); 5.26 (s, 2H); 4.94 (quin, J = 8.9 Hz, 1H) ; 3.83 (s, 3H); 3.27 (s, 3H) ; 2.88 (m, 2H); 2.40 (m, 2H) ; 1.90 (m, 2H). mp: 162-180 °C.

### Example 112: 5-[(E)-[(1,1-dioxo-1,2-benzothiazol-3-yl)-methyl-hydrazono]methyl]-3-isopropyl-1-(methoxymethyl)benzimidazol-2-one

The compound was prepared using the same procedure as described in example 111 starting from 5-[(E)-[(1,1-dioxo-1,2-benzothiazol-3-yl)-methyl-hydrazono]methyl]-3-isopropyl-1H-benzimidazol-2-one (example 26, step E), (250.0 mg; 0.63 mmol; 1.00 eq.) and chloromethyl methyl ether (72 µL; 0.94 mmol; 1.50 eq.) giving 5-[(E)-[(1,1-dioxo-1,2-benzothiazol-3-yl)-methyl-hydrazono]methyl]-3-isopropyl-1-(methoxymethyl)benzimidazol-2-one (238.0 mg; 82 %) as a yellow solid. ¹H NMR (DMSO-d₆, 400 MHz): δ 8.91 (dd, J = 1.3 Hz, J = 7.0 Hz, 1H); 8.55 (s, 1H); 8.11 (dd, J = 1.5 Hz, J = 6.8 Hz, 1H); 7.89 (m, 2H) ; 7.82 (d, J = 0.9 Hz, 1H); 7.65 (dd, J = 1.2 Hz, J = 8.3 Hz, 1H); 7.43 (d, J = 8.1 Hz, 1H); 5.27 (s, 2H) ; 4.72 (sept., J = 7.0 Hz, 1H); 3.81 (s, 3H); 3.28 (s, 3H); 1.54 (d, J = 7.0 Hz, 6H). mp: 232°C.

### Example 113: [3-cyclobutyl-5-[(E)-[(1,1-dioxo-1,2-benzothiazol-3-yl)-methyl-hydrazono]methyl]-2-oxo-benzimidazol-1-yl]methyl 2,2-dimethylpropanoate

The compound was prepared using the same procedure as described in example 111 starting from 3-cyclobutyl-5-[(E)-[(1,1-dioxo-1,2-benzothiazol-3-yl)-methyl-hydrazono]methyl]-1H-benzimidazol-2-one (example 28, step F), (100.0 mg; 0.24 mmol; 1.00 eq.) and chloromethyl pivalate (43 µL; 0.29 mmol; 1.20 eq.) giving [3-cyclobutyl-5-[(E)-[(1,1-dioxo-1,2-benzothiazol-3-yl)-methyl-hydrazono]methyl]-2-oxo-benzimidazol-1-yl]methyl 2,2-dimethylpropanoate (51.2 mg; 40 %) as a pale yellow solid. ¹H NMR (DMSO-d₆, 300 MHz): δ 8.92 (m, 1H); 8.55 (s, 1H); 8.10 (m, 1H); 7.88 (m, 3H); 7.68 (dd, J = 1.2 Hz, J = 8.3 Hz, 1H); 7.46 (d, J = 8.3 Hz, 1H); 5.90 (s, 2H); 4.93 (quint., J = 8.9 Hz, 1H); 3.82 (s, 3H); 2.86 (m, 2H); 2.41 (m, 2H); 1.90 (m, 2H); 1.12 (s, 9H). mp: 245-265°C.

### Example 114: [3-cyclobutyl-5-[(E)-[(1,1-dioxo-1,2-benzothiazol-3-yl)-methyl-hydrazono]methyl]-2-oxo-benzimidazol-1-yl]methylbutanoate

The compound was prepared using the same procedure as described in example 111 starting from 3-cyclobutyl-5-[(E)-[(1,1-dioxo-1,2-benzothiazol-3-yl)-methyl-hydrazono]methyl]-1H-benzimidazol-2-one (example 28, step F), (100.0 mg; 0.24 mmol; 1.00 eq.) and chloromethyl butyrate (37 µL; 0.29 mmol; 1.20 eq.) giving [3-cyclobutyl-5-[(E)-[(1,1-dioxo-1,2-benzothiazol-3-yl)-methyl-hydrazono]methyl]-2-oxo-benzimidazol-1-yl]methyl butanoate (10.5 mg; 8 %) as a white solid. ¹H NMR (DMSO-d₆, 300 MHz): δ 8.92 (m, 1H); 8.56 (s, 1H); 8.11 (m, 1H); 7.89 (m, 3H); 7.69 (dd, J = 1.3 Hz, J = 8.3 Hz, 1H); 7.48 (d, J = 8.3 Hz, 1H); 5.91 (s, 2H); 4.93 (m, 1H); 3.82 (s, 3H); 2.86 (m, 2H); 2.41 (m, 2H); 2.32 (t, J = 7.3 Hz, 2H); 1.90 (m, 2H); 1.53 (sext., J = 7.3 Hz, 2H); 0.85 (t, J = 7.3 Hz, 3H).

### Example 115: 3-cyclobutyl-5-[(E)-[(1,1-dioxo-1,2-benzothiazol-3-yl)-methyl-hydrazono]methyl]-1-(2-methoxyethoxymethyl)benzimidazol-2-one

The compound was prepared using the same procedure as described in example 111 starting from 3-cyclobutyl-5-[(E)-[(1,1-dioxo-1,2-benzothiazol-3-yl)-methyl-hydrazono]methyl]-1H-benzimidazol-2-one (example 28, step F), (100.0 mg; 0.24 mmol; 1.00 eq.) and 1-(chloromethoxy)-2-methoxy-ethane (34 µL; 0.29 mmol; 1.20 eq.) giving 3-cyclobutyl-5-[(E)-[(1,1-dioxo-1,2-benzothiazol-3-yl)-methyl-hydrazono] methyl]-1-(2-methoxyethoxymethyl)benzimidazol-2-one (72.2 mg; 59 %) as a yellow solid. ¹H NMR (DMSO-d₆, 300 MHz): δ 8.93 (d, J = 7.3 Hz, 1H); 8.56 (s, 1H); 8.11 (m, 1H); 7.88 (m, 3H); 7.66 (dd, J = 1.2 Hz, J = 8.3 Hz, 1H); 7.43 (d, J = 8.3 Hz, 1H); 5.32 (s, 2H); 4.94 (t, J = 8.9 Hz, 1H); 3.82 (s, 3H); 3.61 (dd, J = 3.7 Hz, J = 5.7 Hz, 2H); 3.42 (m, 2H); 3.19 (s, 3H); 2.87 (dt, J = 2.3, J = 9.7 Hz, 2H); 2.36 (m, 2H); 1.91 (m, 2H). mp: 161-172°C.

### Example 116: 3-cyclobutyl-5-[(E)-[(1,1-dioxo-1,2-benzothiazol-3-yl)-methyl-hydrazono]methyl]-1-(ethoxymethyl)benzimidazol-2-one

The compound was prepared using the same procedure as described in example 111 starting from 3-cyclobutyl-5-[(E)-[(1,1-dioxo-1,2-benzothiazol-3-yl)-methyl-hydrazono]methyl]-1H-benzimidazol-2-one (example 28, step F), (100.0 mg; 0.24 mmol; 1.00 eq.) and chloromethyl ethyl ether (28 µL; 0.29 mmol; 1.20 eq.) giving 3-cyclobutyl-5-[(E)-[(1,1-dioxo-1,2-benzothiazol-3-yl)-methyl-hydrazono]methyl]-1-(ethoxymethyl)benzimidazol-2-one (60.5 mg; 53 %) as a yellow solid. ¹H NMR (DMSO-d₆, 300 MHz): δ 8.92 (m, 1H); 8.56 (s, 1H); 8.13 (m, 1H); 7.88 (m, 3H); 7.70 (m, 1H); 7.43 (d, J = 8.3 Hz, 1H); 5.29 (s, 2H); 4.93 (m, 1H); 3.82 (s, 3H); 3.52 (q, J = 6.9 Hz, 2H); 2.86 (m, 2H); 2.39 (m, 2H); 1.90 (m, 2H); 1.10 (t, J = 7.0 Hz, 3H). mp: 119-142°C.

### Example 117: [3-cyclobutyl-5-[(E)-[(1,1-dioxo-1,2-benzothiazol-3-yl)-methyl-hydrazono]methyl]-2-oxo-benzimidazol-1-yl]methyl N,N-dimethylcarbamate

The compound was prepared using the same procedure as described in example 52 starting from 3-cyclobutyl-5-[(E)-[(1,1-dioxo-1,2-benzothiazol-3-yl)-methyl-hydrazono]methyl]-1H-benzimidazol-2-one (example 28, step F), (100.0 mg; 0.24 mmol; 1.00 eq.) and (N,N-dimethyl)carbamoyloxymethyl chloride (40.3 mg; 0.29 mmol; 1.20 eq.) giving [3-cyclobutyl-5-[(E)-[(1,1-dioxo-1,2-benzothiazol-3-yl)-methyl-hydrazono]methyl]-2-oxo-benzimidazol-1-yl]methyl N,N-dimethylcarbamate (40.0 mg; 30 %) as a beige solid. ¹H NMR (DMSO-d₆, 500 MHz): δ 8.93 (d, J = 7.4 Hz, 1H); 8.56 (s, 1H) ; 8.11 (d, J = 6.9 Hz, 1H); 7.89 (m, 3H); 7.69 (dd, J = 1.0 Hz, J = 8.1 Hz, 1H); 7.55 (d, J = 8.3 Hz, 1H); 5.86 (s, 2H); 4.93 (quint., J = 8.9 Hz, 1H); 3.82 (s, 3H); 2.87 (m, 2H); 2.83 (s, 3H); 2.81 (s, 3H); 2.39 (m, 2H); 1.90 (m, 2H). mp: 265°C.

### Example 118: N-methyl-N-[(E)-(2-methylthiazol-5-yl)methyleneamino]-1,1-dioxo-1,2-benzothiazol-3-amine

The compound was prepared using the same procedure as described in example 1 step C starting from 1-(1,1-dioxo-1,2-benzothiazol-3-yl)-1-methyl-hydrazine (example 1, step B), (60.0 mg; 0.28 mmol; 1.00 eq.) and 2-methyl-1,3-thiazole-5-carbaldehyde (39.7 mg; 0.31 mmol; 1.10 eq.) giving N-methyl-N-[(E)-(2-methylthiazol-5-yl)methyleneamino]-1,1-dioxo-1,2-benzothiazol-3-amine (54.0 mg; 58 %) as an orange solid. 1H NMR (DMSO-d₆, 400 MHz): δ 8.72 (m, 1H) ; 8.69 (s, 1H) ; 8.12 (s, 1H) ; 8.09 (m, 1H) ; 7.91 (m, 2H) ; 3.76 (s, 3H); 2.76 (s, 3H). mp: 248 °C.

### Example 119: 6-diethoxyphosphoryl-N-[(E)-(4-fluoro-3-methoxy-phenyl)methylene amino]-N-isobutyl-1,1-dioxo-1,2-benzothiazol-3-amine

6-bromo-N-[(E)-(4-fluoro-3-methoxy-phenyl)methyleneamino]-N-isobutyl-1,1-dioxo-1,2-benzothiazol-3-amine (example 63, step A), (1.00 g; 2.14 mmol; 1.00 eq.) was suspended into toluene (20.00 mL). Triethylamine (297 µL; 2.14 mmol; 1.00 eq.), diethyl phosphite (353.9 mg; 2.56 mmol; 1.20 eq.) and 1,1'-bis(diphenylphosphino)ferrocene-palladium(II)dichloride dichloromethane complex (87.2 mg; 0.11 mmol; 0.05 eq.) were added and the mixture was stirred at 90°C overnight. Toluene was removed and the residue was taken up into DCM, washed twice with water, dried over MgSO₄, filtered and concentrated. The residue was purified by chromatography (column SiO₂ 50g, flow rate 50ml/min, gradient from 70/30 Cyclohexane / EtOAc to 100% EtOAc) to give 6-diethoxyphosphoryl-N-[(E)-(4-fluoro-3-methoxy-phenyl)methyleneamino]-N-isobutyl-1,1-dioxo-1,2-benzothiazol-3-amine (570.0 mg; 51 %) as an orange solid. 1H NMR (DMSO-d6, 500 MHz): δ 8.97 (dd, J = 2.5 Hz, J = 8.2 Hz, 1H); 8.57 (s, 1H) ; 8.27 (m, 2H) ; 7.67 (dd, J = 1.8 Hz, J = 8.4 Hz, 1H); 7.54 (ddd, J = 1.9 Hz, J = 4.4 Hz, J = 8.4 Hz, 1H); 7.40 (dd, J = 8.4 Hz, J = 11.1 Hz, 1H); 4.29 (d, J = 7.7 Hz, 2H); 4.12 (m, 4H); 3.98 (s, 3H) ; 2.28 (m, J = 6.9 Hz, 1H); 1.27 (t, J = 7.0 Hz, 6H); 0.99 (d, J = 6.6 Hz, 6H). mp: 85 °C.

### Assays

### Effect of compounds on the Cell-Based TEAD-GAL4 Transactivation Assay

To identify inhibitors of YAP-TEAD interaction, a transient transactivation assays was carried out into HEK293 cell line (HEK293 GripTite^{™} 293 MSR (Invitrogen R795-07)) using plasmids containing the full length TEAD1 sequences, the full length YAP mutant (S127A, S397A) and a luciferase gene reporter. All expression constructs were performed into pSG5 backbone plasmid in which the SV40 promoter was replaced by the CMV promoter. The TEAD1 construct was prepared by cloning the full length human TEAD1 cDNA in fusion with the Gal4 DNA Binding Domain (AA 1-148) into pSG5_CMV to create the TEAD1(FL)_hum_pSG5Gal4_CMV. The full length YAP mutant (S127A, S397A) was also cloned into pSG5_CMV to create the YAP(FL)_hum_pSG5_mutS127A_S397A_CMV. The reporter plasmid GAL4(5xRE)_TK(-105/+56)_pGL3-Basic contains 5 copies of the GAL4 responsive element (5'-TCGGAGGACAGTACTCC-3') upstream of the thymidine kinase (TK) promoter (-105/+56) inserted in a pGL3-Basic vector. To evaluate the selectivity of our compounds in blocking the YAP-TEAD interaction, a counter-screening protein-protein interaction transactivation assays was also established. Briefly, two other expression plasmids, the pBD-P53 which express the P53 (AA 72-390) in fusion with Gal4 and the pAD-SV40T which express the SV40 large T antigen (AA 84-708,AgT), were created. HEK293 cells were seeded at 5 X 10⁴ cells/well in 96 well plates (assay plates) in DMEM medium containing 4.5 g/L D-glucose, 10% high inactivated (HI) fetal bovine serum 1% Glutamax, 1% non-essential amino acid and 1% sodium pyruvate and 0.5% penicillin/streptomycin 37°C in a humidified atmosphere of 8% CO2. After 24 h, transfections are performed using Jet-PEI as transfectant (101B-010 Polyplus Transfection), according to the instructions of the manufacturer (N/P = 10) and a ratio YAP/TEAD=10 (per well: 50 ng of YAP(FL), 5 ng of TEAD(FL) and 50 ng of reporter plasmid). Six hours after cell transfection, the plates ('assay plate') were washed with 100 µl PBS per well and cell were treated with compounds in semi-log from 0.1 µM to 30µM or DMSO 0.5% as control in DMEM 4,5g/L D-Glucose without phenol red, 0,3% BSA, 1% glutamax, 1% NEAA, 1% sodium pyruvate. Twenty-four hours later, the luciferase activity was determined after addition of 100 µl of Steady Glo^{™} luciferase assay system (Promega E2550) according to the manufacturer's directions. Luciferase activity in cell extracts is measured by reading luminescence using the Envision device.

The cellular response was determined by fitting the concentration response curves using a 3-parameter curve fit equation and determining the concentration that inhibited the luciferase activity by 50 %.

**Table 1: TEAD-GAL4 Transactivation activity**

| **EX** | **IC50 (µM)** | **Max Act. (%)** | **EX** | **IC50 (µM)** | **Max Act. (%)** |
|---|---|---|---|---|---|
| Ex 1 | | 54 | Ex 16 | | 55 |
| Ex 2 | 1.11 | 55 | Ex 17 | 2.75 | 55 |
| Ex 3 | | 57 | Ex 18 | | 58 |
| Ex 4 | 11.88 | 59 | Ex 19 | | 52 |
| Ex 5 | | 71 | Ex 20 | | 53 |
| Ex 6 | 1.07 | 51 | Ex 21 | | 70 |
| Ex 7 | | 56 | Ex 22 | | 70 |
| Ex 8 | 5.19 | 49 | Ex 23 | | 83 |
| Ex 9 | 3.94 | 55 | Ex 24 | | 67 |
| Ex 10 | | 52 | Ex 25 | | 57 |
| Ex 11 | 2.85 | 67 | Ex 26 | 0.62 | 60 |
| Ex 12 | 1.56 | 54 | Ex 27 | 2.92 | 62 |
| Ex 13 | 3.35 | 76 | Ex 28 | 3.06 | 57 |
| Ex 14 | | 58 | Ex 29 | | 52 |
| Ex 15 | | 65 | Ex 30 | | 62 |
| Ex 31 | | 54 | Ex 67 | | 58 |
| Ex 32 | | 71 | Ex 68 | 4.50 | 86 |
| Ex 33 | | 66 | Ex 69 | 1.04 | 96 |
| Ex 34 | | 60 | Ex 70 | 1.58 | 69 |
| Ex 35 | | 69 | Ex 71 | | 80 |
| Ex 36 | | 57 | Ex 72 | | 91 |
| Ex 37 | | 58 | Ex 73 | | 59 |
| Ex 38 | | 61 | Ex 74 | | 57 |
| Ex 39 | | 51 | Ex 75 | | 71 |
| Ex 40 | 5.33 | 45 | Ex 76 | | 53 |
| Ex 41 | | 58 | Ex 77 | | 64 |
| Ex 42 | | 51 | Ex 78 | | 66 |
| Ex 43 | 2.78 | 58 | Ex 79 | | 52 |
| Ex 44 | | 67 | Ex 80 | 1.07 | 95 |
| Ex 45 | | 69 | Ex 81 | | 88 |
| Ex 46 | | 68 | Ex 82 | | 62 |
| Ex 47 | 3.40 | 65 | Ex 83 | | 69 |
| Ex 48 | | 67 | Ex 84 | 1.67 | 79 |
| Ex 49 | 3.11 | 71 | Ex 85 | | 57 |
| Ex 50 | 3.03 | 86 | Ex 86 | 1.31 | 72 |
| Ex 51 | | 83 | Ex 87 | | 69 |
| Ex 52 | | 71 | Ex 88 | | 79 |
| Ex 53 | | 63 | Ex 89 | | 59 |
| Ex 54 | | 53 | Ex 90 | | 72 |
| Ex 55 | | 53 | Ex 91 | | 80 |
| Ex 56 | 2.31 | 58 | Ex 92 | 0.86 | 95 |
| Ex 57 | 0.53 | 54 | Ex 93 | | 59 |
| Ex 58 | 3.42 | 59 | Ex 94 | 3.31 | 87 |
| Ex 59 | 3.55 | 89 | Ex 95 | 1.35 | 78 |
| Ex 60 | | 70 | Ex 96 | 10.44 | 81 |
| Ex 61 | | 63 | Ex 97 | 2.77 | 53 |
| Ex 62 | 1.98 | 93 | Ex 98 | 3.92 | 69 |
| Ex 63 | 1.08 | 95 | Ex 99 | 4.14 | 61 |
| Ex 64 | | 84 | Ex 100 | | 57 |
| Ex 65 | 4.04 | 77 | Ex 101 | 5.29 | 90 |
| Ex 66 | | 61 | Ex 102 | 3.01 | 91 |
| Ex 103 | | 69 | Ex 112 | | 59 |
| Ex 104 | | 72 | Ex 113 | 4.00 | 46 |
| Ex 105 | 4.94 | 62 | Ex 114 | 4.95 | 74 |
| Ex 106 | 0.94 | 91 | Ex 115 | | 65 |
| Ex 107 | 2.14 | 53 | Ex 116 | 5.03 | 49 |
| Ex 108 | | 70 | Ex 117 | | 60 |
| Ex 109 | 0.70 | 90 | Ex 118 | 3.97 | 55 |
| Ex 110 | 3.23 | 65 | Ex 119 | | 60 |
| Ex 111 | 8.57 | 68 | | | |

### Inhibition of malignant mesothelioma tumor cell growth

The tumor cell growth inhibitory activity of the YAP-TEAD interaction inhibitors was evaluated in NCI-H2052 mesothelioma cell line harboring a NF2 mutation. This cell line was selected based on its mutational status and the ability of a siRNA directed against YAP, TAZ or TEAD1-4 to inhibit cell proliferation. The nuclear localization of YAP at confluence was also taken into account. Based on these observations we were able to classify several "YAP dependent cells" where YAP is clearly nuclear and in which a cell growth inhibition is observed by using siRNA (NCI-H2052, SKOV-3, ACHN, A549) and the "YAP independent cells" where YAP is preferentially located in the cytoplasm and in which no inhibition of cell growth is observed by using a siRNA (SW620, Met-5a). 10000 cells/well were plated in a 96-well black plate with clear flat bottom TC-Treated Imaging plate (Falcon #353219) in regular medium (as suggested from ATCC for each cell line) with serum, which was replaced the day after with starvation medium containing 1 % serum. After one day growth in the starvation medium, cells were incubated with compounds. The starting concentration was 30 µM and serial dilutions in DMSO and medium were performed until 0.1µM to achieve a final DMSO concentration of 0.5%. The cells were then allowed to grow for 3 days, and then, EdU (Invitrogen, Molecular Probe) was added in each well at a final concentration of 10 µM and the cells were returned to the incubator for additional 24h. Starvation medium was removed and 100 µl of PFA 4% containing Hoechst dye was adding in each well to fix the cells. Plates were then incubated at rt for 15 minutes, washed twice with PBS and the cells were permeabilized by adding 100 µl per well of triton-100 containing 0.3% BSA. After 20 minutes cells were washed with PBS and the EdU detection was performed according to the instructions of the manufacturer. Image acquisition was performed using the ImageXpress Micro and analysed using the MetaXpress software (Molecular Device).

Results were expressed as a percent of inhibition (%) of the cell proliferation values obtained with 0.5 % DMSO treatment alone. The cellular response was determined by fitting the concentration response curves using a 3 parameter curve fit equation and determining the concentration that inhibited cell growth between 50% and 100%. Compounds (Ex 28, 50, 65 and 68) inhibited NCI-H2052 mesothelioma cell proliferation (NF2 mutated cell line; Fig 1) without showing any effect in the Met5A cell line, a "YAP-independent cell line", Fig 1. In addition ours compounds inhibited other mesothelioma and non small cell lung cancer cell growth without affecting proliferation of the "YAP independent" cell line, Met-5a (data not shown).

### References

- Aebi et al., WO 2016/055394
- Aiguade et al., WO 2013/068554
- Aranyos et al., JACS, 1999, 121(18), 4369-4378
- Avruch et a/., Cell Cycle 2012, 1090-1096
- Badouel et al., Curr Opin Cell Biol 2009, 21, 837-43
- Baldwin et al., WO 2008/156817
- Bandarage et al., Bioorg. Med. Chem.Lett., 2009, 19(17), 5191-5194
- Bianchi et al., Nat Genet 1994, 6, 185-192
- Bianchi et al., Natl Acad. Sci. USA, 1995, 92, 10854-10858
- Blanchet et al., Journal of Organic Chemistry, 72(9), 3199-3206; 2007
- Blaquiere et a/., WO 2015/025025
- Bott et al., Nat Genet 2011, 43, 668-672
- Burns et al., WO 2009/029998
- Carbone et al., Clin Cancer Res 2012, 18, 598-604
- Chad et al., Cancer Res 2010, 70, 8517-25
- Chen et a/., JACS, 2014, 136(49), 17337-17342
- Dastbaravardeh et al., Org. Lett., 2012, 14(7), 1930-1933
- De Christofaro et al., Eur J Cancer 2011, 926-933
- Deguen et a/., Int J Cancer 1998, 77, 554-560
- Deniau et a/., Tetrahedron, 2001, 57(13), 2581-2588
- Differding et a/., Helvetica Chimica Acta, 72(6), 1248-52; 1989
- Dong et a/., Cell, 2007, 130: 1120-1133
- Dutta et al., U.S. Pat. Appl. Publ., 20140309427
- Dzhevakov et al., Adv. Synth. Catal., 2016, 358(6), 977-983
- Ebright et al., WO 2013/192352
- Forbe et al., Nucleic Acids Res 2011, 39, D945-950
- Fuchida et al., Bul. Chem. Soc. Jp., 2015, 88(6) 784-791
- Garcia et a/., Chem. Commun. (Cambridge, United Kingdom), 2016, 52(58), 9059-9062
- Gouault et al., Journal of Pharmacy and Pharmacology, 2001, 53(7), 981-985
- Goulet. et al., WO 00/04013
- Green et al., Protective Group in Organic Synthesis, Wiley, third edition
- Haffner et a/., Bioorganic & Medicinal Chemistry Letters, 20(23), 6989-6992; 2010
- Haffner et al., Bioorganic & Medicinal Chemistry Letters, 20(23), 6983-6988; 2010
- Harvey et al., Nat Rev Cancer, 2013,13, 246-257
- Hong W et a/., Cell Dev Biol 2012, 23, 785-793
- Huang et al., Cell 2005, 122, 421-34
- Hudlicky Reductions in Organic Chemistry ACS monograph 188 second edition
- Keglevich et al., Heteroatom Chem., 2012, 23(6), 574-582
- Kim et al., Bioorg. Med. Chem. Lett., 2008, 18(14), 4181-4185
- Krasavin, M. et al Syn. Commun., 2005, 35(19), 2587-2595
- Kurian et al., Bioorganic & Medicinal Chemistry Letters, 24(17), 4176-4180; 2014
- Lei et al., Mol Cell Biol 2008, 28, 2426-36)
- Li et al., Org. Proc. Res. Dev., 2016, 20(8), 1489-1499
- Liu et al., Molecules, 2012, 17, 4545-4559
- Loghmani-Khouzani et al., Journal of Chemical Research, Synopses, (2), 80-81; 2001
- Luehr et al., Bioorg. Med. Chem.,2010, 18(4), 1388-1395
- Meerpoel et al., WO 2012/084804
- Meyers et al., Bioorg. Med. Chem. Lett., 2010, 20(5), 1543-1547
- Mukhina et al., Angew. Chem., Inter. Ed., 2015, 54(39), 11516-11520
- Murakami et al., Cancer Res 2011, 71, 873-883
- Murphy et al., Org. Lett., 2007, 9(5), 757-760
- Naidu et al. WO 2014/028384
- Omura et a/., Tetrahedron, 1978, 34 (11), 1651-1660
- Park et al., Environ Health Perspect 2011, 119, 514-518
- Perez et al., WO 2010/100139
- Raw et al., Tetrahedron Letters, 52(50), 6775-6778; 2011
- Ruttledge et al., Nat Genet 1994, 6, 180-184
- Sakai et al. Chemistry Letters, 2015, 44(11), 1503-1505
- Sam et al., JACS, 1972, 94, 4024-4025
- Schneider et al., Organic Letters, 13(14), 3588-3591; 2011
- Schumacher et al., WO 2007/038367
- Sekido et al., Cancer Res 1995, 55, 1227
- Sekido et al., Pathol Int 2011, 61, 331-344
- Steinhardt et al., Hum. Pathol 2008, 39, 1582-9
- Subramanyam et al., U.S. 5,306,818
- Sun et al., 2008, JOC, 73(18), 7361-7364
- Timmer et al., Chem. Commun. (Cambridge, United Kingdom), 2016, 52(83), 12326-12329
- Tohma et al., Adv. Syn. Catal.,2004, 346, 111-124
- Traynelis et al., WO 2014/025942
- Tumaneng K et al., Curr Biol, 2013, 22, R368-379
- Tzchucke et al., Org. Lett., 2007, 9(5), 761-764
- Wang L et al., Tumour Biol 2014, 14, 463-468
- Wang et al., Cancer Sci 2010, 101, 1279-85
- Winton. et al., Journal of Heterocyclic Chemistry, 1984, 21(3), 889-91
- Xie etal., Eur. J. Med. Chem., 2016, 115, 132-140
- Yokoyama et al., Carcinogenesis 2008, 29, 2139-2146
- Yu Fx et al., Genes Dev 2013, 27, 355-371
- Zeng et al., Cancer Cell 2008, 13, 188-192
- Zhang et al., Bioorg. Med; Chem. Lett., 2008, 18(23), 6067-6070
- Zhang et al., J. Med. Chem., 2009, 52(18) 5703-5711
- Zhao et al., Cancer Res 2009, 69, 1089-98
- Zhao et al., Gens Dev 2008, 22, 1962-71
- Zhao et al. Genes Dev 2007, 21: 2747-2761
- Zhou et al., Oncogene 2011, 30, 2181-86
- Zil'berman et al., Russ. Chem. Rev., 1984, 53, 900-912

## Claims

1. A compound of formula (I): 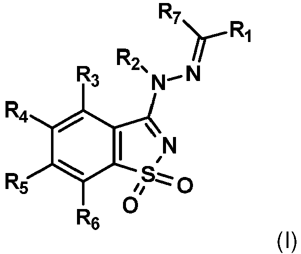 or a pharmaceutically acceptable salt thereof, wherein:
R₁ is an aryl group optionally substituted with one or more substituents independently selected from halo, hydroxyl, alkoxyl, alkylthio, OCF₃, alkyl, cycloalkyl, alkyl-R₈, -O-alkyl-R₈, -NR₉R₁₀, -CO-NR₉R₁₀, oxo, thioxo and phenyl;
R₂ is an alkyl optionally substituted by one or two R₈ or -aryl-R₈;
R₃, R₄, R₅, R₆ are each independently H, halo, hydroxyl, alkyl, alkoxyl, alkoxyl substituted with boronic acid or a boronic acid derivative, hydroxycarbonyl, aryl, -O-aryl, boronic acid or a boronic acid derivative, phosphonic acid or phosphonic acid derivative, alkyl-R₈, -O-alkyl-R₈, -COOR₁₁, -NO₂, -NR₉R₁₀ or -CO-NR₉R₁₀;
R₇ is H or alkyl; or
R₂ and R₇ are bound together to form a 5 or 6-membered heterocycle;
R₈ is halo, hydroxyl, alkoxyl, -NR₉R₁₀, -CO-X-R₁₁, -CN, -CF₃, aryl, cycloalkyl, -O-alkyl-alkoxyl, -O-CO-alkyl, -CO-NR₉R₁₀ or -O-CO-NR₉R₁₀;
R₉ and R₁₀ are each independently H, alkyl or -CO-alkyl, or form together with the nitrogen atom a 3 to 6-membered cyclic group;
X is -O- or -NR₁₂-;
R₁₁ is H or alkyl;
R₁₂ is H, alkyl or hydroxyalkyl;
and when X is -NR₁₂-, R₁₁ and R₁₂ can be bound together to form a 4- to 7-membered cyclic group;
wherein aryl is an aromatic or partially unsaturated (hetero)cycle comprising 1, 2 or 3 rings, which can be fused;
with the proviso that R₁ is not 3,4-dimethoxyphenyl or 3-methoxy-4-hydroxyphenyl, and
with the proviso that R₁ is a substituted aryl group when R₂ and R₇ are bound together to form a 5 or 6-member heterocycle and R₃, R₄, R₅ and R₆ are each H.

2. The compound of claim 1, or a pharmaceutically acceptable salt thereof, wherein R₁ represents: 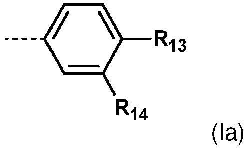 and R₁₃ and R₁₄ are each independently selected from H, halo, hydroxyl, alkoxyl, alkylthio, -OCF₃, alkyl, cycloalkyl, alkyl-R₈, -O-alkyl-R₈, -NR₉R₁₀ and -CO-NR₉R₁₀, with the proviso that when R₁₄ is methoxyl, then R₁₃ is not hydroxyl or methoxyl.

3. The compound of claim 2, selected from:
- N-[(E)-(4-fluorophenyl)methyleneamino]-N-methyl-1,1-dioxo-1,2-benzothiazol-3-amine;
- 2-[3-[(E)-[(1,1-dioxo-1,2-benzothiazol-3-yl)-methyl-hydrazono]methyl]phenoxy] ethanol;
- N-methyl-N-[(E)-(4-morpholinophenyl)methyleneamino]-1,1-dioxo-1,2-benzothiazol-3-amine;
- N-[(E)-[3-(dimethylamino)phenyl]methyleneamino]-N-methyl-1,1-dioxo-1,2-benzothiazol-3-amine;
- N-[(E)-(4-fluoro-3-methoxy-phenyl)methyleneamino]-1,1-dioxo-N-[2-(2-pyridyl)ethyl]-1,2-benzothiazol-3-amine;
- methyl 2-[3-[(E)-[(1,1-dioxo-1,2-benzothiazol-3-yl)-methyl-hydrazono] methyl]phenyl] acetate;
- N-[(E)-(3-ethoxy-4-fluoro-phenyl)methyleneamino]-N-methyl-1,1-dioxo-1,2-benzothiazol-3-amine;
- N-[(E)-(4-fluoro-3-propoxy-phenyl)methyleneamino]-N-methyl-1,1-dioxo-1,2-benzothiazol-3-amine;
- N-[(E)-(4-fluoro-3-isopropoxy-phenyl)methyleneamino]-N-methyl-1,1-dioxo-1,2-benzothiazol-3-amine;
- 2-[5-[{E)-[(1,1-dioxo-1,2-benzothiazol-3-yl)-methyl-hydrazono]methyl]-2-fluoro-phenoxy]ethanol;
- N-[(E)-[4-fluoro-3-(2-methoxyethoxy)phenyl]methyleneamino]-N-methyl-1,1-dioxo-1,2-benzothiazol-3-amine;
- N-[(E)-[3-fluoro-5-methoxy-4-(2-methoxyethoxy)phenyl]methyleneamino]-N-methyl-1,1-dioxo-1,2-benzothiazol-3-amine;
- 5-[(E)-[(1,1-dioxo-1,2-benzothiazol-3-yl)-methyl-hydrazono]methyl]-2-fluoro-benzamide;
- 2-(dimethylamino)-5-[(E)-[(1,1-dioxo-1,2-benzothiazol-3-yl)-methyl-hydrazono]methyl] benzamide;
- [4-[(E)-[(1,1-dioxo-1,2-benzothiazol-3-yl)-methyl-hydrazono]methyl]-2-methoxyphenyl]methanol;
- 2-[4-[(E)-[(1,1-dioxo-1,2-benzothiazol-3-yl)-methyl-hydrazono]methyl]-2-methoxy-phenyl]-N-methyl-acetamide;
- N-[(E)-[4-(2-methoxyethoxy)phenyl]methyleneamino]-N-methyl-1,1-dioxo-1,2-benzothiazol-3-amine;
- N-[(E)-[3-(2-methoxyethoxy)phenyl]methyleneamino]-N-methyl-1,1-dioxo-1,2-benzothiazol-3-amine;
- N-ethyl-N-[(E)-(4-fluoro-3-methoxy-phenyl)methyleneamino]-1,1-dioxo-1,2-benzothiazol-3-amine;
- N-[(E)-(4-fluoro-3-methoxy-phenyl)methyleneamino]-N-isopropyl-1,1-dioxo-1,2-benzothiazol-3-amine;
- N-[(E)-(4-fluoro-3-methoxy-phenyl)methyleneamino]-N-isobutyl-1,1-dioxo-1,2-benzothiazol-3-amine;
- 2-[(1,1-dioxo-1,2-benzothiazol-3-yl)-[(E)-(4-fluoro-3-methoxy-phenyl) methyleneamino]amino]ethanol;
- N-[(E)-(4-fluoro-3-methoxy-phenyl)methyleneamino]-N-(2-methoxyethyl)-1,1-dioxo-1,2-benzothiazol-3-amine;
- N-(2-ethoxyethyl)-N-[(E)-(4-fluoro-3-methoxy-phenyl)methyleneamino]-1,1-dioxo-1,2-benzothiazol-3-amine;
- 3-[(1,1-dioxo-1,2-benzothiazol-3-yl)-[(E)-(4-fluoro-3-methoxy-phenyl) methyleneamino]amino]propan-1-ol;
- 2-(chloromethyl)-3-[(1,1-dioxo-1,2-benzothiazol-3-yl)-[(E)-(4-fluoro-3-methoxyphenyl)methyleneamino]amino]propan-1-ol;
- N-[(E)-(4-fluoro-3-methoxy-phenyl)methyleneamino]-1,1-dioxo-N-(2-phenylethyl)-1,2-benzothiazol-3-amine;
- N-[(E)-(4-fluoro-3-methoxy-phenyl)methyleneamino]-N-methyl-5-nitro-1,1-dioxo-1,2-benzothiazol-3-amine;
- N-[3-[[(E)-(4-fluoro-3-methoxy-phenyl)methyleneamino]-methyl-amino]-1,1-dioxo-1,2-benzothiazol-5-yl]acetamide;
- N3-[(E)-(4-fluoro-3-methoxy-phenyl)methyleneamino]-N3-methyl-1,1-dioxo-1,2-benzothiazole-3,6-diamine;
- 3-[[(E)-(4-fluoro-3-methoxy-phenyl)methyleneamino]-methyl-amino]-1,1-dioxo-1,2-benzothiazol-6-yl]boronic acid;
- 3-[[(E)-(4-fluoro-3-methoxy-phenyl)methyleneamino]-methyl-amino]-1,1-dioxo-1,2-benzothiazol-6-ol;
- 6-benzyloxy-N-[(E)-(4-fluoro-3-methoxy-phenyl)methyleneamino]-N-methyl-1,1-dioxo-1,2-benzothiazol-3-amine;
- 5-ethoxy-N-[(E)-(4-fluoro-3-methoxy-phenyl)methyleneamino]-N-methyl-1,1-dioxo-1,2-benzothiazol-3-amine;
- N-[(E)-(4-fluoro-3-methoxy-phenyl)methyleneamino]-5-isopropoxy-N-methyl-1,1-dioxo-1,2-benzothiazol-3-amine;
- N-[(E)-(4-fluoro-3-methoxy-phenyl)methyleneamino]-N-methyl-1,1-dioxo-5-phenoxy-1,2-benzothiazol-3-amine;
- N-[(E)-(4-fluoro-3-methoxy-phenyl)methyleneamino]-5-(2-methoxyethoxy)-N-methyl-1,1-dioxo-1,2-benzothiazol-3-amine;
- 5-[2-(dimethylamino)ethoxy]-N-[(E)-(4-fluoro-3-methoxy-phenyl)methyleneamino]-N-methyl-1,1-dioxo-1,2-benzothiazol-3-amine;
- 2-[[3-[[(E)-(4-fluoro-3-methoxy-phenyl)methyleneamino]-methyl-amino]-1,1-dioxo-1,2-benzothiazol-5-yl]oxy]-N,N-dimethyl-acetamide;
- 6-(5,5-dimethyl-1,3,2-dioxaborinan-2-yl)-N-[(E)-(4-fluoro-3-methoxy-phenyl) methyleneamino]-N-methyl-1,1-dioxo-1,2-benzothiazol-3-amine;
- 6-diethoxyphosphoryl-N-[(E)-(4-fluoro-3-methoxy-phenyl)methyleneamino]-N-methyl-1,1-dioxo-1,2-benzothiazol-3-amine;
- [3-[ethyl-[(E)-(4-fluoro-3-methoxy-phenyl)methyleneamino]amino]-1,1-dioxo-1,2-benzothiazol-6-yl]boronic acid;
- [3-[[(E)-(4-fluoro-3-methoxy-phenyl)methyleneamino]-isobutyl-amino]-1,1-dioxo-1,2-benzothiazol-6-yl]boronic acid;
- [3-[[(E)-(4-fluorophenyl)methyleneamino]-methyl-amino]-1,1-dioxo-1,2-benzothiazol-6-yl]boronic acid;
- 2-[3-[[(E)-(4-fluoro-3-methoxy-phenyl)methyleneamino]-methyl-amino]-1,1-dioxo-1,2-benzothiazol-6-yl]-6-methyl-1,3,6,2-dioxazaborocane-4,8-dione;
- 2-[3-[[(E)-(4-fluoro-3-methoxy-phenyl)methyleneamino]-isobutyl-amino]-1,1-dioxo-1,2-benzothiazol-6-yl]-6-methyl-1,3,6,2-dioxazaborocane-4,8-dione;
- [3-[[(E)-(4-fluoro-3-methoxy-phenyl)methyleneamino]-(2-methoxyethyl)amino]-1,1-dioxo-1,2-benzothiazol-6-yl]boronic acid
- N-[(E)-[4-fluoro-3-(trifluoromethoxy)phenyl]methyleneamino]-N-methyl-1,1-dioxo-1,2-benzothiazol-3-amine
- [3-[cyclobutylmethyl-[(E)-(4-fluoro-3-methoxy-phenyl)methyleneamino]amino]-1,1-dioxo-1,2-benzothiazol-6-yl]boronic acid
- [5-fluoro-3-[[(E)-(4-fluoro-3-methoxy-phenyl)methyleneamino]-isobutyl-amino]-1,1-dioxo-1,2-benzothiazol-6-yl]boronic acid
- N-[(E)-(4-fluoro-3-methylsulfanyl-phenyl)methyleneamino]-N-methyl-1,1-dioxo-1,2-benzothiazol-3-amine
- N-[(E)-[3-(dimethylamino)-4-fluoro-phenyl]methyleneamino]-N-methyl-1,1-dioxo-1,2-benzothiazol-3-amine
- 3-[[(E)-(4-fluoro-3-methoxy-phenyl)methyleneamino]-isobutyl-amino]-1,1-dioxo-1,2-benzothiazol-6-ol
- [3-[[(E)-(4-fluoro-3-methoxy-phenyl)methyleneamino]-isobutyl-amino]-1,1-dioxo-1,2-benzothiazol-6-yl]oxymethylboronic acid
- 5-ethoxy-N-[(E)-(4-fluoro-3-methoxy-phenyl)methyleneamino]-N-isobutyl-1,1-dioxo-1,2-benzothiazol-3-amine
- [3-[[(E)-(4-fluoro-3-methoxy-phenyl)methyleneamino]-isobutyl-amino]-1,1-dioxo-1,2-benzothiazol-5-yl]boronic acid
- [3-[[(E)-(4-fluoro-3-methoxy-phenyl)methyleneamino]-isobutyl-amino]-1,1-dioxo-1,2-benzothiazol-5-yl]oxymethylboronic acid
- 4-[[3-[[(E)-(4-fluoro-3-methoxy-phenyl)methyleneamino]-isobutyl-amino]-1,1-dioxo-1,2-benzothiazol-6-yl]oxy]butan-1-ol
- [3-[[(E)-(4-fluoro-3-methoxy-phenyl)methyleneamino]-isopropyl-amino]-1,1-dioxo-1,2-benzothiazol-6-yl]boronic acid
- 1-(azetidin-1-yl)-2-[[3-[[(E)-(4-fluoro-3-methoxy-phenyl)methyleneamino]-isobutyl-amino]-1,1 -dioxo-1,2-benzothiazol-5-yl]oxy]ethanone
- 2-[[3-[[(E)-(4-fluoro-3-methoxy-phenyl)methyleneamino]-isobutyl-amino]-1,1-dioxo-1,2-benzothiazol-6-yl]oxy]acetamide
- 2-[[3-[[(E)-(4-fluoro-3-methoxy-phenyl)methyleneamino]-isobutyl-amino]-1,1-dioxo-1,2-benzothiazol-5-yl]oxy]-N-methyl-acetamide
- 2-[[3-[[(E)-(4-fluoro-3-methoxy-phenyl)methyleneamino]-isobutyl-amino]-1,1-dioxo-1,2-benzothiazol-6-yl]oxymethyl]-6-methyl-1,3,6,2-dioxazaborocane-4,8-dione
- N-[(E)-(4-fluoro-3-methylsulfanyl-phenyl)methyleneamino]-N-isobutyl-1,1-dioxo-1,2-benzothiazol-3-amine
- 3-[(1,1-dioxo-1,2-benzothiazol-3-yl)-[(E)-(4-fluoro-3-methoxy-phenyl)methyleneamino] amino]-2,2-dimethyl-propan-1-ol
- [3-[6-{4-fluoro-3-methoxy-phenyl)-3-methyl-4,5-dihydro-3H-pyridazin-2-yl]-1,1-dioxo-1,2-benzothiazol-6-yl]boronic acid
- 3-[6-(4-fluoro-3-methoxy-phenyl)-4,4-dimethyl-3,5-dihydropyridazin-2-yl]-1,2-benzothiazole 1,1-dioxide
- 6-diethoxyphosphoryl-N-[(E)-(4-fluoro-3-methoxy-phenyl)methyleneamino]-N-isobutyl-1,1-dioxo-1,2-benzothiazol-3-amine
and pharmaceutically acceptable salts of these compounds.

4. The compound of claim 1, or a pharmaceutically acceptable salt thereof, wherein R₁ represents: 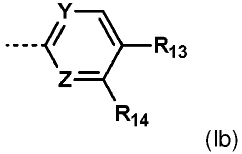 one of Y and Z is N and the other is CH, and
R₁₃ and R₁₄ are each independently selected from H, halo, hydroxyl, alkoxyl, alkylthio, alkyl, cycloalkyl, alkyl-R₈ and -O-alkyl-R₈.

5. The compound of claim 4, selected from:
- 6-[(E)-[(1,1-dioxo-1,2-benzothiazol-3-yl)-methyl-hydrazono]methyl]-4-methoxy-pyridin-3-ol;
- N-[(E)-(6-chloro-5-fluoro-2-pyridyl)methyleneamino]-N-methyl-1,1-dioxo-1,2-benzothiazol-3-amine;
and pharmaceutically acceptable salts of these compounds.

6. The compound of claim 1, or a pharmaceutically acceptable salt thereof, wherein R₁ represents: 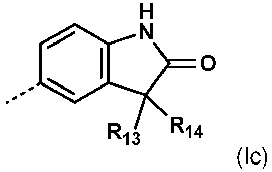 and R₁₃ and R₁₄ are each independently selected from H, halo, hydroxyl, alkyl, alkoxyl, alkoxyalkoxyl, alkylthio, alkyl, cycloalkyl, alkyl-R₈ and -O-alkyl-R₈, or R₁₃ and R₁₄ can be bound together to form a 3- to 6-membered cycle.

7. The compound of claim 6, selected from:
- 5-[(E)-[(1,1-dioxo-1,2-benzothiazol-3-yl)-methyl-hydrazono]methyl]-3,3-dimethyl-indolin-2-one;
- 5-[(E)-[(1,1-dioxo-1,2-benzothiazol-3-yl)-methyl-hydrazono]methyl]-3,3-diethyl-indolin-2-one;
- 5-[(E)-[(1,1-dioxo-1,2-benzothiazol-3-yl)-ethyl-hydrazono]methyl]-3,3-diethyl-indolin-2-one
- [3-[[(E)-(3,3-diethyl-2-oxo-indolin-5-yl)methyleneamino]-(2-methoxyethyl)amino]-1,1-dioxo-1,2-benzothiazol-6-yl]boronic acid
- [3-[[(E)-(3,3-diethyl-2-oxo-indolin-5-yl)methyleneamino]-isobutyl-amino]-1,1-dioxo-1,2-benzothiazol-6-yl]boronic acid
- 3,3-diethyl-5-[(E)-[(6-hydroxy-1,1-dioxo-1,2-benzothiazol-3-yl)-isobutyl-hydrazono] methyl]indolin-2-one
- 5-[(E)-[(6-ethoxy-1,1-dioxo-1,2-benzothiazol-3-yl)-isobutyl-hydrazono]methyl]-3,3-diethyl-indolin-2-one
- 3,3-diethyl-5-[(E)-[isobutyl-(6-methoxy-1,1-dioxo-1,2-benzothiazol-3-yl)hydrazono] methyl]indolin-2-one
- 3,3-diethyl-5-[(E)-[isobutyl-(6-isopropoxy-1,1-dioxo-1,2-benzothiazol-3-yl)hydrazono] methyl]indolin-2-one
- 5-[(E)-[(5-ethoxy-1,1-dioxo-1,2-benzothiazol-3-yl)-isobutyl-hydrazono]methyl]-3,3-diethyl-indolin-2-one
and pharmaceutically acceptable salts of these compounds.

8. The compound of claim 1, or a pharmaceutically acceptable salt thereof, wherein R₁ represents: 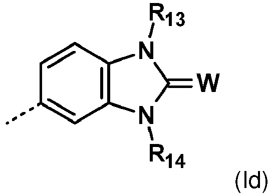 wherein W is O or S, and
R₁₃ and R₁₄ are each independently selected from H, alkyl, cycloalkyl, and alkyl-R₈, and R₈ is preferably hydroxyl, alkoxyl, alkoxyalkoxyl, -O-CO-alkyl, -CO-NR₉R₁₀ or -CF₃.

9. The compound of claim 8, selected from:
- 5-[(E)-[(1,1-dioxo-1,2-benzothiazol-3-yl)-isobutyl-hydrazono]methyl]-3-methyl-1H-benzimidazol-2-one;
- 5-[(E)-[(1,1-dioxo-1,2-benzothiazol-3-yl)-methyl-hydrazono]methyl]-3-ethyl-1H-benzimidazol-2-one;
- 5-[(E)-[(1,1-dioxo-1,2-benzothiazol-3-yl)-methyl-hydrazono]methyl]-3-propyl-1H-benzimidazol-2-one;
- 5-[(E)-[(1,1-dioxo-1,2-benzothiazol-3-yl)-methyl-hydrazono]methyl]-3-isopropyl-1H-benzimidazol-2-one;
- 3-cyclopropyl-5-[(E)-[(1,1-dioxo-1,2-benzothiazol-3-yl)-methyl-hydrazono] methyl]-1H-benzimidazol-2-one;
- 3-cyclobutyl-5-[(E)-[(1,1-dioxo-1,2-benzothiazol-3-yl)-methyl-hydrazono]methyl]-1H-benzimidazol-2-one;
- 5-[(E)-[(1,1-dioxo-1,2-benzothiazol-3-yl)-methyl-hydrazono]methyl]-3-isobutyl-1H-benzimidazol-2-one;
- 5-[(E)-[(1,1-dioxo-1,2-benzothiazol-3-yl)-methyl-hydrazono]methyl]-3-(2,2,2-trifluoroethyl)-1H-benzimidazol-2-one;
- [3-[isobutyl-[(E)-(3-methyl-2-oxo-1H-benzimidazol-5-yl)methyleneamino]amino]-1,1-dioxo-1,2-benzothiazol-6-yl]boronic acid;
- [3-[[(E)-(3-ethyl-2-oxo-1H-benzimidazol-5-yl)methyleneamino]-methyl-amino]-1,1-dioxo-1,2-benzothiazol-6-yl]boronic acid;
- [3-[[(E)-(3-isopropyl-2-oxo-1H-benzimidazol-5-yl)methyleneamino]-methyl-amino]-1,1-dioxo-1,2-benzothiazol-6-yl]boronic acid;
- 3-methyl-5-[(E)-[methyl-(5-methyl-1,1-dioxo-1,2-benzothiazol-3-yl) hydrazono]methyl] - 1H-benzimidazol-2-one;
- [3-[isobutyl-[(E)-(3-isopropyl-2-oxo-1H-benzimidazol-5-yl)methyleneamino]amino]-1,1-dioxo-1,2-benzothiazol-6-yl]boronic acid
- 3-[[(E)-(3-isopropyl-2-oxo-1H-benzimidazol-5-yl)methyleneamino]-(2-methoxyethyl) amino]-1,1-dioxo-1,2-benzothiazol-6-yl]boronic acid
- 3-cyclobutyl-5-[(E)-[(1,1-dioxo-1,2-benzothiazol-3-yl)-methyl-hydrazono]methyl]-1H-benzimidazole-2-thione
- [3-[ethyl-[(E)-(3-isopropyl-2-oxo-1H-benzimidazol-5-yl)methyleneamino]amino]-1,1-dioxo-1,2-benzothiazol-6-yl]boronic acid
- [3-[[(E)-(3-cyclobutyl-2-oxo-1H-benzimidazol-5-yl)methyleneamino]-methyl-amino]-1,1-dioxo-1,2-benzothiazol-6-yl]boronic acid
- [3-[[(E)-(3-cyclobutyl-2-oxo-1H-benzimidazol-5-yl)methyleneamino]-ethyl-amino]-1,1-dioxo-1,2-benzothiazol-6-yl]boronic acid
- [3-[[(E)-(3-cyclobutyl-2-oxo-1H-benzimidazol-5-yl)methyleneamino]-(2-methoxyethyl) amino]-1,1-dioxo-1,2-benzothiazol-6-yl]boronic acid
- [3-[ethyl-[(E)-(3-ethyl-2-oxo-1H-benzimidazol-5-yl)methyleneamino]amino]-1,1-dioxo-1,2-benzothiazol-6-yl]boronic acid
- [3-[[(E)-(3-ethyl-2-oxo-1H-benzimidazol-5-yl)methyleneamino]-isobutyl-amino]-1,1-dioxo-1,2-benzothiazol-6-yl]boronic acid
- 3-cyclobutyl-5-[(E)-[(1,1-dioxo-1,2-benzothiazol-3-yl)-methyl-hydrazono]methyl]-1-(methoxymethyl)benzimidazol-2-one
- 5-[(E)-[(1,1-dioxo-1,2-benzothiazol-3-yl)-methyl-hydrazono]methyl]-3-isopropyl-1-(methoxymethyl)benzimidazol-2-one
- [3-cyclobutyl-5-[(E)-[(1,1-dioxo-1,2-benzothiazol-3-yl)-methyl-hydrazono]methyl]-2-oxo-benzimidazol-1-yl]methyl 2,2-dimethylpropanoate
- [3-cyclobutyl-5-[(E)-[(1,1-dioxo-1,2-benzothiazol-3-yl)-methyl-hydrazono]methyl]-2-oxo-benzimidazol-1-yl]methyl butanoate
- 3-cyclobutyl-5-[(E)-[(1,1-dioxo-1,2-benzothiazol-3-yl)-methyl-hydrazono]methyl]-1-(2-methoxyethoxymethyl)benzimidazol-2-one
- 3-cyclobutyl-5-[(E)-[(1,1-dioxo-1,2-benzothiazol-3-yl)-methyl-hydrazono]methyl]-1-(ethoxymethyl)benzimidazol-2-one
- [3-cyclobutyl-5-[(E)-[(1,1 -dioxo-1,2-benzothiazol-3-yl)-methyl-hydrazono]methyl]-2-oxo-benzimidazol-1-yl]methyl N,N-dimethylcarbamate
and pharmaceutically acceptable salts of these compounds.

10. The compound of claim 1, or a pharmaceutically acceptable salt thereof, wherein R₁ represents: 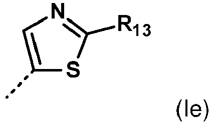 and R₁₃ is H, alkyl, cycloalkyl, phenyl, alkyl-R₈ or -NR₉R₁₀.

11. The compound of claim 10, selected from:
- N-[(E)-(2-aminothiazol-5-yl)methyleneamino]-N-methyl-1,1-dioxo-1,2-benzothiazol-3-amine;
- N-methyl-1,1-dioxo-N-[(E)-(2-pyrrolidin-1-ylthiazol-5-yl)methyleneamino]-1,2-benzothiazol-3-amine;
- N-methyl-N-[(E)-(2-morpholinothiazol-5-yl)methyleneamino]-1,1-dioxo-1,2-benzothiazol-3-amine;
- N-methyl-1,1-dioxo-N-[(E)-(2-phenylthiazol-5-yl)methyleneamino]-1,2-benzothiazol-3-amine;
- N-[(E)-[2-(azetidin-1-yl)thiazol-5-yl]methyleneamino]-N-methyl-1,1-dioxo-1,2-benzothiazol-3-amine;
- N-methyl-N-[(E)-(2-methylthiazol-5-yl)methyleneamino]-1,1-dioxo-1,2-benzothiazol-3-amine
and pharmaceutically acceptable salts of these compounds.

12. The compound of claim 1, or a pharmaceutically acceptable salt thereof, wherein R₁ represents: 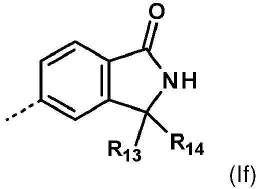 and R₁₃ and R₁₄ are each independently selected from H, halo, hydroxyl, alkoxyl, alkylthio, alkyl, cycloalkyl, alkyl-R₈ and -O-alkyl-R₈ or R₁₃ and R₁₄ can be bound together to form a 3- to 6-membered cycle.

13. The compound claim 12, selected from:
- 5-[(E)-[(1,1-dioxo-1,2-benzothiazol-3-yl)-methyl-hydrazono]methyl]-3-methyl-isoindolin-1-one;
- 5-[(E)-[(1,1-dioxo-1,2-benzothiazol-3-yl)-methyl-hydrazono]methyl]-3,3-dimethyl-isoindolin-1-one;
- 5-[(E)-[(1,1-dioxo-1,2-benzothiazol-3-yl)-ethyl-hydrazono]methyl]-3,3-dimethyl-isoindolin-1-one
- 5'-[(E)-[(1,1-dioxo-1,2-benzothiazol-3-yl)-methyl-hydrazono]methyl]spiro[cyclopropane-1,3'-isoindoline]-1'-one
and pharmaceutically acceptable salts of these compounds.

14. The compound of claim 1, or a pharmaceutically acceptable salt thereof, of formula (II): 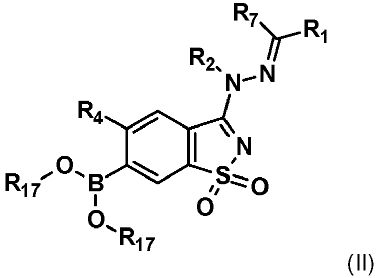 wherein R₁, R₂, R₄, and R₇ are as defined in claim 1, and each R₁₇ independently is H or alkyl, or the two OR₁₇ together form a 5- to 8-membered heterocycle such as 4,4,5,5-tetramethyl-1,3,2-dioxaborolan-2-yl, 5,5-dimethyl-1,3,2-dioxaborinan-2-yl or N-methyl-1,3,6,2-dioxazaborocane-4,8-dione.

15. The compound of claim 14, or a pharmaceutically acceptable salt thereof, of formula (IIa): 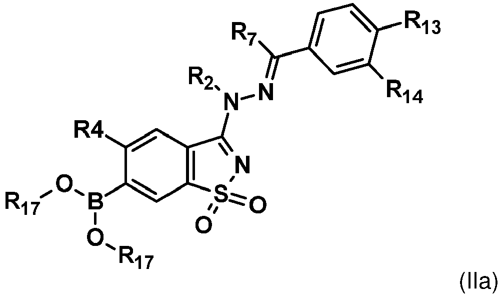 wherein:
R₂ and R₇ are as defined in claim 1;
R₄ is H, alkyl, or halo;
R₁₃ and R₁₄ are each independently selected from H, halo, hydroxyl, alkoxyl, alkyl, cycloalkyl, alkyl-R₈, -O-alkyl-R₈, -NR₉R₁₀ and -CO-NR₉R₁₀, with the proviso that when R₁₄ is methoxyl, then R₁₃ is not hydroxyl or methoxyl;
R₈ is hydroxyl, alkoxyl, cycloalkyl or -CF₃;
each R₁₇ is as defined in claim 14.

16. The compound of claim 14, or a pharmaceutically acceptable salt thereof, of formula (IId): 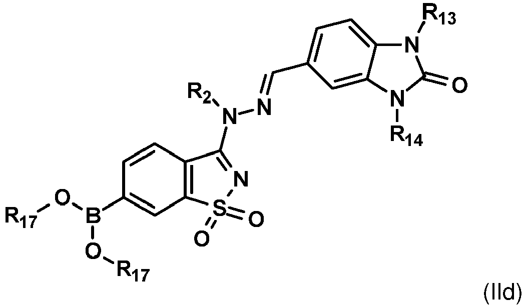 wherein:
R₂ is a linear or branched (C₁-C₄)alkyl or alkyl-R₈;
R₁₃ and R₁₄ are each independently selected from H, alkyl, cycloalkyl, and alkyl-R₈;
R₈ is hydroxyl, alkoxyl, cycloalkyl or -CF₃;
each R₁₇ is as defined in claim 14.

17. The compound of claim 1, or a pharmaceutically acceptable salt thereof, of formula (III): 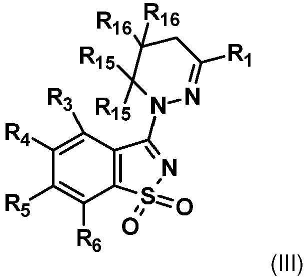 wherein R₁, R₃, R₄, R₅, and R₆ are as defined in claim 1, and R₁₅, R₁₆ are each independently H, alkyl, alkyl-R₈ or -aryl-R₈..

18. A compound of one of claims 1 to 17, for use as a medicament.

19. A compound of one of claims 1 to 17, for use in the treatment of a cancer indication where YAP is localized in the nucleus of the tumor cells, such as lung, thyroid, ovarian, colorectal, prostate, pancreas, esophagus, liver, breast and skin cancer, more particularly in the treatment of malignant mesothelioma.

20. A pharmaceutical composition comprising a compound of one of claims 1 to 17 or a pharmaceutically acceptable salt thereof, and a pharmaceutically acceptable excipient.

## Patentansprüche

1. Verbindung der Formel (I): 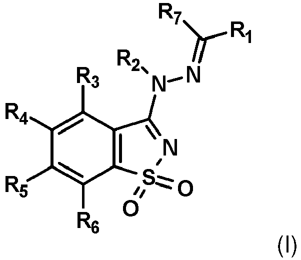 oder ein pharmazeutisch akzeptables Salz davon, wobei:
R₁ eine Arylgruppe ist, die gegebenenfalls mit einem oder mehrere Substituenten unabhängig ausgewählt aus Halogen, Hydroxyl, Alkoxyl, Alkylthio, OCF₃, Alkyl, Cycloalkyl, Alkyl-R₈, -O-Alkyl-R₈, -NR₉R₁₀, -CO-NR₉R₁₀, Oxo, Thioxo und Phenyl substituiert ist,
R₂ ein Alkyl ist, das gegebnenefalls mit einem oder zwei R₈ oder -Aryl-R₈ substituiert ist,
R₃, R₄, R₅, R₆ jeweils unabhängig H, Halogen, Hydroxyl, Alkyl, Alkoxyl, mit Borsäure oder einem Borsäurederivat substituiertes Alkoxyl, Hydroxycarbonyl, Aryl, -O-Aryl, Borsäure oder
ein Borsäurederivat, Phosphonsäure oder Phosphonsäurederivat, Alkyl-R₈, -O-Alkyl-R₈, -COOR₁₁, -NO₂, -NR₉R₁₀ oder -CO-NR₉R₁₀ sind,
R₇ H oder Alkyl ist, oder
R₂ und R₇ miteinander verbunden sind, um einen 5- oder 6-gliedrigen Heterozyklus zu bilden,
R₈ Halogen, Hydroxyl, Alkoxyl, -NR₉R₁₀, -CO-X-R₁₁, -CN, -CF₃, Aryl, Cycloalkyl, -O-Alkylalkoxyl, -O-CO-Alkyl, -CO-NR₉R₁₀ oder -O-CO-NR₉R₁₀ ist,
Rg und R₁₀ jeweils unabhängig H, Alkyl oder -CO-Alkyl sind oder zusammen mit dem Stickstoffatom eine 3- bis 6-gliedrige zyklische Gruppe bilden,
X -O- oder -NR₁₂- ist,
R₁₁ H oder Alkyl ist,
R₁₂ H, Alkyl oder Hydroxyalkyl ist,
und wenn X -NR₁₂- ist, R₁₁ und R₁₂ miteinander verbunden sein können, um eine 4- bis 7-gliedrige zyklische Gruppe zu bilden,
wobei Aryl ein aromatischer oder teilweise ungesättigter (Hetero-)Zyklus ist, umfassend 1, 2 oder 3 Ringe, die fusioniert sein können,
mit der Maßgabe, dass R₁ nicht 3,4-Dimethoxyphenyl oder 3-Methoxy-4-hydroxyphenyl ist, und
mit der Maßgabe, dass R₁ eine substituierte Arylgruppe ist, wenn R₂ und R₇ miteinander verbunden sind, um einen 5- oder 6-gliedrigen Heterozyklus zu bilden, und R₃, R₄, R₅ und
R₆ jeweils H sind.

2. Verbindung nach Anspruch 1, oder ein pharmazeutisch akzeptables Salz davon, wobei R₁ für: 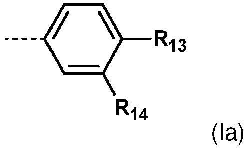 steht und R₁₃ und R₁₄ jeweils unabhängig ausgewählt sind aus H, Halogen, Hydroxyl, Alkoxyl, Alkylthio, -OCF₃, Alkyl, Cycloalkyl, Alkyl-R₈, -O-Alkyl-R₈, -NR₉R₁₀ und -CO-NR₉R₁₀, mit der Maßgabe, dass R₁₃ nicht Hydroxyl oder Methoxyl ist, wenn R₁₄ Methoxyl ist.

3. Verbindung nach Anspruch 2, ausgewählt aus:
- N-[(E)-(4-Fluorphenyl)methylenamino]-N-methyl-1,1 -dioxo-1,2-benzothiazol-3-amin,
- 2-[3-[(E)-[(1,1-Dioxo-1,2-benzothiazol-3-yl)-methyl-hydrazono]methyl]phenoxy]-ethanol,
- N-Methyl-N-[(E)-(4-morpholinophenyl)methylenamino]-1,1 -dioxo-1,2-benzothiazol-3-amin,
- N-[(E)-[3-(Dimethylamino)phenyl]methylenamino]-N-methyl-1,1-dioxo-1,2-benzothiazol-3-amin,
- N-[(E)-(4-Fluor-3-methoxy-phenyl)methylenamino]-1,1-dioxo-N-[2-(2-pyridyl)ethyl]-1,2-benzothiazol-3-amin,
- Methyl-2-[3-[(E)-[(1,1 -dioxo-1,2-benzothiazol-3-yl)-methyl-hydrazono]methyl]phenyl]acetat,
- N-[(E)-(3-Ethoxy-4-fluor-phenyl)methylenamino]-N-methyl-1,1-dioxo-1,2-benzothiazol-3-amin,
- N-[(E)-(4-Fluor-3-propoxy-phenyl)methylenamino]-N-methyl-1,1-dioxo-1,2-benzothiazol-3-amin,
- N-[(E)-(4-Fluor-3-isopropoxy-phenyl)methylenamino]-N-methyl-1,1-dioxo-1,2-benzothiazol-3-amin,
- 2-[5-[(E)-[(1,1-Dioxo-1,2-benzothiazol-3-yl)-methyl-hydrazono]methyl]-2-fluor-phenoxy]ethanol,
- N-[(E)-[4-Fluor-3-(2-methoxyethoxy)phenyl]methylenamino]-N-methyl-1,1 -dioxo-1,2-benzothiazol-3-amin,
- N-[(E)-[3-Fluor-5-methoxy-4-(2-methoxyethoxy)phenyl]methylenamino]-N-methyl-1,1-dioxo-1,2-benzothiazol-3-amin,
- 5-[(E)-[(1,1-Dioxo-1,2-benzothiazol-3-yl)-methyl-hydrazono]methyl]-2-fluor-benzamid,
- 2-(Dimethylamino)-5-[(E)-[(1,1-dioxo-1,2-benzothiazol-3-yl)-methyl-hydrazono]methyl]-benzamid,
- [4-[(E)-[(1,1-Dioxo-1,2-benzothiazol-3-yl)-methyl-hydrazono]methyl]-2-methoxyphenyl]methanol,
- 2-[4-[(E)-[(1,1-Dioxo-1,2-benzothiazol-3-yl)-methyl-hydrazono]methyl]-2-methoxy-phenyl]-N-methyl-acetamid,
- N-[(E)-[4-(2-Methoxyethoxy)phenyl]methylenamino]-N-methyl-1,1 -dioxo-1,2-benzothiazol-3-amin,
- N-[(E)-[3-(2-Methoxyethoxy)phenyl]methylenamino]-N-methyl-1,1 -dioxo-1,2-benzothiazol-3-amin,
- N-Ethyl-N-[(E)-(4-fluor-3-methoxy-phenyl)methylenamino]-1,1-dioxo-1,2-benzothiazol-3-amin,
- N-[(E)-(4-Fluor-3-methoxy-phenyl)methylenamino]-N-isopropyl-1,1-dioxo-1,2-benzothiazol-3-amin,
- N-[(E)-(4-Fluor-3-methoxy-phenyl)methylenamino]-N-isobutyl-1,1-dioxo-1,2-benzothiazol-3-amin,
- 2-[(1,1-Dioxo-1,2-benzothiazol-3-yl)-[(E)-(4-fluor-3-methoxy-phenyl) methylenamino]amino]ethanol,
- N-[(E)-(4-Fluor-3-methoxy-phenyl)methylenamino]-N-(2-methoxyethyl)-1,1 -dioxo-1,2-benzothiazol-3-amin,
- N-(2-Ethoxyethyl)-N-[(E)-(4-fluor-3-methoxy-phenyl)methylenamino]-1,1-dioxo-1,2-benzothiazol-3-amin,
- 3-[(1,1-Dioxo-1,2-benzothiazol-3-yl)-[(E)-(4-fluor-3-methoxy-phenyl) methylenamino]amino]propan-1-ol,
- 2-(Chloromethyl)-3-[(1,1-dioxo-1,2-benzothiazol-3-yl)-[(E)-(4-fluor-3-methoxy-phenyl)methylenamino]amino]propan-1-ol,
- N-[(E)-(4-Fluor-3-methoxy-phenyl)methylenamino]-1,1-dioxo-N-(2-phenylethyl)-1,2-benzothiazol-3-amin,
- N-[(E)-(4-Fluor-3-methoxy-phenyl)methylenamino]-N-methyl-5-nitro-1,1-dioxo-1,2-benzothiazol-3-amin,
- N-[3-[[(E)-(4-Fluor-3-methoxy-phenyl)methylenamino]-methyl-amino]-1,1-dioxo-1,2-benzothiazol-5-yl]acetamid,
- N3-[(E)-(4-Fluor-3-methoxy-phenyl)methylenamino]-N3-methyl-1,1 -dioxo-1,2-benzothiazole-3,6-diamin,
- 3-[[(E)-(4-Fluor-3-methoxy-phenyl)methylenamino]-methyl-amino]-1,1-dioxo-1,2-benzothiazol-6-yl]-Borsäure,
- 3-[[(E)-(4-Fluor-3-methoxy-phenyl)methylenamino]-methyl-amino]-1,1-dioxo-1,2-benzothiazol-6-ol,
- 6-Benzyloxy-N-[(E)-(4-fluor-3-methoxy-phenyl)methylenamino]-N-methyl-1,1-dioxo-1,2-benzothiazol-3-amin,
- 5-Ethoxy-N-[(E)-(4-fluor-3-methoxy-phenyl)methylenamino]-N-methyl-1,1-dioxo-1,2-benzothiazol-3-amin,
- N-[(E)-(4-Fluor-3-methoxy-phenyl)methylenamino]-5-isopropoxy-N-methyl-1,1-dioxo-1,2-benzothiazol-3-amin,
- N-[(E)-(4-Fluor-3-methoxy-phenyl)methylenamino]-N-methyl-1,1-dioxo-5-phenoxy-1,2-benzothiazol-3-amin,
- N-[(E)-(4-Fluor-3-methoxy-phenyl)methylenamino]-5-(2-methoxyethoxy)-N-methyl-1,1-dioxo-1,2-benzothiazol-3-amin,
- 5-[2-(Dimethylamino)ethoxy]-N-[(E)-(4-fluor-3-methoxy-phenyl)methylenamino]-N-methyl-1,1-dioxo-1,2-benzothiazol-3-amin,
- 2-[[3-[[(E)-(4-Fluor-3-methoxy-phenyl)methylenamino]-methyl-amino]-1,1-dioxo-1,2-benzothiazol-5-yl]oxy]-N,N-dimethyl-acetamid,
- 6-(5,5-Dimethyl-1,3,2-dioxaborinan-2-yl)-N-[(E)-(4-fluor-3-methoxy-phenyl) methylenamino]-N-methyl-1,1-dioxo-1,2-benzothiazol-3-amin,
- 6-Diethoxyphosphoryl-N-[(E)-(4-fluor-3-methoxy-phenyl)methylenamino]-N-methyl-1,1-dioxo-1,2-benzothiazol-3-amin,
- [3-[Ethyl-[(E)-(4-fluor-3-methoxy-phenyl)methylenamino]amino]-1,1-dioxo-1,2-benzothiazol-6-yl]-Borsäure,
- [3-[[(E)-(4-Fluor-3-methoxy-phenyl)methylenamino]-isobutyl-amino]-1,1-dioxo-1,2-benzothiazol-6-yl]-Borsäure,
- [3-[[(E)-(4-Fluorphenyl)methylenamino]-methyl-amino]-1,1-dioxo-1,2-benzothiazol-6-yl]-Borsäure,
- 2-[3-[[(E)-(4-Fluor-3-methoxy-phenyl)methylenamino]-methyl-amino]-1,1-dioxo-1,2-benzothiazol-6-yl]-6-methyl-1,3,6,2-dioxazaborocan-4,8-dion,
- 2-[3-[[(E)-(4-Fluor-3-methoxy-phenyl)methylenamino]-isobutyl-amino]-1,1-dioxo-1,2-benzothiazol-6-yl]-6-methyl-1,3,6,2-dioxazaborocan-4,8-dion,
- [3-[[(E)-(4-Fluor-3-methoxy-phenyl)methylenamino]-(2-methoxyethyl)amino]-1,1-dioxo-1,2-benzothiazol-6-yl]-Borsäure
- N-[(E)-[4-Fluor-3-(trifluormethoxy)phenyl]methylenamino]-N-methyl-1,1-dioxo-1,2-benzothiazol-3-amin
- [3-[Cyclobutylmethyl-[(E)-(4-fluor-3-methoxy-phenyl)methylenamino]amino]-1,1-dioxo-1,2-benzothiazol-6-yl]-Borsäure
- [5-Fluor-3-[[(E)-(4-fluor-3-methoxy-phenyl)methylenamino]-isobutyl-amino]-1,1-dioxo-1,2-benzothiazol-6-yl]-Borsäure
- N-[(E)-(4-Fluor-3-methylsulfanyl-phenyl)methylenamino]-N-methyl-1,1 -dioxo-1,2-benzothiazol-3-amin
- N-[(E)-[3-(Dimethylamino)-4-fluor-phenyl]methylenamino]-N-methyl-1,1 -dioxo-1,2-benzothiazol-3-amin
- 3-[[(E)-(4-Fluor-3-methoxy-phenyl)methylenamino]-isobutyl-amino]-1,1 -dioxo-1,2-benzothiazol-6-ol
- [3-[[(E)-(4-Fluor-3-methoxy-phenyl)methylenamino]-isobutyl-amino]-1,1-dioxo-1,2-benzothiazol-6-yl]oxymethyl-Borsäure
- 5-Ethoxy-N-[(E)-(4-fluor-3-methoxy-phenyl)methylenamino]-N-isobutyl-1,1-dioxo-1,2-benzothiazol-3-amin
- [3-[[(E)-(4-Fluor-3-methoxy-phenyl)methylenamino]-isobutyl-amino]-1,1-dioxo-1,2-benzothiazol-5-yl]-Borsäure
- [3-[[(E)-(4-Fluor-3-methoxy-phenyl)methylenamino]-isobutyl-amino]-1,1-dioxo-1,2-benzothiazol-5-yl]oxymethyl-Borsäure
- 4-[[3-[[(E)-(4-Fluor-3-methoxy-phenyl)methylenamino]-isobutyl-amino]-1,1-dioxo-1,2-benzothiazol-6-yl]oxy]butan-1-ol,
- [3-[[(E)-(4-Fluor-3-methoxy-phenyl)methylenamino]-isopropyl-amino]-1,1-dioxo-1,2-benzothiazol-6-yl]-Borsäure,
- 1-(Azetidin-1-yl)-2-[[3-[[(E)-(4-fluor-3-methoxy-phenyl)methylenamino]-isobutyl-amino]-1,1-dioxo-1,2-benzothiazol-5-yl]oxy]ethanon,
- 2-[[3-[[(E)-(4-Fluor-3-methoxy-phenyl)methylenamino]-isobutyl-amino]-1,1-dioxo-1,2-benzothiazol-6-yl]oxy]acetamid,
- 2-[[3-[[(E)-(4-Fluor-3-methoxy-phenyl)methylenamino]-isobutyl-amino]-1,1-dioxo-1,2-benzothiazol-5-yl]oxy]-N-methyl-acetamid,
- 2-[[3-[[(E)-(4-Fluor-3-methoxy-phenyl)methylenamino]-isobutyl-amino]-1,1-dioxo-1,2-benzothiazol-6-yl]oxymethyl]-6-methyl-1,3,6,2-dioxazaborocan-4,8-dion,
- N-[(E)-(4-Fluor-3-methylsulfanyl-phenyl)methylenamino]-N-isobutyl-1,1-dioxo-1,2-benzothiazol-3-amin,
- 3-[(1,1-Dioxo-1,2-benzothiazol-3-yl)-[(E)-(4-fluor-3-methoxyphenyl)methylenamino]amino]-2,2-dimethyl-propan-1-ol,
- [3-[6-(4-Fluor-3-methoxy-phenyl)-3-methyl-4,5-dihydro-3H-pyridazin-2-yl]-1,1-dioxo-1,2-benzothiazol-6-yl]-Borsäure,
- 3-[6-(4-Fluor-3-methoxy-phenyl)-4,4-dimethyl-3,5-dihydropyridazin-2-yl]-1,2-benzothiazole 1,1-dioxid,
- 6-Diethoxyphosphoryl-N-[(E)-(4-fluor-3-methoxy-phenyl)methylenamino]-N-isobutyl-1,1-dioxo-1,2-benzothiazol-3-amin,
und pharmazeutisch akzeptable Salze dieser Verbindungen.

4. Verbindung nach Anspruch 1, oder ein pharmazeutisch akzeptables Salz davon, wobei R₁ für: 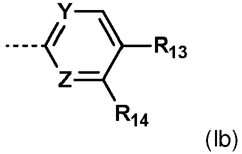 steht, eines von Y und Z N ist und das andere CH ist, und
R₁₃ und R₁₄ jeweils unabhängig ausgewählt sind aus H, Halogen, Hydroxyl, Alkoxyl, Alkylthio, Alkyl, Cycloalkyl, Alkyl-R₈ und -O-Alkyl-R₈-

5. Verbindung nach Anspruch 4, ausgewählt aus:
- 6-[(E)-[(1,1-Dioxo-1,2-benzothiazol-3-yl)-methyl-hydrazono]methyl]-4-methoxy-pyridin-3-ol,
- N-[(E)-(6-Chlor-5-fluor-2-pyridyl)methylenamino]-N-methyl-1,1-dioxo-1,2-benzothiazol-3-amin,
und pharmazeutisch akzeptable Salze dieser Verbindungen.

6. Verbindung nach Anspruch 1, oder ein pharmazeutisch akzeptables Salz davon, wobei R₁ für: 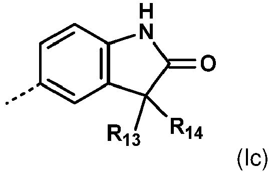 steht und R₁₃ und R₁₄ jeweils unabhängig ausgewählt sind aus H, Halogen, Hydroxyl, Alkyl, Alkoxyl, Alkoxyalkoxyl, Alkylthio, Alkyl, Cycloalkyl, Alkyl-R₈ und -O-Alkyl-R₈, oder R₁₃ und R₁₄ miteinander verbunden sein können, um einen 3- bis 6-gliedrigen Zyklus zu bilden.

7. Verbindung nach Anspruch 6, ausgewählt aus:
- 5-[(E)-[(1,1-Dioxo-1,2-benzothiazol-3-yl)-methyl-hydrazono]methyl]-3,3-dimethyl-indolin-2-on,
- 5-[(E)-[(1,1-Dioxo-1,2-benzothiazol-3-yl)-methyl-hydrazono]methyl]-3,3-diethyl-indolin-2-on,
- 5-[(E)-[(1,1-Dioxo-1,2-benzothiazol-3-yl)-ethyl-hydrazono]methyl]-3,3-diethyl-indolin-2-on
- [3-[[(E)-(3,3-Diethyl-2-oxo-indolin-5-yl)methylenamino]-(2-methoxyethyl)amino]-1,1-dioxo-1,2-benzothiazol-6-yl]-Borsäure
- [3-[[(E)-(3,3-Diethyl-2-oxo-indolin-5-yl)methylenamino]-isobutyl-amino]-1,1-dioxo-1,2-benzothiazol-6-yl]-Borsäure
- 3,3-Diethyl-5-[(E)-[(6-hydroxy-1,1-dioxo-1,2-benzothiazol-3-yl)-isobutyl-hydrazono]-methyl]indolin-2-on
- 5-[(E)-[(6-Ethoxy-1,1 -dioxo-1,2-benzothiazol-3-yl)-isobutyl-hydrazono]methyl]-3,3-diethyl-indolin-2-on
- 3,3-Diethyl-5-[(E)-[isobutyl-(6-methoxy-1,1-dioxo-1,2-benzothiazol-3-yl)hydrazono]-methyl]indolin-2-on
- 3,3-Diethyl-5-[(E)-[isobutyl-(6-isopropoxy-1,1-dioxo-1,2-benzothiazol-3-yl)hydrazono]-methyl]indolin-2-on
- 5-[(E)-[(5-Ethoxy-1,1 -dioxo-1,2-benzothiazol-3-yl)-isobutyl-hydrazono]methyl]-3,3-diethyl-indolin-2-on
und pharmazeutisch akzeptable Salze dieser Verbindungen.

8. Verbindung nach Anspruch 1, oder ein pharmazeutisch akzeptables Salz davon, wobei R₁ für: 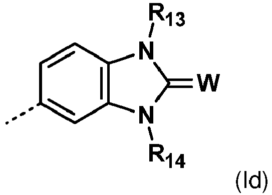 steht, wobei W O oder S ist, und
R₁₃ und R₁₄ jeweils unabhängig ausgewählt sind aus H, Alkyl, Cycloalkyl, und Alkyl-R₈, und R₈ vorzugsweise Hydroxyl, Alkoxyl, Alkoxyalkoxyl, -O-CO-Alkyl, -CO-NR₉R₁₀ oder -CF₃ ist.

9. Verbindung nach Anspruch 8, ausgewählt aus:
- 5-[(E)-[(1,1-Dioxo-1,2-benzothiazol-3-yl)-isobutyl-hydrazono]methyl]-3-methyl-1H-benzimidazol-2-on,
- 5-[(E)-[(1,1-Dioxo-1,2-benzothiazol-3-yl)-methyl-hydrazono]methyl]-3-ethyl-1H-benzimidazol-2-on,
- 5-[(E)-[(1,1-Dioxo-1,2-benzothiazol-3-yl)-methyl-hydrazono]methyl]-3-propyl-1H-benzimidazol-2-on,
- 5-[(E)-[(1,1-Dioxo-1,2-benzothiazol-3-yl)-methyl-hydrazono]methyl]-3-isopropyl-1H-benzimidazol-2-on,
- 3-Cyclopropyl-5-[(E)-[(1,1-dioxo-1,2-benzothiazol-3-yl)-methyl-hydrazono]methyl]-1H-benzimidazol-2-on,
- 3-Cyclobutyl-5-[(E)-[(1,1-dioxo-1,2-benzothiazol-3-yl)-methyl-hydrazono]methyl]-1H-benzimidazol-2-on,
- 5-[(E)-[(1,1-Dioxo-1,2-benzothiazol-3-yl)-methyl-hydrazono]methyl]-3-isobutyl-1H-benzimidazol-2-on,
- 5-[(E)-[(1,1-Dioxo-1,2-benzothiazol-3-yl)-methyl-hydrazono]methyl]-3-(2,2,2-trifluorethyl)-1H-benzimidazol-2-on,
- [3-[Isobutyl-[(E)-(3-methyl-2-oxo-1H-benzimidazol-5-yl)methylenamino]amino]-1,1-dioxo-1,2-benzothiazol-6-yl]-Borsäure,
- [3-[[(E)-(3-Ethyl-2-oxo-1H-benzimidazol-5-yl)methylenamino]-methyl-amino]-1,1-dioxo-1,2-benzothiazol-6-yl]-Borsäure,
- [3-[[(E)-(3-Isopropyl-2-oxo-1H-benzimidazol-5-yl)methylenamino]-methyl-amino]-1,1-dioxo-1,2-benzothiazol-6-yl]-Borsäure,
- 3-Methyl-5-[(E)-[methyl-(5-methyl-1,1-dioxo-1,2-benzothiazol-3-yl) hydrazono]methyl]
- 1H-benzimidazol-2-on,
- [3-[lsobutyl-[(E)-(3-isopropyl-2-oxo-1H-benzimidazol-5-yl)methylenamino]amino]-1,1-dioxo-1,2-benzothiazol-6-yl]-Borsäure
- 3-[[(E)-(3-Isopropyl-2-oxo-1H-benzimidazol-5-yl)methylenamino]-(2-methoxyethyl)-amino]-1,1-dioxo-1,2-benzothiazol-6-yl]-Borsäure
- 3-Cyclobutyl-5-[(E)-[(1,1-dioxo-1,2-benzothiazol-3-yl)-methyl-hydrazono]methyl]-1H-benzimidazole-2-thion
- [3-[Ethyl-[(E)-(3-isopropyl-2-oxo-1H-benzimidazol-5-yl)methylenamino]amino]-1,1-dioxo-1,2-benzothiazol-6-yl]-Borsäure
- [3-[[(E)-(3-Cyclobutyl-2-oxo-1H-benzimidazol-5-yl)methylenamino]-methyl-amino]-1,1-dioxo-1,2-benzothiazol-6-yl]-Borsäure
- [3-[[(E)-(3-Cyclobutyl-2-oxo-1H-benzimidazol-5-yl)methylenamino]-ethyl-amino]-1,1-dioxo-1,2-benzothiazol-6-yl]-Borsäure
- [3-[[(E)-(3-Cyclobutyl-2-oxo-1H-benzimidazol-5-yl)methylenamino]-(2-methoxyethyl)-amino]-1,1-dioxo-1,2-benzothiazol-6-yl]-Borsäure
- [3-[Ethyl-[(E)-(3-ethyl-2-oxo-1H-benzimidazol-5-yl)methylenamino]amino]-1,1-dioxo-1,2-benzothiazol-6-yl]-Borsäure
- [3-[[(E)-(3-Ethyl-2-oxo-1H-benzimidazol-5-yl)methylenamino]-isobutyl-amino]-1,1-dioxo-1,2-benzothiazol-6-yl]-Borsäure
- 3-Cyclobutyl-5-[(E)-[(1,1-dioxo-1,2-benzothiazol-3-yl)-methyl-hydrazono]methyl]-1-(methoxymethyl)benzimidazol-2-on
- 5-[(E)-[(1,1-Dioxo-1,2-benzothiazol-3-yl)-methyl-hydrazono]methyl]-3-isopropyl-1-(methoxymethyl)benzimidazol-2-on
- [3-Cyclobutyl-5-[(E)-[(1,1-dioxo-1,2-benzothiazol-3-yl)-methyl-hydrazono]methyl]-2-oxo-benzimidazol-1-yl]methyl 2,2-dimethylpropanoat
- [3-Cyclobutyl-5-[(E)-[(1,1-dioxo-1,2-benzothiazol-3-yl)-methyl-hydrazono]methyl]-2-oxo-benzimidazol-1-yl]methylbutanoat
- 3-Cyclobutyl-5-[(E)-[(1,1-dioxo-1,2-benzothiazol-3-yl)-methyl-hydrazono]methyl]-1-(2-methoxyethoxymethyl)benzimidazol-2-on
- 3-Cyclobutyl-5-[(E)-[(1,1-dioxo-1,2-benzothiazol-3-yl)-methyl-hydrazono]methyl]-1-(ethoxymethyl)benzimidazol-2-on
- [3-Cyclobutyl-5-[(E)-[(1,1-dioxo-1,2-benzothiazol-3-yl)-methyl-hydrazono]methyl]-2-oxo-benzimidazol-1-yl]methyl N,N-dimethylcarbamat
und pharmazeutisch akzeptable Salze dieser Verbindungen.

10. Verbindung nach Anspruch 1, oder ein pharmazeutisch akzeptables Salz davon, wobei R₁ für: 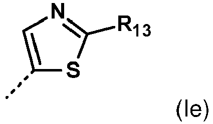 steht und R₁₃ H, Alkyl, Cycloalkyl, Phenyl, Alkyl-R₈ oder -NR₉R₁₀ ist.

11. Verbindung nach Anspruch 10, ausgewählt aus:
- N-[(E)-(2-Aminothiazol-5-yl)methylenamino]-N-methyl-1,1-dioxo-1,2-benzothiazol-3-amin,
- N-Methyl-1,1-dioxo-N-[(E)-(2-pyrrolidin-1-ylthiazol-5-yl)methylenamino]-1,2-benzothiazol-3-amin,
- N-Methyl-N-[(E)-(2-morpholinothiazol-5-yl)methylenamino]-1,1-dioxo-1,2-benzothiazol-3-amin,
- N-Methyl-1,1-dioxo-N-[(E)-(2-phenylthiazol-5-yl)methylenamino]-1,2-benzothiazol-3-amin,
- N-[(E)-[2-(Azetidin-1-yl)thiazol-5-yl]methylenamino]-N-methyl-1,1 -dioxo-1,2-benzothiazol-3-amin,
- N-Methyl-N-[(E)-(2-methylthiazol-5-yl)methylenamino]-1,1-dioxo-1,2-benzothiazol-3-amin
und pharmazeutisch akzeptable Salze dieser Verbindungen.

12. Verbindung nach Anspruch 1, oder ein pharmazeutisch akzeptables Salz davon, wobei R₁ für: 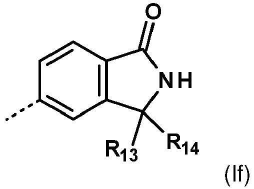 steht und R₁₃ und R₁₄ jeweils unabhängig ausgewählt sind aus H, Halogen, Hydroxyl, Alkoxyl, Alkylthio, Alkyl, Cycloalkyl, Alkyl-R₈ und -O-Alkyl-R₈, oder R₁₃ und R₁₄ miteinander verbunden sein können, um einen 3- bis 6-gliedrigen Zyklus zu bilden.

13. Verbindung nach Anspruch 12, ausgewählt aus:
- 5-[(E)-[(1,1-Dioxo-1,2-benzothiazol-3-yl)-methyl-hydrazono]methyl]-3-methyl-isoindolin-1-on,
- 5-[(E)-[(1,1-Dioxo-1,2-benzothiazol-3-yl)-methyl-hydrazono]methyl]-3,3-dimethyl-isoindolin-1-on,
- 5-[(E)-[(1,1-Dioxo-1,2-benzothiazol-3-yl)-ethyl-hydrazono]methyl]-3,3-dimethyl-isoindolin-1-on
- 5'-[(E)-[(1,1-Dioxo-1,2-benzothiazol-3-yl)-methyl-hydrazono]methyl]spiro-[cyclopropan-1,3'-isoindolin]-1'-on
und pharmazeutisch akzeptable Salze dieser Verbindungen.

14. Verbindung nach Anspruch 1, oder ein pharmazeutisch akzeptables Salz davon, der Formel (II): 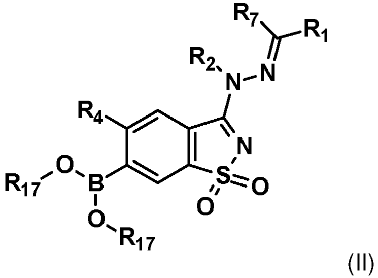 wobei R₁, R₂, R₄, und R₇ wie in Anspruch 1 definiert sind, und jedes R₁₇ unabhängig H oder Alkyl ist, oder die zwei OR₁₇ zusammen einen 5- bis 8-gliedrigen Heterozyklus bilden, etwa 4,4,5,5-Tetramethyl-1,3,2-dioxaborolan-2-yl, 5,5-Dimethyl-1,3,2-dioxaborinan-2-yl oder N-Methyl-1,3,6,2-dioxazaborocan-4,8-dion.

15. Verbindung nach Anspruch 14, oder ein pharmazeutisch akzeptables Salz davon, der Formel (IIa): 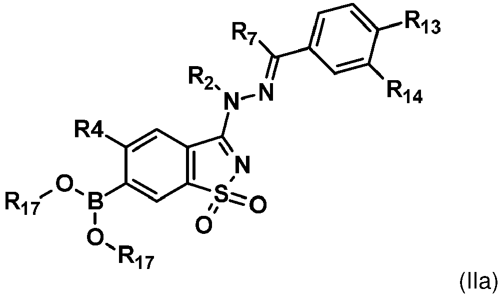 wobei:
R₂ und R₇ wie in Anspruch 1 definiert sind,
R₄ H, Alkyl oder Halogen ist,
R₁₃ und R₁₄ jeweils unabhängig ausgewählt sind aus H, Halogen, Hydroxyl, Alkoxyl, Alkyl, Cycloalkyl, Alkyl-R₈, -O-Alkyl-R₈, -NR₉R₁₀ und -CO-NR₉R₁₀, mit der Maßgabe, dass R₁₃ nicht Hydroxyl oder Methoxyl ist, wenn R₁₄ Methoxyl ist,
R₈ Hydroxyl, Alkoxyl, Cycloalkyl oder -CF₃ ist,
jedes R₁₇ wie in Anspruch 14 ist.

16. Verbindung nach Anspruch 14, oder ein pharmazeutisch akzeptables Salz davon, der Formel (IId): 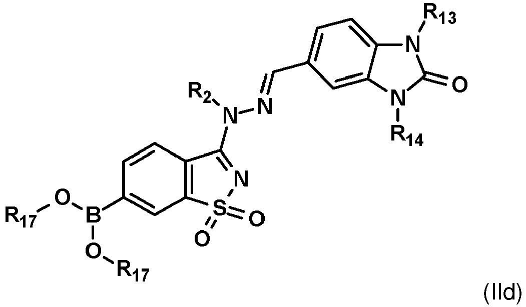 wobei:
R₂ ein lineares oder verzweigtes (C₁-C₄)-Alkyl oder Alkyl-R₈ ist,
R₁₃ und R₁₄ jeweils unabhängig ausgewählt sind aus H, Alkyl, Cycloalkyl und Alkyl-R₈,
R₈ Hydroxyl, Alkoxyl, Cycloalkyl oder -CF₃ ist,
jedes R₁₇ wie in Anspruch 14 definiert ist.

17. Verbindung nach Anspruch 1, oder ein pharmazeutisch akzeptables Salz davon, der Formel (III): 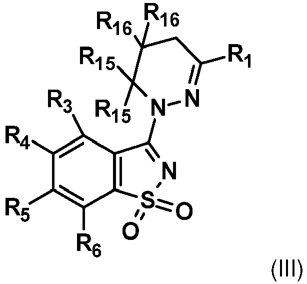 wobei R₁, R₃, R₄, R₅ und R₆ wie in Anspruch 1 definiert sind, und R₁₅, R₁₆ jeweils unabhängig H, Alkyl, Alkyl-R₈ oder -Aryl-R₈ sind.

18. Verbindung nach einem der Ansprüche 1 bis 17, zur Verwendung als ein Medikament.

19. Verbindung nach einem der Ansprüche 1 bis 17, zur Verwendung in der Behandlung einer Krebsindikation, bei welcher YAP in dem Nukleus der Tumorzellen lokalisiert wird, wie etwa Lungen-, Schilddrüsen-, Eierstock-, Kolorektal-, Prostata-, Pankreas-, Speiseröhren-, Leber, Brust- und Hautkrebs, noch konkreter in der Behandlung eines bösartigen Mesothelioms.

20. Pharmazeutische Zusammensetzung, umfassend eine Verbindung nach einem der Ansprüche 1 bis 17 oder ein pharmazeutisch akzeptables Salz davon sowie einen pharmazeutisch akzeptablen Exzipienten.

## Revendications

1. Composé de formule (I) : 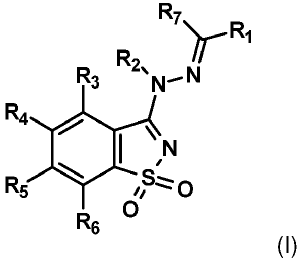 ou sel pharmaceutiquement acceptable de celui-ci, dans laquelle :
R₁ est un groupe aryle éventuellement substitué par un ou plusieurs substituants indépendamment choisis parmi halo, hydroxy, alcoxy, alkylthio, OCF₃, alkyle, cycloalkyle, alkyl-R₈, -O-alkyl-R₈, -NR₉R₁₀, -CO-NR₉R₁₀, oxo, thioxo et phényle ;
R₂ est un alkylé éventuellement substitué par un ou deux R₈ ou -aryl-R₈ ;
R₃, R₄, R₅, R₆ sont chacun indépendamment H, halo, hydroxy, alkyle, alcoxy, alcoxy substitué par l'acide boronique ou un dérivé d'acide boronique, hydroxycarbonyle, aryle, -O-aryle, l'acide boronique ou un dérivé d'acide boronique, l'acide phosphonique ou un dérivé d'acide phosphonique, alkyl-R₈, -O-alkyl-R₈, -COOR₁₁, -NO₂, -NR₉R₁₀ ou -CO-NR₉R₁₀ ;
R₇ est H or alkyle ; ou
R₂ et R₇ sont liés ensemble pour former un hétérocycle à 5 ou 6 chaînons ;
R₈ est halo, hydroxy, alcoxy, -NR₉R₁₀, -CO-X-R₁₁, -CN, -CF₃, aryle, cycloalkyle, -O-alkyl-alcoxy, -O-CO-alkyle, -CO-NR₉R₁₀ ou -O-CO-NR₉R_{10 ;}
R₉ et R₁₀ sont chacun indépendamment H, alkylé ou -CO-alkyle, ou forment avec l'atome d'azote un groupe cyclique ayant de 3 à 6 chaînons ;
X est -O- ouy -NR₁₂- ;
R₁₁ est H ou alkylé;
R₁₂ est H, alkylé ou hydroxyalkyle ;
et lorsque X est -NR₁₂-, R₁₁ et R₁₂ peuvent être liés ensemble pour former un groupe cyclique ayant de 4 à 7 chaînons ;
où aryle est un (hétéro)cycle aromatique ou partiellement insaturé comprenant 1, 2 ou 3 noyaux, qui peuvent être fusionnés ;
étant entendu que R₁ n'est pas un 3,4-dimethoxyphényle ou un 3-méthoxy-4-hydroxyphényle, et
étant entendu que R₁ est un groupe aryle substitué lorsque R₂ et R₇ sont liés ensemble pour former un hétérocycle à 5 ou 6 chaînons et R₃, R₄, R₅ et R₆ sont chacun H.

2. Composé selon la revendication 1, ou sel pharmaceutiquement acceptable de celui-ci, où R₁ représente : 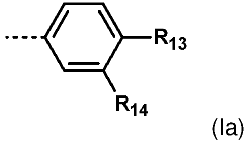 et R₁₃ et R₁₄ sont chacun indépendamment choisis parmi H, halo, hydroxy, alcoxy, alkylthio, -OCF₃, alkyle, cycloalkyle, alkyl-R₈, -O-alkyl-R₈, -NR₉R₁₀ et -CO-NR₉R₁₀, étant entendu que lorsque R₁₄ est méthoxy, alors R₁₃ n'est pas hydroxyl ou méthoxy.

3. Composé selon la revendication 2, choisi parmi :
- N-[(E)-(4-fluorophényl)méthylèneamino]-N-méthyl-1,1-dioxo-1,2-benzothiazol-3-amine ;
- 2-[3-[(E)-[(1,1-dioxo-1,2-benzothiazol-3-yl)-méthyl-hydrazono]méthyl]phénoxy] éthanol ;
- N-méthyl-N-[(E)-(4-morpholinophényl)méthylèneamino]-1,1-dioxo-1,2-benzothiazol-3-amine ;
- N-[(E)-[3-(diméthylamino)phényl]méthylèneamino]-N-méthyl-1,1-dioxo-1,2-benzothiazol-3-amine ;
- N-[(E)-(4-fluoro-3-méthoxy-phényl)méthylèneamino]-1,1-dioxo-N-[2-(2-pyridyl)éthyl]-1,2-benzothiazol-3-amine ;
- Acétate de 2-[3-[(E)-[(1,1-dioxo-1,2-benzothiazol-3-yl)-méthyl-hydrazono] méthyl]phényl]méthyle ;
- N-[(E)-(3-éthoxy-4-fluoro-phényl)méthylèneamino]-N-méthyl-1,1-dioxo-1,2-benzothiazol-3-amine;
- N-[(E)-(4-fluoro-3-propoxy-phényl)méthylèneamino]-N-méthyl-1,1-dioxo-1,2-benzothiazol-3-amine;
- N-[(E)-(4-fluoro-3-isopropoxy-phényl)méthylèneamino]-N-méthyl-1,1 -dioxo-1,2-benzothiazol-3-amine;
- 2-[5-[(E)-[(1,1-dioxo-1,2-benzothiazol-3-yl)-méthyl-hydrazono]méthyl]-2-fluoro-phenoxy]éthanol;
- N-[(E)-[4-fluoro-3-(2-méthoxyéthoxy)phényl]méthylèneamino]-N-méthyl-1,1-dioxo-1,2-benzothiazol-3-amine;
- N-[(E)-[3-fluoro-5-méthoxy-4-(2-méthoxyéthoxy)phényl]méthylèneamino]-N-méthyl-1,1-dioxo-1,2-benzothiazol-3-amine;
- 5-[(E)-[(1,1-dioxo-1,2-benzothiazol-3-yl)-méthyl-hydrazono]méthyl]-2-fluoro-benzamide;
- 2-(diméthylamino)-5-[(E)-[(1,1-dioxo-1,2-benzothiazol-3-yl)-méthyl-hydrazono]méthyl] benzamide;
- [4-[(E)-[(1,1-dioxo-1,2-benzothiazol-3-yl)-méthyl-hydrazono]méthyl]-2-méthoxy-phényl]méthanol;
- 2-[4-[(E)-[(1,1 -dioxo-1,2-benzothiazol-3-yl)-méthyl-hydrazono]méthyl]-2-méthoxy-phényl]-N-méthyl-acétamide;
- N-[(E)-[4-(2-méthoxyéthoxy)phényl]méthylèneamino]-N-méthyl-1,1-dioxo-1,2-benzothiazol-3-amine;
- N-[(E)-[3-(2-méthoxyéthoxy)phényl]méthylèneamino]-N-méthyl-1,1-dioxo-1,2-benzothiazol-3-amine;
- N-éthyl-N-[(E)-(4-fluoro-3-méthoxy-phényl)méthylèneamino]-1,1-dioxo-1,2-benzothiazol-3-amine;
- N-[(E)-(4-fluoro-3-méthoxy-phényl)méthylèneamino]-N-isopropyl-1,1-dioxo-1,2-benzothiazol-3-amine;
- N-[(E)-(4-fluoro-3-méthoxy-phényl)méthylèneamino]-N-isobutyl-1,1-dioxo-1,2-benzothiazol-3-amine;
- 2-[(1,1-dioxo-1,2-benzothiazol-3-yl)-[(E)-(4-fluoro-3-méthoxy-phényl) méthylèneamino]amino]éthanol;
- N-[(E)-(4-fluoro-3-méthoxy-phényl)méthylèneamino]-N-(2-méthoxyéthyl)-1,1-dioxo-1,2-benzothiazol-3-amine;
- N-(2-éthoxyéthyl)-N-[(E)-(4-fluoro-3-méthoxy-phényl)méthylèneamino]-1,1-dioxo-1,2-benzothiazol-3-amine;
- 3-[(1,1-dioxo-1,2-benzothiazol-3-yl)-[(E)-(4-fluoro-3-méthoxy-phényl) méthylèneamino]amino]propan-1-ol;
- 2-(chlorométhyl)-3-[(1,1-dioxo-1,2-benzothiazol-3-yl)-[(E)-(4-fluoro-3-méthoxy-phényl)méthylèneamino]amino]propan-1-ol;
- N-[(E)-(4-fluoro-3-méthoxy-phényl)méthylèneamino]-1,1-dioxo-N-(2-phényléthyl)-1,2-benzothiazol-3-amine;
- N-[(E)-(4-fluoro-3-méthoxy-phényl)méthylèneamino]-N-méthyl-5-nitro-1,1 -dioxo-1,2-benzothiazol-3-amine;
- N-[3-[[(E)-(4-fluoro-3-méthoxy-phényl)méthylèneamino]-méthyl-amino]-1,1-dioxo-1,2-benzothiazol-5-yl]acétamide;
- N3-[(E)-(4-fluoro-3-méthoxy-phényl)méthylèneamino]-N3-méthyl-1,1-dioxo-1,2-benzothiazole-3,6-diamine;
- Acide 3-[[(E)-(4-fluoro-3-méthoxy-phényl)méthylèneamino]-méthyl-amino]-1,1-dioxo-1,2-benzothiazol-6-yl]boronique;
- 3-[[(E)-(4-fluoro-3-méthoxy-phényl)méthylèneamino]-méthyl-amino]-1,1-dioxo-1,2-benzothiazol-6-ol;
- 6-benzyloxy-N-[(E)-(4-fluoro-3-méthoxy-phényl)méthylèneamino]-N-méthyl-1,1-dioxo-1,2-benzothiazol-3-amine ;
- 5-éthoxy-N-[(E)-(4-fluoro-3-méthoxy-phényl)méthylèneamino]-N-méthyl-1,1-dioxo-1,2-benzothiazol-3-amine ;
- N-[(E)-(4-fluoro-3-méthoxy-phényl)méthylèneamino]-5-isopropoxy-N-méthyl-1,1-dioxo-1,2-benzothiazol-3-amine ;
- N-[(E)-(4-fluoro-3-méthoxy-phényl)méthylèneamino]-N-méthyl-1,1-dioxo-5-phénoxy-1,2-benzothiazol-3-amine ;
- N-[(E)-(4-fluoro-3-méthoxy-phényl)méthylèneamino]-5-(2-méthoxyéthoxy)-N-méthyl-1,1-dioxo-1,2-benzothiazol-3-amine ;
- 5-[2-(diméthylamino)éthoxy]-N-[(E)-(4-fluoro-3-méthoxy-phényl)méthylèneamino]-N-méthyl-1,1-dioxo-1,2-benzothiazol-3-amine ;
- 2-[[3-[[(E)-(4-fluoro-3-méthoxy-phényl)méthylèneamino]-méthyl-amino]-1,1-dioxo-1,2-benzothiazol-5-yl]oxy]-N,N-diméthyl-acétamide ;
- 6-(5,5-diméthyl-1,3,2-dioxaborinan-2-yl)-N-[(E)-(4-fluoro-3-méthoxy-phényl) méthylèneamino]-N-méthyl-1,1-dioxo-1,2-benzothiazol-3-amine ;
- 6-diéthoxyphosphoryl-N-[(E)-(4-fluoro-3-méthoxy-phényl)méthylèneamino]-N-méthyl-1,1-dioxo-1,2-benzothiazol-3-amine ;
- Acide [3-[éthyl-[(E)-(4-fluoro-3-méthoxy-phényl)méthylèneamino]amino]-1,1-dioxo-1,2-benzothiazol-6-yl]boronique ;
- Acide [3-[[(E)-(4-fluoro-3-méthoxy-phényl)méthylèneamino]-isobutyl-amino]-1,1-dioxo-1,2-benzothiazol-6-yl]boronique ;
- Acide [3-[[(E)-(4-fluorophényl)méthylèneamino]-méthyl-amino]-1,1-dioxo-1,2-benzothiazol-6-yl]boronique ;
- 2-[3-[[(E)-(4-fluoro-3-méthoxy-phényl)méthylèneamino]-méthyl-amino]-1,1-dioxo-1,2-benzothiazol-6-yl]-6-méthyl-1,3,6,2-dioxazaborocane-4,8-dione ;
- 2-[3-[[(E)-(4-fluoro-3-méthoxy-phényl)méthylèneamino]-isobutyl-amino]-1,1-dioxo-1,2-benzothiazol-6-yl]-6-méthyl-1,3,6,2-dioxazaborocane-4,8-dione ;
- Acide [3-[[(E)-(4-fluoro-3-méthoxy-phényl)méthylèneamino]-(2-méthoxyéthyl)amino]-1,1-dioxo-1,2-benzothiazol-6-yl]boronique ;
- N-[(E)-[4-fluoro-3-(trifluorométhoxy)phényl]méthylèneamino]-N-méthyl-1,1 -dioxo-1,2-benzothiazol-3-amine ;
- Acide [3-[cyclobutylméthyl-[(E)-(4-fluoro-3-méthoxy-phényl)méthylèneamino]amino]-1,1-dioxo-1,2-benzothiazol-6-yl]boronique ;
- Acide [5-fluoro-3-[[(E)-(4-fluoro-3-méthoxy-phényl)méthylèneamino]-isobutyl-amino]-1,1-dioxo-1,2-benzothiazol-6-yl]boronique ;
- N-[(E)-(4-fluoro-3-méthylsulfanyl-phényl)méthylèneamino]-N-méthyl-1,1-dioxo-1,2-benzothiazol-3-amine ;
- N-[(E)-[3-(diméthylamino)-4-fluoro-phényl]méthylèneamino]-N-méthyl-1,1-dioxo-1,2-benzothiazol-3-amine ;
- 3-[[(E)-(4-fluoro-3-méthoxy-phényl)méthylèneamino]-isobutyl-amino]-1,1-dioxo-1,2-benzothiazol-6-ol ;
- Acide [3-[[(E)-(4-fluoro-3-méthoxy-phényl)méthylèneamino]-isobutyl-amino]-1,1-dioxo-1,2-benzothiazol-6-yl]oxyméthylboronique ;
- 5-éthoxy-N-[(E)-(4-fluoro-3-méthoxy-phényl)méthylèneamino]-N-isobutyl-1,1-dioxo-1,2-benzothiazol-3-amine ;
- Acide [3-[[(E)-(4-fluoro-3-méthoxy-phényl)méthylèneamino]-isobutyl-amino]-1,1-dioxo-1,2-benzothiazol-5-yl]boronique ;
- Acide [3-[[(E)-(4-fluoro-3-méthoxy-phényl)méthylèneamino]-isobutyl-amino]-1,1-dioxo-1,2-benzothiazol-5-yl]oxyméthylboronique ;
- 4-[[3-[[(E)-(4-fluoro-3-méthoxy-phényl)méthylèneamino]-isobutyl-amino]-1,1-dioxo-1,2-benzothiazol-6-yl]oxy]butan-1-ol ;
- Acide [3-[[(E)-(4-fluoro-3-méthoxy-phényl)méthylèneamino]-isopropyl-amino]-1,1-dioxo-1,2-benzothiazol-6-yl]boronique ;
- 1-(azétidin-1-yl)-2-[[3-[[(E)-(4-fluoro-3-méthoxy-phényl)méthylèneamino]-isobutyl-amino]-1,1-dioxo-1,2-benzothiazol-5-yl]oxy]éthanone ;
- 2-[[3-[[(E)-(4-fluoro-3-méthoxy-phényl)méthylèneamino]-isobutyl-amino]-1,1-dioxo-1,2-benzothiazol-6-yl]oxy]acétamide ;
- 2-[[3-[[(E)-(4-fluoro-3-méthoxy-phényl)méthylèneamino]-isobutyl-amino]-1,1-dioxo-1,2-benzothiazol-5-yl]oxy]-N-méthyl-acétamide ;
- 2-[[3-[[(E)-(4-fluoro-3-méthoxy-phényl)méthylèneamino]-isobutyl-amino]-1,1-dioxo-1,2-benzothiazol-6-yl]oxyméthyl]-6-méthyl-1,3,6,2-dioxazaborocane-4,8-dione ;
- N-[(E)-(4-fluoro-3-méthylsulfanyl-phényl)méthylèneamino]-N-isobutyl-1,1 -dioxo-1,2-benzothiazol-3-amine ;
- 3-[(1,1-dioxo-1,2-benzothiazol-3-yl)-[(E)-(4-fluoro-3-méthoxy-phényl)méthylèneamino] amino]-2,2-diméthyl-propan-1-ol ;
- Acide [3-[6-(4-fluoro-3-méthoxy-phényl)-3-méthyl-4,5-dihydro-3H-pyridazin-2-yl]-1,1-dioxo-1,2-benzothiazol-6-yl]boronique ;
- 3-[6-(4-fluoro-3-méthoxy-phényl)-4,4-diméthyl-3,5-dihydropyridazin-2-yl]-1,2-benzothiazole 1,1-dioxyde ;
- 6-diéthoxyphosphoryl-N-[(E)-(4-fluoro-3-méthoxy-phényl)méthylèneamino]-N-isobutyl-1,1-dioxo-1,2-benzothiazol-3-amine ;
et les sels pharmaceutiquement acceptables de ces composés.

4. Composé selon la revendication 1, ou sel pharmaceutiquement acceptable de celui-ci, où R₁ représente : 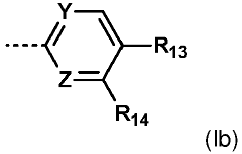 l'un de Y et Z est N et l'autre est CH, et
R₁₃ et R₁₄ sont chacun indépendamment choisis parmi H, halo, hydroxy, alcoxy, alkylthio, alkyle, cycloalkyle, alkyl-R₈ et -O-alkyl-R₈.

5. Composé selon la revendication 4, choisi parmi :
- 6-[(E)-[(1,1-dioxo-1,2-benzothiazol-3-yl)-méthyl-hydrazono]méthyl]-4-méthoxy-pyridin-3-ol ;
- N-[(E)-(6-chloro-5-fluoro-2-pyridyl)méthylèneamino]-N-méthyl-1,1-dioxo-1,2-benzothiazol-3-amine;
et les sels pharmaceutiquement acceptables de ces composés.

6. Composé selon la revendication 1, ou sel pharmaceutiquement acceptable de celui-ci, où R₁ représente : 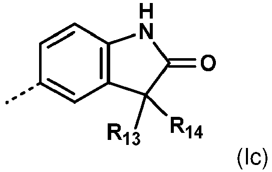 et R₁₃ et R₁₄ sont chacun indépendamment choisis parmi H, halo, hydroxy, alkyle, alcoxy, alcoxyalcoxy, alkylthio, alkyle, cycloalkyle, alkyl-R₈ et -O-alkyl-R₈, ou R₁₃ et R₁₄ peuvent être liés ensemble pour former un cycle ayant 3 à 6 chaînons.

7. Composé selon la revendication 6, choisi parmi :
- 5-[(E)-[(1,1-dioxo-1,2-benzothiazol-3-yl)-méthyl-hydrazono]méthyl]-3,3-diméthyl-indolin-2-one ;
- 5-[(E)-[(1,1-dioxo-1,2-benzothiazol-3-yl)-méthyl-hydrazono]méthyl]-3,3-diéthyl-indolin-2-one ;
- 5-[(E)-[(1,1-dioxo-1,2-benzothiazol-3-yl)-éthyl-hydrazono]méthyl]-3,3-diéthyl-indolin-2-one ;
- Acide [3-[[(E)-(3,3-diéthyl-2-oxo-indolin-5-yl)méthyleneamino]-(2-méthoxyéthyl)amino]-1,1-dioxo-1,2-benzothiazol-6-yl]boronique ;
- Acide [3-[[(E)-(3,3-diéthyl-2-oxo-indolin-5-yl)méthyleneamino]-isobutyl-amino]-1,1-dioxo-1,2-benzothiazol-6-yl]boronique ;
- 3,3-diéthyl-5-[(E)-[(6-hydroxy-1,1-dioxo-1,2-benzothiazol-3-yl)-isobutyl-hydrazono] méthyl]indolin-2-one ;
- 5-[(E)-[(6-éthoxy-1,1-dioxo-1,2-benzothiazol-3-yl)-isobutyl-hydrazono]méthyl]-3,3-diéthyl-indolin-2-one ;
- 3,3-diéthyl-5-[(E)-[isobutyl-(6-methoxy-1,1-dioxo-1,2-benzothiazol-3-yl)hydrazono] méthyl]indolin-2-one ;
- 3,3-diéthyl-5-[(E)-[isobutyl-(6-isopropoxy-1,1-dioxo-1,2-benzothiazol-3-yl)hydrazono] méthyl]indolin-2-one ;
- 5-[(E)-[(5-éthoxy-1,1-dioxo-1,2-benzothiazol-3-yl)-isobutyl-hydrazono]méthyl]-3,3-diethyl-indolin-2-one ;
et les sels pharmaceutiquement acceptables de ces composés.

8. Composé selon la revendication 1, ou sel pharmaceutiquement acceptable de celui-ci, où R₁ représente : 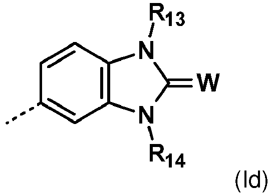 où W est O ou S, et
R₁₃ et R₁₄ sont chacun indépendamment choisis parmi H, alkyle, cycloalkyle, et alkyl-R₈, et R₈ est de préférence hydroxy, alcoxy, alcoxyalcoxy, -O-CO-alkyl, -CO-NR₉R₁₀ ou -CF₃.

9. Composé selon la revendication 8, choisi parmi :
- 5-[(E)-[(1,1-dioxo-1,2-benzothiazol-3-yl)-isobutyl-hydrazono]méthyl]-3-méthyl-1H-benzimidazol-2-one ;
- 5-[(E)-[(1,1-dioxo-1,2-benzothiazol-3-yl)-méthyl-hydrazono]méthyl]-3-ethyl-1H-benzimidazol-2-one ;
- 5-[(E)-[(1,1-dioxo-1,2-benzothiazol-3-yl)-méthyl-hydrazono]méthyl]-3-propyl-1H-benzimidazol-2-one ;
- 5-[(E)-[(1,1-dioxo-1,2-benzothiazol-3-yl)-méthyl-hydrazono]méthyl]-3-isopropyl-1H-benzimidazol-2-one ;
- 3-cyclopropyl-5-[(E)-[(1,1-dioxo-1,2-benzothiazol-3-yl)-méthyl-hydrazono]méthyl]-1H-benzimidazol-2-one ;
- 3-cyclobutyl-5-[(E)-[(1,1-dioxo-1,2-benzothiazol-3-yl)-méthyl-hydrazono]méthyl]-1H-benzimidazol-2-one ;
- 5-[(E)-[(1,1-dioxo-1,2-benzothiazol-3-yl)-méthyl-hydrazono]méthyl]-3-isobutyl-1H-benzimidazol-2-one ;
- 5-[(E)-[(1,1-dioxo-1,2-benzothiazol-3-yl)-méthyl-hydrazono]méthyl]-3-(2,2,2-trifluoroéthyl)-1H-benzimidazol-2-one ;
- Acide [3-[isobutyl-[(E)-(3-méthyl-2-oxo-1H-benzimidazol-5-yl)méthylèneamino]amino]-1,1-dioxo-1,2-benzothiazol-6-yl]boronique ;
- Acide [3-[[(E)-(3-éthyl-2-oxo-1H-benzimidazol-5-yl)méthylèneamino]-méthyl-amino]-1,1-dioxo-1,2-benzothiazol-6-yl]boronique ;
- Acide [3-[[(E)-(3-isopropyl-2-oxo-1H-benzimidazol-5-yl)méthylèneamino]-méthyl-amino]-1,1-dioxo-1,2-benzothiazol-6-yl]boronique ;
- 3-méthyl-5-[(E)-[méthyl-(5-méthyl-1,1-dioxo-1,2-benzothiazol-3-yl)hydrazono]méthyl]-1H-benzimidazol-2-one ;
- Acide [3-[isobutyl-[(E)-(3-isopropyl-2-oxo-1H-benzimidazol-5-yl)-méthylèneamino]amino]-1,1-dioxo-1,2-benzothiazol-6-yl]boronique ;
- Acide 3-[[(E)-(3-isopropyl-2-oxo-1H-benzimidazol-5-yl)méthylèneamino]-(2-méthoxyéthyl)amino]-1,1-dioxo-1,2-benzothiazol-6-yl]boronique ;
- 3-cyclobutyl-5-[(E)-[(1,1-dioxo-1,2-benzothiazol-3-yl)-méthyl-hydrazono]méthyl]-1H-benzimidazole-2-thione ;
- Acide [3-[éthyl-[(E)-(3-isopropyl-2-oxo-1H-benzimidazol-5-yl)méthylèneamino]amino]-1,1-dioxo-1,2-benzothiazol-6-yl]boronique ;
- Acide [3-[[(E)-(3-cyclobutyl-2-oxo-1H-benzimidazol-5-yl)méthylèneamino]-méthyl-amino]-1,1-dioxo-1,2-benzothiazol-6-yl]boronique ;
- Acide [3-[[(E)-(3-cyclobutyl-2-oxo-1H-benzimidazol-5-yl)méthylèneamino]-éthyl-amino]-1,1-dioxo-1,2-benzothiazol-6-yl]boronique ;
- Acide [3-[[(E)-(3-cyclobutyl-2-oxo-1H-benzimidazol-5-yl)méthylèneamino]-(2-méthoxyéthyl) amino]-1,1-dioxo-1,2-benzothiazol-6-yl]boronique ;
- Acide [3-[éthyl-[(E)-(3-éthyl-2-oxo-1H-benzimidazol-5-yl)méthylèneamino]amino]-1,1-dioxo-1,2-benzothiazol-6-yl]boronique ;
- Acide [3-[[(E)-(3-éthyl-2-oxo-1H-benzimidazol-5-yl)méthylèneamino]-isobutyl-amino]-1,1-dioxo-1,2-benzothiazol-6-yl]boronique ;
- 3-cyclobutyl-5-[(E)-[(1,1-dioxo-1,2-benzothiazol-3-yl)-méthyl-hydrazono]méthyl]-1-(méthoxyméthyl)benzimidazol-2-one ;
- 5-[(E)-[(1,1-dioxo-1,2-benzothiazol-3-yl)-méthyl-hydrazono]méthyl]-3-isopropyl-1-(méthoxyméthyl)benzimidazol-2-one ;
- 2,2-diméthylpropanoate de [3-cyclobutyl-5-[(E)-[(1,1-dioxo-1,2-benzothiazol-3-yl)-méthyl-hydrazono]méthyl]-2-oxo-benzimidazol-1-yl]méthyle ;
- Butanoate de [3-cyclobutyl-5-[(E)-[(1,1-dioxo-1,2-benzothiazol-3-yl)-méthyl-hydrazono]méthyl]-2-oxo-benzimidazol-1-yl]méthyle ;
- 3-cyclobutyl-5-[(E)-[(1,1-dioxo-1,2-benzothiazol-3-yl)-méthyl-hydrazono]méthyl]-1-(2-méthoxyéthoxyméthyl)benzimidazol-2-one ;
- 3-cyclobutyl-5-[(E)-[(1,1-dioxo-1,2-benzothiazol-3-yl)-méthyl-hydrazono]méthyl]-1-(éthoxyméthyl)benzimidazol-2-one ;
- N,N-diméthylcarbamate de [3-cyclobutyl-5-[(E)-[(1,1-dioxo-1,2-benzothiazol-3-yl)-méthyl-hydrazono]méthyl]-2-oxo-benzimidazol-1-yl]methyle ;
et les sels pharmaceutiquement acceptables de ces composés.

10. Composé selon la revendication 1, ou sel pharmaceutiquement acceptable de celui-ci, où R₁ représente : 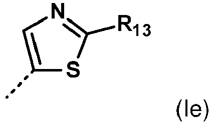 et R₁₃ est H, alkyle, cycloalkyle, phényle, alkyl-R₈ ou -NR₉R₁₀.

11. Composé selon la revendication 10, choisi parmi :
- N-[(E)-(2-aminothiazol-5-yl)méthylèneamino]-N-méthyl-1,1-dioxo-1,2-benzothiazol-3-amine ;
- N-méthyl-1,1-dioxo-N-[(E)-(2-pyrrolidin-1-ylthiazol-5-yl)méthylèneamino]-1,2-benzothiazol-3-amine ;
- N-méthyl-N-[(E)-(2-morpholinothiazol-5-yl)méthylèneamino]-1,1-dioxo-1,2-benzothiazol-3-amine ;
- N-méthyl-1,1-dioxo-N-[(E)-(2-phénylthiazol-5-yl)méthylèneamino]-1,2-benzothiazol-3-amine;
- N-[(E)-[2-(azétidin-1-yl)thiazol-5-yl]méthylèneamino]-N-méthyl-1,1-dioxo-1,2-benzothiazol-3-amine;
- N-méthyl-N-[(E)-(2-méthylthiazol-5-yl)méthylèneamino]-1,1-dioxo-1,2-benzothiazol-3-amine;
et les sels pharmaceutiquement acceptables de ces composés.

12. Composé selon la revendication 1, ou sel pharmaceutiquement acceptable de celui-ci, où R₁ représente : 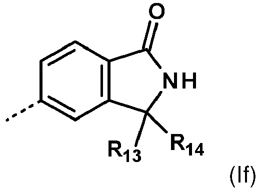 et R₁₃ et R₁₄ sont chacun indépendamment choisis parmi H, halo, hydroxy, alcoxy, alkylthio, alkyle, cycloalkyle, alkyl-R₈ et -O-alkyl-R₈ ou R₁₃ et R₁₄ peuvent être liés ensemble pour former un cycle ayant 3 à 6 chaînons.

13. Composé selon la revendication 12, choisi parmi :
- 5-[(E)-[(1,1-dioxo-1,2-benzothiazol-3-yl)-méthyl-hydrazono]méthyl]-3-methyl-isoindolin-1-one ;
- 5-[(E)-[(1,1-dioxo-1,2-benzothiazol-3-yl)-méthyl-hydrazono]méthyl]-3,3-diméthyl-isoindolin-1-one ;
- 5-[(E)-[(1,1-dioxo-1,2-benzothiazol-3-yl)-éthyl-hydrazono]méthyl]-3,3-diméthyl-isoindolin-1-one ;
- 5'-[(E)-[(1,1-dioxo-1,2-benzothiazol-3-yl)-méthyl-hydrazono]méthyl]spiro[cyclopropane-1,3'-isoindoline]-1'-one ;
et les sels pharmaceutiquement acceptables de ces composés.

14. Composé selon la revendication 1, ou sel pharmaceutiquement acceptable de celui-ci, de formule (II) : 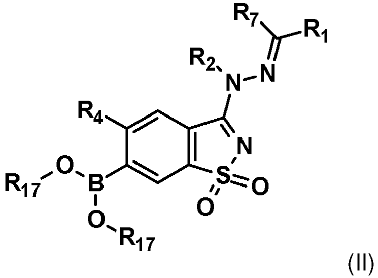 dans laquelle R₁, R₂, R₄, et R₇ sont tels que définis à la revendication 1, et chaque R₁₇ est indépendamment H ou alkyle, ou les deux OR₁₇ forment ensemble un hétérocycle ayant 5 à 8 chaînons tel que 4,4,5,5-tétraméthyl-1,3,2-dioxaborolan-2-yle, 5,5-diméthyl-1,3,2-dioxaborinan-2-yle ou N-méthyl-1,3,6,2-dioxazaborocane-4,8-dione.

15. Composé selon la revendication 14, ou sel pharmaceutiquement acceptable de celui-ci, de formule (IIa) : 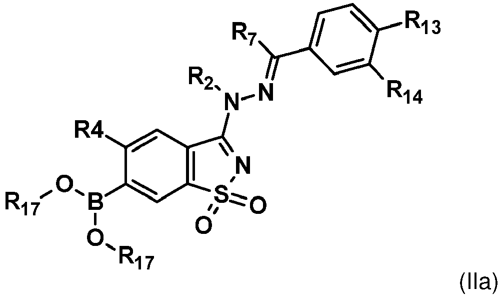 dans laquelle :
R₂ et R₇ sont tels que définis à la revendication 1 ;
R₄ est H, alkyle, ou halo ;
R₁₃ et R₁₄ sont indépendamment choisis parmi H, halo, hydroxy, alcoxy, alkyle, cycloalkyle, alkyl-R₈, -O-alkyl-R₈, -NR₉R₁₀ et -CO-NR₉R₁₀, étant entendu que lorsque R₁₄ est méthoxy, alors R₁₃ n'est pas hydroxy ou méthoxy ;
R₈ est hydroxy, alcoxy, cycloalkyle ou -CF₃ ;
chaque R₁₇ est tel que défini à la revendication 14.

16. Composé selon la revendication 14, ou sel pharmaceutiquement acceptable de celui-ci, de formule (IId) : 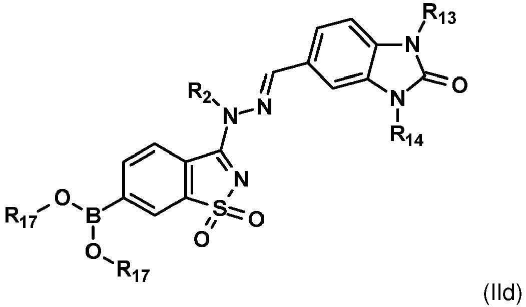 dans laquelle:
R₂ est un (C₁-C₄)alkyle linéaire ou ramifié, ou alkyl-R₈ ;
R₁₃ et R₁₄ sont chacun indépendamment choisis parmi H, alkyle, cycloalkyle, et alkyl-R₈ ;
R₈ is hydroxyl, alkoxyl, cycloalkyl or -CF₃ ;
chaque R₁₇ est tel que défini à la revendication 14.

17. Composé selon la revendication 1, ou sel pharmaceutiquement acceptable de celui-ci, de formule (III) : 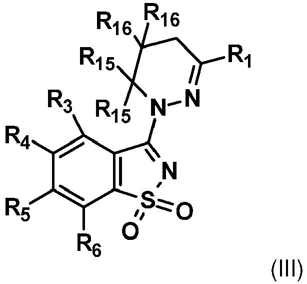 dans laquelle R₁, R₃, R₄, R₅, et R₆ sont tells que définis à la revendication 1, et R₁₅, R₁₆ sont chacun indépendamment H, alkyle, alkyl-R₈ ou -aryl-R₈..

18. Composé selon l'une des revendications 1 à 17, pour utilisation comme médicament.

19. Composé selon l'une des revendications 1 à 17, pour utilisation dans le traitement d'une indication cancéreuse où YAP est localisée dans le noyau des cellules tumorales, telle que le cancer du poumon, le cancer de la thyroïde, le cancer des ovaires, le cancer colorectla, le cancer de la prostate, le cancer du pancréas, le cancer de l'oesaphage, le cnacer du foie, le cancer du sein et le cancer de la peau, plus particulièrement dans le traitement des mésothéliomes malins.

20. Composition pharmaceutique comprenant un composé selon l'une des revendications 1 à 17 ou un sel pharmceutiquement acceptable de celui-ci, et un excipient pharmceutiquement acceptable.
